# EUROPEAN PATENT APPLICATION

(11) **EP 2 014 675 A1**
(43) Date of publication of application: **14.01.2009**
(21) Application number: 08014683.0
(22) Date of filing: 11.08.2004
(51) Int. Cl.: C07K 14/47, C12N 15/12, C07K 16/18, C12N 15/62, A61K 38/17, A61K 39/395, C12Q 1/68, G01N 33/53

(54) **Compositions and methods for the treatment of immune related diseases**

(30) Priority: 11.08.2003 US 493546 P
(62) Divisional of application: 04781002.3
(71) Applicant: Genentech, Inc., South San Francisco CA 94080-4990 (US)
(72) Inventor: Abbas, Alexander, Belmont, CA 94002 (US); Clark, Hilary, San Francisco, CA 94131 (US); Ouyang, Wenjun, Foster City, CA 94404 (US); Williams, Mickey P., Emerald Hills, CA 94062 (US); Wood, William I., Cupertino, CA 95014 (US); Wu, Thomas D., San Francisco, CA 94110 (US)
(74) Representative: Woolley, Lindsey Claire

(57) **Abstract**

The present invention related to compositions containing novel proteins and methods of using those compositions for the diagnosis and treatment of immune related diseases.

## Description

### PRIORTY

This application claims priority to U.S. Provisional Application No.: 60/493,546 filed August 11, 2003, to which U.S. Provisional Applications claim priority under 35 U.S.C. §119, the entire disclosure of which is hereby incorporated by reference in its entirety.

### FIELD OF THE INVENTION

The present invention relates to compositions and methods useful for the diagnosis and treatment of immune related diseases.

### BACKGROUND OF THE INVENTION

Immune related and inflammatory diseases are the manifestation or consequence of fairly complex, often multiple interconnected biological pathways which in normal physiology are critical to respond to insult or injury, initiate repair from insult or injury, and mount innate and acquired defense against foreign organisms. Disease or pathology occurs when these normal physiological pathways cause additional insult or injury either as directly related to the intensity of the response, as a consequence of abnormal regulation or excessive stimulation, as a reaction to self, or as a combination of these.

Though the genesis of these diseases often involves multistep pathways and often multiple different biological systems/pathways, intervention at critical points in one or more of these pathways can have an ameliorative or therapeutic effect. Therapeutic intervention can occur by either antagonism of a detrimental process/pathway or stimulation of a beneficial process/pathway.

Many immune related diseases are known and have been extensively studied. Such diseases include immune-mediated inflammatory diseases, non-immune-mediated inflammatory diseases, infectious diseases, immunodeficiency diseases, neoplasia, etc.

T lymphocytes (T cells) are an important component of a mammalian immune response. T cells recognize antigens which are associated with a self-molecule encoded by genes within the major histocompatibility complex (MHC). The antigen may be displayed together with MHC molecules on the surface of antigen presenting cells, virus infected cells, cancer cells, grafts, *etc*. The T cell system eliminates these altered cells which pose a health threat to the host mammal. T cells include helper T cells and cytotoxic T cells. Helper T cells proliferate extensively following recognition of an antigen -MHC complex on an antigen presenting cell. Helper T cells also secrete a variety of cytokines, *i*.*e*., lymphokines, which play a central role in the activation of B cells, cytotoxic T cells and a variety of other cells which participate in the immune response.

CD4 T helper cells play central role in regulating immune system. Under different pathogenic challenges, naive CD4 T cells can differentiate to two different subsets. T helper 1 (Th1) cells produce IFN-gamma, TNF-alpha and LT. Th1 cells and cytokines they produced are important for cellular immunity and critical for clearance of intracellular pathogen invasions. IFN-gamma produced by Th1 cells also helps antibody isotype switch to IgG2a, while the cytokines produced by Th1 cells activate macrophages and promote CTL reaction. In contrast, T helper 2 (Th2) CD4 cells mainly mediate humoral immunity. Th2 cells secrete IL-4, IL-5, IL-6, and IL-13. These cytokines play central in role in promotion of eosinophil development and mast cell activation. Th2 cells also help in B cell development antibody isotype switching to IgE and IgA. Th2 cells and their cytokines are critical for helminthes clearance.

Although Th1 and Th2 cells are necessary for the immune system to fight with various pathogenic invasion, unregulated Th1 and Th2 differentiation could play a role in autoimmune diseases. For example, uncontrolled Th2 differentiation has been demonstrated to be involved in immediate hypersensitivity, allergic reaction and asthma. Th1 cells have been shown to present in diabetes, MS, psoriasis, and lupus. Currently, IL-12 and IL-4 have been identified to be the key cytokines initiating the development of the Th1 and Th2 cells, respectively. Upon binding to its receptor, IL-12 activates Stat4, which then forms a homodimer, migrates into the nucleus and initiates down stream transcription events for Th1 development. IL-4 activates a different Stat molecule, Stat6, which induces transcription factor GATA3 expression. GATA-3 will then promote downstream differentiation of Th2 cells. The differentiation of Th1 and Th2 cells are a dynamic process, at each stage, there are different molecular events happening and different gene expression profiles. For example, at the early stage naive T cells are sensitive to environment stimuli, such as cytokines and costimulatory signals. If they receive the Th2 priming signal, they will quickly shut down the expression of the IL-12 receptor b2 chain expression and block further Th1 development. However, at the late stage of Th1 development, applying Th2 differentiation cytokines will fail to switch cells to a Th2 type. In this experiment, we mapped the gene expression profiles during the whole process of Th1 and Th2 development. We isolated naive CD4 T cells from normal human donors. Th1 cells were generated by stimulation of T cells with anti-CD3 and CD-28 plus IL-12, and anti-IL-4 antibody. Th2 cells were generated by similar TCR stimulation plus IL-4, anti-IL12, and anti-IFN-g antibodies. The undifferentiated T cells were generated by TCR stimulation, and neutralizing antibodies for IL-12, IL-4 and IFN-gamma. T cells were expanded on day 3 of primary activation with 5 volumes of fresh media. The fully differentiated Th1 and Th2 cells were then restimulated by anti-CD3 and anti-CD28. RNA was purified at different stages of T cell development, and RNA isolated for gene chip based expression analysis. Comparing gene expression profiles enabled us to identified genes preferentially expressed in Th1 or Th2 cell at different stages. These genes could play very important roles in the initiation of Th1/Th2 differentiation, maintenance of Th1/Th2 phenotype, activation of Thl/Th2 cells, and effector functions, such as cytokine production, of Th1/Th2 cells. These genes could also serve as molecular markers to identify and target specific Th1 and Th2 subsets. Thus, these genes are potential therapeutic targets for many autoimmune diseases.

Autoimmune related diseases could be treated by suppressing the immune response. Using neutralizing antibodies that inhibit molecules having immune stimulatory activity would be beneficial in the treatment of immune-mediated and inflammatory diseases. Molecules which inhibit the immune response can be utilized (proteins directly or via the use of antibody agonists) to inhibit the immune response and thus ameliorate immune related disease.

Despite the above identified advances in T cell research, there is a great need for additional diagnostic and therapeutic agents capable of detecting the presence of a T cell mediated disorders in a mammal and for effectively reducing these disorders. Accordingly, it is an objective of the present invention to identify polypeptides that are overexpressed in activated T cells as compared to resting T cells, and to use those polypeptides, and their encoding nucleic acids, to produce compositions of matter useful in the therapeutic treatment and diagnostic detection of T cell mediated disorders in mammals.

### SUMMARY OF THE INVENTION

### A. Embodiments

The present invention concerns compositions and methods useful for the diagnosis and treatment of immune related disease in mammals, including humans. The present invention is based on the identification of proteins (including agonist and antagonist antibodies) which are a result of stimulation of the immune response in mammals. Immune related diseases can be treated by suppressing or enhancing the immune response. Molecules that enhance the immune response stimulate or potentiate the immune response to an antigen. Molecules which stimulate the immune response can be used therapeutically where enhancement of the immune response would be beneficial. Alternatively, molecules that suppress the immune response attenuate or reduce the immune response to an antigen (e.g., neutralizing antibodies) can be used therapeutically where attenuation of the immune response would be beneficial (e.g., inflammation). Accordingly, the PRO polypeptides, agonists and antagonists thereof are also useful to prepare medicines and medicaments for the treatment of immune-related and inflammatory diseases. In a specific aspect, such medicines and medicaments comprise a therapeutically effective amount of a PRO polypeptide, agonist or antagonist thereof with a pharmaceutically acceptable carrier. Preferably, the admixture is sterile.

In a further embodiment, the invention concerns a method of identifying agonists or antagonists to a PRO polypeptide which comprises contacting the PRO polypeptide with a candidate molecule and monitoring a biological activity mediated by said PRO polypeptide. Preferably, the PRO polypeptide is a native sequence PRO polypeptide. In a specific aspect, the PRO agonist or antagonist is an anti-PRO antibody.

In another embodiment, the invention concerns a composition of matter comprising a PRO polypeptide or an agonist or antagonist antibody which binds the polypeptide in admixture with a carrier or excipient. In one aspect, the composition comprises a therapeutically effective amount of the polypeptide or antibody. In another aspect, when the composition comprises an immune stimulating molecule, the composition is useful for: (a) increasing infiltration of inflammatory cells into a tissue of a mammal in need thereof, (b) stimulating or enhancing an immune response in a mammal in need thereof, (c) increasing the proliferation of T-lymphocytes in a mammal in need thereof in response to an antigen, (d) stimulating the activity of T-lymphocytes or (e) increasing the vascular permeability. In a further aspect, when the composition comprises an immune inhibiting molecule, the composition is useful for: (a) decreasing infiltration of inflammatory cells into a tissue of a mammal in need thereof, (b) inhibiting or reducing an immune response in a mammal in need thereof, (c) decreasing the activity of T-lymphocytes or (d) decreasing the proliferation of T-lymphocytes in a mammal in need thereof in response to an antigen. In another aspect, the composition comprises a further active ingredient, which may, for example, be a further antibody or a cytotoxic or chemotherapeutic agent. Preferably, the composition is sterile.

In another embodiment, the invention concerns a method of treating an immune related disorder in a mammal in need thereof, comprising administering to the mammal an effective amount of a PRO polypeptide, an agonist thereof, or an antagonist thereto. In a preferred aspect, the immune related disorder is selected from the group consisting of: systemic lupus erythematosis, rheumatoid arthritis, osteoarthritis, juvenile chronic arthritis, spondyloarthropathies, systemic sclerosis, idiopathic inflammatory myopathies, Sjögren's syndrome, systemic vasculitis, sarcoidosis, autoimmune hemolytic anemia, autoimmune thrombocytopenia, thyroiditis, diabetes mellitus, immune-mediated renal disease, demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious, autoimmune chronic active hepatitis, primary biliary cirrhosis, granulomatous hepatitis, and sclerosing cholangitis, inflammatory bowel disease, gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease.

In another embodiment, the invention provides an antibody which specifically binds to any of the above or below described polypeptides. Optionally, the antibody is a monoclonal antibody, humanized antibody, antibody fragment or single-chain antibody. In one aspect, the present invention concerns an isolated antibody which binds a PRO polypeptide. In another aspect, the antibody mimics the activity of a PRO polypeptide (an agonist antibody) or conversely the antibody inhibits or neutralizes the activity of a PRO polypeptide (an antagonist antibody). In another aspect, the antibody is a monoclonal antibody, which preferably has nonhuman complementarity determining region (CDR) residues and human framework region (FR) residues. The antibody may be labeled and may be immobilized on a solid support. In a further aspect, the antibody is an antibody fragment, a monoclonal antibody, a single-chain antibody, or an anti-idiotypic antibody.

In yet another embodiment, the present invention provides a composition comprising an anti-PRO antibody in admixture with a pharmaceutically acceptable carrier. In one aspect, the composition comprises a therapeutically effective amount of the antibody. Preferably, the composition is sterile. The composition may be administered in the form of a liquid pharmaceutical formulation, which may be preserved to achieve extended storage stability. Alternatively, the antibody is a monoclonal antibody, an antibody fragment, a humanized antibody, or a single-chain antibody.

In a further embodiment, the invention concerns an article of manufacture, comprising:
(a) a composition of matter comprising a PRO polypeptide or agonist or antagonist thereof;
(b) a container containing said composition; and
(c) a label affixed to said container, or a package insert included in said container referring to the use of said PRO polypeptide or agonist or antagonist thereof in the treatment of an immune related disease. The composition may comprise a therapeutically effective amount of the PRO polypeptide or the agonist or antagonist thereof.

In yet another embodiment, the present invention concerns a method of diagnosing an immune related disease in a mammal, comprising detecting the level of expression of a gene encoding a PRO polypeptide (a) in a test sample of tissue cells obtained from the mammal, and (b) in a control sample of known normal tissue cells of the same cell type, wherein a higher or lower expression level in the test sample as compared to the control sample indicates the presence of immune related disease in the mammal from which the test tissue cells were obtained.

In another embodiment, the present invention concerns a method of diagnosing an immune disease in a mammal, comprising (a) contacting an anti-PRO antibody with a test sample of tissue cells obtained from the mammal, and (b) detecting the formation of a complex between the antibody and a PRO polypeptide, in the test sample; wherein the formation of said complex is indicative of the presence or absence of said disease. The detection may be qualitative or quantitative, and may be performed in comparison with monitoring the complex formation in a control sample of known normal tissue cells of the same cell type. A larger quantity of complexes formed in the test sample indicates the presence or absence of an immune disease in the mammal from which the test tissue cells were obtained. The antibody preferably carries a detectable label. Complex formation can be monitored, for example, by light microscopy, flow cytometry, fluorimetry, or other techniques known in the art. The test sample is usually obtained from an individual suspected of having a deficiency or abnormality of the immune system.

In another embodiment, the invention provides a method for determining the presence of a PRO polypeptide in a sample comprising exposing a test sample of cells suspected of containing the PRO polypeptide to an anti-PRO antibody and determining the binding of said antibody to said cell sample. In a specific aspect, the sample comprises a cell suspected of containing the PRO polypeptide and the antibody binds to the cell. The antibody is preferably detectably labeled and/or bound to a solid support.

In another embodiment, the present invention concerns an immune-related disease diagnostic kit, comprising an anti-PRO antibody and a carrier in suitable packaging. The kit preferably contains instructions for using the antibody to detect the presence of the PRO polypeptide. Preferably the carrier is pharmaceutically acceptable.

In another embodiment, the present invention concerns a diagnostic kit, containing an anti-PRO antibody in suitable packaging. The kit preferably contains instructions for using the antibody to detect the PRO polypeptide.

In another embodiment, the invention provides a method of diagnosing an immune-related disease in a mammal which comprises detecting the presence or absence or a PRO polypeptide in a test sample of tissue cells obtained from said mammal, wherein the presence or absence of the PRO polypeptide in said test sample is indicative of the presence of an immune-related disease in said mammal.

In another embodiment, the present invention concerns a method for identifying an agonist of a PRO polypeptide comprising:
(a) contacting cells and a test compound to be screened under conditions suitable for the induction of a cellular response normally induced by a PRO polypeptide; and
(b) determining the induction of said cellular response to determine if the test compound is an effective agonist, wherein the induction of said cellular response is indicative of said test compound being an effective agonist.

In another embodiment, the invention concerns a method for identifying a compound capable of inhibiting the activity of a PRO polypeptide comprising contacting a candidate compound with a PRO polypeptide under conditions and for a time sufficient to allow these two components to interact and determining whether the activity of the PRO polypeptide is inhibited. In a specific aspect, either the candidate compound or the PRO polypeptide is immobilized on a solid support. In another aspect, the non-immobilized component carries a detectable label. In a preferred aspect, this method comprises the steps of:
(a) contacting cells and a test compound to be screened in the presence of a PRO polypeptide under conditions suitable for the induction of a cellular response normally induced by a PRO polypeptide; and
(b) determining the induction of said cellular response to determine if the test compound is an effective antagonist.

In another embodiment, the invention provides a method for identifying a compound that inhibits the expression of a PRO polypeptide in cells that normally express the polypeptide, wherein the method comprises contacting the cells with a test compound and determining whether the expression of the PRO polypeptide is inhibited. In a preferred aspect, this method comprises the steps of:
(a) contacting cells and a test compound to be screened under conditions suitable for allowing expression of the PRO polypeptide; and
(b) determining the inhibition of expression of said polypeptide.

In yet another embodiment, the present invention concerns a method for treating an immune-related disorder in a mammal that suffers therefrom comprising administering to the mammal a nucleic acid molecule that codes for either (a) a PRO polypeptide, (b) an agonist of a PRO polypeptide or (c) an antagonist of a PRO polypeptide, wherein said agonist or antagonist may be an anti-PRO antibody. In a preferred embodiment, the mammal is human. In another preferred embodiment, the nucleic acid is administered via *ex vivo* gene therapy. In a further preferred embodiment, the nucleic acid is comprised within a vector, more preferably an adenoviral, adeno-associated viral, lentiviral or retroviral vector.

In yet another aspect, the invention provides a recombinant viral particle comprising a viral vector consisting essentially of a promoter, nucleic acid encoding (a) a PRO polypeptide, (b) an agonist polypeptide of a PRO polypeptide, or (c) an antagonist polypeptide of a PRO polypeptide, and a signal sequence for cellular secretion of the polypeptide, wherein the viral vector is in association with viral structural proteins. Preferably, the signal sequence is from a mammal, such as from a native PRO polypeptide.

In a still further embodiment, the invention concerns an *ex vivo* producer cell comprising a nucleic acid construct that expresses retroviral structural proteins and also comprises a retroviral vector consisting essentially of a promoter, nucleic acid encoding (a) a PRO polypeptide, (b) an agonist polypeptide of a PRO polypeptide or (c) an antagonist polypeptide of a PRO polypeptide, and a signal sequence for cellular secretion of the polypeptide, wherein said producer cell packages the retroviral vector in association with the structural proteins to produce recombinant retroviral particles.

In a still further embodiment, the invention provides a method of increasing the activity of T-lymphocytes in a mammal comprising administering to said mammal (a) a PRO polypeptide, (b) an agonist of a PRO polypeptide, or (c) an antagonist of a PRO polypeptide, wherein the activity of T-lymphocytes in the mammal is increased.

In a still further embodiment, the invention provides a method of decreasing the activity of T-lymphocytes in a mammal comprising administering to said mammal (a) a PRO polypeptide, (b) an agonist of a PRO polypeptide, or (c) an antagonist of a PRO polypeptide, wherein the activity of T-lymphocytes in the mammal is decreased.

In a still further embodiment, the invention provides a method of increasing the proliferation of T-lymphocytes in a mammal comprising administering to said mammal (a) a PRO polypeptide, (b) an agonist of a PRO polypeptide, or (c) an antagonist of a PRO polypeptide, wherein the proliferation of T-lymphocytes in the mammal is increased.

In a still further embodiment, the invention provides a method of decreasing the proliferation of T-lymphocytes in a mammal comprising administering to said mammal (a) a PRO polypeptide, (b) an agonist of a PRO polypeptide, or (c) an antagonist of a PRO polypeptide, wherein the proliferation of T-lymphocytes in the mammal is decreased.

### B. Additional Embodiments

In other embodiments of the present invention, the invention provides vectors comprising DNA encoding any of the herein described polypeptides. Host cell comprising any such vector are also provided. By way of example, the host cells may be CHO cells, *E. coli,* or yeast. A process for producing any of the herein described polypeptides is further provided and comprises culturing host cells under conditions suitable for expression of the desired polypeptide and recovering the desired polypeptide from the cell culture.

In other embodiments, the invention provides chimeric molecules comprising any of the herein described polypeptides fused to a heterologous polypeptide or amino acid sequence. Example of such chimeric molecules comprise any of the herein described polypeptides fused to an epitope tag sequence or a Fc region of an immunoglobulin.

In another embodiment, the invention provides an antibody which specifically binds to any of the above or below described polypeptides. Optionally, the antibody is a monoclonal antibody, humanized antibody, antibody fragment or single-chain antibody.

In yet other embodiments, the invention provides oligonucleotide probes useful for isolating genomic and cDNA nucleotide sequences or as antisense probes, wherein those probes may be derived from any of the above or below described nucleotide sequences.

In other embodiments, the invention provides an isolated nucleic acid molecule comprising a nucleotide sequence that encodes a PRO polypeptide.

In one aspect, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule encoding a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein, or (b) the complement of the DNA molecule of (a).

In other aspects, the isolated nucleic acid molecule comprises a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule comprising the coding sequence of a full-length PRO polypeptide cDNA as disclosed herein, the coding sequence of a PRO polypeptide lacking the signal peptide as disclosed herein, the coding sequence of an extracellular domain of a transmembrane PRO polypeptide, with or without the signal peptide, as disclosed herein or the coding sequence of any other specifically defined fragment of the full-length amino acid sequence as disclosed herein, or (b) the complement of the DNA molecule of (a).

In a further aspect, the invention concerns an isolated nucleic acid molecule comprising a nucleotide sequence having at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity to (a) a DNA molecule that encodes the same mature polypeptide encoded by any of the human protein cDNAs as disclosed herein, or (b) the complement of the DNA molecule of (a).

Another aspect the invention provides an isolated nucleic acid molecule comprising a nucleotide sequence encoding a PRO polypeptide which is either transmembrane domain-deleted or transmembrane domain-inactivated, or is complementary to such encoding nucleotide sequence, wherein the transmembrane domain(s) of such polypeptide are disclosed herein. Therefore, soluble extracellular domains of the herein described PRO polypeptides are contemplated.

Another embodiment is directed to fragments of a PRO polypeptide coding sequence, or the complement thereof, that may find use as, for example, hybridization probes, for encoding fragments of a PRO polypeptide that may optionally encode a polypeptide comprising a binding site for an anti-PRO antibody or as antisense oligonucleotide probes. Such nucleic acid fragments are usually at least about 20 nucleotides in length, alternatively at least about 30 nucleotides in length, alternatively at least about 40 nucleotides in length, alternatively at least about 50 nucleotides in length, alternatively at least about 60 nucleotides in length, alternatively at least about 70 nucleotides in length, alternatively at least about 80 nucleotides in length, alternatively at least about 90 nucleotides in length, alternatively at least about 100 nucleotides in length, alternatively at least about 110 nucleotides in length, alternatively at least about 120 nucleotides in length, alternatively at least about 130 nucleotides in length, alternatively at least about 140 nucleotides in length, alternatively at least about 150 nucleotides in length, alternatively at least about 160 nucleotides in length, alternatively at least about 170 nucleotides in length, alternatively at least about 180 nucleotides in length, alternatively at least about 190 nucleotides in length, alternatively at least about 200 nucleotides in length, alternatively at least about 250 nucleotides in length, alternatively at least about 300 nucleotides in length, alternatively at least about 350 nucleotides in length, alternatively at least about 400 nucleotides in length, alternatively at least about 450 nucleotides in length, alternatively at least about 500 nucleotides in length, alternatively at least about 600 nucleotides in length, alternatively at least about 700 nucleotides in length, alternatively at least about 800 nucleotides in length, alternatively at least about 900 nucleotides in length and alternatively at least about 1000 nucleotides in length, wherein in this context the term "about" means the referenced nucleotide sequence length plus or minus 10% of that referenced length. It is noted that novel fragments of a PRO polypeptide-encoding nucleotide sequence may be determined in a routine manner by aligning the PRO polypeptide-encoding nucleotide sequence with other known nucleotide sequences using any of a number of well known sequence alignment programs and determining which PRO polypeptide-encoding nucleotide sequence fragment(s) are novel. All of such PRO polypeptide-encoding nucleotide sequences are contemplated herein. Also contemplated are the PRO polypeptide fragments encoded by these nucleotide molecule fragments, preferably those PRO polypeptide fragments that comprise a binding site for an anti-PRO antibody.

In another embodiment, the invention provides isolated PRO polypeptide encoded by any of the isolated nucleic acid sequences herein above identified.

In a certain aspect, the invention concerns an isolated PRO polypeptide, comprising an amino acid sequence having at least about 80% amino acid sequence identity, alternatively at least about 81 % amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a PRO polypeptide having a full-length amino acid sequence as disclosed herein, an amino acid sequence lacking the signal peptide as disclosed herein, an extracellular domain of a transmembrane protein, with or without the signal peptide, as disclosed herein or any other specifically defined fragment of the full-length amino acid sequence as disclosed herein.

In a further aspect, the invention concerns an isolated PRO polypeptide comprising an amino acid sequence having at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to an amino acid sequence encoded by any of the human protein cDNAs as disclosed herein.

In a specific aspect, the invention provides an isolated PRO polypeptide without the N-terminal signal sequence and/or the initiating methionine and is encoded by a nucleotide sequence that encodes such an amino acid sequence as herein before described. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

Another aspect the invention provides an isolated PRO polypeptide which is either transmembrane domain-deleted or transmembrane domain-inactivated. Processes for producing the same are also herein described, wherein those processes comprise culturing a host cell comprising a vector which comprises the appropriate encoding nucleic acid molecule under conditions suitable for expression of the PRO polypeptide and recovering the PRO polypeptide from the cell culture.

In yet another embodiment, the invention concerns agonists and antagonists of a native PRO polypeptide as defined herein. In a particular embodiment, the agonist or antagonist is an anti-PRO antibody or a small molecule.

In a further embodiment, the invention concerns a method of identifying agonists or antagonists to a PRO polypeptide which comprise contacting the PRO polypeptide with a candidate molecule and monitoring a biological activity mediated by said PRO polypeptide. Preferably, the PRO polypeptide is a native PRO polypeptide.

In a still further embodiment, the invention concerns a composition of matter comprising a PRO polypeptide, or an agonist or antagonist of a PRO polypeptide as herein described, or an anti-PRO antibody, in combination with a carrier. Optionally, the carrier is a pharmaceutically acceptable carrier.

Another embodiment of the present invention is directed to the use of a PRO polypeptide, or an agonist or antagonist thereof as herein before described, or an anti-PRO antibody, for the preparation of a medicament useful in the treatment of a condition which is responsive to the PRO polypeptide, an agonist or antagonist thereof or an anti-PRO antibody.

### BRIEF DESCRIPTION OF THE DRAWINGS

SEQ ID NOs 1-6464 show the nucleic acids of the invention and their encoded PRO polypeptides. Also included, for convenience is a List of Figures attached hereto as Appendix A, in which each Figure number corresponds to the same number SEQ ID NO: in the sequence listing. For example, Figure 1 equals SEQ ID NO:1 of the sequence listing.

### DETAILED DESCRIPTION OF THE PREFERRED EMBODIMENTS

### I. Definitions

The terms "PRO polypeptide" and "PRO" as used herein and when immediately followed by a numerical designation refer to various polypeptides, wherein the complete designation (i.e., PRO/number) refers to specific polypeptide sequences as described herein. The terms "PRO/number polypeptide" and "PRO/number" wherein the term "number" is provided as an actual numerical designation as used herein encompass native sequence polypeptides and polypeptide variants (which are further defined herein). The PRO polypeptides described herein may be isolated from a variety of sources, such as from human tissue types or from another source, or prepared by recombinant or synthetic methods. The term "PRO polypeptide" refers to each individual PRO/number polypeptide disclosed herein. All disclosures in this specification which refer to the "PRO polypeptide" refer to each of the polypeptides individually as well as jointly. For example, descriptions of the preparation of, purification of, derivation of, formation of antibodies to or against, administration of, compositions containing, treatment of a disease with, etc., pertain to each polypeptide of the invention individually. The term "PRO polypeptide" also includes variants of the PRO/number polypeptides disclosed herein.

A "native sequence PRO polypeptide" comprises a polypeptide having the same amino acid sequence as the corresponding PRO polypeptide derived from nature. Such native sequence PRO polypeptides can be isolated from nature or can be produced by recombinant or synthetic means. The term "native sequence PRO polypeptide" specifically encompasses naturally-occurring truncated or secreted forms of the specific PRO polypeptide (e.g., an extracellular domain sequence), naturally-occurring variant forms (e.g., alternatively spliced forms) and naturally-occurring allelic variants of the polypeptide. In various embodiments of the invention, the native sequence PRO polypeptides disclosed herein are mature or full-length native sequence polypeptides comprising the full-length amino acids sequences shown in the accompanying figures. Start and stop codons are shown in bold font and underlined in the figures. However, while the PRO polypeptide disclosed in the accompanying figures are shown to begin with methionine residues designated herein as amino acid position 1 in the figures, it is conceivable and possible that other methionine residues located either upstream or downstream from the amino acid position 1 in the figures may be employed as the starting amino acid residue for the PRO polypeptides.

The PRO polypeptide "extracellular domain" or "ECD" refers to a form of the PRO polypeptide which is essentially free of the transmembrane and cytoplasmic domains. Ordinarily, a PRO polypeptide ECD will have less than 1% of such transmembrane and/or cytoplasmic domains and preferably, will have less than 0.5% of such domains. It will be understood that any transmembrane domains identified for the PRO polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified herein. Optionally, therefore, an extracellular domain of a PRO polypeptide may contain from about 5 or fewer amino acids on either side of the transmembrane domain/extracellular domain boundary as identified in the Examples or specification and such polypeptides, with or without the associated signal peptide, and nucleic acid encoding them, are contemplated by the present invention.

The approximate location of the "signal peptides" of the various PRO polypeptides disclosed herein are shown in the present specification and/or the accompanying figures. It is noted, however, that the C-terminal boundary of a signal peptide may vary, but most likely by no more than about 5 amino acids on either side of the signal peptide C-terminal boundary as initially identified herein, wherein the C-terminal boundary of the signal peptide may be identified pursuant to criteria routinely employed in the art for identifying that type of amino acid sequence element (e.g., Nielsen et al., Prot. Eng. 10:1-6 (1997) and von Heinje et al., Nucl. Acids. Res. 14:4683-4690 (1986)). Moreover, it is also recognized that, in some cases, cleavage of a signal sequence from a secreted polypeptide is not entirely uniform, resulting in more than one secreted species. These mature polypeptides, where the signal peptide is cleaved within no more than about 5 amino acids on either side of the C-terminal boundary of the signal peptide as identified herein, and the polynucleotides encoding them, are contemplated by the present invention.

"PRO polypeptide variant" means an active PRO polypeptide as defined above or below having at least about 80% amino acid sequence identity with a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Such PRO polypeptide variants include, for instance, PRO polypeptides wherein one or more amino acid residues are added, or deleted, at the N- or C-terminus of the full-length native amino acid sequence. Ordinarily, a PRO polypeptide variant will have at least about 80% amino acid sequence identity, alternatively at least about 81% amino acid sequence identity, alternatively at least about 82% amino acid sequence identity, alternatively at least about 83% amino acid sequence identity, alternatively at least about 84% amino acid sequence identity, alternatively at least about 85% amino acid sequence identity, alternatively at least about 86% amino acid sequence identity, alternatively at least about 87% amino acid sequence identity, alternatively at least about 88% amino acid sequence identity, alternatively at least about 89% amino acid sequence identity, alternatively at least about 90% amino acid sequence identity, alternatively at least about 91% amino acid sequence identity, alternatively at least about 92% amino acid sequence identity, alternatively at least about 93% amino acid sequence identity, alternatively at least about 94% amino acid sequence identity, alternatively at least about 95% amino acid sequence identity, alternatively at least about 96% amino acid sequence identity, alternatively at least about 97% amino acid sequence identity, alternatively at least about 98% amino acid sequence identity and alternatively at least about 99% amino acid sequence identity to a full-length native sequence PRO polypeptide sequence as disclosed herein, a PRO polypeptide sequence lacking the signal peptide as disclosed herein, an extracellular domain of a PRO polypeptide, with or without the signal peptide, as disclosed herein or any other specifically defined fragment of a full-length PRO polypeptide sequence as disclosed herein. Ordinarily, PRO variant polypeptides are at least about 10 amino acids in length, alternatively at least about 20 amino acids in length, alternatively at least about 30 amino acids in length, alternatively at least about 40 amino acids in length, alternatively at least about 50 amino acids in length, alternatively at least about 60 amino acids in length, alternatively at least about 70 amino acids in length, alternatively at least about 80 amino acids in length, alternatively at least about 90 amino acids in length, alternatively at least about 100 amino acids in length, alternatively at least about 150 amino acids in length, alternatively at least about 200 amino acids in length, alternatively at least about 300 amino acids in length, or more.

"Percent (%) amino acid sequence identity" with respect to the PRO polypeptide sequences identified herein is defined as the percentage of amino acid residues in a candidate sequence that are identical with the amino acid residues in the specific PRO polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table I below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table 1 below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations using this method, Tables 2 and 3 demonstrate how to calculate the % amino acid sequence identity of the amino acid sequence designated "Comparison Protein" to the amino acid sequence designated "PRO", wherein "PRO" represents the amino acid sequence of a hypothetical PRO polypeptide of interest, "Comparison Protein" represents the amino acid sequence of a polypeptide against which the "PRO" polypeptide of interest is being compared, and "X, "Y" and "Z" each represent different hypothetical amino acid residues.

Unless specifically stated otherwise, all % amino acid sequence identity values used herein are obtained as described in the immediately preceding paragraph using the ALIGN-2 computer program. However, % amino acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % amino acid sequence identity value is determined by dividing (a) the number of matching identical amino acid residues between the amino acid sequence of the PRO polypeptide of interest having a sequence derived from the native PRO polypeptide and the comparison amino acid sequence of interest (i.e., the sequence against which the PRO polypeptide of interest is being compared which may be a PRO variant polypeptide) as determined by WU-BLAST-2 by (b) the total number of amino acid residues of the PRO polypeptide of interest. For example, in the statement "a polypeptide comprising an the amino acid sequence A which has or having at least 80% amino acid sequence identity to the amino acid sequence B", the amino acid sequence A is the comparison amino acid sequence of interest and the amino acid sequence B is the amino acid sequence of the PRO polypeptide of interest.

Percent amino acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCB1-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y
where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

"PRO variant polynucleotide" or "PRO variant nucleic acid sequence" means a nucleic acid molecule which encodes an active PRO polypeptide as defined below and which has at least about 80% nucleic acid sequence identity with a nucleotide acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal peptide, as disclosed herein or any other fragment of a full-length PRO polypeptide sequence as disclosed herein. Ordinarily, a PRO variant polynucleotide will have at least about 80% nucleic acid sequence identity, alternatively at least about 81% nucleic acid sequence identity, alternatively at least about 82% nucleic acid sequence identity, alternatively at least about 83% nucleic acid sequence identity, alternatively at least about 84% nucleic acid sequence identity, alternatively at least about 85% nucleic acid sequence identity, alternatively at least about 86% nucleic acid sequence identity, alternatively at least about 87% nucleic acid sequence identity, alternatively at least about 88% nucleic acid sequence identity, alternatively at least about 89% nucleic acid sequence identity, alternatively at least about 90% nucleic acid sequence identity, alternatively at least about 91% nucleic acid sequence identity, alternatively at least about 92% nucleic acid sequence identity, alternatively at least about 93% nucleic acid sequence identity, alternatively at least about 94% nucleic acid sequence identity, alternatively at least about 95% nucleic acid sequence identity, alternatively at least about 96% nucleic acid sequence identity, alternatively at least about 97% nucleic acid sequence identity, alternatively at least about 98% nucleic acid sequence identity and alternatively at least about 99% nucleic acid sequence identity with a nucleic acid sequence encoding a full-length native sequence PRO polypeptide sequence as disclosed herein, a full-length native sequence PRO polypeptide sequence lacking the signal peptide as disclosed herein, an extracellular domain of a PRO polypeptide, with or without the signal sequence, as disclosed herein or any other fragment of a full-length PRO polypeptide sequence as disclosed herein. Variants do not encompass the native nucleotide sequence.

Ordinarily, PRO variant polynucleotides are at least about 30 nucleotides in length, alternatively at least about 60 nucleotides in length, alternatively at least about 90 nucleotides in length, alternatively at least about 120 nucleotides in length, alternatively at least about 150 nucleotides in length, alternatively at least about 180 nucleotides in length, alternatively at least about 210 nucleotides in length, alternatively at least about 240 nucleotides in length, alternatively at least about 270 nucleotides in length, alternatively at least about 300 nucleotides in length, alternatively at least about 450 nucleotides in length, alternatively at least about 600 nucleotides in length, alternatively at least about 900 nucleotides in length, or more.

"Percent (%) nucleic acid sequence identity" with respect to PRO-encoding nucleic acid sequences identified herein is defined as the percentage of nucleotides in a candidate sequence that are identical with the nucleotides in the PRO nucleic acid sequence of interest, after aligning the sequences and introducing gaps, if necessary, to achieve the maximum percent sequence identity. Alignment for purposes of determining percent nucleic acid sequence identity can be achieved in various ways that are within the skill in the art, for instance, using publicly available computer software such as BLAST, BLAST-2, ALIGN or Megalign (DNASTAR) software. For purposes herein, however, % nucleic acid sequence identity values are generated using the sequence comparison computer program ALIGN-2, wherein the complete source code for the ALIGN-2 program is provided in Table 1 below. The ALIGN-2 sequence comparison computer program was authored by Genentech, Inc. and the source code shown in Table I below has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Table I below. The ALIGN-2 program should be compiled for use on a UNIX operating system, preferably digital UNIX V4.0D. All sequence comparison parameters are set by the ALIGN-2 program and do not vary.

In situations where ALIGN-2 is employed for nucleic acid sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program ALIGN-2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C. As examples of % nucleic acid sequence identity calculations, Tables 4 and 5, demonstrate how to calculate the % nucleic acid sequence identity of the nucleic acid sequence designated "Comparison DNA" to the nucleic acid sequence designated "PRO-DNA", wherein "PRO-DNA" represents a hypothetical PRO-encoding nucleic acid sequence of interest, "Comparison DNA" represents the nucleotide sequence of a nucleic acid molecule against which the "PRO-DNA" nucleic acid molecule of interest is being compared, and "N", "L" and "V" each represent different hypothetical nucleotides.

Unless specifically stated otherwise, all % nucleic acid sequence identity values used herein are obtained as described in the immediately preceding paragraph using the ALIGN-2 computer program. However, % nucleic acid sequence identity values may also be obtained as described below by using the WU-BLAST-2 computer program (Altschul et al., Methods in Enzymology 266:460-480 (1996)). Most of the WU-BLAST-2 search parameters are set to the default values. Those not set to default values, i.e., the adjustable parameters, are set with the following values: overlap span = 1, overlap fraction = 0.125, word threshold (T) = 11, and scoring matrix = BLOSUM62. When WU-BLAST-2 is employed, a % nucleic acid sequence identity value is determined by dividing (a) the number of matching identical nucleotides between the nucleic acid sequence of the PRO polypeptide-encoding nucleic acid molecule of interest having a sequence derived from the native sequence PRO polypeptide-encoding nucleic acid and the comparison nucleic acid molecule of interest (i.e., the sequence against which the PRO polypeptide-encoding nucleic acid molecule of interest is being compared which may be a variant PRO polynucleotide) as determined by WU-BLAST-2 by (b) the total number of nucleotides of the PRO polypeptide-encoding nucleic acid molecule of interest. For example, in the statement "an isolated nucleic acid molecule comprising a nucleic acid sequence A which has or having at least 80% nucleic acid sequence identity to the nucleic acid sequence B", the nucleic acid sequence A is the comparison nucleic acid molecule of interest and the nucleic acid sequence B is the nucleic acid sequence of the PRO polypeptide-encoding nucleic acid molecule of interest.

Percent nucleic acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov or otherwise obtained from the National Institute of Health, Bethesda, MD. NCBI-BLAST2 uses several search parameters, wherein all of those search parameters are set to default values including, for example, unmask = yes, strand = all, expected occurrences = 10, minimum low complexity length = 15/5, multi-pass e-value = 0.01, constant for multi-pass = 25, dropoff for final gapped alignment = 25 and scoring matrix = BLOSUM62.

In situations where NCBI-BLAST2 is employed for sequence comparisons, the % nucleic acid sequence identity of a given nucleic acid sequence C to, with, or against a given nucleic acid sequence D (which can alternatively be phrased as a given nucleic acid sequence C that has or comprises a certain % nucleic acid sequence identity to, with, or against a given nucleic acid sequence D) is calculated as follows:
100 times the fraction W/Z
where W is the number of nucleotides scored as identical matches by the sequence alignment program NCBI-BLAST2 in that program's alignment of C and D, and where Z is the total number of nucleotides in D. It will be appreciated that where the length of nucleic acid sequence C is not equal to the length of nucleic acid sequence D, the % nucleic acid sequence identity of C to D will not equal the % nucleic acid sequence identity of D to C.

In other embodiments, PRO variant polynucleotides are nucleic acid molecules that encode an active PRO polypeptide and which are capable of hybridizing, preferably under stringent hybridization and wash conditions, to nucleotide sequences encoding a full-length PRO polypeptide as disclosed herein. PRO variant polypeptides may be those that are encoded by a PRO variant polynucleotide.

"Isolated," when used to describe the various polypeptides disclosed herein, means polypeptide that has been identified and separated and/or recovered from a component of its natural environment. Contaminant components of its natural environment are materials that would typically interfere with diagnostic or therapeutic uses for the polypeptide, and may include enzymes, hormones, and other proteinaceous or non-proteinaceous solutes. In preferred embodiments, the polypeptide will be purified (1) to a degree sufficient to obtain at least 15 residues of N-terminal or internal amino acid sequence by use of a spinning cup sequenator, or (2) to homogeneity by SDS-PAGE under non-reducing or reducing conditions using Coomassie blue or, preferably, silver stain. Isolated polypeptide includes polypeptide *in situ* within recombinant cells, since at least one component of the PRO polypeptide natural environment will not be present. Ordinarily, however, isolated polypeptide will be prepared by at least one purification step.

An "isolated" PRO polypeptide-encoding nucleic acid or other polypeptide-encoding nucleic acid is a nucleic acid molecule that is identified and separated from at least one contaminant nucleic acid molecule with which it is ordinarily associated in the natural source of the polypeptide-encoding nucleic acid. An isolated polypeptide-encoding nucleic acid molecule is other than in the form or setting in which it is found in nature. Isolated polypeptide-encoding nucleic acid molecules therefore are distinguished from the specific polypeptide-encoding nucleic acid molecule as it exists in natural cells. However, an isolated polypeptide-encoding nucleic acid molecule includes polypeptide-encoding nucleic acid molecules contained in cells that ordinarily express the polypeptide where, for example, the nucleic acid molecule is in a chromosomal location different from that of natural cells.

The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, the synthetic oligonucleotide adaptors or linkers are used in accordance with conventional practice.

The term "antibody" is used in the broadest sense and specifically covers, for example, single anti-PRO monoclonal antibodies (including agonist, antagonist, and neutralizing antibodies), anti-PRO antibody compositions with polyepitopic specificity, single chain anti-PRO antibodies, and fragments of anti-PRO antibodies (see below). The term "monoclonal antibody" as used herein refers to an antibody obtained from a population of substantially homogeneous antibodies, i.e., the individual antibodies comprising the population are identical except for possible naturally-occurring mutations that may be present in minor amounts.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, may be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) employ 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with washes at 42°C in 0.2 x SSC (sodium chloride/sodium citrate) and 50% formamide at 55°C, followed by a high-stringency wash consisting of 0.1 x SSC containing EDTA at 55°C.

"Moderately stringent conditions" may be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in I x SSC at about 37-50°C. The skilled artisan will recognize how to adjust the temperature, ionic strength, etc. as necessary to accommodate factors such as probe length and the like.

The term "epitope tagged" when used herein refers to a chimeric polypeptide comprising a PRO polypeptide fused to a "tag polypeptide". The tag polypeptide has enough residues to provide an epitope against which an antibody can be made, yet is short enough such that it does not interfere with activity of the polypeptide to which it is fused. The tag polypeptide preferably also is fairly unique so that the antibody does not substantially cross-react with other epitopes. Suitable tag polypeptides generally have at least six amino acid residues and usually between about 8 and 50 amino acid residues (preferably, between about 10 and 20 amino acid residues).

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (i.e., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

"Active" or "activity" for the purposes herein refers to form(s) of a PRO polypeptide which retain a biological and/or an immunological activity of native or naturally-occurring PRO, wherein "biological" activity refers to a biological function (either inhibitory or stimulatory) caused by a native or naturally-occurring PRO other than the ability to induce the production of an antibody against an antigenic epitope possessed by a native or naturally-occurring PRO and an "immunological" activity refers to the ability to induce the production of an antibody against an antigenic epitope possessed by a native or naturally-occurring PRO.

The term "antagonist" is used in the broadest sense, and includes any molecule that partially or fully blocks, inhibits, or neutralizes a biological activity of a native PRO polypeptide disclosed herein. In a similar manner, the term "agonist" is used in the broadest sense and includes any molecule that mimics a biological activity of a native PRO polypeptide disclosed herein. Suitable agonist or antagonist molecules specifically include agonist or antagonist antibodies or antibody fragments, fragments or amino acid sequence variants of native PRO polypeptides, peptides, antisense oligonucleotides, small organic molecules, etc. Methods for identifying agonists or antagonists of a PRO polypeptide may comprise contacting a PRO polypeptide with a candidate agonist or antagonist molecule and measuring a detectable change in one or more biological activities normally associated with the PRO polypeptide.

"Treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents includes simultaneous (concurrent) and consecutive administration in any order.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

"Antibody fragments" comprise a portion of an intact antibody, preferably the antigen binding or variable region of the intact antibody. Examples of antibody fragments include Fab, Fab', F(ab')₂, and Fv fragments; diabodies; linear antibodies (Zapata et al., Protein Eng. 8(10): 1057-1062 [1995]); single-chain antibody molecules; and multispecific antibodies formed from antibody fragments.

Papain digestion of antibodies produces two identical antigen-binding fragments, called "Fab" fragments, each with a single antigen-binding site, and a residual "Fc" fragment, a designation reflecting the ability to crystallize readily. Pepsin treatment yields an F(ab')₂ fragment that has two antigen-combining sites and is still capable of cross-linking antigen.

"Fv" is the minimum antibody fragment which contains a complete antigen-recognition and - binding site. This region consists of a dimer of one heavy- and one light-chain variable domain in tight, non-covalent association. It is in this configuration that the three CDRs of each variable domain interact to define an antigen-binding site on the surface of the V_{H}-V_{L} dimer. Collectively, the six CDRs confer antigen-binding specificity to the antibody. However, even a single variable domain (or half of an Fv comprising only three CDRs specific for an antigen) has the ability to recognize and bind antigen, although at a lower affinity than the entire binding site.

The Fab fragment also contains the constant domain of the light chain and the first constant domain (CH1) of the heavy chain. Fab fragments differ from Fab' fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. F(ab')₂ antibody fragments originally were produced as pairs of Fab' fragments which have hinge cysteines between them. Other chemical couplings of antibody fragments are also known.

The "light chains" of antibodies (immunoglobulins) from any vertebrate species can be assigned to one of two clearly distinct types, called kappa and lambda, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these may be further divided into subclasses (isotypes), e.g., IgG1, IgG2, IgG3, IgG4, IgA, and IgA2.

"Single-chain Fv" or "sFv" antibody fragments comprise the V_{H} and V_{L} domains of antibody, wherein these domains are present in a single polypeptide chain. Preferably, the Fv polypeptide further comprises a polypeptide linker between the V_{H} and V_{L} domains which enables the sFv to form the desired structure for antigen binding. For a review of sFv, see Pluckthun in The Pharmacology of Monoclonal Antibodies, vol. 113, Rosenburg and Moore eds., Springer-Verlag, New York, pp. 269-315 (1994).

The term "diabodies" refers to small antibody fragments with two antigen-binding sites, which fragments comprise a heavy-chain variable domain (V_{H}) connected to a light-chain variable domain (V_{L}) in the same polypeptide chain (V_{H}-V_{L}). By using a linker that is too short to allow pairing between the two domains on the same chain, the domains are forced to pair with the complementary domains of another chain and create two antigen-binding sites. Diabodies are described more fully in, for example, EP 404,097; WO 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody in situ within recombinant cells since at least one component of the antibody's natural environment will not be present. Ordinarily, however, isolated antibody will be prepared by at least one purification step.

An antibody that "specifically binds to" or is "specific for" a particular polypeptide or an epitope on a particular polypeptide is one that binds to that particular polypeptide or epitope on a particular polypeptide without substantially binding to any other polypeptide or polypeptide epitope.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

The term "immune related disease" means a disease in which a component of the immune system of a mammal causes, mediates or otherwise contributes to a morbidity in the mammal. Also included are diseases in which stimulation or intervention of the immune response has an ameliorative effect on progression of the disease. Included within this term are immune-mediated inflammatory diseases, non-immune-mediated inflammatory diseases, infectious diseases, immunodeficiency diseases, neoplasia, *etc*.

The term "T cell mediated disease" means a disease in which T cells directly or indirectly mediate or otherwise contribute to a morbidity in a mammal. The T cell mediated disease may be associated with cell mediated effects, lymphokine mediated effects, *etc*., and even effects associated with B cells if the B cells are stimulated, for example, by the lymphokines secreted by T cells.

Examples of immune-related and inflammatory diseases, some of which are immune or T cell mediated, which can be treated according to the invention include systemic lupus erythematosis, rheumatoid arthritis, juvenile chronic arthritis, spondyloarthropathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease. Infectious diseases including viral diseases such as AIDS (HIV infection), hepatitis A, B, C, D, and E, herpes, *etc*., bacterial infections, fungal infections, protozoal infections and parasitic infections.

The term "effective amount" is a concentration or amount of a PRO polypeptide and/or agonist/antagonist which results in achieving a particular stated purpose. An "effective amount" of a PRO polypeptide or agonist or antagonist thereof may be determined empirically. Furthermore, a "therapeutically effective amount" is a concentration or amount of a PRO polypeptide and/or agonist/antagonist which is effective for achieving a stated therapeutic effect. This amount may also be determined empirically.

The term "cytotoxic agent" as used herein refers to a substance that inhibits or prevents the function of cells and/or causes destruction of cells. The term is intended to include radioactive isotopes (*e*.*g*., I¹³¹, I¹²⁵, Y⁹⁰ and Re¹⁸⁶), chemotherapeutic agents, and toxins such as enzymatically active toxins of bacterial, fungal, plant or animal origin, or fragments thereof.

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, e.g., paclitaxel (Taxol, Bristol-Myers Squibb Oncology, Princeton, NJ), and doxetaxel (Taxotere, Rhône-Poulenc Rorer, Antony, France), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), melphalan and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially cancer cell overexpressing any of the genes identified herein, either *in vitro* or *in vivo*. Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al. (WB Saunders: Philadelphia, 1995), especially p. 13.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-α and -β; mullerian-inhibiting substance; mouse gonadotropin-associated peptide; inhibin; activin; vascular endothelial growth factor; integrin; thrombopoietin (TPO); nerve growth factors such as NGF-β; platelet-growth factor; transforming growth factors (TGFs) such as TGF-α and TGF-β; insulin-like growth factor-I and -II; erythropoietin (EPO); osteoinductive factors; interferons such as interferon-α, -β, and -γ; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1α, IL-2, IL-3, IL-4, IL-5, IL-6, IL-7, IL-8, IL-9, IL-11, IL-12; a tumor necrosis factor such as TNF-α or TNF-β; and other polypeptide factors including LIF and kit ligand (KL). As used herein, the term cytokine includes proteins from natural sources or from recombinant cell culture and biologically active equivalents of the native sequence cytokines.

As used herein, the term "immunoadhesin" designates antibody-like molecules which combine the binding specificity of a heterologous protein (an "adhesin") with the effector functions of immunoglobulin constant domains. Structurally, the immunoadhesins comprise a fusion of an amino acid sequence with the desired binding specificity which is other than the antigen recognition and binding site of an antibody (*i*.*e*., is "heterologous"), and an immunoglobulin constant domain sequence. The adhesin part of an immunoadhesin molecule typically is a contiguous amino acid sequence comprising at least the binding site of a receptor or a ligand. The immunoglobulin constant domain sequence in the immunoadhesin may be obtained from any immunoglobulin, such as IgG-1, IgG-2, IgG-3, or IgG-4 subtypes, IgA (including IgA-1 and IgA-2), IgE, IgD or IgM.

As used herein, the term "inflammatory cells" designates cells that enhance the inflammatory response such as mononuclear cells, eosinophils, macrophages, and polymorphonuclear neutrophils (PMN).

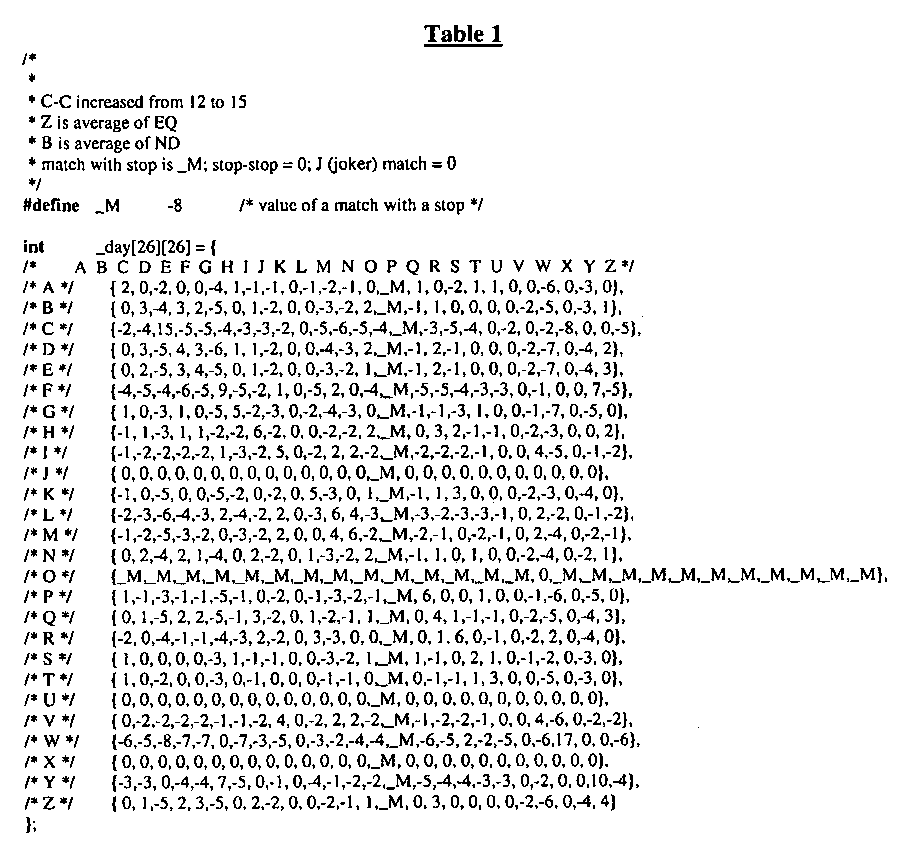

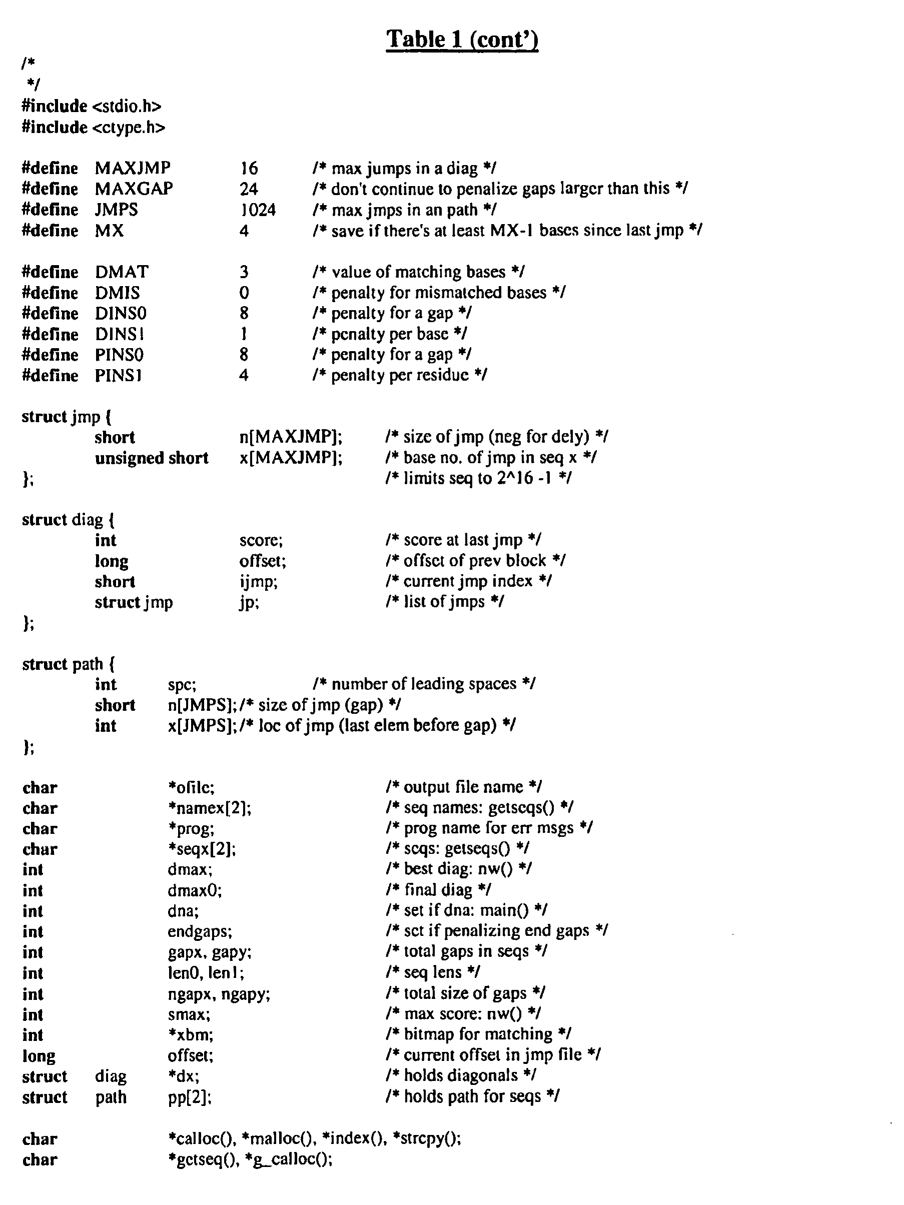

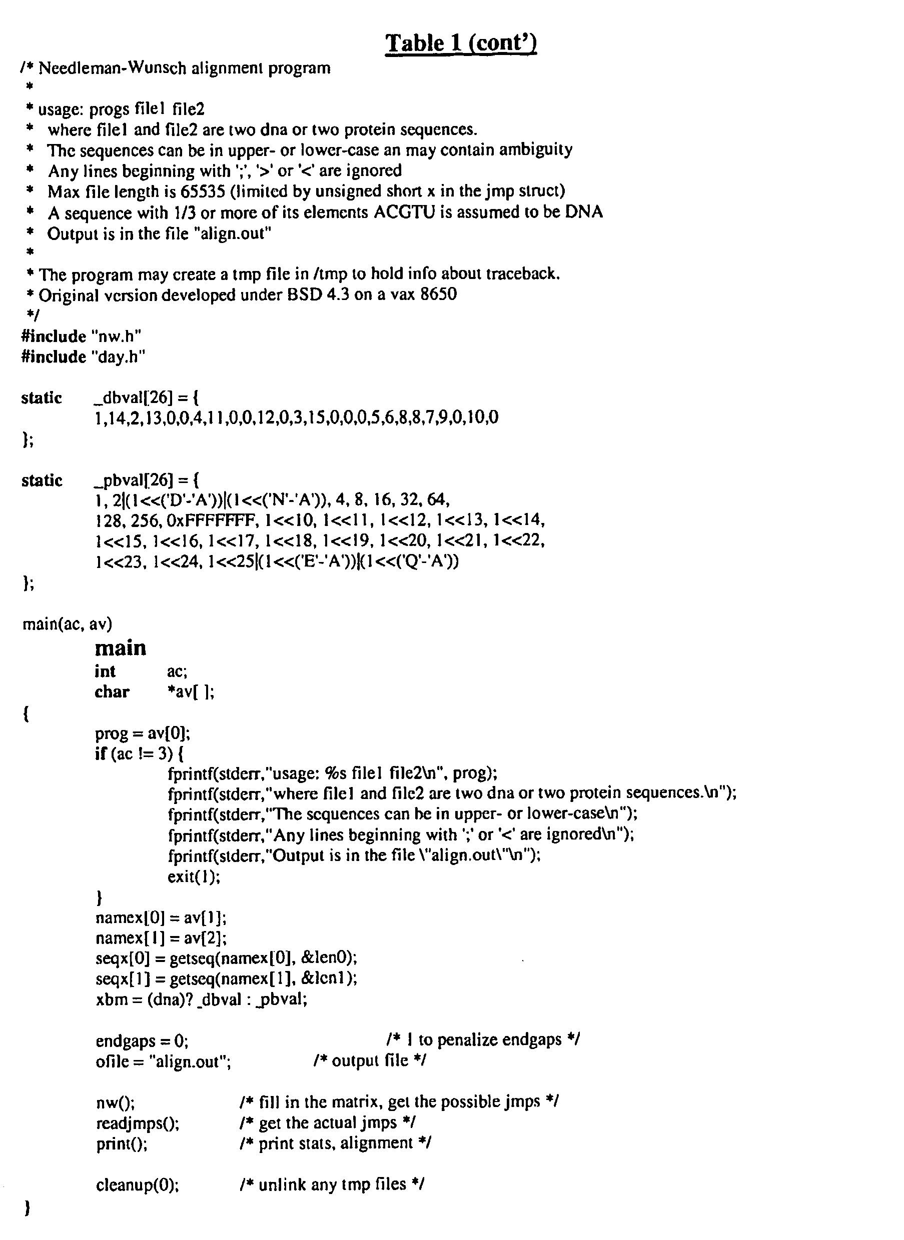

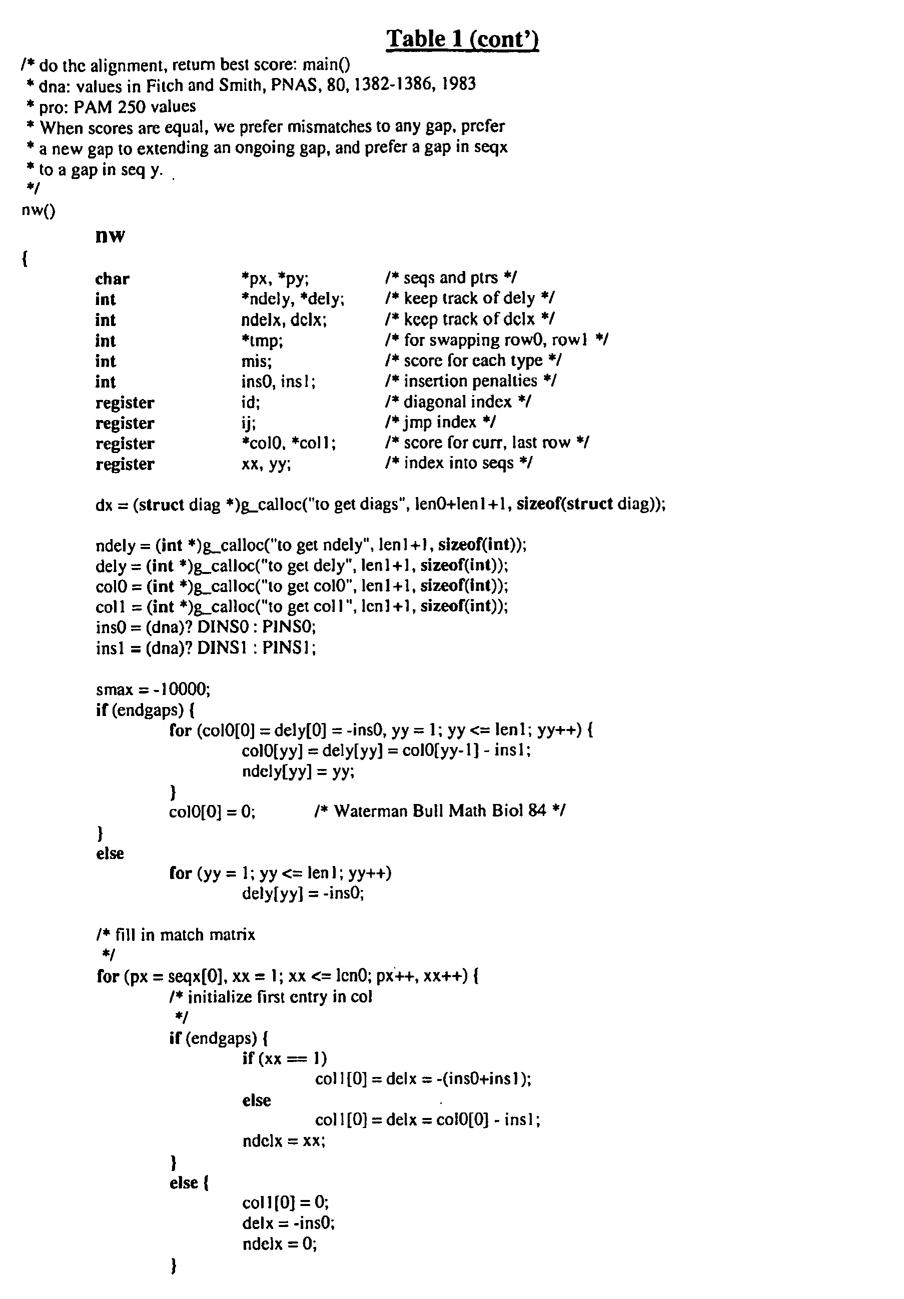

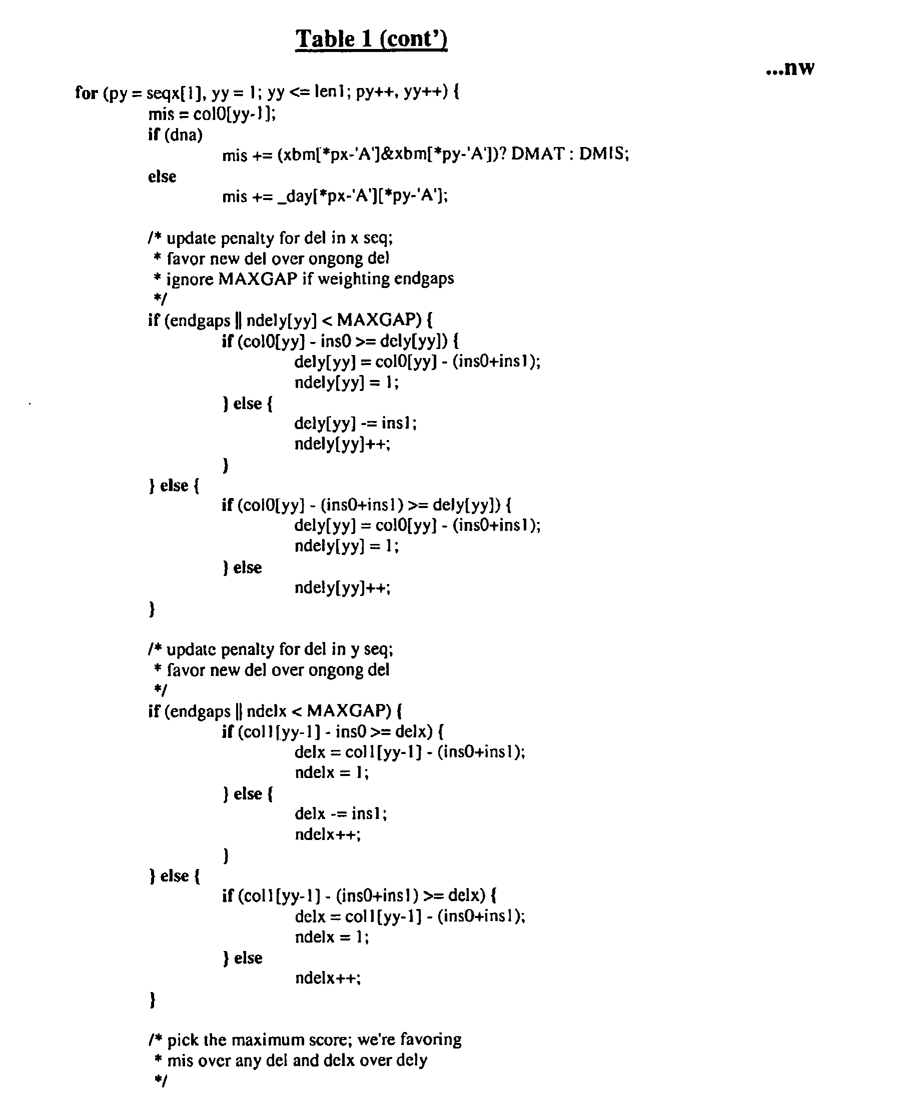

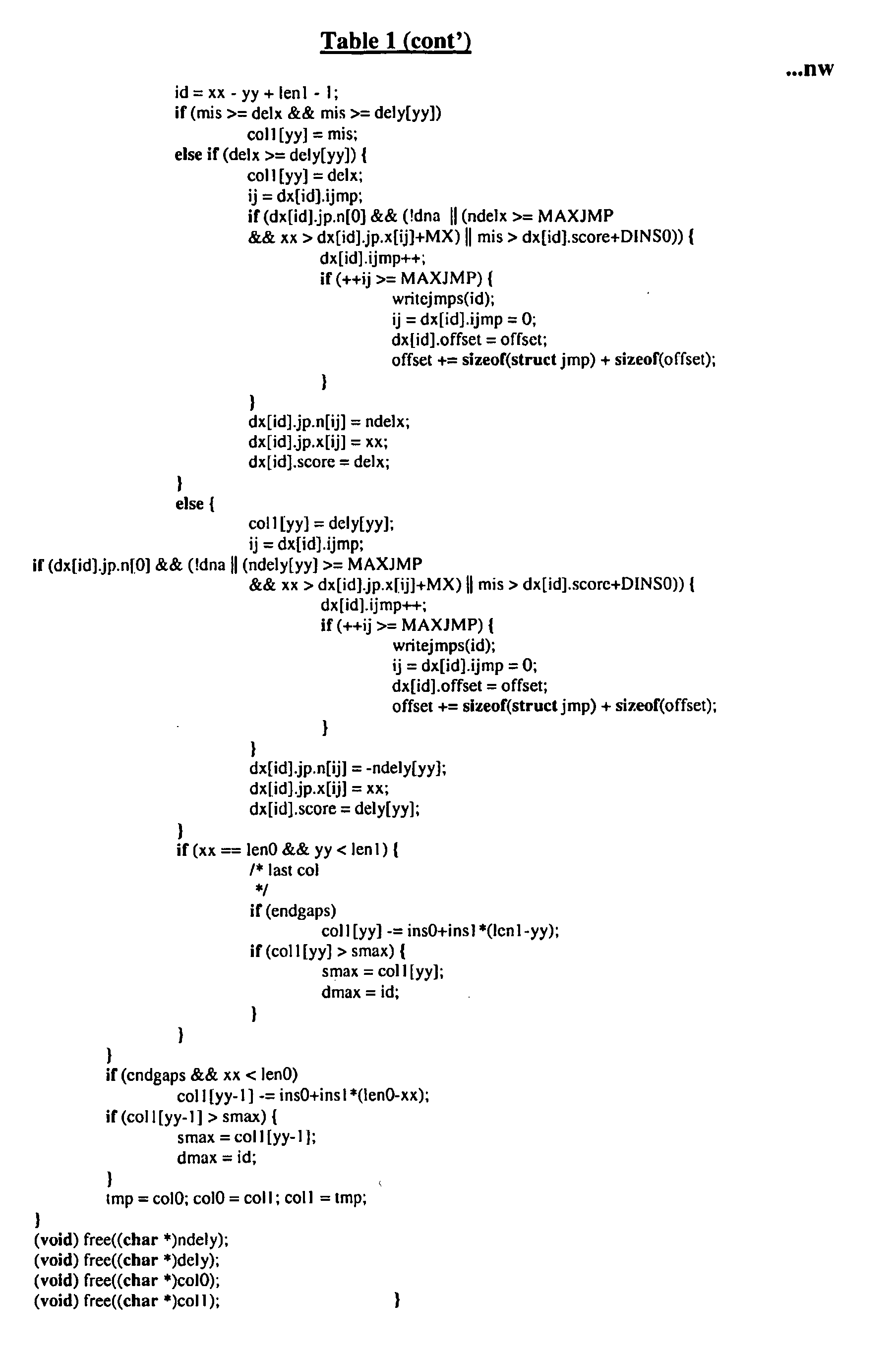

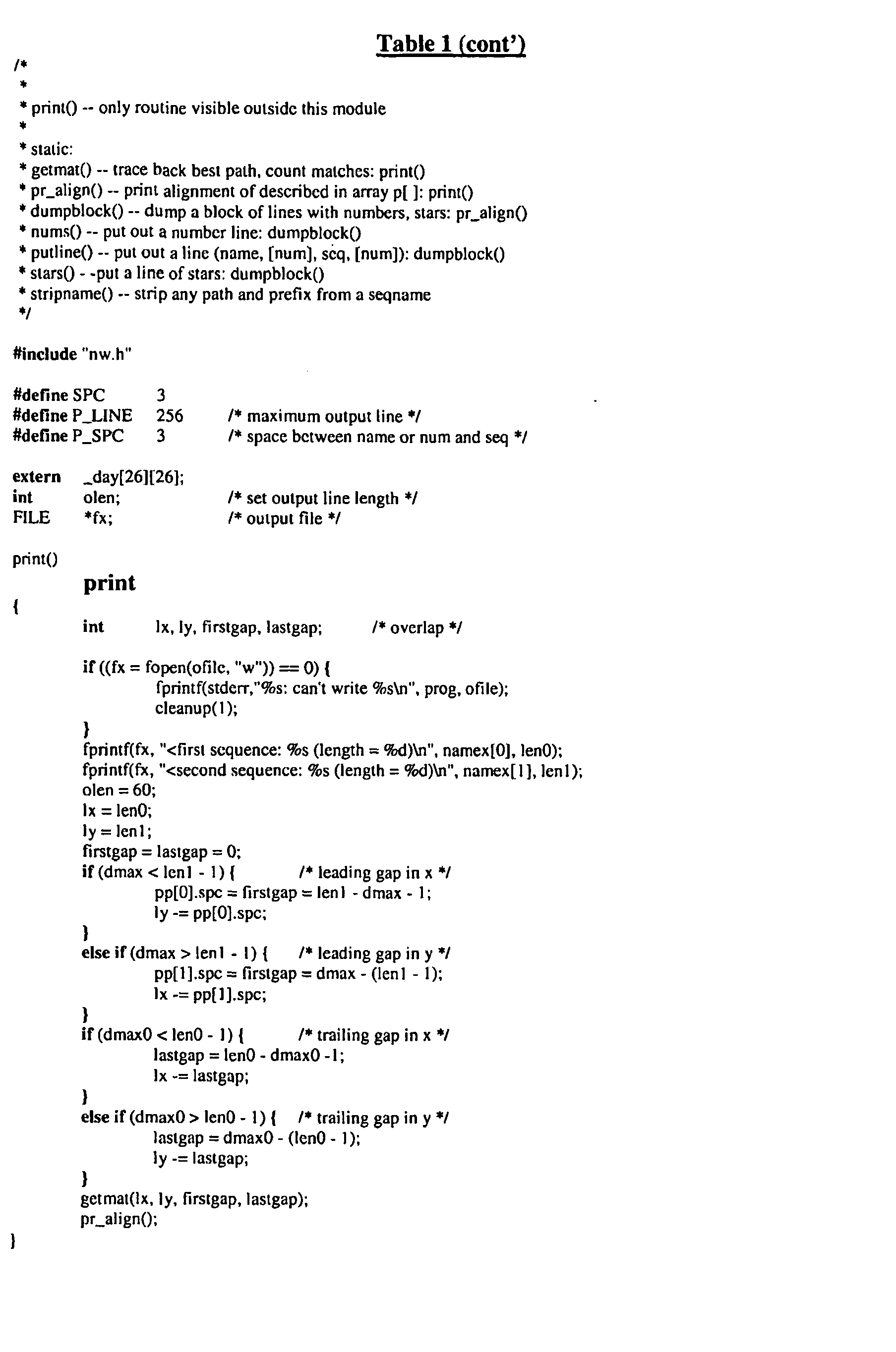

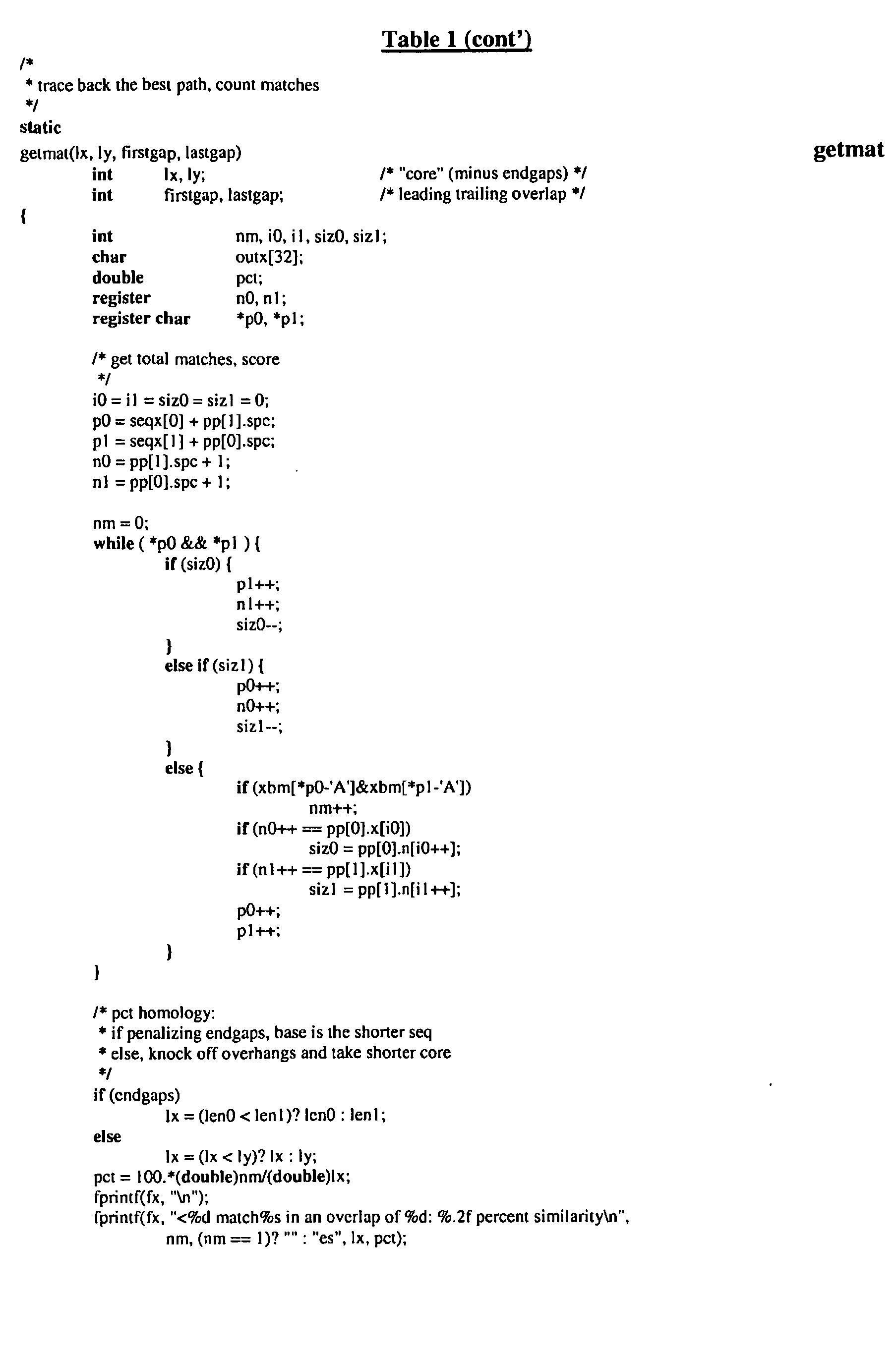

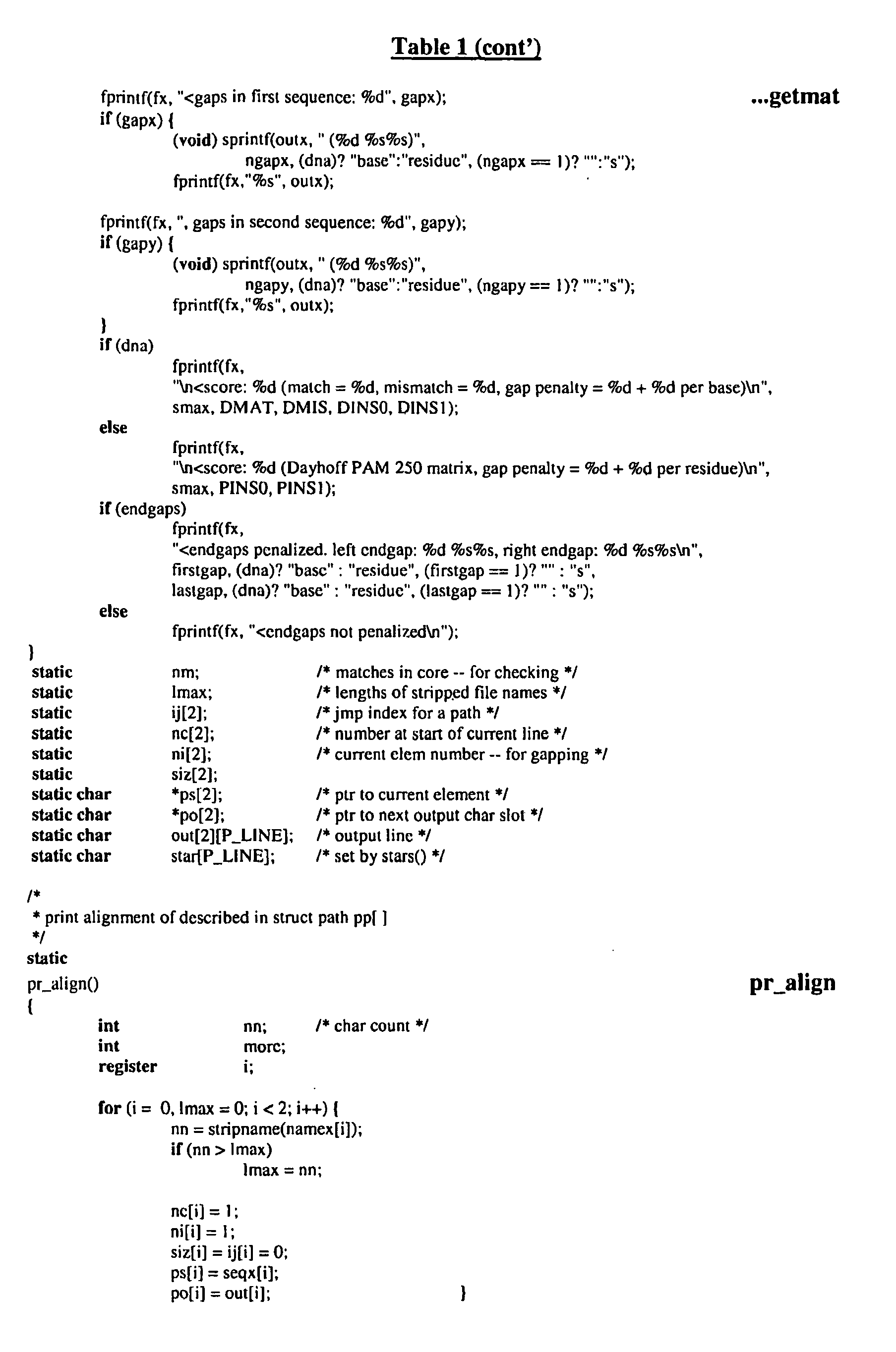

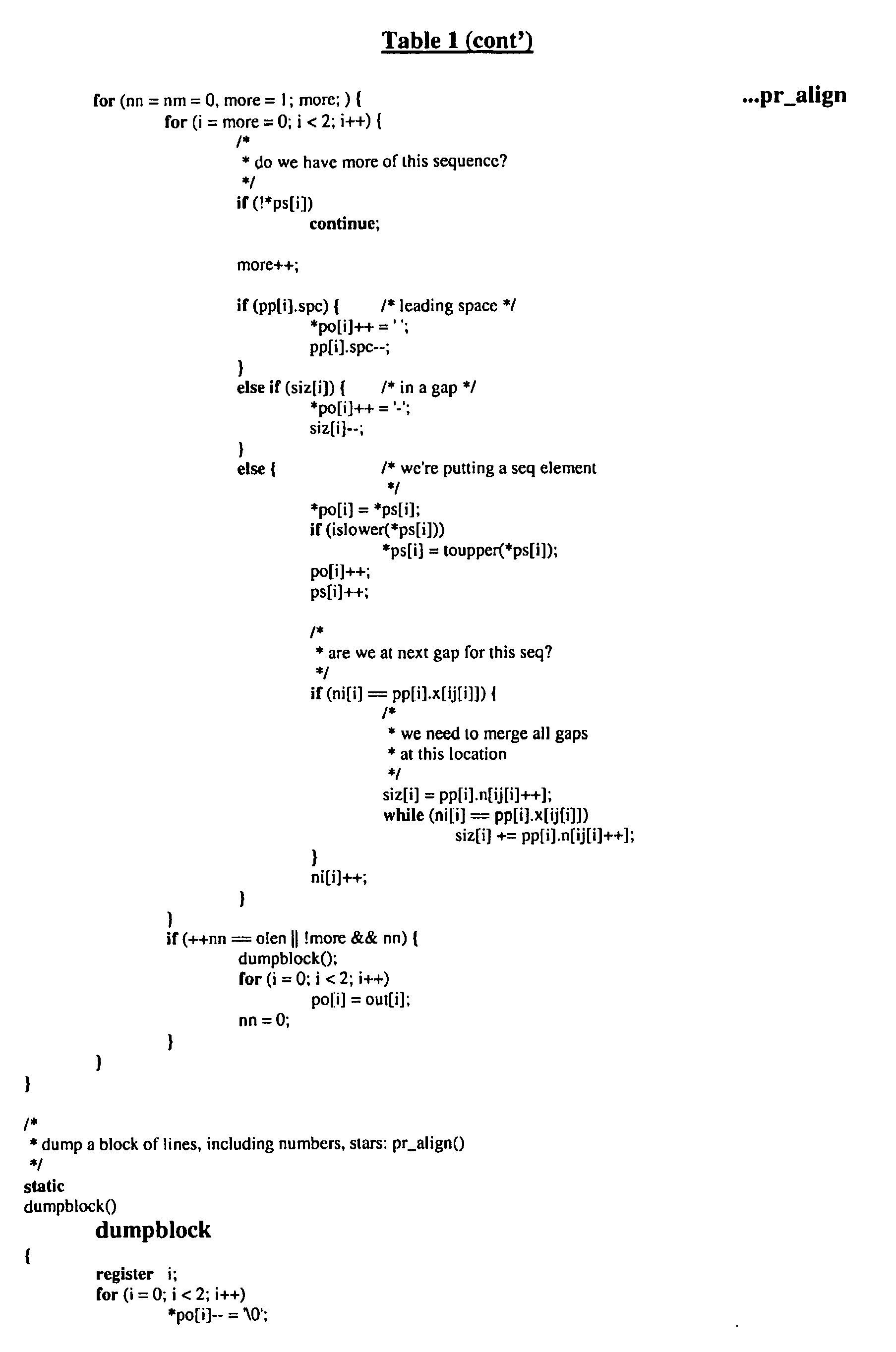

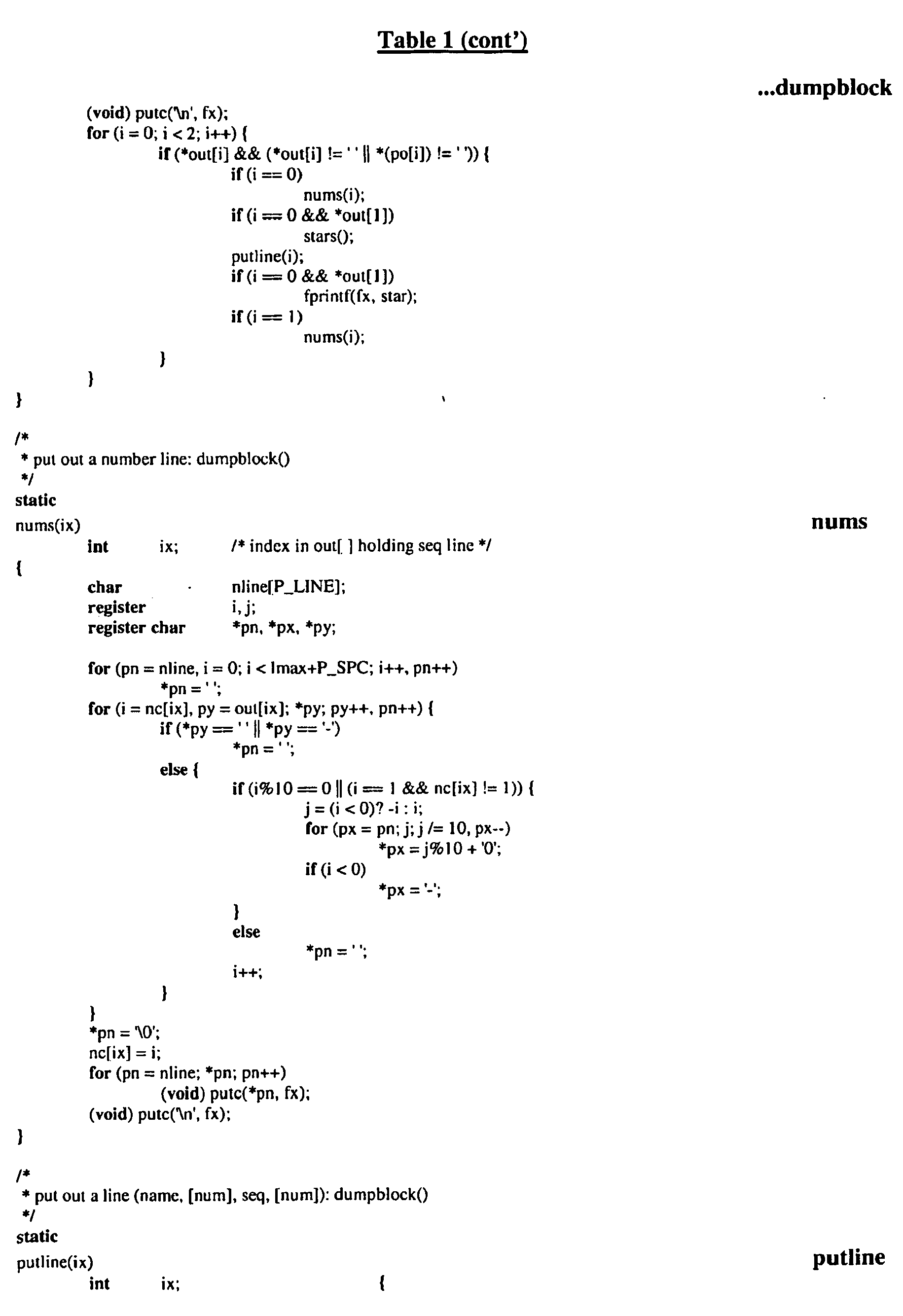

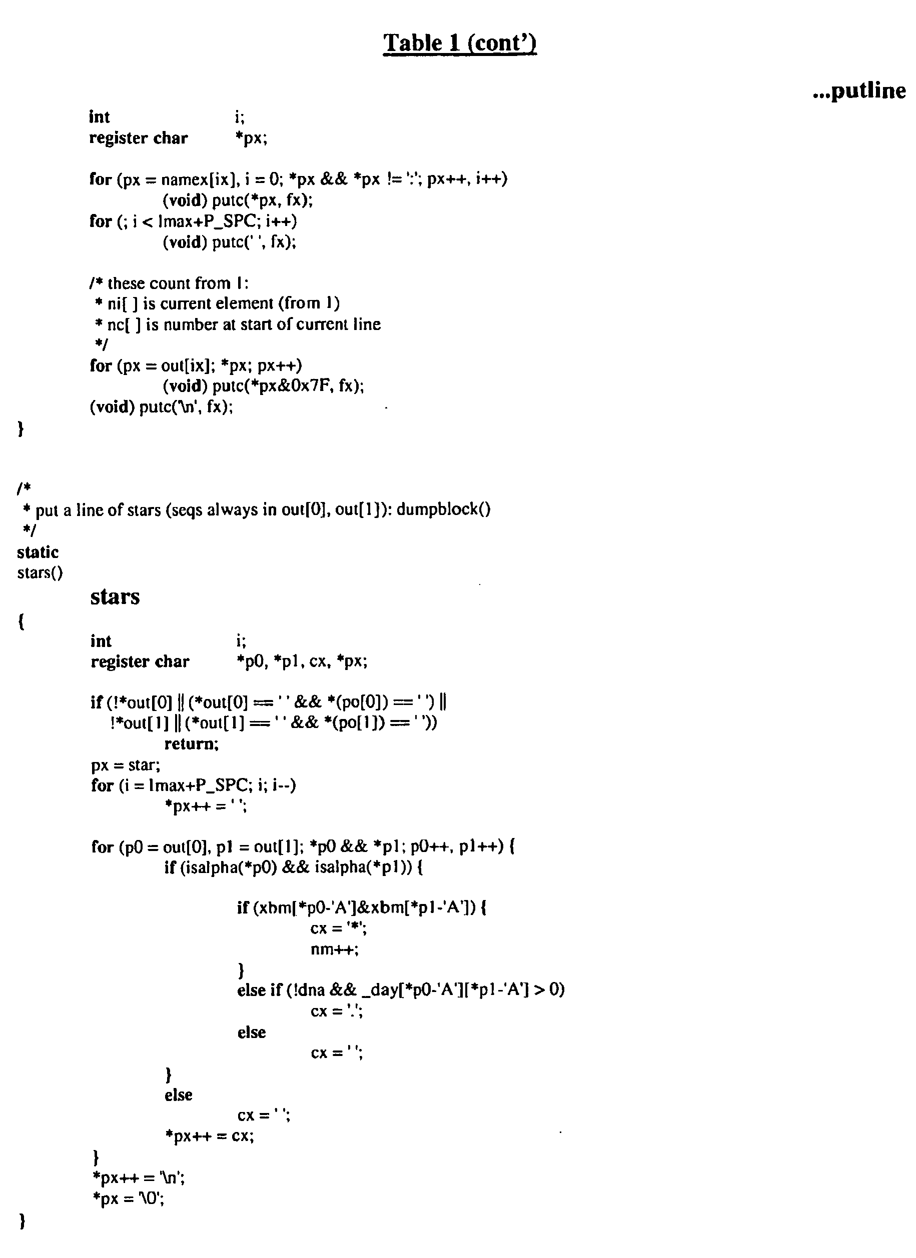

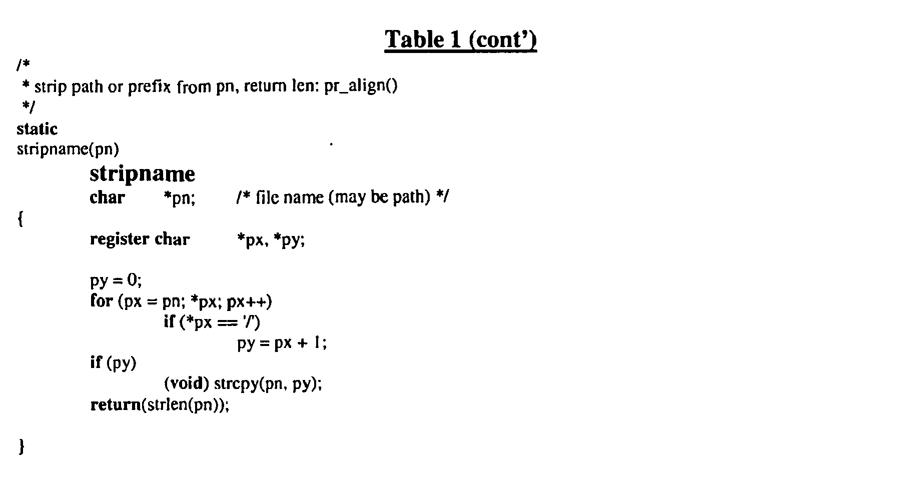

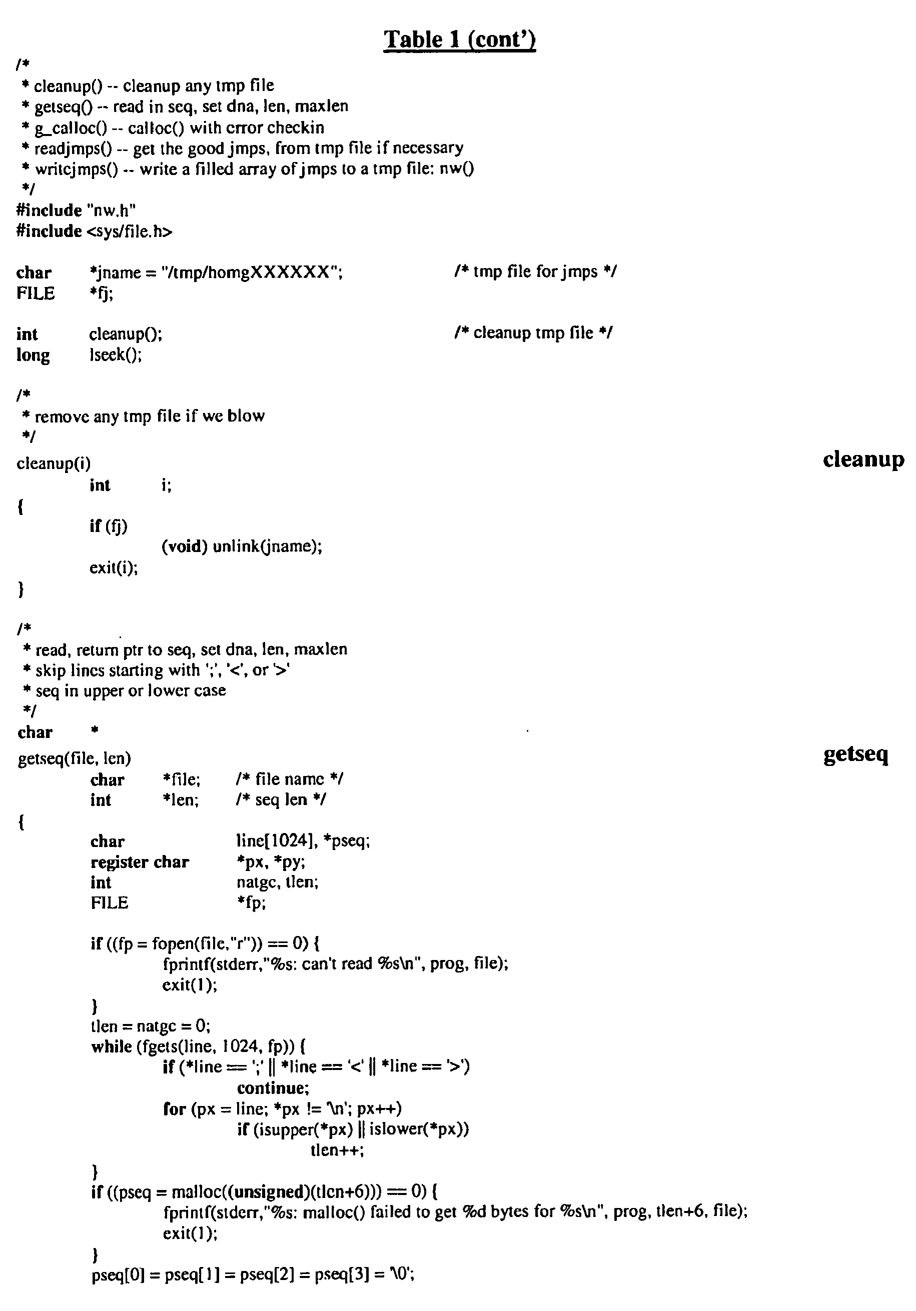

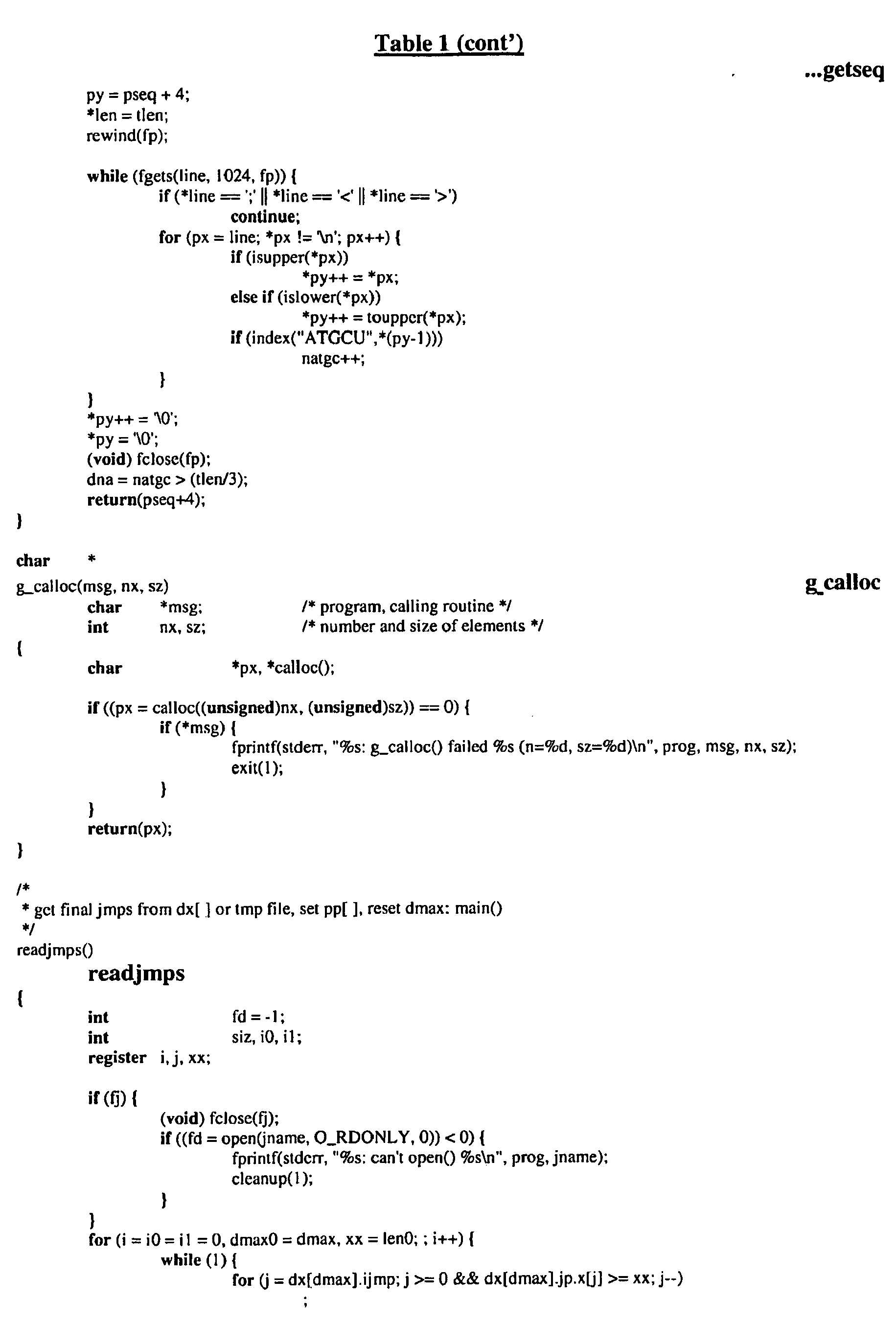

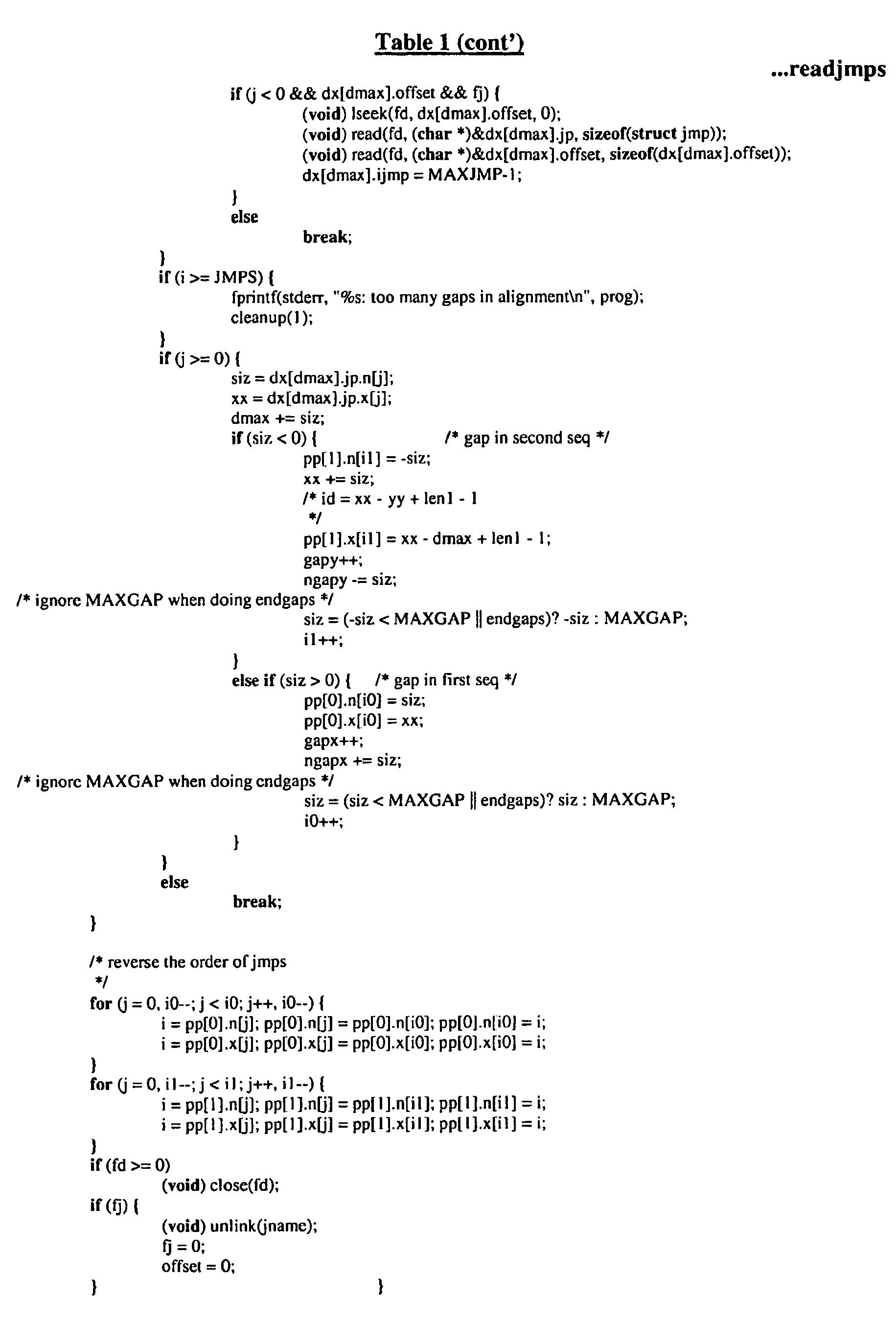

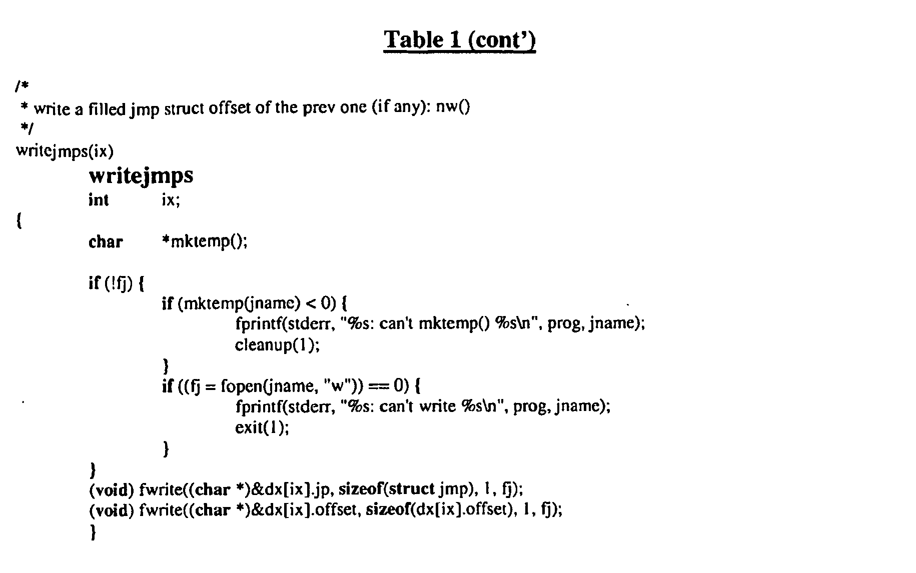

**Table 2**

| | | |
|---|---|---|
| PRO | XXXXXXXXXXXXXXX | (Length = 15 amino acids) |
| Comparison Protein | XXXXXXXXXXXX | (Length = 12 amino acids) |
| % amino acid sequence identity = (the number of identically matching amino acid residues between the two polypeptide sequences as determined by ALIGN-2) divided by (the total number of amino acid residues of the PRO polypeptide) = 5 divided by 15 = 33.3% | | |

**Table 3**

| | | |
|---|---|---|
| PRO | XXXXXXXXXX | (Length = 10 amino acids) |
| Comparison Protein | XXXXXYYYYYYZZYZ | (Length = 15 amino acids) |
| % amino acid sequence identity = (the number of identically matching amino acid residues between the two polypeptide sequences as determined by ALIGN-2) divided by (the total number of amino acid residues of the PRO polypeptide) = 5 divided by 10 = 50% | | |

**Table 4**

| | | |
|---|---|---|
| PRO-DNA | NNNNNNNNNNNNNN | (Length = 14 nucleotides) |
| Comparison DNA | NNNNNNLLLLLLLLLL | (Length = 16 nucleotides) |
| % nucleic acid sequence identity = (the number of identically matching nucleotides between the two nucleic acid sequences as determined by ALIGN-2) divided by (the total number of nucleotides of the PRO-DNA nucleic acid sequence) = 6 divided by 14 = 42.9% | | |

**Table 5**

| | | |
|---|---|---|
| PRO-DNA | NNNNNNNNNNNN | (Length = 12 nucleotides) |
| Comparison DNA | NNNNLLLVV | (Length = 9 nucleotides) |
| % nucleic acid sequence identity = (the number of identically matching nucleotides between the two nucleic acid sequences as determined by ALIGN-2) divided by (the total number of nucleotides of the PRO-DNA nucleic acid sequence) = 4 divided by 12 = 33.3% | | |

### II. Compositions and Methods of the Invention

### A. Full-Length PRO Polypeptides

The present invention provides newly identified and isolated nucleotide sequences encoding polypeptides referred to in the present application as PRO polypeptides. In particular, cDNAs encoding various PRO polypeptides have been identified and isolated, as disclosed in further detail in the Examples below. However, for sake of simplicity, in the present specification the protein encoded by the full length native nucleic acid molecules disclosed herein as well as all further native homologues and variants included in the foregoing definition of PRO, will be referred to as "PRO/number", regardless of their origin or mode of preparation.

As disclosed in the Examples below, various cDNA clones have been disclosed. The predicted amino acid sequence can be determined from the nucleotide sequence using routine skill. For the PRO polypeptides and encoding nucleic acids described herein, Applicants have identified what is believed to be the reading frame best identifiable with the sequence information available at the time.

### B. PRO Polypeptide Variants

In addition to the full-length native sequence PRO polypeptides described herein, it is contemplated that PRO variants can be prepared. PRO variants can be prepared by introducing appropriate nucleotide changes into the PRO DNA, and/or by synthesis of the desired PRO polypeptide. Those skilled in the art will appreciate that amino acid changes may alter post-translational processes of the PRO, such as changing the number or position of glycosylation sites or altering the membrane anchoring characteristics.

Variations in the native full-length sequence PRO or in various domains of the PRO described herein, can be made, for example, using any of the techniques and guidelines for conservative and non-conservative mutations set forth, for instance, in U.S. Patent No. 5,364,934. Variations may be a substitution, deletion or insertion of one or more codons encoding the PRO that results in a change in the amino acid sequence of the PRO as compared with the native sequence PRO. Optionally, the variation is by substitution of at least one amino acid with any other amino acid in one or more of the domains of the PRO. Guidance in determining which amino acid residue may be inserted, substituted or deleted without adversely affecting the desired activity may be found by comparing the sequence of the PRO with that of homologous known protein molecules and minimizing the number of amino acid sequence changes made in regions of high homology. Amino acid substitutions can be the result of replacing one amino acid with another amino acid having similar structural and/or chemical properties, such as the replacement of a leucine with a serine, i.e., conservative amino acid replacements. Insertions or deletions may optionally be in the range of about 1 to 5 amino acids. The variation allowed may be determined by systematically making insertions, deletions or substitutions of amino acids in the sequence and testing the resulting variants for activity exhibited by the full-length or mature native sequence.

PRO polypeptide fragments are provided herein. Such fragments may be truncated at the N-terminus or C-terminus, or may lack internal residues, for example, when compared with a full length native protein. Certain fragments lack amino acid residues that are not essential for a desired biological activity of the PRO polypeptide.

PRO fragments may be prepared by any of a number of conventional techniques. Desired peptide fragments may be chemically synthesized. An alternative approach involves generating PRO fragments by enzymatic digestion, e.g., by treating the protein with an enzyme known to cleave proteins at sites defined by particular amino acid residues, or by digesting the DNA with suitable restriction enzymes and isolating the desired fragment. Yet another suitable technique involves isolating and amplifying a DNA fragment encoding a desired polypeptide fragment, by polymerase chain reaction (PCR). Oligonucleotides that define the desired termini of the DNA fragment are employed at the 5' and 3' primers in the PCR. Preferably, PRO polypeptide fragments share at least one biological and/or immunological activity with the native PRO polypeptide disclosed herein.

In particular embodiments, conservative substitutions of interest are shown in Table 6 under the heading of preferred substitutions. If such substitutions result in a change in biological activity, then more substantial changes, denominated exemplary substitutions in Table 6, or as further described below in reference to amino acid classes, are introduced and the products screened.

**Table 6**

| Original Residue | Exemplary Substitutions | Preferred Substitutions |
|---|---|---|
| Ala (A) | val; leu; ile | val |
| Arg (R) | lys; gln; asn | lys |
| Asn (N) | gln; his; lys; arg | gln |
| Asp (D) | glu | glu |
| Cys (C) | ser | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe; norleucine | leu |
| Leu (L) | norleucine; ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala; norleucine | leu |

Substantial modifications in function or immunological identity of the PRO polypeptide are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: norleucine, met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class. Such substituted residues also may be introduced into the conservative substitution sites or, more preferably, into the remaining (non-conserved) sites.

The variations can be made using methods known in the art such as oligonucleotide-mediated (site-directed) mutagenesis, alanine scanning, and PCR mutagenesis. Site-directed mutagenesis [Carter et al., Nucl. Acids Res., 13:4331 (1986); Zoller et al., Nucl. Acids Res., 10:6487 (1987)], cassette mutagenesis [Wells et al., Gene, 34:315 (1985)], restriction selection mutagenesis [Wells et al., Philos. Trans. R. Soc. London SerA, 317:415 (1986)] or other known techniques can be performed on the cloned DNA to produce the PRO variant DNA.

Scanning amino acid analysis can also be employed to identify one or more amino acids along a contiguous sequence. Among the preferred scanning amino acids are relatively small, neutral amino acids. Such amino acids include alanine, glycine, serine, and cysteine. Alanine is typically a preferred scanning amino acid among this group because it eliminates the side-chain beyond the beta-carbon and is less likely to alter the main-chain conformation of the variant [Cunningham and Wells, Science, 244: 1081-1085 (1989)]. Alanine is also typically preferred because it is the most common amino acid. Further, it is frequently found in both buried and exposed positions [Creighton, The Proteins, (W.H. Freeman & Co., N.Y.); Chothia, J. Mol. Biol., 150:1 (1976)]. If alanine substitution does not yield adequate amounts of variant, an isoteric amino acid can be used.

### C. Modifications of PRO

Covalent modifications of PRO are included within the scope of this invention. One type of covalent modification includes reacting targeted amino acid residues of a PRO polypeptide with an organic derivatizing agent that is capable of reacting with selected side chains or the N- or C- terminal residues of the PRO. Derivatization with bifunctional agents is useful, for instance, for crosslinking PRO to a water-insoluble support matrix or surface for use in the method for purifying anti-PRO antibodies, and vice-versa. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl or threonyl residues, methylation of the α-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the PRO polypeptide included within the scope of this invention comprises altering the native glycosylation pattern of the polypeptide. "Altering the native glycosylation pattern" is intended for purposes herein to mean deleting one or more carbohydrate moieties found in native sequence PRO (either by removing the underlying glycosylation site or by deleting the glycosylation by chemical and/or enzymatic means), and/or adding one or more glycosylation sites that are not present in the native sequence PRO. In addition, the phrase includes qualitative changes in the glycosylation of the native proteins, involving a change in the nature and proportions of the various carbohydrate moieties present.

Addition of glycosylation sites to the PRO polypeptide may be accomplished by altering the amino acid sequence. The alteration may be made, for example, by the addition of, or substitution by, one or more serine or threonine residues to the native sequence PRO (for O-linked glycosylation sites). The PRO amino acid sequence may optionally be altered through changes at the DNA level, particularly by mutating the DNA encoding the PRO polypeptide at preselected bases such that codons are generated that will translate into the desired amino acids.

Another means of increasing the number of carbohydrate moieties on the PRO polypeptide is by chemical or enzymatic coupling of glycosides to the polypeptide. Such methods are described in the art, e.g., in WO 87/05330 published 11 September 1987, and in Aplin and Wriston, CRC Crit. Rev. Biochem., pp. 259-306 (1981).

Removal of carbohydrate moieties present on the PRO polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo- and exo-glycosidases as described by Thotakura et al., Meth. Enzymol., 138:350 (1987).

Another type of covalent modification of PRO comprises linking the PRO polypeptide to one of a variety of nonproteinaceous polymers, e.g., polyethylene glycol (PEG), polypropylene glycol, or polyoxyalkylenes, in the manner set forth in U.S. Patent Nos. 4,640,835; 4,496,689; 4,301,144; 4,670,417; 4,791,192 or 4,179,337.

The PRO of the present invention may also be modified in a way to form a chimeric molecule comprising PRO fused to another, heterologous polypeptide or amino acid sequence.

In one embodiment, such a chimeric molecule comprises a fusion of the PRO with a tag polypeptide which provides an epitope to which an anti-tag antibody can selectively bind. The epitope tag is generally placed at the amino- or carboxyl- terminus of the PRO. The presence of such epitope-tagged forms of the PRO can be detected using an antibody against the tag polypeptide. Also, provision of the epitope tag enables the PRO to be readily purified by affinity purification using an anti-tag antibody or another type of affinity matrix that binds to the epitope tag. Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; an alpha-tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

In an alternative embodiment, the chimeric molecule may comprise a fusion of the PRO with an immunoglobulin or a particular region of an immunoglobulin. For a bivalent form of the chimeric molecule (also referred to as an "immunoadhesin"), such a fusion could be to the Fc region of an IgG molecule. The Ig fusions preferably include the substitution of a soluble (transmembrane domain deleted or inactivated) form of a PRO polypeptide in place of at least one variable region within an Ig molecule. In a particularly preferred embodiment, the immunoglobulin fusion includes the hinge, CH2 and CH3, or the hinge, CH1, CH2 and CH3 regions of an IgG1 molecule. For the production of immunoglobulin fusions see also US Patent No. 5,428,130 issued June 27, 1995.

### D. Preparation of PRO

The description below relates primarily to production of PRO by culturing cells transformed or transfected with a vector containing PRO nucleic acid. It is, of course, contemplated that alternative methods, which are well known in the art, may be employed to prepare PRO. For instance, the PRO sequence, or portions thereof, may be produced by direct peptide synthesis using solid-phase techniques [see, e.g., Stewart et al., Solid-Phase Peptide Synthesis, W.H. Freeman Co., San Francisco, CA (1969); Merrifield, J. Am. Chem. Soc., 85:2149-2154 (1963)]. *In vitro* protein synthesis may be performed using manual techniques or by automation. Automated synthesis may be accomplished, for instance, using an Applied Biosystems Peptide Synthesizer (Foster City, CA) using manufacturer's instructions. Various portions of the PRO may be chemically synthesized separately and combined using chemical or enzymatic methods to produce the full-length PRO.

### 1. Isolation of DNA Encoding PRO

DNA encoding PRO may be obtained from a cDNA library prepared from tissue believed to possess the PRO mRNA and to express it at a detectable level. Accordingly, human PRO DNA can be conveniently obtained from a cDNA library prepared from human tissue, such as described in the Examples. The PRO-encoding gene may also be obtained from a genomic library or by known synthetic procedures (e.g., automated nucleic acid synthesis).

Libraries can be screened with probes (such as antibodies to the PRO or oligonucleotides of at least about 20-80 bases) designed to identify the gene of interest or the protein encoded by it. Screening the cDNA or genomic library with the selected probe may be conducted using standard procedures, such as described in Sambrook et al., Molecular Cloning: A Laboratory Manual (New York: Cold Spring Harbor Laboratory Press, 1989). An alternative means to isolate the gene encoding PRO is to use PCR methodology [Sambrook et al., supra; Dieffenbach et al., PCR Primer: A Laboratory Manual (Cold Spring Harbor Laboratory Press, 1995)].

The Examples below describe techniques for screening a cDNA library. The oligonucleotide sequences selected as probes should be of sufficient length and sufficiently unambiguous that false positives are minimized. The oligonucleotide is preferably labeled such that it can be detected upon hybridization to DNA in the library being screened. Methods of labeling are well known in the art, and include the use of radiolabels like ³²P-labeled ATP, biotinylation or enzyme labeling. Hybridization conditions, including moderate stringency and high stringency, are provided in Sambrook et al., supra.

Sequences identified in such library screening methods can be compared and aligned to other known sequences deposited and available in public databases such as GenBank or other private sequence databases. Sequence identity (at either the amino acid or nucleotide level) within defined regions of the molecule or across the full-length sequence can be determined using methods known in the art and as described herein.

Nucleic acid having protein coding sequence may be obtained by screening selected cDNA or genomic libraries using the deduced amino acid sequence disclosed herein for the first time, and, if necessary, using conventional primer extension procedures as described in Sambrook et al., supra, to detect precursors and processing intermediates of mRNA that may not have been reverse-transcribed into cDNA.

### 2. Selection and Transformation of Host Cells

Host cells are transfected or transformed with expression or cloning vectors described herein for PRO production and cultured in conventional nutrient media modified as appropriate for inducing promoters, selecting transformants, or amplifying the genes encoding the desired sequences. The culture conditions, such as media, temperature, pH and the like, can be selected by the skilled artisan without undue experimentation. In general, principles, protocols, and practical techniques for maximizing the productivity of cell cultures can be found in Mammalian Cell Biotechnology: a Practical Approach, M. Butler, ed. (IRL Press, 1991) and Sambrook et al., supra.

Methods of eukaryotic cell transfection and prokaryotic cell transformation are known to the ordinarily skilled artisan, for example, CaCl₂, CaPO₄, liposome-mediated and electroporation. Depending on the host cell used, transformation is performed using standard techniques appropriate to such cells. The calcium treatment employing calcium chloride, as described in Sambrook et al., supra, or electroporation is generally used for prokaryotes. Infection with *Agrobacterium tumefaciens* is used for transformation of certain plant cells, as described by Shaw et al., Gene, 23:315 (1983) and WO 89/05859 published 29 June 1989. For mammalian cells without such cell walls, the calcium phosphate precipitation method of Graham and van der Eb, Virology, 52:456-457 (1978) can be employed. General aspects of mammalian cell host system transfections have been described in U.S. Patent No. 4,399,216. Transformations into yeast are typically carried out according to the method of Van Solingen et al., J. Bact., 130:946 (1977) and Hsiao et al., Proc. Natl. Acad. Sci. (USA), 76:3829 (1979). However, other methods for introducing DNA into cells, such as by nuclear microinjection, electroporation, bacterial protoplast fusion with intact cells, or polycations, e.g., polybrene, polyornithine, may also be used. For various techniques for transforming mammalian cells, see Keown et al., Methods in Enzymology, 185:527-537 (1990) and Mansour et al., Nature, 336:348-352 (1988).

Suitable host cells for cloning or expressing the DNA in the vectors herein include prokaryote, yeast, or higher eukaryote cells. Suitable prokaryotes include but are not limited to eubacteria, such as Gram-negative or Gram-positive organisms, for example, Enterobacteriaceae such as *E. coli.* Various *E. coli* strains are publicly available, such as *E. coli* K12 strain MM294 (ATCC 31,446); *E. coli* X1776 (ATCC 31,537); *E. coli* strain W3110 (ATCC 27,325) and K5 772 (ATCC 53,635). Other suitable prokaryotic host cells include Enterobacteriaceae such as *Escherichia*, e.g., *E. coli, Enterobacter, Erwinia, Klebsiella, Proteus, Salmonella*, e.g., *Salmonella typhimurium, Serratia*, e.g., *Serratia marcescans*, and *Shigella*, as well as *Bacilli* such as *B. subtilis* and *B. licheniformis* (e.g., *B. licheniformis* 41 P disclosed in DD 266,710 published 12 April 1989), *Pseudomonas* such as *P. aeruginosa*, and *Streptomyces*. These examples are illustrative rather than limiting. Strain W3110 is one particularly preferred host or parent host because it is a common host strain for recombinant DNA product fermentations. Preferably, the host cell secretes minimal amounts of proteolytic enzymes. For example, strain W3110 may be modified to effect a genetic mutation in the genes encoding proteins endogenous to the host, with examples of such hosts including *E. coli* W3110 strain 1A2, which has the complete genotype *tonA* ; *E. coli* W3110 strain 9E4, which has the complete genotype *tonA ptr3; E. coli* W3110 strain 27C7 (ATCC 55,244), which has the complete genotype *tonA ptr3 phoA E15 (argF-lac)169 degP ompT kan*^{r}; *E. coli* W3110 strain 37D6, which has the complete genotype *tonA ptr3 phoA E15 (argF-lac)169 degP ompT rbs7 i*/*vG kan^{r}*; *E. coli* W3110 strain 40B4, which is strain 37D6 with a non-kanamycin resistant *degP* deletion mutation; and an *E. coli* strain having mutant periplasmic protease disclosed in U.S. Patent No. 4,946,783 issued 7 August 1990. Alternatively, *in vitro* methods of cloning, e.g., PCR or other nucleic acid polymerase reactions, are suitable.

In addition to prokaryotes, eukaryotic microbes such as filamentous fungi or yeast are suitable cloning or expression hosts for PRO-encoding vectors. *Saccharomyces cerevisiae* is a commonly used lower eukaryotic host microorganism. Others include *Schizosaccharomyces pombe* (Beach and Nurse, Nature, 290: 140 [1981]; EP 139,383 published 2 May 1985); *Kluyveromyces* hosts (U.S. Patent No. 4,943,529; Fleer et al., Bio/Technology, 9:968-975 (1991)) such as, e.g., *K. lactis* (MW98-8C, CBS683, CBS4574; Louvencourt et al., J. Bacteriol., 154(2):737-742 [1983]), *K. fragilis* (ATCC 12,424), *K. bulgaricus* (ATCC 16,045), *K. wickeramii* (ATCC 24,178), *K. waltii* (ATCC 56,500), *K. drosophilarum* (ATCC 36,906; Van den Berg et al., Bio/Technology, 8:135 (1990)), *K. thermotolerans*, and *K. marxianus; yarrowia* (EP 402,226); *Pichia pastoris* (EP 183,070; Sreekrishna et al., J. Basic Microbiol., 28:265-278 [1988]); *Candida; Trichoderma reesia* (EP 244,234); *Neurospora crassa* (Case et al., Proc. Natl. Acad. Sci. USA, 76:5259-5263 [1979]); *Schwanniomyces* such as *Schwanniomyces occidentalis* (EP 394,538 published 31 October 1990); and filamentous fungi such as, e.g., *Neurospora, Penicillium, Tolypocladium* (WO 91/00357 published 10 January 1991), and *Aspergillus* hosts such as *A. nidulans* (Ballance et al., Biochem. Biophys. Res. Commun., 112:284-289 [1983]; Tilburn et al., Gene, 26:205-221 [1983]; Yelton et al., Proc. Natl. Acad. Sci. USA, 81: 1470-1474 [1984]) and *A. niger* (Kelly and Hynes, EMBO J., 4:475-479 [1985]). Methylotropic yeasts are suitable herein and include, but are not limited to, yeast capable of growth on methanol selected from the genera consisting of *Hansenula, Candida, Kloeckera, Pichia, Saccharomyces, Torulopsis*, and *Rhodotorula*. A list of specific species that are exemplary of this class of yeasts may be found in C. Anthony, The Biochemistry of Methylotrophs, 269 (1982).

Suitable host cells for the expression of glycosylated PRO are derived from multicellular organisms. Examples of invertebrate cells include insect cells such as Drosophila S2 and Spodoptera Sf9, as well as plant cells. Examples of useful mammalian host cell lines include Chinese hamster ovary (CHO) and COS cells. More specific examples include monkey kidney CV1 line transformed by SV40 (COS-7, ATCC CRL 1651); human embryonic kidney line (293 or 293 cells subcloned for growth in suspension culture, Graham et al., J. Gen Virol., 36:59 (1977)); Chinese hamster ovary cells/-DHFR (CHO, Urlaub and Chasin, Proc. Natl. Acad. Sci. USA, 77:4216 (1980)); mouse sertoli cells (TM4, Mather, Biol. Reprod., 23:243-251 (1980)); human lung cells (W138, ATCC CCL 75); human liver cells (Hep G2, HB 8065); and mouse mammary tumor (MMT 060562, ATCC CCL51). The selection of the appropriate host cell is deemed to be within the skill in the art.

### 3. Selection and Use of a Replicable Vector

The nucleic acid (e.g., cDNA or genomic DNA) encoding PRO may be inserted into a replicable vector for cloning (amplification of the DNA) or for expression. Various vectors are publicly available. The vector may, for example, be in the form of a plasmid, cosmid, viral particle, or phage. The appropriate nucleic acid sequence may be inserted into the vector by a variety of procedures. In general, DNA is inserted into an appropriate restriction endonuclease site(s) using techniques known in the art. Vector components generally include, but are not limited to, one or more of a signal sequence, an origin of replication, one or more marker genes, an enhancer element, a promoter, and a transcription termination sequence. Construction of suitable vectors containing one or more of these components employs standard ligation techniques which are known to the skilled artisan.

The PRO may be produced recombinantly not only directly, but also as a fusion polypeptide with a heterologous polypeptide, which may be a signal sequence or other polypeptide having a specific cleavage site at the N-terminus of the mature protein or polypeptide. In general, the signal sequence may be a component of the vector, or it may be a part of the PRO-encoding DNA that is inserted into the vector. The signal sequence may be a prokaryotic signal sequence selected, for example, from the group of the alkaline phosphatase, penicillinase, lpp, or heat-stable enterotoxin II leaders. For yeast secretion the signal sequence may be, e.g., the yeast invertase leader, alpha factor leader (including *Saccharomyces* and *Kluyveromyces* α-factor leaders, the latter described in U.S. Patent No. 5,010,182), or acid phosphatase leader, the *C. albicans* glucoamylase leader (EP 362,179 published 4 April 1990), or the signal described in WO 90/13646 published 15 November 1990. In mammalian cell expression, mammalian signal sequences may be used to direct secretion of the protein, such as signal sequences from secreted polypeptides of the same or related species, as well as viral secretory leaders.

Both expression and cloning vectors contain a nucleic acid sequence that enables the vector to replicate in one or more selected host cells. Such sequences are well known for a variety of bacteria, yeast, and viruses. The origin of replication from the plasmid pBR322 is suitable for most Gram-negative bacteria, the 2µ plasmid origin is suitable for yeast, and various viral origins (SV40, polyoma, adenovirus, VSV or BPV) are useful for cloning vectors in mammalian cells.

Expression and cloning vectors will typically contain a selection gene, also termed a selectable marker. Typical selection genes encode proteins that (a) confer resistance to antibiotics or other toxins, e.g., ampicillin, neomycin, methotrexate, or tetracycline, (b) complement auxotrophic deficiencies, or (c) supply critical nutrients not available from complex media, e.g., the gene encoding D-alanine racemase for *Bacilli.*

An example of suitable selectable markers for mammalian cells are those that enable the identification of cells competent to take up the PRO-encoding nucleic acid, such as DHFR or thymidine kinase. An appropriate host cell when wild-type DHFR is employed is the CHO cell line deficient in DHFR activity, prepared and propagated as described by Urlaub et al., Proc. Natl. Acad. Sci. USA, 77:4216 (1980). A suitable selection gene for use in yeast is the *trp*1 gene present in the yeast plasmid YRp7 [Stinchcomb et al., Nature, 282:39 (1979); Kingsman et al., Gene, 7:141 (1979); Tschemper et al., Gene, 10:157 (1980)]. The *trp*1 gene provides a selection marker for a mutant strain of yeast lacking the ability to grow in tryptophan, for example, ATCC No. 44076 or PEP4-1 [Jones, Genetics, 85:12 (1977)].

Expression and cloning vectors usually contain a promoter operably linked to the PRO-encoding nucleic acid sequence to direct mRNA synthesis. Promoters recognized by a variety of potential host cells are well known. Promoters suitable for use with prokaryotic hosts include the β-lactamase and lactose promoter systems [Chang et al., Nature, 275:615 (1978); Goeddel et al., Nature, 281:544 (1979)], alkaline phosphatase, a tryptophan (trp) promoter system [Goeddel, Nucleic Acids Res., 8:4057 (1980); EP 36,776], and hybrid promoters such as the tac promoter [deBoer et al., Proc. Natl. Acad. Sci. USA, 80:21-25 (1983)]. Promoters for use in bacterial systems also will contain a Shine-Dalgarno (S.D.) sequence operably linked to the DNA encoding PRO.

Examples of suitable promoting sequences for use with yeast hosts include the promoters for 3-phosphoglycerate kinase [Hitzeman et al., J. Biol. Chem., 255:2073 (1980)] or other glycolytic enzymes [Hess et al., J. Adv. Enzyme Reg., 7:149 (1968); Holland, Biochemistry, 17:4900 (1978)], such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase.

Other yeast promoters, which are inducible promoters having the additional advantage of transcription controlled by growth conditions, are the promoter regions for alcohol dehydrogenase 2, isocytochrome C, acid phosphatase, degradative enzymes associated with nitrogen metabolism, metallothionein, glyceraldehyde-3-phosphate dehydrogenase, and enzymes responsible for maltose and galactose utilization. Suitable vectors and promoters for use in yeast expression are further described in EP 73,657.

PRO transcription from vectors in mammalian host cells is controlled, for example, by promoters obtained from the genomes of viruses such as polyoma virus, fowlpox virus (UK 2,211,504 published 5 July 1989), adenovirus (such as Adenovirus 2), bovine papilloma virus, avian sarcoma virus, cytomegalovirus, a retrovirus, hepatitis-B virus and Simian Virus 40 (SV40), from heterologous mammalian promoters, e.g., the actin promoter or an immunoglobulin promoter, and from heat-shock promoters, provided such promoters are compatible with the host cell systems.

Transcription of a DNA encoding the PRO by higher eukaryotes may be increased by inserting an enhancer sequence into the vector. Enhancers are cis-acting elements of DNA, usually about from 10 to 300 bp, that act on a promoter to increase its transcription. Many enhancer sequences are now known from mammalian genes (globin, elastase, albumin, α-fetoprotein, and insulin). Typically, however, one will use an enhancer from a eukaryotic cell virus. Examples include the SV40 enhancer on the late side of the replication origin (bp 100-270), the cytomegalovirus early promoter enhancer, the polyoma enhancer on the late side of the replication origin, and adenovirus enhancers. The enhancer may be spliced into the vector at a position 5' or 3' to the PRO coding sequence, but is preferably located at a site 5' from the promoter.

Expression vectors used in eukaryotic host cells (yeast, fungi, insect, plant, animal, human, or nucleated cells from other multicellular organisms) will also contain sequences necessary for the termination of transcription and for stabilizing the mRNA. Such sequences are commonly available from the 5' and, occasionally 3', untranslated regions of eukaryotic or viral DNAs or cDNAs. These regions contain nucleotide segments transcribed as polyadenylated fragments in the untranslated portion of the mRNA encoding PRO.

Still other methods, vectors, and host cells suitable for adaptation to the synthesis of PRO in recombinant vertebrate cell culture are described in Gething et al., Nature, 293:620-625 (1981); Mantei et al., Nature, 281:40-46 (1979); EP 117,060; and EP 117,058.

### 4. Detecting Gene Amplification/Expression

Gene amplification and/or expression may be measured in a sample directly, for example, by conventional Southern blotting, Northern blotting to quantitate the transcription of mRNA [Thomas, Proc. Natl. Acad. Sci. USA, 77:5201-5205 (1980)], dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes. The antibodies in turn may be labeled and the assay may be carried out where the duplex is bound to a surface, so that upon the formation of duplex on the surface, the presence of antibody bound to the duplex can be detected.

Gene expression, alternatively, may be measured by immunological methods, such as immunohistochemical staining of cells or tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence PRO polypeptide or against a synthetic peptide based on the DNA sequences provided herein or against exogenous sequence fused to PRO DNA and encoding a specific antibody epitope.

### 5. Purification of Polypeptide

Forms of PRO may be recovered from culture medium or from host cell lysates. If membrane-bound, it can be released from the membrane using a suitable detergent solution (e.g. Triton-X 100) or by enzymatic cleavage. Cells employed in expression of PRO can be disrupted by various physical or chemical means, such as freeze-thaw cycling, sonication, mechanical disruption, or cell lysing agents.

It may be desired to purify PRO from recombinant cell proteins or polypeptides. The following procedures are exemplary of suitable purification procedures: by fractionation on an ion-exchange column; ethanol precipitation; reverse phase HPLC; chromatography on silica or on a cation-exchange resin such as DEAE; chromatofocusing; SDS-PAGE; ammonium sulfate precipitation; gel filtration using, for example, Sephadex G-75; protein A Sepharose columns to remove contaminants such as IgG; and metal chelating columns to bind epitope-tagged forms of the PRO. Various methods of protein purification may be employed and such methods are known in the art and described for example in Deutscher, Methods in Enzymology, 182 (1990); Scopes, Protein Purification: Principles and Practice, Springer-Verlag, New York (1982). The purification step(s) selected will depend, for example, on the nature of the production process used and the particular PRO produced.

### E. Tissue Distribution

The location of tissues expressing the PRO can be identified by determining mRNA expression in various human tissues. The location of such genes provides information about which tissues are most likely to be affected by the stimulating and inhibiting activities of the PRO polypeptides. The location of a gene in a specific tissue also provides sample tissue for the activity blocking assays discussed below.

As noted before, gene expression in various tissues may be measured by conventional Southern blotting, Northern blotting to quantitate the transcription of mRNA (Thomas, Proc. Natl. Acad. Sci. USA, 77:5201-5205 [1980]), dot blotting (DNA analysis), or *in situ* hybridization, using an appropriately labeled probe, based on the sequences provided herein. Alternatively, antibodies may be employed that can recognize specific duplexes, including DNA duplexes, RNA duplexes, and DNA-RNA hybrid duplexes or DNA-protein duplexes.

Gene expression in various tissues, alternatively, may be measured by immunological methods, such as immunohistochemical staining of tissue sections and assay of cell culture or body fluids, to quantitate directly the expression of gene product. Antibodies useful for immunohistochemical staining and/or assay of sample fluids may be either monoclonal or polyclonal, and may be prepared in any mammal. Conveniently, the antibodies may be prepared against a native sequence of a PRO polypeptide or against a synthetic peptide based on the DNA sequences encoding the PRO polypeptide or against an exogenous sequence fused to a DNA encoding a PRO polypeptide and encoding a specific antibody epitope. General techniques for generating antibodies, and special protocols for Northern blotting and *in situ* hybridization are provided below.

### F. Antibody Binding Studies

The activity of the PRO polypeptides can be further verified by antibody binding studies, in which the ability of anti-PRO antibodies to inhibit the effect of the PRO polypeptides, respectively, on tissue cells is tested. Exemplary antibodies include polyclonal, monoclonal, humanized, bispecific, and heteroconjugate antibodies, the preparation of which will be described hereinbelow.

Antibody binding studies may be carried out in any known assay method, such as competitive binding assays, direct and indirect sandwich assays, and immunoprecipitation assays. Zola, Monoclonal Antibodies: A Manual of Techniques, pp.147-158 (CRC Press, Inc., 1987).

Competitive binding assays rely on the ability of a labeled standard to compete with the test sample analyte for binding with a limited amount of antibody. The amount of target protein in the test sample is inversely proportional to the amount of standard that becomes bound to the antibodies. To facilitate determining the amount of standard that becomes bound, the antibodies preferably are insolubilized before or after the competition, so that the standard and analyte that are bound to the antibodies may conveniently be separated from the standard and analyte which remain unbound.

Sandwich assays involve the use of two antibodies, each capable of binding to a different immunogenic portion, or epitope, of the protein to be detected. In a sandwich assay, the test sample analyte is bound by a first antibody which is immobilized on a solid support, and thereafter a second antibody binds to the analyte, thus forming an insoluble three-part complex. See, *e.g.,* US Pat No. 4,376,110. The second antibody may itself be labeled with a detectable moiety (direct sandwich assays) or may be measured using an anti-immunoglobulin antibody that is labeled with a detectable moiety (indirect sandwich assay). For example, one type of sandwich assay is an ELISA assay, in which case the detectable moiety is an enzyme.

For immunohistochemistry, the tissue sample may be fresh or frozen or may be embedded in paraffin and fixed with a preservative such as formalin, for example.

### G. Cell-Based Assays

Cell-based assays and animal models for immune related diseases can be used to further understand the relationship between the genes and polypeptides identified herein and the development and pathogenesis of immune related disease.

In a different approach, cells of a cell type known to be involved in a particular immune related disease are transfected with the cDNAs described herein, and the ability of these cDNAs to stimulate or inhibit immune function is analyzed. Suitable cells can be transfected with the desired gene, and monitored for immune function activity. Such transfected cell lines can then be used to test the ability of poly- or monoclonal antibodies or antibody compositions to inhibit or stimulate immune function, for example to modulate T-cell proliferation or inflammatory cell infiltration. Cells transfected with the coding sequences of the genes identified herein can further be used to identify drug candidates for the treatment of immune related diseases.

In addition, primary cultures derived from transgenic animals (as described below) can be used in the cell-based assays herein, although stable cell lines are preferred. Techniques to derive continuous cell lines from transgenic animals are well known in the art (see, *e.g.,* Small et al., Mol. Cell. Biol. 5: 642-648 [1985]).

One suitable cell based assay is the mixed lymphocyte reaction (MLR). Current Protocols in Immunology, unit 3.12; edited by J E Coligan, A M Kruisbeek, D H Marglies, E M Shevach, W Strober, National Institutes of Health, Published by John Wiley & Sons, Inc. In this assay, the ability of a test compound to stimulate or inhibit the proliferation of activated T cells is assayed. A suspension of responder T cells is cultured with allogeneic stimulator cells and the proliferation of T cells is measured by uptake of tritiated thymidine. This assay is a general measure of T cell reactivity. Since the majority of T cells respond to and produce IL-2 upon activation, differences in responsiveness in this assay in part reflect differences in IL-2 production by the responding cells. The MLR results can be verified by a standard lymphokine (IL-2) detection assay. *Current Protocols in Immunology,* above, 3.15, 6.3.

A proliferative T cell response in an MLR assay may be due to direct mitogenic properties of an assayed molecule or to external antigen induced activation. Additional verification of the T cell stimulatory activity of the PRO polypeptides can be obtained by a costimulation assay. T cell activation requires an antigen specific signal mediated through the T-cell receptor (TCR) and a costimulatory signal mediated through a second ligand binding interaction, for example, the B7 (CD80, CD86)/CD28 binding interaction. CD28 crosslinking increases lymphokine secretion by activated T cells. T cell activation has both negative and positive controls through the binding of ligands which have a negative or positive effect. CD28 and CTLA-4 are related glycoproteins in the Ig superfamily which bind to B7. CD28 binding to B7 has a positive costimulation effect of T cell activation; conversely, CTLA-4 binding to B7 has a T cell deactivating effect. Chambers, C. A. and Allison, J. P., Curr. Opin. Immunol. (1997) 9:396. Schwartz, R. H., Cell (1992) 71:1065; Linsey, P. S. and Ledbetter, J. A., Annu. Rev. Immunol. (1993) 11:191; June, C. H. et al, Immunol. Today (1994) 15:321; Jenkins, M. K., Immunity (1994) 1:405. In a costimulation assay, the PRO polypeptides are assayed for T cell costimulatory or inhibitory activity.

Direct use of a stimulating compound as in the invention has been validated in experiments with 4-1BB glycoprotein, a member of the tumor necrosis factor receptor family, which binds to a ligand (4-1BBL) expressed on primed T cells and signals T cell activation and growth. Alderson, M. E. et al., J. Immunol. (1994) 24:2219.

The use of an agonist stimulating compound has also been validated experimentally. Activation of 4-1BB by treatment with an agonist anti-4-1BB antibody enhances eradication of tumors. Hellstrom, I. and Hellstrom, K. E., Crit. Rev. Immunol. (1998) 18:1. Immunoadjuvant therapy for treatment of tumors, described in more detail below, is another example of the use of the stimulating compounds of the invention.

Alternatively, an immune stimulating or enhancing effect can also be achieved by administration of a PRO which has vascular permeability enhancing properties. Enhanced vascular permeability would be beneficial to disorders which can be attenuated by local infiltration of immune cells (*e.g*., monocytes, eosinophils, PMNs) and inflammation.

On the other hand, PRO polypeptides, as well as other compounds of the invention, which are direct inhibitors of T cell proliferation/activation, lymphokine secretion, and/or vascular permeability can be directly used to suppress the immune response. These compounds are useful to reduce the degree of the immune response and to treat immune related diseases characterized by a hyperactive, superoptimal, or autoimmune response. This use of the compounds of the invention has been validated by the experiments described above in which CTLA-4 binding to receptor B7 deactivates T cells. The direct inhibitory compounds of the invention function in an analogous manner. The use of compound which suppress vascular permeability would be expected to reduce inflammation. Such uses would be beneficial in treating conditions associated with excessive inflammation.

Alternatively, compounds, *e.g*., antibodies, which bind to stimulating PRO polypeptides and block the stimulating effect of these molecules produce a net inhibitory effect and can be used to suppress the T cell mediated immune response by inhibiting T cell proliferation/activation and/or lymphokine secretion. Blocking the stimulating effect of the polypeptides suppresses the immune response of the mammal. This use has been validated in experiments using an anti-IL2 antibody. In these experiments, the antibody binds to IL2 and blocks binding of IL2 to its receptor thereby achieving a T cell inhibitory effect.

### H. Animal Models

The results of the cell based in vitro assays can be further verified using *in vivo* animal models and assays for T-cell function. A variety of well known animal models can be used to further understand the role of the genes identified herein in the development and pathogenesis of immune related disease, and to test the efficacy of candidate therapeutic agents, including antibodies, and other antagonists of the native polypeptides, including small molecule antagonists. The *in vivo* nature of such models makes them predictive of responses in human patients. Animal models of immune related diseases include both non-recombinant and recombinant (transgenic) animals. Non-recombinant animal models include, for example, rodent, *e.g.,* murine models. Such models can be generated by introducing cells into syngeneic mice using standard techniques, *e.g*., subcutaneous injection, tail vein injection, spleen implantation, intraperitoneal implantation, implantation under the renal capsule, *etc.*

Graft-versus-host disease occurs when immunocompetent cells are transplanted into immunosuppressed or tolerant patients. The donor cells recognize and respond to host antigens. The response can vary from life threatening severe inflammation to mild cases of diarrhea and weight loss. Graft-versus-host disease models provide a means of assessing T cell reactivity against MHC antigens and minor transplant antigens. A suitable procedure is described in detail in Current Protocols in Immunology, above, unit 4.3.

An animal model for skin allograft rejection is a means of testing the ability of T cells to mediate *in vivo* tissue destruction and a measure of their role in transplant rejection. The most common and accepted models use murine tail-skin grafts. Repeated experiments have shown that skin allograft rejection is mediated by T cells, helper T cells and killer-effector T cells, and not antibodies. Auchincloss, H. Jr. and Sachs, D. H., Fundamental Immunology, 2nd ed., W. E. Paul ed., Raven Press, NY, 1989, 889-992. A suitable procedure is described in detail in *Current Protocols in Immunology,* above, unit 4.4. Other transplant rejection models which can be used to test the compounds of the invention are the allogeneic heart transplant models described by Tanabe, M. et al, Transplantation (1994) 58:23 and Tinubu, S. A. et al, J. Immunol. (1994) 4330-4338.

Animal models for delayed type hypersensitivity provides an assay of cell mediated immune function as well. Delayed type hypersensitivity reactions are a T cell mediated *in vivo* immune response characterized by inflammation which does not reach a peak until after a period of time has elapsed after challenge with an antigen. These reactions also occur in tissue specific autoimmune diseases such as multiple sclerosis (MS) and experimental autoimmune encephalomyelitis (EAE, a model for MS). A suitable procedure is described in detail in *Current Protocols in Immunology,* above, unit 4.5.

EAE is a T cell mediated autoimmune disease characterized by T cell and mononuclear cell inflammation and subsequent demyelination of axons in the central nervous system. EAE is generally considered to be a relevant animal model for MS in humans. Bolton, C., Multiple Sclerosis (1995) 1:143. Both acute and relapsing-remitting models have been developed. The compounds of the invention can be tested for T cell stimulatory or inhibitory activity against immune mediated demyelinating disease using the protocol described in *Current Protocols in Immunology,* above, units 15.1 and 15.2. See also the models for myelin disease in which oligodendrocytes or Schwann cells are grafted into the central nervous system as described in Duncan, I. D. et al, Molec. Med. Today (1997) 554-561.

Contact hypersensitivity is a simple delayed type hypersensitivity *in vivo* assay of cell mediated immune function. In this procedure, cutaneous exposure to exogenous haptens which gives rise to a delayed type hypersensitivity reaction which is measured and quantitated. Contact sensitivity involves an initial sensitizing phase followed by an elicitation phase. The elicitation phase occurs when the T lymphocytes encounter an antigen to which they have had previous contact. Swelling and inflammation occur, making this an excellent model of human allergic contact dermatitis. A suitable procedure is described in detail in Current Protocols in Immunology, Eds. J. E. Cologan, A. M. Kruisbeek, D. H. Margulies, E. M. Shevach and W. Strober, John Wiley & Sons, Inc., 1994, unit 4.2. See also Grabbe, S. and Schwarz, T, Immun. Today 19 (1): 37-44 (1998).

An animal model for arthritis is collagen-induced arthritis. This model shares clinical, histological and immunological characteristics of human autoimmune rheumatoid arthritis and is an acceptable model for human autoimmune arthritis. Mouse and rat models are characterized by synovitis, erosion of cartilage and subchondral bone. The compounds of the invention can be tested for activity against autoimmune arthritis using the protocols described in *Current Protocols in Immunology,* above, units 15.5. See also the model using a monoclonal antibody to CD18 and VLA-4 integrins described in Issekutz, A.C. et al., Immunology (1996) 88:569.

A model of asthma has been described in which antigen-induced airway hyper-reactivity, pulmonary eosinophilia and inflammation are induced by sensitizing an animal with ovalbumin and then challenging the animal with the same protein delivered by aerosol. Several animal models (guinea pig, rat, non-human primate) show symptoms similar to atopic asthma in humans upon challenge with aerosol antigens. Murine models have many of the features of human asthma. Suitable procedures to test the compounds of the invention for activity and effectiveness in the treatment of asthma are described by Wolyniec, W. W. et al, Am. J. Respir. Cell Mol. Biol. (1998) 18:777 and the references cited therein.

Additionally, the compounds of the invention can be tested on animal models for psoriasis like diseases. Evidence suggests a T cell pathogenesis for psoriasis. The compounds of the invention can be tested in the scid/scid mouse model described by Schon, M. P. et al, Nat. Med. (1997) 3:183, in which the mice demonstrate histopathologic skin lesions resembling psoriasis. Another suitable model is the human skin/scid mouse chimera prepared as described by Nickoloff, B. J. et al, Am. J. Path. (1995) 146:580.

Recombinant (transgenic) animal models can be engineered by introducing the coding portion of the genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, *e.g*., baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (*e.g*., Van der Putten et al., Proc. Natl. Acad. Sci. USA 82, 6148-615 [1985]); gene targeting in embryonic stem cells (Thompson et al., Cell 56, 313-321 [1989]); electroporation of embryos (Lo, Mol. Cel. Biol. 3, 1803-1814 [1983]); sperm-mediated gene transfer (Lavitrano et al., Cell 57, 717-73 [1989]). For review, see, for example, U.S. Patent No. 4,736,866.

For the purpose of the present invention, transgenic animals include those that carry the transgene only in part of their cells ("mosaic animals"). The transgene can be integrated either as a single transgene, or in concatamers, *e.g*., head-to-head or head-to-tail tandems. Selective introduction of a transgene into a particular cell type is also possible by following, for example, the technique of Lasko et al., Proc. Natl. Acad. Sci. USA 89, 6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry.

The animals may be further examined for signs of immune disease pathology, for example by histological examination to determine infiltration of immune cells into specific tissues. Blocking experiments can also be performed in which the transgenic animals are treated with the compounds of the invention to determine the extent of the T cell proliferation stimulation or inhibition of the compounds. In these experiments, blocking antibodies which bind to the PRO polypeptide, prepared as described above, are administered to the animal and the effect on immune function is determined.

Alternatively, "knock out" animals can be constructed which have a defective or altered gene encoding a polypeptide identified herein, as a result of homologous recombination between the endogenous gene encoding the polypeptide and altered genomic DNA encoding the same polypeptide introduced into an embryonic cell of the animal. For example, cDNA encoding a particular polypeptide can be used to clone genomic DNA encoding that polypeptide in accordance with established techniques. A portion of the genomic DNA encoding a particular polypeptide can be deleted or replaced with another gene, such as a gene encoding a selectable marker which can be used to monitor integration. Typically, several kilobases of unaltered flanking DNA (both at the 5' and 3' ends) are included in the vector [see *e.g.,* Thomas and Capecchi, Cell, 51:503 (1987) for a description of homologous recombination vectors]. The vector is introduced into an embryonic stem cell line (*e.g.,* by electroporation) and cells in which the introduced DNA has homologously recombined with the endogenous DNA are selected [see *e.g.,* Li et al., Cell, 69:915 (1992)]. The selected cells are then injected into a blastocyst of an animal (*e.g.,* a mouse or rat) to form aggregation chimeras [see *e.g.,* Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, for their ability to defend against certain pathological conditions and for their development of pathological conditions due to absence of the polypeptide.

### I. ImmunoAdjuvant Therapy

In one embodiment, the immunostimulating compounds of the invention can be used in immunoadjuvant therapy for the treatment of tumors (cancer). It is now well established that T cells recognize human tumor specific antigens. One group of tumor antigens, encoded by the MAGE, BAGE and GAGE families of genes, are silent in all adult normal tissues , but are expressed in significant amounts in tumors, such as melanomas, lung tumors, head and neck tumors, and bladder carcinomas DeSmet et al., (1996) Proc. Natl. Acad. Sci. USA, 93:7149. It has been shown that costimulation of T cells induces tumor regression and an antitumor response both *in vitro* and *in vivo.* Melero, I. et al., Nature Medicine (1997) 3:682; Kwon, E. D. et al., Proc. Natl. Acad. Sci. USA (1997) 94: 8099; Lynch, D. H. et al, Nature Medicine (1997) 3:625; Finn, O. J. and Lotze, M. T., J. Immunol. (1998) 21:114. The stimulatory compounds of the invention can be administered as adjuvants, alone or together with a growth regulating agent, cytotoxic agent or chemotherapeutic agent, to stimulate T cell proliferation/activation and an antitumor response to tumor antigens. The growth regulating, cytotoxic, or chemotherapeutic agent may be administered in conventional amounts using known administration regimes. Immunostimulating activity by the compounds of the invention allows reduced amounts of the growth regulating, cytotoxic, or chemotherapeutic agents thereby potentially lowering the toxicity to the patient.

### J. Screening Assays for Drug Candidates

Screening assays for drug candidates are designed to identify compounds that bind to or complex with the polypeptides encoded by the genes identified herein or a biologically active fragment thereof, or otherwise interfere with the interaction of the encoded polypeptides with other cellular proteins. Such screening assays will include assays amenable to high-throughput screening of chemical libraries, making them particularly suitable for identifying small molecule drug candidates. Small molecules contemplated include synthetic organic or inorganic compounds, including peptides, preferably soluble peptides, (poly)peptide-immunoglobulin fusions, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. The assays can be performed in a variety of formats, including protein-protein binding assays, biochemical screening assays, immunoassays and cell based assays, which are well characterized in the art. All assays are common in that they call for contacting the drug candidate with a polypeptide encoded by a nucleic acid identified herein under conditions and for a time sufficient to allow these two components to interact.

In binding assays, the interaction is binding and the complex formed can be isolated or detected in the reaction mixture. In a particular embodiment, the polypeptide encoded by the gene identified herein or the drug candidate is immobilized on a solid phase, *e.g*., on a microtiter plate, by covalent or non-covalent attachments. Non-covalent attachment generally is accomplished by coating the solid surface with a solution of the polypeptide and drying. Alternatively, an immobilized antibody, *e.g*., a monoclonal antibody, specific for the polypeptide to be immobilized can be used to anchor it to a solid surface. The assay is performed by adding the non-immobilized component, which may be labeled by a detectable label, to the immobilized component, *e.g*., the coated surface containing the anchored component. When the reaction is complete, the non-reacted components are removed, *e.g.,* by washing, and complexes anchored on the solid surface are detected. When the originally non-immobilized component carries a detectable label, the detection of label immobilized on the surface indicates that complexing occurred. Where the originally non-immobilized component does not carry a label, complexing can be detected, for example, by using a labelled antibody specifically binding the immobilized complex.

If the candidate compound interacts with but does not bind to a particular protein encoded by a gene identified herein, its interaction with that protein can be assayed by methods well known for detecting protein-protein interactions. Such assays include traditional approaches, such as, cross-linking, co-immunoprecipitation, and co-purification through gradients or chromatographic columns. In addition, protein-protein interactions can be monitored by using a yeast-based genetic system described by Fields and co-workers [Fields and Song, Nature (London) 340, 245-246 (1989); Chien et al., Proc. Natl. Acad. Sci. USA 88, 9578-9582 (1991)] as disclosed by Chevray and Nathans, Proc. Natl. Acad. Sci. USA 89, 5789-5793 (1991). Many transcriptional activators, such as yeast GAL4, consist of two physically discrete modular domains, one acting as the DNA-binding domain, while the other one functioning as the transcription activation domain. The yeast expression system described in the foregoing publications (generally referred to as the "two-hybrid system") takes advantage of this property, and employs two hybrid proteins, one in which the target protein is fused to the DNA-binding domain of GAL4, and another, in which candidate activating proteins are fused to the activation domain. The expression of a GAL1-*lac*Z reporter gene under control of a GAL4-activated promoter depends on reconstitution of GAL4 activity via protein-protein interaction. Colonies containing interacting polypeptides are detected with a chromogenic substrate for β-galactosidase. A complete kit (MATCHMAKER^{™}) for identifying protein-protein interactions between two specific proteins using the two-hybrid technique is commercially available from Clontech. This system can also be extended to map protein domains involved in specific protein interactions as well as to pinpoint amino acid residues that are crucial for these interactions.

In order to find compounds that interfere with the interaction of a gene identified herein and other intra- or extracellular components can be tested, a reaction mixture is usually prepared containing the product of the gene and the intra- or extracellular component under conditions and for a time allowing for the interaction and binding of the two products. To test the ability of a test compound to inhibit binding, the reaction is run in the absence and in the presence of the test compound. In addition, a placebo may be added to a third reaction mixture, to serve as positive control. The binding (complex formation) between the test compound and the intra- or extracellular component present in the mixture is monitored as described above. The formation of a complex in the control reaction(s) but not in the reaction mixture containing the test compound indicates that the test compound interferes with the interaction of the test compound and its reaction partner.

### K. Compositions and Methods for the Treatment of Immune Related Diseases

The compositions useful in the treatment of immune related diseases include, without limitation, proteins, antibodies, small organic molecules, peptides, phosphopeptides, antisense and ribozyme molecules, triple helix molecules, *etc.* that inhibit or stimulate immune function, for example, T cell proliferation/activation, lymphokine release, or immune cell infiltration.

For example, antisense RNA and RNA molecules act to directly block the translation of mRNA by hybridizing to targeted mRNA and preventing protein translation. When antisense DNA is used, oligodeoxyribonucleotides derived from the translation initiation site, *e.g*., between about -10 and +10 positions of the target gene nucleotide sequence, are preferred.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details see, *e.g.,* Rossi, Current Biology 4, 469-471 (1994), and PCT publication No. WO 97/33551 (published September 18, 1997).

Nucleic acid molecules in triple helix formation used to inhibit transcription should be single-stranded and composed of deoxynucleotides. The base composition of these oligonucleotides is designed such that it promotes triple helix formation via Hoogsteen base pairing rules, which generally require sizeable stretches of purines or pyrimidines on one strand of a duplex. For further details see, e.g., PCT publication No. WO 97/33551, *supra.*

These molecules can be identified by any or any combination of the screening assays discussed above and/or by any other screening techniques well known for those skilled in the art.

### L. Anti-PRO Antibodies

The present invention further provides anti-PRO antibodies. Exemplary antibodies include polyclonal, monoclonal, humanized, bispecific, and heteroconjugate antibodies.

### I. Polyclonal Antibodies

The anti-PRO antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the PRO polypeptide or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

### 2. Monoclonal Antibodies

The anti-PRO antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the PRO polypeptide or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell [Goding, Monoclonal Antibodies: Principles and Practice, Academic Press, (1986) pp. 59-103]. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies [Kozbor, J. Immunol., 133:3001 (1984); Brodeur et al., Monoclonal Antibody Production Techniques and Applications, Marcel Dekker, Inc., New York, (1987) pp. 51-63].

The culture medium in which the hybridoma cells are cultured can then be assayed for the presence of monoclonal antibodies directed against PRO. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. The DNA also may be modified, for example, by substituting the coding sequence for human heavy and light chain constant domains in place of the homologous murine sequences [U.S. Patent No. 4,816,567; Morrison et al., supra] or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

### 3. Human and Humanized Antibodies

The anti-PRO antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the method of Winter and co-workers [Jones et al., Nature, 321:522-525 (1986); Riechmann et al., Nature, 332:323-327 (1988); Verhoeyen et al., Science, 239:1534-1536 (1988)], by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technoloey 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995).

The antibodies may also be affinity matured using known selection and/or mutagenesis methods as described above. Preferred affinity matured antibodies have an affinity which is five times, more preferably 10 times, even more preferably 20 or 30 times greater than the starting antibody (generally murine, humanized or human) from which the matured antibody is prepared.

### 4. Bispecific Antibodies

Bispecific antibodies are monoclonal, preferably human or humanized, antibodies that have binding specificities for at least two different antigens. In the present case, one of the binding specificities is for the PRO, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities [Milstein and Cuello, Nature, 305:537-539 (1983)]. Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. F(ab')₂ bispecific antibodies). Techniques for generating bispecific antibodies from antibody fragments have been described in the literature. For example, bispecific antibodies can be prepared can be prepared using chemical linkage. Brennan et al., Science 229:81 (1985) describe a procedure wherein intact antibodies are proteolytically cleaved to generate F(ab')₂ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from E. *coli* and chemically coupled to form bispecific antibodies. Shalaby et al., J. Exp. Med. 175:217-225 (1992) describe the production of a fully humanized bispecific antibody F(ab')₂ molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various technique for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain (V_{H}) connected to a light-chain variable domain (V_{L}) by a linker which is too short to allow pairing between the two domains on the same chain. Accordingly, the V_{H} and V_{L} domains of one fragment are forced to pair with the complementary V_{L} and V_{H} domains of another fragment, thereby forming two antigen-binding sites. Another strategy for making bispecific antibody fragments by the use of single-chain Fv (sFv) dimers has also been reported. See, Gruber et al., J. Immunol. 152:5368 (1994). Antibodies with more than two valencies are contemplated. For example, trispecific antibodies can be prepared. Tutt et al., J. Immunol. 147:60 (1991).

Exemplary bispecific antibodies may bind to two different epitopes on a given PRO polypeptide herein. Alternatively, an anti-PRO polypeptide arm may be combined with an arm which binds to a triggering molecule on a leukocyte such as a T-cell receptor molecule (e.g. CD2, CD3, CD28, or B7), or Fc receptors for IgG (FcyR), such as FcγRI (CD64), FcγRII (CD32) and FcγRIII (CD16) so as to focus cellular defense mechanisms to the cell expressing the particular PRO polypeptide. Bispecific antibodies may also be used to localize cytotoxic agents to cells which express a particular PRO polypeptide. These antibodies possess a PRO-binding arm and an arm which binds a cytotoxic agent or a radionuclide chelator, such as EOTUBE, DPTA, DOTA, or TETA. Another bispecific antibody of interest binds the PRO polypeptide and further binds tissue factor (TF).

### 5. Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells [U.S. Patent No. 4,676,980], and for treatment of HIV infection [WO 91/00360; WO 92/200373; EP 03089]. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

### 6. Effector Function Engineering

It may be desirable to modify the antibody of the invention with respect to effector function, so as to enhance, *e.g*., the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff et al. Cancer Research, 53: 2560-2565 (1993). Alternatively, an antibody can be engineered that has dual Fc regions and may thereby have enhanced complement lysis and ADCC capabilities. See Stevenson et al., Anti-Cancer Drug Design, 3: 219-230 (1989).

### 7. Immunoconjugates

The invention also pertains to immunoconjugates comprising an antibody conjugated to a cytotoxic agent such as a chemotherapeutic agent, toxin (*e.g.,* an enzymatically active toxin of bacterial, fungal, plant, or animal origin, or fragments thereof), or a radioactive isotope (*i.e.,* a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include ²¹²Bi ¹³¹I, ¹³¹In, ⁹⁰Y, and ¹⁸⁶Re.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-Iabeled 1-isothiocyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" *(e.g.,* avidin) that is conjugated to a cytotoxic agent (*e.g.,* a radionucleotide).

### 8. Immunoliposomes

The antibodies disclosed herein may also be formulated as immunoliposomes. Liposomes containing the antibody are prepared by methods known in the art, such as described in Epstein et al., Proc. Natl. Acad. Sci. USA, 82: 3688 (1985); Hwang et al., Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al ., J. Biol. Chem., 257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

### M. Pharmaceutical Compositions

The active PRO molecules of the invention (e.g., PRO polypeptides, anti-PRO antibodies, and/or variants of each) as well as other molecules identified by the screening assays disclosed above, can be administered for the treatment of immune related diseases, in the form of pharmaceutical compositions.

Therapeutic formulations of the active PRO molecule, preferably a polypeptide or antibody of the invention, are prepared for storage by mixing the active molecule having the desired degree of purity with optional pharmaceutically acceptable carriers, excipients or stabilizers (Remington's Pharmaceutical Sciences 16th edition, Osol, A. Ed. [1980]), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (*e.g*., Zn-protein complexes); and/or non-ionic surfactants such as TWEEN^{™}, PLURONICS^{™} or polyethylene glycol (PEG).

Compounds identified by the screening assays disclosed herein can be formulated in an analogous manner, using standard techniques well known in the art.

Lipofections or liposomes can also be used to deliver the PRO molecule into cells. Where antibody fragments are used, the smallest inhibitory fragment which specifically binds to the binding domain of the target protein is preferred. For example, based upon the variable region sequences of an antibody, peptide molecules can be designed which retain the ability to bind the target protein sequence. Such peptides can be synthesized chemically and/or produced by recombinant DNA technology (see, *e.g.,* Marasco et al., Proc. Natl. Acad. Sci. USA 90, 7889-7893 [1993]).

The formulation herein may also contain more than one active compound as necessary for the particular indication being treated, preferably those with complementary activities that do not adversely affect each other. Alternatively, or in addition, the composition may comprise a cytotoxic agent, cytokine or growth inhibitory agent. Such molecules are suitably present in combination in amounts that are effective for the purpose intended.

The active PRO molecules may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for *in vivo* administration must be sterile. This is readily accomplished by filtration through sterile filtration membranes.

Sustained-release preparations or the PRO molecules may be prepared. Suitable examples of sustained-release preparations include semipermeable matrices of solid hydrophobic polymers containing the antibody, which matrices are in the form of shaped articles, e.g., films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ-ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

### N. Methods of Treatment

It is contemplated that the polypeptides, antibodies and other active compounds of the present invention may be used to treat various immune related diseases and conditions, such as T cell mediated diseases, including those characterized by infiltration of inflammatory cells into a tissue, stimulation of T-cell proliferation, inhibition of T-cell proliferation, increased or decreased vascular permeability or the inhibition thereof.

Exemplary conditions or disorders to be treated with the polypeptides, antibodies and other compounds of the invention, include, but are not limited to systemic lupus erythematosis, rheumatoid arthritis, juvenile chronic arthritis, osteoarthritis, spondyloarthropathies, systemic sclerosis (scleroderma), idiopathic inflammatory myopathies (dermatomyositis, polymyositis), Sjögren's syndrome, systemic vasculitis, sarcoidosis, autoimmune hemolytic anemia (immune pancytopenia, paroxysmal nocturnal hemoglobinuria), autoimmune thrombocytopenia (idiopathic thrombocytopenic purpura, immune-mediated thrombocytopenia), thyroiditis (Grave's disease, Hashimoto's thyroiditis, juvenile lymphocytic thyroiditis, atrophic thyroiditis), diabetes mellitus, immune-mediated renal disease (glomerulonephritis, tubulointerstitial nephritis), demyelinating diseases of the central and peripheral nervous systems such as multiple sclerosis, idiopathic demyelinating polyneuropathy or Guillain-Barré syndrome, and chronic inflammatory demyelinating polyneuropathy, hepatobiliary diseases such as infectious hepatitis (hepatitis A, B, C, D, E and other non-hepatotropic viruses), autoimmune chronic active hepatitis, primary biliary cirrhosis, granulomatous hepatitis, and sclerosing cholangitis, inflammatory bowel disease (ulcerative colitis: Crohn's disease), gluten-sensitive enteropathy, and Whipple's disease, autoimmune or immune-mediated skin diseases including bullous skin diseases, erythema multiforme and contact dermatitis, psoriasis, allergic diseases such as asthma, allergic rhinitis, atopic dermatitis, food hypersensitivity and urticaria, immunologic diseases of the lung such as eosinophilic pneumonias, idiopathic pulmonary fibrosis and hypersensitivity pneumonitis, transplantation associated diseases including graft rejection and graft -versus-host-disease.

In systemic lupus erythematosus, the central mediator of disease is the production of auto-reactive antibodies to self proteins/tissues and the subsequent generation of immune-mediated inflammation. Antibodies either directly or indirectly mediate tissue injury. Though T lymphocytes have not been shown to be directly involved in tissue damage, T lymphocytes are required for the development of auto-reactive antibodies. The genesis of the disease is thus T lymphocyte dependent. Multiple organs and systems are affected clinically including kidney, lung, musculoskeletal system, mucocutaneous, eye, central nervous system, cardiovascular system, gastrointestinal tract, bone marrow and blood.

Rheumatoid arthritis (RA) is a chronic systemic autoimmune inflammatory disease that mainly involves the synovial membrane of multiple joints with resultant injury to the articular cartilage. The pathogenesis is T lymphocyte dependent and is associated with the production of rheumatoid factors, auto-antibodies directed against self IgG, with the resultant formation of immune complexes that attain high levels in joint fluid and blood. These complexes in the joint may induce the marked infiltrate of lymphocytes and monocytes into the synovium and subsequent marked synovial changes; the joint space/fluid if infiltrated by similar cells with the addition of numerous neutrophils. Tissues affected are primarily the joints, often in symmetrical pattern. However, extra-articular disease also occurs in two major forms. One form is the development of extra-articular lesions with ongoing progressive joint disease and typical lesions of pulmonary fibrosis, vasculitis, and cutaneous ulcers. The second form of extra-articular disease is the so called Felty's syndrome which occurs late in the RA disease course, sometimes after joint disease has become quiescent, and involves the presence of neutropenia, thrombocytopenia and splenomegaly. This can be accompanied by vasculitis in multiple organs with formations of infarcts, skin ulcers and gangrene. Patients often also develop rheumatoid nodules in the subcutis tissue overlying affected joints; the nodules late stage have necrotic centers surrounded by a mixed inflammatory cell infiltrate. Other manifestations which can occur in RA include: pericarditis, pleuritis, coronary arteritis, intestitial pneumonitis with pulmonary fibrosis, keratoconjunctivitis sicca, and rhematoid nodules.

Juvenile chronic arthritis is a chronic idiopathic inflammatory disease which begins often at less than 16 years of age. Its phenotype has some similarities to RA; some patients which are rhematoid factor positive are classified as juvenile rheumatoid arthritis. The disease is sub-classified into three major categories: pauciarticular, polyarticular, and systemic. The arthritis can be severe and is typically destructive and leads to joint ankylosis and retarded growth. Other manifestations can include chronic anterior uveitis and systemic amyloidosis.

Spondyloarthropathies are a group of disorders with some common clinical features and the common association with the expression of HLA-B27 gene product. The disorders include: ankylosing sponylitis, Reiter's syndrome (reactive arthritis), arthritis associated with inflammatory bowel disease, spondylitis associated with psoriasis, juvenile onset spondyloarthropathy and undifferentiated spondyloarthropathy. Distinguishing features include sacroileitis with or without spondylitis; inflammatory asymmetric arthritis; association with HLA-B27 (a serologically defined allele of the HLA-B locus of class I MHC); ocular inflammation, and absence of autoantibodies associated with other rheumatoid disease. The cell most implicated as key to induction of the disease is the CD8+ T lymphocyte, a cell which targets antigen presented by class I MHC molecules. CD8+ T cells may react against the class I MHC allele HLA-B27 as if it were a foreign peptide expressed by MHC class I molecules. It has been hypothesized that an epitope of HLA-B27 may mimic a bacterial or other microbial antigenic epitope and thus induce a CD8+ T cells response.

Systemic sclerosis (scleroderma) has an unknown etiology. A hallmark of the disease is induration of the skin; likely this is induced by an active inflammatory process. Scleroderma can be localized or systemic; vascular lesions are common and endothelial cell injury in the microvasculature is an early and important event in the development of systemic sclerosis; the vascular injury may be immune mediated. An immunologic basis is implied by the presence of mononuclear cell infiltrates in the cutaneous lesions and the presence of anti-nuclear antibodies in many patients. ICAM-1 is often upregulated on the cell surface of fibroblasts in skin lesions suggesting that T cell interaction with these cells may have a role in the pathogenesis of the disease. Other organs involved include: the gastrointestinal tract: smooth muscle atrophy and fibrosis resulting in abnormal peristalsis/motility; kidney: concentric subendothelial intimal proliferation affecting small arcuate and interlobular arteries with resultant reduced renal cortical blood flow, results in proteinuria, azotemia and hypertension; skeletal muscle: atrophy, interstitial fibrosis; inflammation; lung: interstitial pneumonitis and interstitial fibrosis; and heart: contraction band necrosis, scarring/fibrosis.

Idiopathic inflammatory myopathies including dermatomyositis, polymyositis and others are disorders of chronic muscle inflammation of unknown etiology resulting in muscle weakness. Muscle injury/inflammation is often symmetric and progressive. Autoantibodies are associated with most forms. These myositis-specific autoantibodies are directed against and inhibit the function of components, proteins and RNA's, involved in protein synthesis.

Sjögren's syndrome is due to immune-mediated inflammation and subsequent functional destruction of the tear glands and salivary glands. The disease can be associated with or accompanied by inflammatory connective tissue diseases. The disease is associated with autoantibody production against Ro and La antigens, both of which are small RNA-protein complexes. Lesions result in keratoconjunctivitis sicca, xerostomia, with other manifestations or associations including bilary cirrhosis, peripheral or sensory neuropathy, and palpable purpura.

Systemic vasculitis are diseases in which the primary lesion is inflammation and subsequent damage to blood vessels which results in ischemia/necrosis/degeneration to tissues supplied by the affected vessels and eventual end-organ dysfunction in some cases. Vasculitides can also occur as a secondary lesion or sequelae to other immune-inflammatory mediated diseases such as rheumatoid arthritis, systemic sclerosis, etc., particularly in diseases also associated with the formation of immune complexes. Diseases in the primary systemic vasculitis group include: systemic necrotizing vasculitis: polyarteritis nodosa, allergic angiitis and granulomatosis, polyangiitis; Wegener's granulomatosis; lymphomatoid granulomatosis; and giant cell arteritis. Miscellaneous vasculitides include: mucocutaneous lymph node syndrome (MLNS or Kawasaki's disease), isolated CNS vasculitis, Behet's disease, thromboangiitis obliterans (Buerger's disease) and cutaneous necrotizing venulitis. The pathogenic mechanism of most of the types of vasculitis listed is believed to be primarily due to the deposition of immunoglobulin complexes in the vessel wall and subsequent induction of an inflammatory response either via ADCC, complement activation, or both.

Sarcoidosis is a condition of unknown etiology which is characterized by the presence of epithelioid granulomas in nearly any tissue in the body; involvement of the lung is most common. The pathogenesis involves the persistence of activated macrophages and lymphoid cells at sites of the disease with subsequent chronic sequelae resultant from the release of locally and systemically active products released by these cell types.

Autoimmune hemolytic anemia including autoimmune hemolytic anemia, immune pancytopenia, and paroxysmal noctural hemoglobinuria is a result of production of antibodies that react with antigens expressed on the surface of red blood cells (and in some cases other blood cells including platelets as well) and is a reflection of the removal of those antibody coated cells via complement mediated lysis and/or ADCC/Fc-receptor-mediated mechanisms.

In autoimmune thrombocytopenia including thrombocytopenic purpura, and immune-mediated thrombocytopenia in other clinical settings, platelet destruction/removal occurs as a result of either antibody or complement attaching to platelets and subsequent removal by complement lysis, ADCC or FC-receptor mediated mechanisms.

Thyroiditis including Grave's disease, Hashimoto's thyroiditis, juvenile lymphocytic thyroiditis, and atrophic thyroiditis, are the result of an autoimmune response against thyroid antigens with production of antibodies that react with proteins present in and often specific for the thyroid gland. Experimental models exist including spontaneous models: rats (BUF and BB rats) and chickens (obese chicken strain); inducible models: immunization of animals with either thyroglobulin, thyroid microsomal antigen (thyroid peroxidase).

Type I diabetes mellitus or insulin-dependent diabetes is the autoimmune destruction of pancreatic islet β cells; this destruction is mediated by auto-antibodies and auto-reactive T cells. Antibodies to insulin or the insulin receptor can also produce the phenotype of insulin-non-responsiveness.

Immune mediated renal diseases, including glomerulonephritis and tubulointerstitial nephritis, are the result of antibody or T lymphocyte mediated injury to renal tissue either directly as a result of the production of autoreactive antibodies or T cells against renal antigens or indirectly as a result of the deposition of antibodies and/or immune complexes in the kidney that are reactive against other, non-renal antigens. Thus other immune-mediated diseases that result in the formation of immune-complexes can also induce immune mediated renal disease as an indirect sequelae. Both direct and indirect immune mechanisms result in inflammatory response that produces/induces lesion development in renal tissues with resultant organ function impairment and in some cases progression to renal failure. Both humoral and cellular immune mechanisms can be involved in the pathogenesis of lesions.

Demyelinating diseases of the central and peripheral nervous systems, including Multiple Sclerosis; idiopathic demyelinating polyneuropathy or Guillain-Barré syndrome; and Chronic Inflammatory Demyelinating Polyneuropathy, are believed to have an autoimmune basis and result in nerve demyelination as a result of damage caused to oligodendrocytes or to myelin directly. In MS there is evidence to suggest that disease induction and progression is dependent on T lymphocytes. Multiple Sclerosis is a demyelinating disease that is T lymphocyte-dependent and has either a relapsing-remitting course or a chronic progressive course. The etiology is unknown; however, viral infections, genetic predisposition, environment, and autoimmunity all contribute. Lesions contain infiltrates of predominantly T lymphocyte mediated, microglial cells and infiltrating macrophages; CD4+ T lymphocytes are the predominant cell type at lesions. The mechanism of oligodendrocyte cell death and subsequent demyelination is not known but is likely T lymphocyte driven.

Inflammatory and Fibrotic Lung Disease, including Eosinophilic Pneumonias; Idiopathic Pulmonary Fibrosis, and Hypersensitivity Pneumonitis may involve a disregulated immune-inflammatory response. Inhibition of that response would be of therapeutic benefit.

Autoimmune or Immune-mediated Skin Disease including Bullous Skin Diseases, Erythema Multiforme, and Contact Dermatitis are mediated by auto-antibodies, the genesis of which is T lymphocyte-dependent.

Psoriasis is a T lymphocyte-mediated inflammatory disease. Lesions contain infiltrates of T lymphocytes, macrophages and antigen processing cells, and some neutrophils.

Allergic diseases, including asthma; allergic rhinitis; atopic dermatitis; food hypersensitivity; and urticaria are T lymphocyte dependent. These diseases are predominantly mediated by T lymphocyte induced inflammation, IgE mediated-inflammation or a combination of both.

Transplantation associated diseases, including Graft rejection and Graft-Versus-Host-Disease (GVHD) are T lymphocyte-dependent; inhibition of T lymphocyte function is ameliorative.

Other diseases in which intervention of the immune and/or inflammatory response have benefit are infectious disease including but not limited to viral infection (including but not limited to AIDS, hepatitis A, B, C, D, E and herpes) bacterial infection, fungal infections, and protozoal and parasitic infections (molecules (or derivatives/agonists) which stimulate the MLR can be utilized therapeutically to enhance the immune response to infectious agents), diseases of immunodeficiency (molecules/derivatives/agonists) which stimulate the MLR can be utilized therapeutically to enhance the immune response for conditions of inherited, acquired, infectious induced (as in HIV infection), or iatrogenic (*i.e*., as from chemotherapy) immunodeficiency, and neoplasia.

It has been demonstrated that some human cancer patients develop an antibody and/or T lymphocyte response to antigens on neoplastic cells. It has also been shown in animal models of neoplasia that enhancement of the immune response can result in rejection or regression of that particular neoplasm. Molecules that enhance the T lymphocyte response in the MLR have utility *in vivo* in enhancing the immune response against neoplasia. Molecules which enhance the T lymphocyte proliferative response in the MLR (or small molecule agonists or antibodies that affected the same receptor in an agonistic fashion) can be used therapeutically to treat cancer. Molecules that inhibit the lymphocyte response in the MLR also function *in vivo* during neoplasia to suppress the immune response to a neoplasm; such molecules can either be expressed by the neoplastic cells themselves or their expression can be induced by the neoplasm in other cells. Antagonism of such inhibitory molecules (either with antibody, small molecule antagonists or other means) enhances immune-mediated tumor rejection.

Additionally, inhibition of molecules with proinflammatory properties may have therapeutic benefit in reperfusion injury; stroke; myocardial infarction; atherosclerosis; acute lung injury; hemorrhagic shock; burn; sepsis/septic shock; acute tubular necrosis; endometriosis; degenerative joint disease and pancreatis.

The compounds of the present invention, *e.g*., polypeptides or antibodies, are administered to a mammal, preferably a human, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation (intranasal, intrapulmonary) routes. Intravenous or inhaled administration of polypeptides and antibodies is preferred.

In immunoadjuvant therapy, other therapeutic regimens, such administration of an anti-cancer agent, may be combined with the administration of the proteins, antibodies or compounds of the instant invention. For example, the patient to be treated with a the immunoadjuvant of the invention may also receive an anti-cancer agent (chemotherapeutic agent) or radiation therapy. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the immunoadjuvant or may be given simultaneously therewith. Additionally, an anti-estrogen compound such as tamoxifen or an anti-progesterone such as onapristone (see, EP 616812) may be given in dosages known for such molecules.

It may be desirable to also administer antibodies against other immune disease associated or tumor associated antigens, such as antibodies which bind to CD20, CD11a, CD18, ErbB2, EGFR, ErbB3, ErbB4, or vascular endothelial factor (VEGF). Alternatively, or in addition, two or more antibodies binding the same or two or more different antigens disclosed herein may be coadministered to the patient. Sometimes, it may be beneficial to also administer one or more cytokines to the patient. In one embodiment, the PRO polypeptides are coadministered with a growth inhibitory agent. For example, the growth inhibitory agent may be administered first, followed by a PRO polypeptide. However, simultaneous administration or administration first is also contemplated. Suitable dosages for the growth inhibitory agent are those presently used and may be lowered due to the combined action (synergy) of the growth inhibitory agent and the PRO polypeptide.

For the treatment or reduction in the severity of immune related disease, the appropriate dosage of an a compound of the invention will depend on the type of disease to be treated, as defined above, the severity and course of the disease, whether the agent is administered for preventive or therapeutic purposes, previous therapy, the patient's clinical history and response to the compound, and the discretion of the attending physician. The compound is suitably administered to the patient at one time or over a series of treatments.

For example, depending on the type and severity of the disease, about 1 µg/kg to 15 mg/kg (*e.g*., 0.1-20 mg/kg) of polypeptide or antibody is an initial candidate dosage for administration to the patient, whether, for example, by one or more separate administrations, or by continuous infusion. A typical daily dosage might range from about I µg/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

### O. Articles of Manufacture

In another embodiment of the invention, an article of manufacture containing materials (*e.g*., comprising a PRO molecule) useful for the diagnosis or treatment of the disorders described above is provided. The article of manufacture comprises a container and an instruction. Suitable containers include, for example, bottles, vials, syringes, and test tubes. The containers may be formed from a variety of materials such as glass or plastic. The container holds a composition which is effective for diagnosing or treating the condition and may have a sterile access port (for example the container may be an intravenous solution bag or a vial having a stopper pierceable by a hypodermic injection needle). The active agent in the composition is usually a polypeptide or an antibody of the invention. An instruction or label on, or associated with, the container indicates that the composition is used for diagnosing or treating the condition of choice. The article of manufacture may further comprise a second container comprising a pharmaceutically-acceptable buffer, such as phosphate-buffered saline, Ringer's solution and dextrose solution. It may further include other materials desirable from a commercial and user standpoint, including other buffers, diluents, filters, needles, syringes, and package inserts with instructions for use.

### P. Diagnosis and Prognosis of Immune Related Disease

Cell surface proteins, such as proteins which are overexpressed in certain immune related diseases, are excellent targets for drug candidates or disease treatment. The same proteins along with secreted proteins encoded by the genes amplified in immune related disease states find additional use in the diagnosis and prognosis of these diseases. For example, antibodies directed against the protein products of genes amplified in multiple sclerosis, rheumatoid arthritis, or another immune related disease, can be used as diagnostics or prognostics.

For example, antibodies, including antibody fragments, can be used to qualitatively or quantitatively detect the expression of proteins encoded by amplified or overexpressed genes ("marker gene products"). The antibody preferably is equipped with a detectable, e.g., fluorescent label, and binding can be monitored by light microscopy, flow cytometry, fluorimetry, or other techniques known in the art. These techniques are particularly suitable, if the overexpressed gene encodes a cell surface protein Such binding assays are performed essentially as described above.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for *in situ* detection.

The following examples are offered for illustrative purposes only, and are not intended to limit the scope of the present invention in any way.

All patent and literature references cited in the present specification are hereby incorporated by reference in their entirety.

### EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA.

### EXAMPLE 1: Microarray analysis of stimulated T-cells

Nucleic acid microarrays, often containing thousands of gene sequences, are useful for identifying differentially expressed genes in diseased tissues as compared to their normal counterparts. Using nucleic acid microarrays, test and control mRNA samples from test and control tissue samples are reverse transcribed and labeled to generate cDNA probes. The cDNA probes are then hybridized to an array of nucleic acids immobilized on a solid support. The array is configured such that the sequence and position of each member of the array is known. For example, a selection of genes known to be expressed in certain disease states may be arrayed on a solid support. Hybridization of a labeled probe with a particular array member indicates that the sample from which the probe was derived expresses that gene. If the hybridization signal of a probe from a test (for example, activated CD4+ T cells) sample is greater than hybridization signal of a probe from a control (for example, non-stimulated CD4 + T cells) sample, the gene or genes overexpressed in the test tissue are identified. The implication of this result is that an overexpressed protein in a test tissue is useful not only as a diagnostic marker for the presence of a disease condition, but also as a therapeutic target for treatment of a disease condition.

The methodology of hybridization of nucleic acids and microarray technology is well known in the art. In one example, the specific preparation of nucleic acids for hybridization and probes, slides, and hybridization conditions are all detailed in PCT Patent Application Serial No. PCT/US01/10482, filed on March 30, 2001 and which is herein incorporated by reference.

When CD4+ T cells mature from thymus and enter into the peripheral lymph system, they usually maintain their naive phenotype before encountering antigens specific for their T cell receptor [Sprent et al., Annu Rev lmmunol. (2002); 20:551-79]. The binding to specific antigens presented by APC, causes T cell activation. Depending on the environment and cytokine stimulation, CD4+ T cells differentiate into a Th1 or Th2 phenotype and become effector or memory cells [Sprent et al., Annu Rev Immunol. (2002); 20:551-79 and Murphy et al., Nat Rev Immunol. (2002) Dec;2(12):933-44]. This process is known as primary activation. Having undergone primary activation, CD4+ T cells become effector or memory cells, they maintain their phenotype as Th1 or Th2. Once these cells encounter antigen again, they undergo secondary activation, but this time the response to antigen will be quicker than the primary activation and results in the production of effector cytokines as determined by the primary activation [Sprent et al., Annu Rev Immunol, (2002); 20:551-79 and Murphy et al., Annu Rev Immunol. 2000;18:451-94].

Studies have found during the primary and secondary activation of CD4 + T cells the expression of certain genes is variable [Rogge et al., Nature Genetics. 25, 96 - 101 (2000) and Ouyang et al., Proc Natl Acad Sci U S A. (1999) Mar 30;96(7):3888-93]. The present study represents a model to identify differentially expressed genes during the primary and secondary activation response *in vitro.*

For primary activation conditions, naïve T cells were activated by anti-CD3, anti-CD28 and specific cytokines (experimental conditions are described below). This primary activation was termed condition (a). RNA isolated from cells in this condition can provide information about what genes are differentially regulated during the primary activation, and what cytokines affect gene expression during Th1 and Th2 development. After primary activation, the CD4+ T cells were maintained in culture for a week. However, as the previous activation and cytokine treatment has been imprinted into these cells and they have become either effector or memory cells. During this period, because there are no APCs or antigens, the CD4+ T cells enter a resting stage. This resting stage, termed condition (b) (with experimental conditions described below), provides information about the differences between naive vs. memory cells, and resting memory Th1 vs. resting memory Th2 cells. The resting memory Th1 and Th2 cells then undergo secondary activation under condition (c) and condition (d), with both conditions being described below. These conditions provide information about the differences between activated naive and activated memory T cells, and the differences between activated memory Th 1 vs. activated memory Th2 cells. This study demonstrates differential gene expression during different stages of CD4 T cell activation and differentiation. As we know, many autoimmune diseases are caused by memory Th1 and Th2 cells. The data now provide us opportunity to find markers to identify these cells and specifically target these cells as a new therapeutic approach.

In this experiment, CD4+ T cells were purified from a single donor using the RossetteSep^{™} protocol (Stem Cell Technologies, Vancouver BC) which contains anti-CD8, anti-CD16, anti-CD19, anti-CD36 and anti-CD56 antibodies used to produce a population of isolated CD4 + T cells with the modification to the protocol of using 1.3 ml reagent/25ml blood. The isolated CD4+ T cells were washed by PBS (0.5% BSA) twice and counted. Naïve CD4+ T cells were further isolated by Miltenyi CD45RO beads (Miltenyi Biotec) through the autoMACS^{™} depletion program and the purity of the cells was determined by FACS analysis. Experiments proceeded only with >90% cell pure CD4+ T cells. At this point RNA was extracted from 50 x 10^6 CD4+ T cells for use as a baseline control. The remainder of the cells were stimulated by plate bound anti-CD3 and anti-CD28 at 20 x 10^6 cells / 6 ml T cell media / well of a 6 well plate.

On Day 1, to induce Th 1 differentiation, IL-12 (1 ng/ml) and anti-IL-4 (1 µ/ml)were added. For Th2 differentiation, IL-4 (5 ng/ml), anti-IL-12 (0.5 µg/ml), and anti-IFN-g were added. For Th0 cells, anti-IL-12 (0.5 µg/ml), anti-IL-4 (1 µg/ml) and anti -IFN-gamma (0.1 µg/ml) were added. All reagents were from R&D Systems (R & D Systems Inc. Minneapolis, MN).

On Day 2, cells from one well per condition were harvested for RNA purification to obtain a 48hr time point (condition (a)). On Day 3, the cells were expanded 4 fold by removing the media used for differentiation, and adding fresh media plus IL-2 and cultured for 4 days. On Day 7, the cells were washed and counted, and the cytokine profiles were examined by intracellular cytokine staining and ELISA to determine if differentiation was complete. Half of the cells were harvested and RNA purified to determine the expression of genes in the resting state (condition (b)). IL-4 and IFN-gamma producing cells were enriched for by using the Miltenyi^{™} cytokine assay kit. The isolated IL-4 or IFN-gamma producing cells were expanded for two more weeks by using similar conditions as above.

On Day 21, cells were harvested and subject to intracellular cytokine staining and ELISA for cytokine production analysis. The remainder of the cells were re-stimulated by anti-CD3 and anti-CD28 (secondary activation). Cells were harvested at 12 hr (condition (c)) and 48 hr (condition (d)) for RNA purification. From the different conditions, RNA was extracted and analysis run on Affimax (Affymetrix Inc. Santa Clara, CA) microarray chips. Non-stimulated cells harvested immediately after purification, were subjected to the same analysis. Genes were compared whose expression was upregulated or downregulated at the different activated conditions vs. resting cells.

Below are the results of these experiments, demonstrating that various PRO polypeptides of the present invention are significantly upregulated or downregulated in isolated stimulated CD4+ T helper cells as compared to unstimulated CD4+ T helper cells or isolated resting CD4+ T helper cells. As Th1 and Th2 cells play a role in normal immune defense during infection, and play a role in immune disorders, this data demonstrate that the PRO polypeptides of the present invention are useful not only as diagnostic markers for the presence of one or more immune disorders, but also serve as therapeutic targets for the treatment of those immune disorders.

SEQ ID NOs 1-6464 show nucleic acids and their encoded proteins show differential expression at (condition (c)) or (condition (d)) vs. unstimulated cells as a normal control, cells that have undergone primary activation, or primary activated cells that had been in resting for 7 days. SEQ ID NO:2955, SEQ ID NO:2855, SEQ ID NO:3487, SEQ ID NO:3088, SEQ ID NO:1319, SEQ ID NO:1629, SEQ ID NO:1733, SEQ ID NO:1561, and SEQ ID NO:1699 are highly overexpressed at (condtion (c)) or (condition (d)) vs. unstimulated cells as a normal control , cells that have undergone primary activation, or primary activated cells that had been in resting for 7 days.

### EXAMPLE 2: Use of PRO as a hybridization probe

The following method describes use of a nucleotide sequence encoding PRO as a hybridization probe.

DNA comprising the coding sequence of full-length or mature PRO as disclosed herein is employed as a probe to screen for homologous DNAs (such as those encoding naturally-occurring variants of PRO) in human tissue cDNA libraries or human tissue genomic libraries.

Hybridization and washing of filters containing either library DNAs is performed under the following high stringency conditions. Hybridization of radiolabeled PRO-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1 x SSC and 0.1% SDS at 42°C.

DNAs having a desired sequence identity with the DNA encoding full-length native sequence PRO can then be identified using standard techniques known in the art.

### EXAMPLE 3: Expression of PRO in E. coli

This example illustrates preparation of an unglycosylated form of PRO by recombinant expression in *E. coli.*

The DNA sequence encoding PRO is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli*; see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO coding region, lambda transcriptional terminator, and an argU gene.

The ligation mixture is then used to transform a selected *E. coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO protein can then be purified using a metal chelating column under conditions that allow tight binding of the protein.

PRO may be expressed in *E. coli* in a poly-His tagged form, using the following procedure. The DNA encoding PRO is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an E. *coli* host based on strain 52 (W3110 fuhA(tonA) Ion galE rpoHts(htpRts) clpP(lacIq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g (NH₄)₂SO₄, 0.71 g sodium citrate•2H2O, 1.07 g KCl, 5.36 g Difco yeast extract, 5.36 g Sheffield hycase SF in 500 mL water, as well as 110 mM MPOS, pH 7.3, 0.55% (w/v) glucose and 7 mM MgSO₄) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1M and 0.02 M, respectively, and the solution is stirred overnight at 4°C. This step results in a denatured protein with all cysteine residues blocked by sulfitolization. The solution is centrifuged at 40,000 rpm in a Beckman Ultracentifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R1/H reversed phase column using a mobile buffer of 0.1% TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

Many of the PRO polypeptides disclosed herein were successfully expressed as described above.

### EXAMPLE 4: Expression of PRO in mammalian cells

This example illustrates preparation of a potentially glycosylated form of PRO by recombinant expression in mammalian cells.

The vector, pRK5 (see EP 307,247, published March 15, 1989), is employed as the expression vector. Optionally, the PRO DNA is ligated into pRK5 with selected restriction enzymes to allow insertion of the PRO DNA using ligation methods such as described in Sambrook et al., supra. The resulting vector is called pRK5-PRO.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 µg pRK5-PRO DNA is mixed with about 1 µg DNA encoding the VA RNA gene [Thimmappaya et al., Cell, 31:543 (1982)] and dissolved in 500 µl of 1 mM Tris-HCl, 0.1 mM EDTA, 0.227 M CaCl₂. To this mixture is added, dropwise, 500 µl of 50 mM HEPES (pH 7.35), 280 mM NaCl, 1.5 mM NaPO₄, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 µCi/ml ³⁵S-cysteine and 200 µCi/ml ³⁵S-methionine. After a 12 hour incubation, the conditioned medium is collected, concentrated on a spin filter, and loaded onto a 15% SDS gel. The processed gel may be dried and exposed to film for a selected period of time to reveal the presence of PRO polypeptide. The cultures containing transfected cells may undergo further incubation (in serum free medium) and the medium is tested in selected bioassays.

In an alternative technique, PRO may be introduced into 293 cells transiently using the dextran sulfate method described by Somparyrac et al., Proc. Natl. Acad. Sci., 12:7575 (1981). 293 cells are grown to maximal density in a spinner flask and 700 µg pRK5-PRO DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 µg/ml bovine insulin and 0.1 µg/ml bovine transferrin. After about four days, the conditioned media is centrifuged and filtered to remove cells and debris. The sample containing expressed PRO can then be concentrated and purified by any selected method, such as dialysis and/or column chromatography.

In another embodiment, PRO can be expressed in CHO cells. The pRK5-PRO can be transfected into CHO cells using known reagents such as CaPO₄ or DEAE-dextran. As described above, the cell cultures can be incubated, and the medium replaced with culture medium (alone) or medium containing a radiolabel such as ³⁵S-methionine. After determining the presence of PRO polypeptide, the culture medium may be replaced with serum free medium. Preferably, the cultures are incubated for about 6 days, and then the conditioned medium is harvested. The medium containing the expressed PRO can then be concentrated and purified by any selected method.

Epitope-tagged PRO may also be expressed in host CHO cells. The PRO may be subcloned out of the pRK5 vector. The subclone insert can undergo PCR to fuse in frame with a selected epitope tag such as a poly-his tag into a Baculovirus expression vector. The poly-his tagged PRO insert can then be subcloned into a SV40 promoter/enhancer containing vector containing a selection marker such as DHFR for selection of stable clones. Finally, the CHO cells can be transfected (as described above) with the SV40 promoter/enhancer containing vector. Labeling may be performed, as described above, to verify expression. The culture medium containing the expressed poly-His tagged PRO can then be concentrated and purified by any selected method, such as by Ni²⁺-chelate affinity chromatography.

PRO may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgG1 constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect^{®} (Quiagen), Dosper^{®} or Fugene^{®} (Boehringer Mannheim). The cells are grown as described in Lucas et al., supra. Approximately 3 x 10⁻⁷ cells are frozen in an ampule for further growth and production as described below.

The ampules containing the plasmid DNA are thawed by placement into water bath and mixed by vortexing. The contents are pipetted into a centrifuge tube containing 10 mL of media and centrifuged at 1000 rpm for 5 minutes. The supernatant is aspirated and the cells are resuspended in 10 mL of selective media (0.2 µm filtered PS20 with 5% 0.2 µm diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x 10⁵ cells/mL. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x 10⁶ cells/mL. On day 0, pH is determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Corning 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 µm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 µl of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

Many of the PRO polypeptides disclosed herein were successfully expressed as described above.

### EXAMPLE 5: Expression of PRO in Yeast

The following method describes recombinant expression of PRO in yeast.

First, yeast expression vectors are constructed for intracellular production or secretion of PRO from the ADH2/GAPDH promoter. DNA encoding PRO and the promoter is inserted into suitable restriction enzyme sites in the selected plasmid to direct intracellular expression of PRO. For secretion, DNA encoding PRO can be cloned into the selected plasmid, together with DNA encoding the ADH2/GAPDH promoter, a native PRO signal peptide or other mammalian signal peptide, or, for example, a yeast alpha-factor or invertase secretory signal/leader sequence, and linker sequences (if needed) for expression of PRO.

Yeast cells, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO can subsequently be isolated and purified by removing the yeast cells from the fermentation medium by centrifugation and then concentrating the medium using selected cartridge filters. The concentrate containing PRO may further be purified using selected column chromatography resins.

Many of the PRO polypeptides disclosed herein were successfully expressed as described above.

### EXAMPLE 6: Expression of PRO in Baculovirus-Infected Insect Cells

The following method describes recombinant expression of PRO in Baculovirus-infected insect cells.

The sequence coding for PRO is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO or the desired portion of the coding sequence of PRO such as the sequence encoding the extracellular domain of a transmembrane protein or the sequence encoding the mature protein if the protein is extracellular is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold^{™} virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PRO can then be purified, for example, by Ni²⁺-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM MgCl₂; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 µm filter. A Ni²⁺-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline A₂₈₀ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching A₂₈₀ baseline again, the column is developed with a 0 to 500 mM Imidazole gradient in the secondary wash buffer. One mL fractions are collected and analyzed by SDS-PAGE and silver staining or Western blot with Ni²⁺-NTA-conjugated to alkaline phosphatase (Qiagen). Fractions containing the eluted His₁₀-tagged PRO are pooled and dialyzed against loading buffer.

Alternatively, purification of the IgG tagged (or Fc tagged) PRO can be performed using known chromatography techniques, including for instance, Protein A or protein G column chromatography.

Many of the PRO polypeptides disclosed herein were successfully expressed as described above.

### EXAMPLE 7: Preparation of Antibodies that Bind PRO

This example illustrates preparation of monoclonal antibodies which can specifically bind PRO.

Techniques for producing the monoclonal antibodies are known in the art and are described, for instance, in Goding, supra. Immunogens that may be employed include purified PRO, fusion proteins containing PRO, and cells expressing recombinant PRO on the cell surface. Selection of the immunogen can be made by the skilled artisan without undue experimentation.

Mice, such as Balb/c, are immunized with the PRO immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO antibodies.

After a suitable antibody titer has been detected, the animals "positive" for antibodies can be injected with a final intravenous injection of PRO. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO. Determination of "positive" hybridoma cells secreting the desired monoclonal antibodies against PRO is within the skill in the art.

The positive hybridoma cells can be injected intraperitoneally into syngeneic Balb/c mice to produce ascites containing the anti-PRO monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

### EXAMPLE 8: Purification of PRO Polypeptides Using Specific Antibodies

Native or recombinant PRO polypeptides may be purified by a variety of standard techniques in the art of protein purification. For example, pro-PRO polypeptide, mature PRO polypeptide, or pre-PRO polypeptide is purified by immunoaffinity chromatography using antibodies specific for the PRO polypeptide of interest. In general, an immunoaffinity column is constructed by covalently coupling the anti-PRO polypeptide antibody to an activated chromatographic resin.

Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE^{™} (Pharmacia LKB Biotechnology). The antibody is coupled to the resin, the resin is blocked, and the derivative resin is washed according to the manufacturer's instructions.

Such an immunoaffinity column is utilized in the purification of PRO polypeptide by preparing a fraction from cells containing PRO polypeptide in a soluble form. This preparation is derived by solubilization of the whole cell or of a subcellular fraction obtained via differential centrifugation by the addition of detergent or by other methods well known in the art. Alternatively, soluble PRO polypeptide containing a signal sequence may be secreted in useful quantity into the medium in which the cells are grown.

A soluble PRO polypeptide-containing preparation is passed over the immunoaffinity column, and the column is washed under conditions that allow the preferential absorbance of PRO polypeptide (e.g., high ionic strength buffers in the presence of detergent). Then, the column is eluted under conditions that disrupt antibody/PRO polypeptide binding (*e*.*g*., a low pH buffer such as approximately pH 2-3, or a high concentration of a chaotrope such as urea or thiocyanate ion), and PRO polypeptide is collected.

### EXAMPLE 9: Drug Screening

This invention is particularly useful for screening compounds by using PRO polypeptides or binding fragment thereof in any of a variety of drug screening techniques. The PRO polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO polypeptide or fragment. Drugs are screened against such transformed cells in competitive binding assays. Such cells, either in viable or fixed form, can be used for standard binding assays. One may measure, for example, the formation of complexes between PRO polypeptide or a fragment and the agent being tested. Alternatively, one can examine the diminution in complex formation between the PRO polypeptide and its target cell or target receptors caused by the agent being tested.

Thus, the present invention provides methods of screening for drugs or any other agents which can affect a PRO polypeptide-associated disease or disorder. These methods comprise contacting such an agent with an PRO polypeptide or fragment thereof and assaying (I) for the presence of a complex between the agent and the PRO polypeptide or fragment, or (ii) for the presence of a complex between the PRO polypeptide or fragment and the cell, by methods well known in the art. In such competitive binding assays, the PRO polypeptide or fragment is typically labeled. After suitable incubation, free PRO polypeptide or fragment is separated from that present in bound form, and the amount of free or uncomplexed label is a measure of the ability of the particular agent to bind to PRO polypeptide or to interfere with the PRO polypeptide/cell complex.

Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO polypeptide, the peptide test compounds are reacted with PRO polypeptide and washed. Bound PRO polypeptide is detected by methods well known in the art. Purified PRO polypeptide can also be coated directly onto plates for use in the aforementioned drug screening techniques. In addition, non-neutralizing antibodies can be used to capture the peptide and immobilize it on the solid support.

This invention also contemplates the use of competitive drug screening assays in which neutralizing antibodies capable of binding PRO polypeptide specifically compete with a test compound for binding to PRO polypeptide or fragments thereof. In this manner, the antibodies can be used to detect the presence of any peptide which shares one or more antigenic determinants with PRO polypeptide.

### EXAMPLE 10: Rational Drug Design

The goal of rational drug design is to produce structural analogs of biologically active polypeptide of interest (*i*.*e*., a PRO polypeptide) or of small molecules with which they interact, e.g., agonists, antagonists, or inhibitors. Any of these examples can be used to fashion drugs which are more active or stable forms of the PRO polypeptide or which enhance or interfere with the function of the PRO polypeptide *in vivo* (*c*.*f*., Hodgson, Bio/Technology, 9: 19-21 (1991)).

In one approach, the three-dimensional structure of the PRO polypeptide, or of a PRO polypeptide-inhibitor complex, is determined by x-ray crystallography, by computer modeling or, most typically, by a combination of the two approaches. Both the shape and charges of the PRO polypeptide must be ascertained to elucidate the structure and to determine active site(s) of the molecule. Less often, useful information regarding the structure of the PRO polypeptide may be gained by modeling based on the structure of homologous proteins. In both cases, relevant structural information is used to design analogous PRO polypeptide-like molecules or to identify efficient inhibitors. Useful examples of rational drug design may include molecules which have improved activity or stability as shown by Braxton and Wells, Biochemistry, 31:7796-7801 (1992) or which act as inhibitors, agonists, or antagonists of native peptides as shown by Athauda et al., J. Biochem., 113:742-746 (1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PRO polypeptide may be made available to perform such analytical studies as X-ray crystallography. In addition, knowledge of the PRO polypeptide amino acid sequence provided herein will provide guidance to those employing computer modeling techniques in place of or in addition to x-ray crystallography.

The foregoing written specification is considered to be sufficient to enable one skilled in the art to practice the invention. The present invention is not to be limited in scope by the construct deposited, since the deposited embodiment is intended as a single illustration of certain aspects of the invention and any constructs that are functionally equivalent are within the scope of this invention. The deposit of material herein does not constitute an admission that the written description herein contained is inadequate to enable the practice of any aspect of the invention, including the best mode thereof, nor is it to be construed as limiting the scope of the claims to the specific illustrations that it represents. Indeed, various modifications of the invention in addition to those shown and described herein will become apparent to those skilled in the art from the foregoing description and fall within the scope of the appended claims.

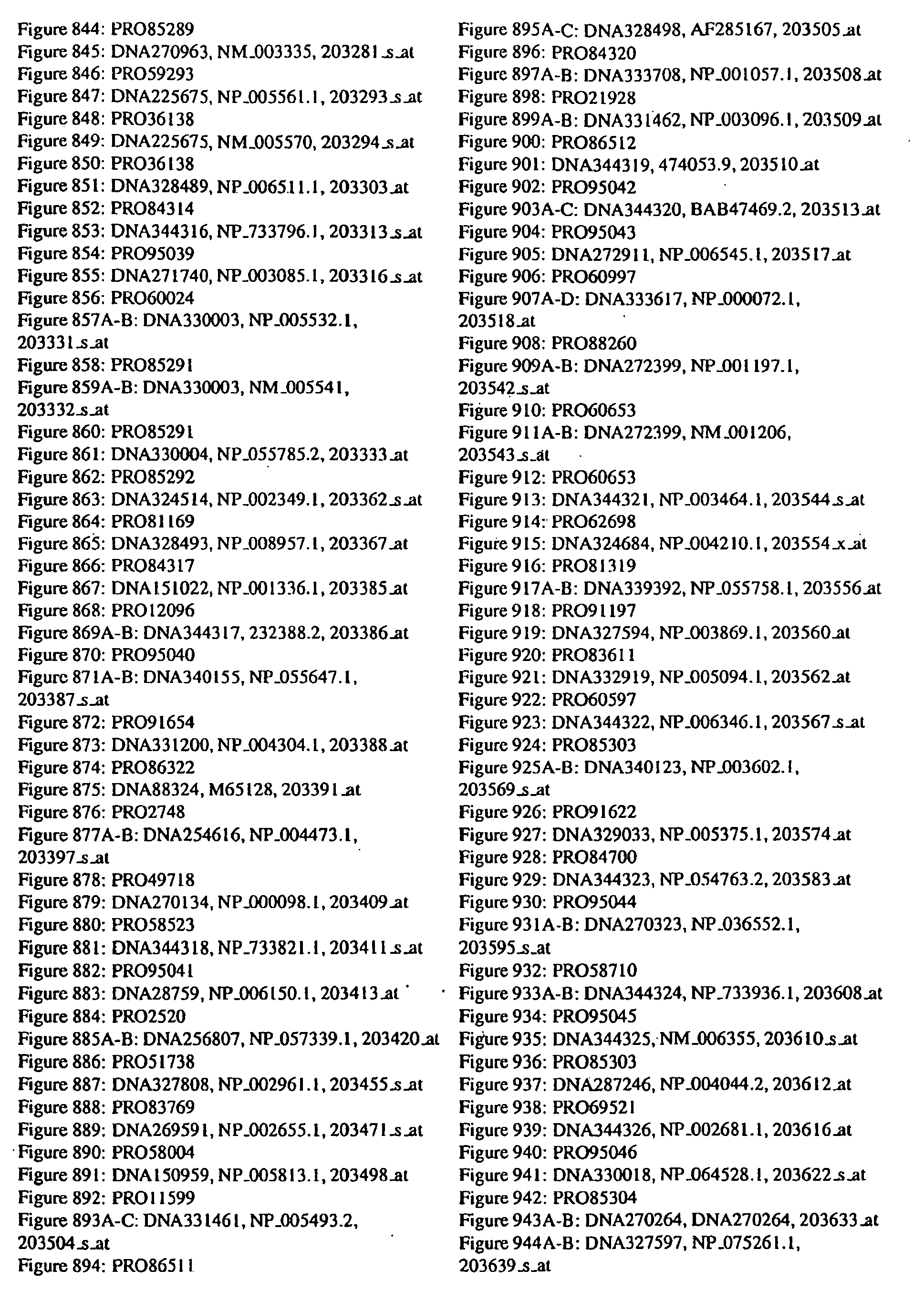

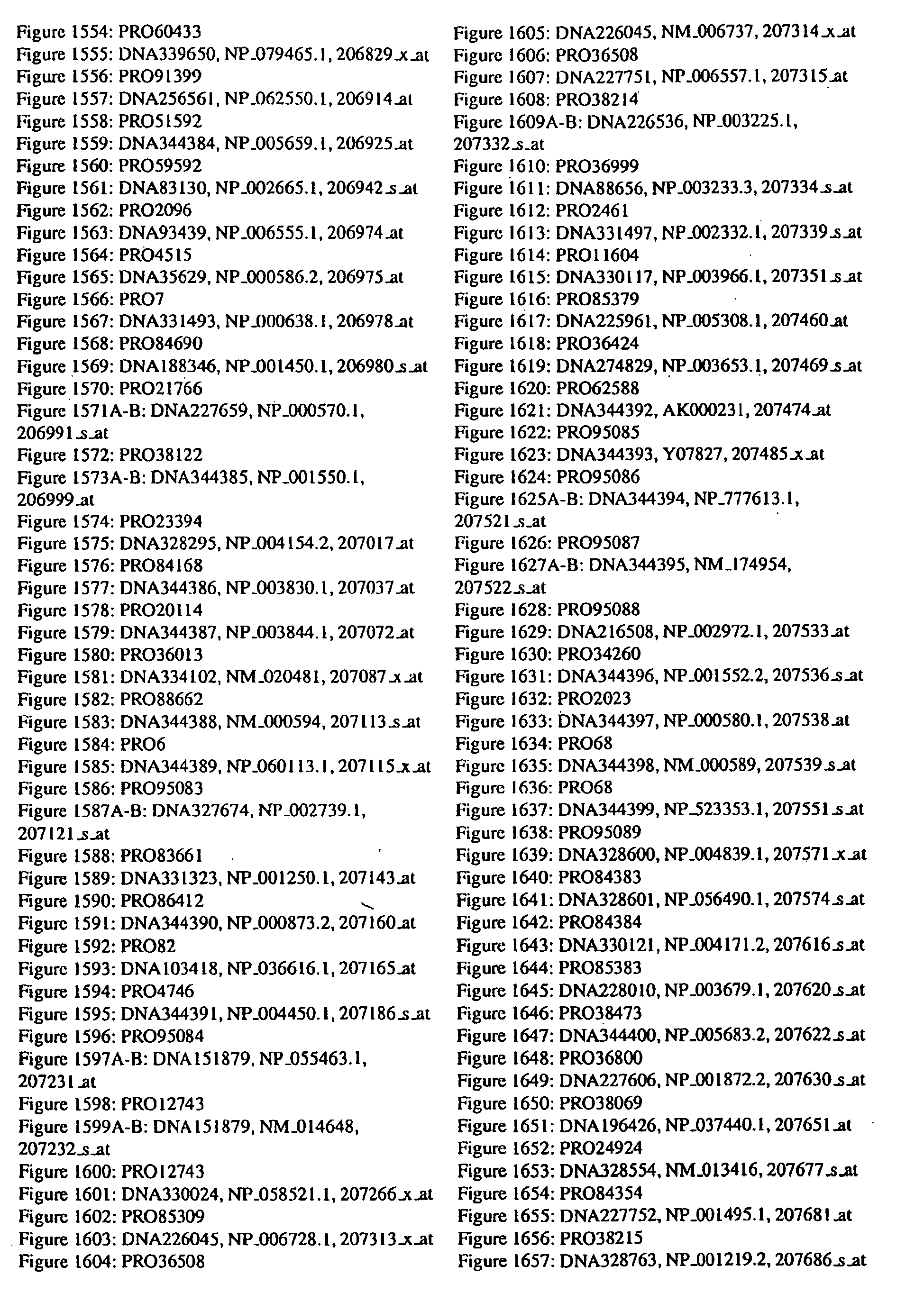

APPENDIX A

List of Figures

Figure 1: DNA344243, U25789, 200012_x_at
Figure 2: PRO94991
Figure 3: DNA326466, NP_004530.1, 200027_at
Figure 4: PRO60800
Figure 5: DNA326324, NP_000972.1, 200029_at
Figure 6: PRO4738
Figure 7: DNA344244, NP_006324.1, 200056_s_at
Figure 8: PRO61385
Figure 9: DNA304680, NP_031381.2, 200064_at
Figure 10: PRO71106
Figure 11: DNA325222, NP_000967.1, 200088_x_at
Figure 12: PRO62236
Figure 13: DNA270963, NP_003326.1, 1294_at
Figure 14: PRO59293
Figure 15: DNA188207, NP_005371.1, 37005_at
Figure 16: PRO21719
Figure 17: DNA333633, NP_055697.1, 38149_at
Figure 18: PRO88275
Figure 19: DNA254127, NP_008925.1, 38241_at
Figure 20: PRO49242
Figure 21A-B: DNA329908, BAA13246.1, 38892_at
Figure 22: PRO85225
Figure 23: DNA327523, NP_004916.1, 39248_at
Figure 24: PRO38028
Figure 25: DNA328357, 1452321.2, 39582_at
Figure 26: PRO84217
Figure 27A-B: DNA273398, NP_056383.1, 41577_at
Figure 28: PRO61398
Figure 29: DNA327526, NP_065727.2, 45288_at
Figure 30: PRO83574
Figure 31: DNA344245, AF177331, 47069_at
Figure 32: PRO94992
Figure 33A-B: DNA335121, NP_066300.1, 47550_at
Figure 34: PRO89524
Figure 35: DNA344246, NP_009093.1, 50221_at
Figure 36: PRO94993
Figure 37A-B: DNA226870, NP_000782.1, 48808_at
Figure 38: PRO37333
Figure 39A-B: DNA194778, NP_055545.1, 200617_at
Figure 40: PRO24056
Figure 41: DNA287245, NP_004175.1, 200628_s_at
Figure 42: PRO69520
Figure 43: DNA287245, NM_004184, 200629_at
Figure 44: PRO69520
Figure 45: DNA327532, NP_002056.2, 200648_s_at
Figure 46: PRO71134
Figure 47: DNA226063, X05130, 200656_s_at
Figure 48: PRO36526
Figure 49: DNA274759, NP_005611.1, 200660_at
Figure 50: PRO62529
Figure 51: DNA324276, NP_000985.1, 200674_s_at
Figure 52: PRO80959
Figure 53: DNA304669, NP_002119.1, 200679_x_at
Figure 54: PRO71096
Figure 55A-B: DNA344247, 7684654.2, 200690_at
Figure 56: PRO94994
Figure 57: DNA344248, NP_004125.3, 200691_s_at
Figure 58: PRO94995
Figure 59: DNA344249, NM_004134, 200692_s_at
Figure 60: PRO94996
Figure 61: DNA324897, NP_006845.1, 200700_s_at
Figure 62: PRO12468
Figure 63: DNA328375, NP_002071.1, 200708_at
Figure 64: PRO80880
Figure 65: DNA327114, NP_006004.1, 200725_x_at
Figure 66: PRO62466
Figure 67: DNA323943, NP_001021.1, 200741_s_at
Figure 68: PRO80676
Figure 69: DNA344250, NP_000382.3, 200742_s_at
Figure 70: PRO94997
Figure 71: DNA304659, NP_002023.1, 200748_s_at
Figure 72: PRO71086
Figure 73: DNA344251, 7762050.6, 200749_at
Figure 74: PRO94998
Figure 75: DNA287207, NP_006316.1, 200750_s_at
Figure 76: PRO39268
Figure 77A-B: DNA344252, NP_001377.1, 200762_at
Figure 78: PRO62709
Figure 79: DNA225584, NP_001145.1, 200782_at
Figure 80: PRO36047
Figure 81: DNA226262, NP_005554.1, 200783_s_at
Figure 82: PRO36725
Figure 83: DNA324060, NP_002530.1, 200790_at
Figure 84: PRO80773
Figure 85: DNA287211, NP_002147.1, 200806_s_at
Figure 86: PRO69492
Figure 87: DNA287211, NM_002156, 200807_s_at
Figure 88: PRO69492
Figure 89: DNA325222, NM_000976, 200809_x_at
Figure 90: PRO62236
Figure 91: DNA269874, NP_001271.1, 200810_s_at
Figure 92: PRO58272
Figure 93: DNA269874, NM_001280, 200811_at
Figure 94: PRO58272
Figure 95: DNA227795, NP_006420.1, 200812_at
Figure 96: PRO38258
Figure 97: DNA189687, NP_000843.1, 200824_at
Figure 98: PRO25845
Figure 99A-B: DNA255281, NP_006380.1,
200825_s_at
Figure 100: PRO50357
Figure 101: DNA88165, M14221, 200838_at
Figure 102: PRO2678
Figure 103: DNA196817, L16510, 200839_s_at
Figure 104: PRO3344
Figure 105: DNA326615, NP_000971.1, 200869_at
Figure 106: PRO82971
Figure 107: DNA226112, NP_002769.1, 200871_s_at

Figure 108: PRO36575
Figure 109: DNA254537, NP_002957.1, 200872_at
Figure 110: PRO49642
Figure 111: DNA254572, NP_006576.1, 200873_s_at
Figure 112: PRO49675
Figure 113: DNA271030, NP_006383.1, 200875_s_at
Figure 114: PRO59358
Figure 115: DNA324107, NP_006421.1, 200877_at
Figure 116: PRO80814
Figure 117: DNA328379, BC015869, 200878_at
Figure 118: PRO84234
Figure 119: DNA329099, 1164406.9, 200880_at
Figure 120: PRO60127
Figure 121: DNA271847, NP_001530.1, 200881_s_at
Figure 122: PRO60127
Figure 123: DNA226124, NP_003135.1, 200890_s_at
Figure 124: PRO36587
Figure 125: DNA325584, NP_002005.1, 200894_s_at
Figure 126: PRO59262
Figure 127: DNA325584, NM_002014, 200895_s_at
Figure 128: PRO59262
Figure 129: DNA272961, NP_004485.1, 200896_x_at
Figure 130: PRO61041
Figure 131A-B: DNA329018, NP_057165.2,
200897_s_at
Figure 132: PRO84693
Figure 133: DNA328380, X64879, 200904_at
Figure 134A-B: DNA329018, NM_016081,
200907_s_at
Figure 135: PRO84693
Figure 136: DNA304665, NP_000995.1, 200909_s_at
Figure 137: PRO71092
Figure 138: DNA272974, NP_005989.1, 200910_at
Figure 139: PRO61054
Figure 140: DNA272695, NP_001722.1, 200920_s_at
Figure 141: PRO60817
Figure 142: DNA272695, NM_001731, 200921_s_at
Figure 143: PRO60817
Figure 144A-B: DNA270430, NP_054706.1,
200931_s_at
Figure 145: PRO58810
Figure 146: DNA325153, NP_150644.1, 200936_at
Figure 147: PRO22907
Figure 148: DNA329925, NP_001528.1, 200942_s_at
Figure 149: PRO85239
Figure 150A-B: DNA287217, NP_001750.1,
200951_s_at
Figure 151: PRO36766
Figure 152A-B: DNA287217, NM_001759,
200952_s_at
Figure 153: PRO36766
Figure 154A-B: DNA226303, D13639, 200953_s_at
Figure 155: PRO36766
Figure 156: DNA324149, NP_000984.1, 200963_x_at
Figure 157: PRO11197
Figure 158A-C: DNA344253, NP_002304.2,
200965_s_at
Figure 159: PRO94999
Figure 160: DNA344254, AL137335, 200992_at
Figure 161: DNA325778, NP_006816.2, 200998_s_at
Figure 162: PRO82248
Figure 163: DNA325778, NM_006825, 200999_s_at
Figure 164: PRO82248
Figure 165: DNA275408, NP_001596.1, 201000_at
Figure 166: PRO63068
Figure 167: DNA328387, NP_001760.1, 201005_at
Figure 168: PRO4769
Figure 169: DNA304713, NP_006463.2, 201008_s_at
Figure 170: PRO71139
Figure 171: DNA304713, NM_006472, 201009_s_at
Figure 172: PRO71139
Figure 173: DNA304713, S73591, 201010_s_at
Figure 174: PRO71139
Figure 175: DNA89242, NP_000691.1, 201012_at
Figure 176: PRO2907
Figure 177: DNA328388, NP_006443.1, 201014_s_at
Figure 178: PRO84240
Figure 179A-B: DNA344255, 1327792.5, 201016_at
Figure 180: PRO95001
Figure 181: DNA328389, NP_006861.1, 201022_s_at
Figure 182: PRO84241
Figure 183: DNA344256, NP_005633.2, 201023_at
Figure 184: PRO95002
Figure 185A-B: DNA329101, NP_056988.2,
201024_x_at
Figure 186: PRO84751
Figure 187: DNA196628, NP_005318.1, 201036_s_at
Figure 188: PRO25105
Figure 189: DNA328391, NP_004408.1, 201041_s_at
Figure 190: PRO84242
Figure 191: DNA344257, NP_006296.1, 201043_s_at
Figure 192: PRO95003
Figure 193: DNA103208, NP_004090.3, 201061_s_at
Figure 194: PRO4538
Figure 195: DNA344258, NP_003810.1, 201064_s_at
Figure 196: PRO62717
Figure 197: DNA344259, NP_001907.2, 201066_at
Figure 198: PRO95004
Figure 199: DNA151675, NP_004791.1, 201078_at
Figure 200: PRO11975
Figure 201: DNA274743, NP_002850.1, 201087_at
Figure 202: PRO62517
Figure 203: DNA254725, NP_002257.1, 201088_at
Figure 204: PRO49824
Figure 205: DNA304719, NP_002296.1, 201105_at
Figure 206: PRO71145
Figure 207: DNA344260, NP_003312.2, 201113_at
Figure 208: PRO95005
Figure 209: DNA326273, NP_001961.1, 201123_s_at
Figure 210: PRO82678
Figure 211: DNA271185, NP_002397.1, 201126_s_at
Figure 212: PRO59502

Figure 213: DNA344261, NP_062543.1, 201132_at
Figure 214: PRO95006
Figure 215A-B: DNA227128, NP_055634.1,
201133_s_at
Figure 216: PRO37591
Figure 217: DNA329104, NP_004085.1, 201144_s_at
Figure 218: PRO69550
Figure 219: DNA344262, NP_000959.2, 201154_x_at
Figure 220: PRO95007
Figure 221A-B: DNA326365, NP_066565.1, 201158_at
Figure 222: PRO82761
Figure 223: DNA334099, NP_003642.2, 201161_s_at
Figure 224: PRO85244
Figure 225: DNA151802, NP_003661.1, 201169_s_at
Figure 226: PRO12890
Figure 227: DNA151802, NM_003670, 201170_s_at
Figure 228: PRO12890
Figure 229: DNA329091, NP_003936.1, 201171_at
Figure 230: PRO11997
Figure 231: DNA323783, NP_006591.1, 201173_x_at
Figure 232: PRO80535
Figure 233A-B: DNA344263, NP_003477.2,
201195_s_at
Figure 234: PRO49192
Figure 235: DNA328400, NP_003842.1, 201200_at
Figure 236: PRO1409
Figure 237: DNA103488, NP_002583.1, 201202_at
Figure 238: PRO4815
Figure 239: DNA344264, NP_005023.2, 201215_at
Figure 240: PRO83378
Figure 241: DNA326974, NP_000958.1, 201217_x_at
Figure 242: PRO83285
Figure 243: DNA327544, NP_002865.1, 201222_s_at
Figure 244: PRO70357
Figure 245: DNA344265, NP_006754.1, 201235_s_at
Figure 246: PRO80725
Figure 247: DNA275049, NP_004930.1, 201241_at
Figure 248: PRO62770
Figure 249: DNA226615, NP_001668.1, 201242_s_at
Figure 250: PRO37078
Figure 251: DNA226615, NM_001677, 201243_s_at
Figure 252: PRO37078
Figure 253: DNA287331, NP_002645.1, 201251_at
Figure 254: PRO69595
Figure 255: DNA324525, NP_000997.1, 201257_x_at
Figure 256: PRO81179
Figure 257: DNA227416, NP_006745.1, 201259_s_at
Figure 258: PRO37879
Figure 259: DNA227416, NM_006754, 201260_s_at
Figure 260: PRO37879
Figure 261: DNA270950, NP_003182.1, 201263_at
Figure 262: PRO59281
Figure 263: DNA97290, NP_002503.1, 201268_at
Figure 264: PRO3637
Figure 265: DNA344266, AF267863, 201276_at
Figure 266: PRO95008
Figure 267: DNA328405, NP_112556.1, 201277_s_at
Figure 268: PRO84252
Figure 269: DNA331290, NP_038474.1, 201285_at
Figure 270: PRO86391
Figure 271: DNA270526, NP_001166.1, 201288_at
Figure 272: PRO58903
Figure 273A-B: DNA327545, NP_001058.2,
201291_s_at
Figure 274: PRO82731
Figure 275A-B: DNA327545, NM_001067, 201292_at
Figure 276: PRO82731
Figure 277A-B: DNA344267, NM_134264,
201294_s_at
Figure 278: PRO95009
Figure 279A-B: DNA226778, AL110269, 201295_s_at
Figure 280: PRO37241
Figure 281: DNA333423, NP_001144.1, 201301_s_at
Figure 282: PRO61325
Figure 283: DNA333423, NM_001153, 201302_at
Figure 284: PRO61325
Figure 285: DNA329106, NP_003013.1, 201311_s_at
Figure 286: PRO83360
Figure 287: DNA329106, NM_003022, 201312_s_at
Figure 288: PRO83360
Figure 289: DNA255078, NP_006426.1, 201315_x_at
Figure 290: PRO50165
Figure 291: DNA274745, NP_006815.1, 201323_at
Figure 292: PRO62518
Figure 293: DNA150781, NP_001414.1, 201324_at
Figure 294: PRO12467
Figure 295: DNA150781, NM_001423, 201325_s_at
Figure 296: PRO12467
Figure 297: DNA329002, NP_001753.1, 201326_at
Figure 298: PRO4912
Figure 299: DNA329002, NM_001762, 201327_s_at
Figure 300: PRO4912
Figure 301A-C: DNA271656, NP_056128.1,
201334_s_at
Figure 302: PRO59943
Figure 303: DNA329107, NP_008818.3, 201367_s_at
Figure 304: PRO84754
Figure 305A-B: DNA329108, 1383643.16, 201368_at
Figure 306: PRO84755
Figure 307: DNA329107, NM_006887, 201369_s_at
Figure 308: PRO84754
Figure 309: DNA329218, NP_055227.1, 201381_x_at
Figure 310: PRO84829
Figure 311: DNA344268, NP_002800.2, 201388_at
Figure 312: PRO63269
Figure 313: DNA326116, NP_057376.1, 201391_at
Figure 314: PRO82542
Figure 315: DNA331447, NP_006614.2, 201397_at
Figure 316: PRO85247
Figure 317: DNA328410, NP_004519.1, 201403_s_at
Figure 318: PRO60174
Figure 319: DNA327072, NP_066357.1, 201406_at

Figure 320: PRO10723
Figure 321: DNA344269, NP_077007.1, 201420_s_at
Figure 322: PRO95010
Figure 323: DNA272286, NP_001743.1, 201432_at
Figure 324: PRO60544
Figure 325A-C: DNA88140, NP_004360.1, 201438_at
Figure 326: PRO2670
Figure 327: DNA344270, NP_071505.1, 201450_s_at
Figure 328: PRO95011
Figure 329: DNA326736, NP_006657.1, 201459_at
Figure 330: PRO83076
Figure 331: DNA226359, NP_002219.1, 201464_x_at
Figure 332: PRO36822
Figure 333: DNA226359, NM_002228, 201466_s_at
Figure 334: PRO36822
Figure 335: DNA328414, NP_003891.1, 201471_s_at
Figure 336: PRO81346
Figure 337: DNA103320, NP_002220.1, 201473_at
Figure 338: PRO4650
Figure 339: DNA325704, NP_004981.2, 201475_x_at
Figure 340: PRO82188
Figure 341: DNA327551, NP_001024.1, 201476_s_at
Figure 342: PRO59289
Figure 343: DNA327551, NM_001033, 201477_s_at
Figure 344: PRO59289
Figure 345: DNA254783, NP_001354.1, 201478_s_at
Figure 346: PRO49881
Figure 347: DNA254783, NM_001363, 201479_at
Figure 348: PRO49881
Figure 349: DNA329940, NP_001805.1, 201487_at
Figure 350: PRO2679
Figure 351: DNA304459, NP_005720.1, 201489_at
Figure 352: PRO37073
Figure 353: DNA304459, NM_005729, 201490_s_at
Figure 354: PRO37073
Figure 355: DNA325920, NP_036243.1, 201491_at
Figure 356: PRO82373
Figure 357: DNA253807, NP_065390.1, 201502_s_at
Figure 358: PRO49210
Figure 359: DNA329941, NP_001543.1, 201508_at
Figure 360: PRO85249
Figure 361: DNA323741, NP_003123.1, 201516_at
Figure 362: PRO80498
Figure 363: DNA344271, NP_073719.1, 201522_x_at
Figure 364: PRO62659
Figure 365: DNA328418, NP_003398.1, 201531_at
Figure 366: PRO84261
Figure 367: DNA329943, NP_009037.1, 201534_s_at
Figure 368: PRO85251
Figure 369: DNA329943, NM_007106, 201535_at
Figure 370: PRO85251
Figure 371: DNA329553, NP_064535.1, 201543_s_at
Figure 372: PRO38313
Figure 373: DNA344272, NP_004121.2, 201554_x_at
Figure 374: PRO95012
Figure 375: DNA272171, NP_002379.2, 201555_at
Figure 376: PRO60438
Figure 377: DNA226291, NP_055047.1, 201557_at
Figure 378: PRO36754
Figure 379A-B: DNA290226, NP_039234.1,
201559_s_at
Figure 380: PRO70317
Figure 381A-B: DNA290226, NM_013943, 201560_at
Figure 382: PRO70317
Figure 383: DNA227478, NP_002157.1, 201565_s_at
Figure 384: PRO37941
Figure 385: DNA150986, D13891, 201566_x_at
Figure 386: PRO0
Figure 387: DNA344273, M75715, 201573_s_at
Figure 388: PRO95013
Figure 389A-B: DNA270995, NP_004721.1, 201574_at
Figure 390: PRO59324
Figure 391: DNA227071, NP_000260.1, 201577_at
Figure 392: PRO37534
Figure 393A-B: DNA329944, AB032988, 201581_at
Figure 394: DNA227013, NP_001560.1, 201587_s_at
Figure 395: PRO37476
Figure 396: DNA150990, NP_003632.1, 201601_x_at
Figure 397: PRO12570
Figure 398: DNA290280, NP_004359.1, 201605_x_at
Figure 399: PRO70425
Figure 400:,DNA329947, NP_536806.1, 201613_s_at
Figure 401: PRO37674
Figure 402: DNA188207, NM_005380, 201621_at
Figure 403: PRO21719
Figure 404: DNA329114, NP_001340.1, 201623_s_at
Figure 405: PRO84759
Figure 406: DNA329114, NM_001349, 201624_at
Figure 407: PRO84759
Figure 408: DNA344274, 7698185.18, 201626_at
Figure 409: PRO95014
Figure 410A-D: DNA344275, U96876, 201627_s_at
Figure 411: DNA344276, NM_004300, 201629_s_at
Figure 412: PRO89350
Figure 413: DNA329115, NP_434702.1, 201631_s_at
Figure 414: PRO84760
Figure 415: DNA326193, NP_085056.1, 201634_s_at
Figure 416: PRO82609
Figure 417: DNA287240, NP_004326.1, 201641_at
Figure 418: PRO29371
Figure 419: DNA88410, NP_005525.1, 201642_at
Figure 420: PRO2778
Figure 421A-B: DNA220748, NP_000201.1, 201656_at
Figure 422: PRO34726
Figure 423: DNA328423, NP_003245.1, 201666_at
Figure 424: PRO2121
Figure 425: DNA344277, NP_683877.1, 201676_x_at
Figure 426: PRO81959
Figure 427: DNA324742, NP_001751.1, 201700_at
Figure 428: PRO81367
Figure 429: DNA270883, NP_001061.1, 201714_at
Figure 430: PRO59218

Figure 431A-B: DNA151806, NP_001422.1,
201718_s_at
Figure 432: PRO12768
Figure 433A-B: DNA151806, NM_001431,
201719_s_at
Figure 434: PRO12768
Figure 435: DNA273759, NP_006014.1, 201725_at
Figure 436: PRO61721
Figure 437: DNA344278, NP_005618.2, 201739_at
Figure 438: PRO86741
Figure 439: DNA326373, NP_008855.1, 201742_x_at
Figure 440: PRO82769
Figure 441A-B: DNA344279, 345309.13, 201749_at
Figure 442: PRO95015
Figure 443: DNA287167, NP_006627.1, 201761_at
Figure 444: PRO59136
Figure 445A-B: DNA150444, NP_055589.1,
201778_s_at
Figure 446: PRO12253
Figure 447A-B: DNA103387, NP_002287.1, 201795_at
Figure 448: PRO4716
Figure 449A-B: DNA272263, NP_006286.1,
201797_s_at
Figure 450: PRO70138
Figure 451: DNA151017, NP_004835.1, 201810_s_at
Figure 452: PRO12841
Figure 453: DNA151017, NM_004844, 201811_x_at
Figure 454: PRO12841
Figure 455: DNA324015, NP_006326.1, 201821_s_at
Figure 456: PRO80735
Figure 457: DNA329952, NP_005854.2, 201830_s_at
Figure 458: PRO85256
Figure 459: DNA304710, NP_001531.1, 201841_s_at
Figure 460: PRO71136
Figure 461: DNA88450, NP_000226.1, 201847_at
Figure 462: PRO2795
Figure 463: DNA254350, NP_004043.2, 201849_at
Figure 464: PRO49461
Figure 465: DNA150725, NP_001738.1, 201850_at
Figure 466: PRO12792
Figure 467: DNA329118, NP_068660.1, 201853_s_at
Figure 468: PRO83123
Figure 469A-B: DNA103553, NP_000167.1,
201865_x_at
Figure 470: PRO4880
Figure 471: DNA272066, NP_002931.1, 201872_s_at
Figure 472: PRO60337
Figure 473A-B: DNA331295, NP_002710.1,
201877_s_at
Figure 474: PRO86394
Figure 475: DNA150805, NP_055703.1, 201889_at
Figure 476: PRO11583
Figure 477: DNA344280, BC028932, 201890_at
Figure 478: DNA329956, NP_000875.1, 201892_s_at
Figure 479: PRO85260
Figure 480: DNA328431, NP_001817.1, 201897_s_at
Figure 481: PRO45093
Figure 482: DNA324310, NP_003356.1, 201903_at
Figure 483: PRO80988
Figure 484: DNA305191, NP_000999.1, 201909_at
Figure 485: PRO71295
Figure 486: DNA275385, NP_002085.1, 201912_s_at
Figure 487: PRO63048
Figure 488: DNA254978, NP_060625.1, 201917_s_at
Figure 489: PRO50067
Figure 490: DNA103328, NP_005406.2, 201920_at
Figure 491: PRO4658
Figure 492: DNA329057, NP_004116.2, 201921_at
Figure 493: PRO84719
Figure 494: DNA227112, NP_006397.1, 201923_at
Figure 495: PRO37575
Figure 496: DNA83046, NP_000565.1, 201925_s_at
Figure 497: PRO2569
Figure 498: DNA83046, NM_000574, 201926_s_at
Figure 499: PRO2569
Figure 500A-B: DNA344281, NP_005906.2, 201930_at
Figure 501: PRO62927
Figure 502: DNA329119, NP_004633.1, 201938_at
Figure 503: PRO4550
Figure 504A-B: DNA329120, NP_002560.1, 201945_at
Figure 505: PRO2752
Figure 506: DNA274167, NP_006422.1, 201946_s_at
Figure 507: PRO62097
Figure 508: DNA274167, NM_006431, 201947_s_at
Figure 509: PRO62097
Figure 510A-B: DNA327563, NP_066945.1, 201963_at
Figure 511: PRO83592
Figure 512: DNA344282, NP_002624.2, 201968_s_at
Figure 513: PRO95016
Figure 514: DNA344283, NP_751896.1, 201970_s_at
Figure 515: PRO95017
Figure 516: DNA344284, NP_002393.1, 202016_at
Figure 517: PRO95018
Figure 518: DNA328437, NP_005792.1, 202021_x_at
Figure 519: PRO84271
Figure 520: DNA300776, NP_000990.1, 202029_x_at
Figure 521: PRO70900
Figure 522: DNA344285, NP_005521.1, 202069_s_at
Figure 523: PRO83596
Figure 524: DNA226116, NP_002990.1, 202071_at
Figure 525: PRO36579
Figure 526: DNA344286, AF070533, 202073_at
Figure 527: PRO95019
Figure 528: DNA289522, NP_004994.1, 202077_at
Figure 529: PRO70276
Figure 530A-B: DNA270923, NP_004808.1, 202085_at
Figure 531: PRO59256
Figure 532: DNA327568, NP_002453.1, 202086_at
Figure 533: PRO57922
Figure 534: DNA271404, NP_001542.1, 202105_at
Figure 535: PRO59703
Figure 536: DNA328440, NP_004517.1, 202107_s_at

Figure 537: PRO84274
Figure 538: DNA344287, NP_003822.2, 202129_s_at
Figure 539: PRO95020
Figure 540: DNA324895, NP_006294.2, 202138_x_at
Figure 541: PRO81501
Figure 542A-B: DNA304479, NP_057124.2, 202194_at
Figure 543: PRO733
Figure 544: DNA329121, NP_079471.1, 202241_at
Figure 545: PRO84763
Figure 546: DNA325711, NP_000066.1, 202246_s_at
Figure 547: PRO4873
Figure 548: DNA294794, NP_002861.1, 202252_at
Figure 549: PRO70754
Figure 550: DNA256533, NP_006105.1, 202264_s_at
Figure 551: PRO51565
Figure 552: DNA150808, NP_002044.1, 202269_x_at
Figure 553: PRO12478
Figure 554: DNA150808, NM_002053, 202270_at
Figure 555: PRO12478
Figure 556: DNA304716, NP_510867.1, 202284_s_at
Figure 557: PRO71142
Figure 558: DNA328274, NP_055706.1, 202290_at
Figure 559: PRO12912
Figure 560: DNA331450, NP_004381.2, 202295_s_at
Figure 561: PRO2682
Figure 562: DNA344288, NP_000584.2, 202307_s_at
Figure 563: PRO36996
Figure 564A-B: DNA329970, NP_000910.2,
202336_s_at
Figure 565: PRO85272
Figure 566: DNA325115, NP_001435.1, 202345_s_at
Figure 567: PRO81689
Figure 568: DNA344289, NP_002807.1, 202352_s_at
Figure 569: PRO58880
Figure 570A-B: DNA254188, NP_004913.1, 202361_at
Figure 571: PRO49300
Figure 572: DNA331297, NP_005953.2, 202364_at
Figure 573: PRO86396
Figure 574A-B: DNA227353, NP_055637.1, 202375_at
Figure 575: PRO37816
Figure 576: DNA344290, 1096863.3, 202377_at
Figure 577: PRO95021
Figure 578: DNA103246, NP_059996.1, 202378_s_at
Figure 579: PRO4576
Figure 580: DNA328449, NP_005462.1, 202382_s_at
Figure 581: PRO60304
Figure 582: DNA150514, NP_065203.1, 202418_at
Figure 583: PRO12304
Figure 584A-C: DNA270933, NP_006757.1, 202423_at
Figure 585: PRO59265
Figure 586A-B: DNA335104, NP_000935.1,
202429_s_at
Figure 587: PRO49644
Figure 588: DNA227121, NP_066928.1, 202430_s_at
Figure 589: PRO37584
Figure 590: DNA66487, NP_002458.1, 202431_s_at
Figure 591: PRO1213
Figure 592A-B: DNA327576, NP_000095.1,
202435_s_at
Figure 593: PRO83600
Figure 594A-B: DNA327576, NM_000104,
202436_s_at
Figure 595: PRO83600
Figure 596A-D: DNA270871, U56438, 202437_s_at
Figure 597A-B: DNA344291, 7685287.117,
202438_x_at
Figure 598: PRO2328
Figure 599A-B: DNA335104, NM_000944,
202457_s_at
Figure 600: PRO49644
Figure 601A-B: DNA329973, NP_055461.1,
202459_s_at
Figure 602: PRO82824
Figure 603A-B: DNA269642, NP_004557.1,
202464_s_at
Figure 604: PRO58054
Figure 605: DNA227921, NP_003789.1, 202468_s_at
Figure 606: PRO38384
Figure 607A-B: DNA329122, NP_067675.1, 202478_at
Figure 608: PRO84764
Figure 609A-B: DNA329122, NM_021643,
202479_s_at
Figure 610: PRO84764
Figure 611: DNA329123, NP_002873.1, 202483_s_at
Figure 612: PRO84765
Figure 613: DNA344292, NP_003918.1, 202484_s_at
Figure 614: PRO95022
Figure 615: DNA324925, NP_036544.1, 202487_s_at
Figure 616: PRO61812
Figure 617A-B: DNA103449, NP_008862.1,
202498_s_at
Figure 618: PRO4776
Figure 619: DNA328451, NP_000007.1, 202502_at
Figure 620: PRO62139
Figure 621: DNA234442, NP_055551.1, 202503_s_at
Figure 622: PRO38852
Figure 623A-B: DNA277809, NP_055582.1,
202523_s_at
Figure 624: PRO64556
Figure 625A-B: DNA277809, NM_014767,
202524_s_at
Figure 626: PRO64556
Figure 627A-B: DNA226870, NM_000791,
202534_x_at
Figure 628: PRO37333
Figure 629: DNA328453, NP_003752.2, 202546_at
Figure 630: PRO84281
Figure 631A-B: DNA344293, NP_008879.2, 202557_at
Figure 632: PRO95023
Figure 633: DNA344294, NP_004166.1, 202567_at
Figure 634: PRO83257
Figure 635: DNA325587, NP_068772.1, 202580_x_at

Figure 636: PRO82083
Figure 637: DNA329979, NP_001062.1, 202589_at
Figure 638: PRO82821
Figure 639: DNA326078, NP_057725.1, 202593_s_at
Figure 640: PRO38464
Figure 641: DNA329125, NP_056159.1, 202594_at
Figure 642: PRO84767
Figure 643: DNA329125, NM_015344, 202595_s_at
Figure 644: PRO84767
Figure 645: DNA274881, NP_001896.1, 202613_at
Figure 646: PRO62626
Figure 647A-B: DNA329980, 1134366.16, 202615_at
Figure 648: PRO85278
Figure 649A-C: DNA344295, NP_036427.1,
202624_s_at
Figure 650: PRO95024
Figure 651A-B: DNA344296, 441144.12, 202625_at
Figure 652: PRO95025
Figure 653: DNA103245, NP_002341.1, 202626_s_at
Figure 654: PRO4575
Figure 655: DNA329126, NP_005025.1, 202635_s_at
Figure 656: PRO84768
Figure 657: DNA59763, NP_000192.1, 202638_s_at
Figure 658: PRO160
Figure 659: DNA289528, NP_004302.1, 202641_at
Figure 660: PRO70286
Figure 661A-B: DNA344297, NP_006281.1,
202643_s_at
Figure 662: PRO12904
Figure 663A-B: DNA344298, NM_006290,
202644_s_at
Figure 664: PRO12904
Figure 665: DNA254129, NP_006001.1, 202655_at
Figure 666: PRO49244
Figure 667A-B: DNA333747, 099914.40, 202663_at
Figure 668: PRO88372
Figure 669: DNA344299, NP_001665.1, 202672_s_at
Figure 670: PRO95026
Figure 671: DNA272801, NP_004483.1, 202678_at
Figure 672: PRO60906
Figure 673: DNA335588, NP_003801.1, 202687_s_at
Figure 674: PRO1096
Figure 675: DNA335588, NM_003810, 202688_at
Figure 676: PRO1096
Figure 677: DNA344300, NP_008869.1, 202690_s_at
Figure 678: PRO41946
Figure 679A-B: DNA150467, NP_055513.1,
202699_s_at
Figure 680: PRO12272
Figure 681: DNA330776, NP_005740.1, 202704_at
Figure 682: PRO58014
Figure 683: DNA326000, NP_004692.1, 202705_at
Figure 684: PRO82442
Figure 685A-B: DNA328459, NP_004332.2, 202715_at
Figure 686: PRO84285
Figure 687A-B: DNA270254, NP_002006.2,
202724_s_at
Figure 688: PRO58642
Figure 689: DNA331298, NP_055271.2, 202730_s_at
Figure 690: PRO81909
Figure 691: DNA344301, NM_145341, 202731_at
Figure 692: PRO95027
Figure 693A-B: DNA344302, BC035058, 202741_at
Figure 694: PRO95028
Figure 695: DNA271973, NP_002722.1, 202742_s_at
Figure 696: PRO60248
Figure 697: DNA344303, BC040437, 202746_at
Figure 698: PRO1189
Figure 699: DNA327192, NP_004858.1, 202747_s_at
Figure 700: PRO1189
Figure 701: DNA227164, Y12478, 202749_at
Figure 702: PRO37627
Figure 703A-C: DNA329129, NP_009134.1,
202759_s_at
Figure 704: PRO84288
Figure 705A-B: DNA344304, NM_147150,
202760_s_at
Figure 706: PRO95029
Figure 707A-B: DNA256782, AL080133, 202761_s_at
Figure 708: PRO51715
Figure 709A-B: DNA328464, 977954.20, 202769_at
Figure 710: PRO84290
Figure 711: DNA226578, NP_004345.1, 202770_s_at
Figure 712: PRO37041
Figure 713: DNA273346, NP_055316.1, 202779_s_at
Figure 714: PRO61349
Figure 715: DNA275337, NP_037365.1, 202786_at
Figure 716: PRO63011
Figure 717: DNA344305, 345245.28, 202789_at
Figure 718: PRO95030
Figure 719: DNA329986, NP_006454.1, 202811_at
Figure 720: PRO61895
Figure 721: DNA328465, NP_005639.1, 202824_s_at
Figure 722: PRO84291
Figure 723: DNA269828, NP_006691.1, 202837_at
Figure 724: PRO58230
Figure 725: DNA329988, NP_036460.1, 202842_s_at
Figure 726: PRO1471
Figure 727: DNA329988, NM_012328, 202843_at
Figure 728: PRO1471
Figure 729: DNA328466, NP_004554.1, 202847_at
Figure 730: PRO84292
Figure 731: DNA227063, NP_002849.1, 202850_at
Figure 732: PRO37526
Figure 733: DNA103394, NP_004198.1, 202855_s_at
Figure 734: PRO4722
Figure 735: DNA103394, NM_004207, 202856_s_at
Figure 736: PRO4722
Figure 737: DNA344306, NP_000575.1, 202859_x_at
Figure 738: PRO74
Figure 739: DNA275144, NP_000128.1, 202862_at
Figure 740: PRO62852

Figure 741: DNA328467, NP_003104.2, 202864_s_at
Figure 742: PRO84293
Figure 743: DNA287289, NP_058132.1, 202869_at
Figure 744: PRO69559
Figure 745: DNA273060, NP_001246.1, 202870_s_at
Figure 746: PRO61125
Figure 747: DNA325334, NP_061931.1, 202887_s_at
Figure 748: PRO81877
Figure 749A-B: DNA333705, NP_004070.3,
202901_x_at
Figure 750: PRO88334
Figure 751A-B: DNA333705, NM_004079,
202902_s_at
Figure 752: PRO88334
Figure 753: DNA332688, NP_510966.1, 202910_s_at
Figure 754: PRO2030
Figure 755A-B: DNA275066, NP_000170.1, 202911_at
Figure 756: PRO62786
Figure 757: DNA83008, NP_001115.1, 202912_at
Figure 758: PRO2032
Figure 759A-B: DNA344307, 7762119.3, 202934_at
Figure 760: PRO95031
Figure 761: DNA344308, NP_056518.2, 202937_x_at
Figure 762: PRO95032
Figure 763: DNA304681, NP_066552.1, 202941_at
Figure 764: PRO71107
Figure 765: DNA269481, NP_001976.1, 202942_at
Figure 766: PRO57901
Figure 767: DNA273320, NP_008950.1, 202954_at
Figure 768: PRO61327
Figure 769: DNA344309, X73427, 202988_s_at
Figure 770: PRO95033
Figure 771: DNA329136, NP_057475.1, 203023_at
Figure 772: PRO84772
Figure 773: DNA270174, NP_000092.1, 203028_s_at
Figure 774: PRO58563
Figure 775A-B: DNA83163, U66702, 203029_s_at
Figure 776: PRO2611
Figure 777A-B: DNA344310, NP_055566.1,
203037_s_at
Figure 778: PRO95034
Figure 779A-B: DNA344311, NP_002835.2, 203038_at
Figure 780: PRO95035
Figure 781A-B: DNA304464, NP_055733.1, 203044_at
Figure 782: PRO71042
Figure 783A-B: DNA328358, NP_005981.1, 203047_at
Figure 784: PRO84218
Figure 785A-B: DNA227821, NP_055666.1, 203068_at
Figure 786: PRO38284
Figure 787: DNA329137, NP_005892.1, 203077_s_at
Figure 788: PRO12879
Figure 789A-B: DNA339385, NP_055568.1, 203082_at
Figure 790: PRO91190
Figure 791: DNA344312, 1386457.26, 203086_at
Figure 792: PRO95036
Figure 793: DNA329138, NP_004511.1, 203087_s_at
Figure 794: PRO84773
Figure 795: DNA344313, AF026030, 203092_at
Figure 796: PRO95037
Figure 797A-B: DNA227949, NP_055062.1,
203096_s_at
Figure 798: PRO38412
Figure 799: DNA329992, NP_002399.1, 203102_s_at
Figure 800: PRO59267
Figure 801: DNA272867, NP_003960.1, 203109_at
Figure 802: PRO60960
Figure 803: DNA150430, NP_006387.1, 203114_at
Figure 804: PRO12770
Figure 805: DNA329994, NP_004707.2, 203118_at
Figure 806: PRO85286
Figure 807: DNA287417, NP_077003.1, 203119_at
Figure 808: PRO69674
Figure 809A-B: DNA226395, NP_000312.1, 203132_at
Figure 810: PRO36858
Figure 811A-B: DNA344314, NP_620309.1, 203140_at
Figure 812: PRO12790
Figure 813: DNA269433, NP_005877.1, 203163_at
Figure 814: PRO57856
Figure 815: DNA340116, NP_000146.2, 203179_at
Figure 816: PRO91615
Figure 817A-B: DNA331303, NP_003129.1,
203182_s_at
Figure 818: PRO86399
Figure 819: DNA304720, NP_062427.1, 203186_s_at
Figure 820: PRO71146
Figure 821A-B: DNA270861, NP_001371.1, 203187_at
Figure 822: PRO59198
Figure 823A-B: DNA344315, AAL56659.1,
203194_s_at
Figure 824: PRO95038
Figure 825: DNA329997, NP_031396.1, 203209_at
Figure 826: PRO61115
Figure 827A-B: DNA328481, NP_057240.1,
203211_s_at
Figure 828: PRO84307
Figure 829: DNA327588, 995529.4, 203213_at
Figure 830: PRO83607
Figure 831: DNA334914, NP_001777.1, 203214_x_at
Figure 832: PRO58324
Figure 833A-C: DNA274481, NP_000323.1,
203231_s_at
Figure 834: PRO62384
Figure 835A-C: DNA274481, NM_000332,
203232_s_at
Figure 836: PRO62384
Figure 837: DNA76514, NP_000409.1, 203233_at
Figure 838: PRO2540
Figure 839: DNA334781, NP_006448.1, 203242_s_at
Figure 840: PRO89234
Figure 841: DNA334781, NM_006457, 203243_s_at
Figure 842: PRO89234
Figure 843: DNA330000, NP_036277.1, 203270_at

Figure 945: PRO83613
Figure 946: DNA254642, NP_004100.1, 203646_at
Figure 947: PRO49743
Figure 948: DNA328507, NP_006395.1, 203650_at
Figure 949: PRO4761
Figure 950: DNA151752, NP_002124.1, 203665_at
Figure 951: PRO12886
Figure 952: DNA88352, NP_002067.1, 203676_at
Figure 953: PRO2759
Figure 954A-B: DNA227646, NP_000288.1, 203688_at
Figure 955: PRO38109
Figure 956A-B: DNA330021, NP_001940.1,
203692_s_at
Figure 957: PRO85306
Figure 958A-B: DNA330021, NM_001949,
203693_s_at
Figure 959: PRO85306
Figure 960A-B: DNA344327, NP_002591.1, 203708_at
Figure 961: PRO10691
Figure 962A-C: DNA331467, NP_002213.1, 203710_at
Figure 963: PRO86516
Figure 964: DNA329144, NM_014878, 203712_at
Figure 965: PRO84779
Figure 966: DNA324183, NP_001926.2, 203716_s_at
Figure 967: PRO80881
Figure 968: DNA330023, NP_001915.1, 203725_at
Figure 969: PRO85308
Figure 970A-B: DNA344328, NP_003613.1,
203736_s_at
Figure 971: PRO95047
Figure 972A-B: DNA325369, NP_055877.2,
203737_s_at
Figure 973: PRO81905
Figure 974: DNA344329, AL834427, 203738_at
Figure 975A-B: DNA274324, NP_006517.1, 203739_at
Figure 976: PRO62242
Figure 977A-B: DNA150748, NP_001105.1,
203741_s_at
Figure 978: PRO12446
Figure 979: DNA344330, 197185.7, 203745_at
Figure 980: PRO58198
Figure 981A-B: DNA325972, NP_001202.3, 203755_at
Figure 982: PRO82417
Figure 983: DNA328509, NP_006739.1, 203761_at
Figure 984: PRO57996
Figure 985: DNA344331, NP_057092.1, 203762_s_at
Figure 986: PRO95049
Figure 987: DNA344332, NM_016008, 203763_at
Figure 988: PRO95050
Figure 989: DNA330025, NP_055565.2, 203764_at
Figure 990: PRO85310
Figure 991: DNA330027, NP_036578.1, 203787_at
Figure 992: PRO85312
Figure 993: DNA274125, NP_071739.1, 203830_at
Figure 994: PRO62061
Figure 995A-B: DNA331113, NP_005914.1,
203836_s_at
Figure 996: PRO60244
Figure 997A-B: DNA344333, U67156, 203837_at
Figure 998: PRO60244
Figure 999A-B: DNA344334, 435717.6, 203843_at
Figure 1000: PRO95051
Figure 1001A-B: DNA325529, NP_536739.1,
203853_s_at
Figure 1002: PRO82037
Figure 1003: DNA275339, NP_005685.1, 203880_at
Figure 1004: PRO63012
Figure 1005: DNA328513, NM_016283, 203893_at
Figure 1006: PRO37815
Figure 1007: DNA151820, NP_000851.1, 203914_x_at
Figure 1008: PRO12194
Figure 1009: DNA82376, NP_002407.1, 203915_at
Figure 1010: PRO1723
Figure 1011: DNA344335, NP_004258.2, 203921_at
Figure 1012: PRO77044
Figure 1013: DNA271676, NP_002052.1, 203925_at
Figure 1014: PRO59961
Figure 1015: DNA344336, NP_002940.2, 203931_s_at
Figure 1016: PRO95052
Figure 1017: DNA88035, NP_002517.1, 203939_at
Figure 1018: PRO2135
Figure 1019: DNA327606, NP_001163.1, 203945_at
Figure 1020: PRO57873
Figure 1021: DNA327606, NM_001172, 203946_s_at
Figure 1022: PRO57873
Figure 1023: DNA344337, NP_005186.2, 203973_s_at
Figure 1024: PRO95053
Figure 1025: DNA227239, NP_003497.1, 203987_at
Figure 1026: PRO37702
Figure 1027: DNA344338, NP_004471.1, 203988_s_at
Figure 1028: PRO95054
Figure 1029: DNA226133, NP_001983.1, 203989_x_at
Figure 1030: PRO36596
Figure 1031A-B: DNA333574, NP_002820.2,
203997_at
Figure 1032: PRO88221
Figure 1033A-B: DNA344339, BC010502,
204009_s_at
Figure 1034: PRO95055
Figure 1035: DNA328516, NP_005833.1, 204011_at
Figure 1036: PRO12323
Figure 1037: DNA344340, NP_001385.1, 204014_at
Figure 1038: PRO49185
Figure 1039: DNA329145, NM_057158, 204015_s_at
Figure 1040: PRO84780
Figure 1041: DNA330033, NP_056492.1, 204019_s_at
Figure 1042: PRO85318
Figure 1043: DNA328271, NP_008988.2, 204026_s_at
Figure 1044: PRO81868
Figure 1045: DNA344341, NP_055390.1, 204030_s_at
Figure 1046: PRO95056
Figure 1047: DNA344342, 7698646.3, 204057_at

Figure 1048: PRO95057
Figure 1049A-B: DNA336315, NP_005035.1,
204060_s_at
Figure 1050: PRO90466
Figure 1051: DNA226737, NP_004576.1, 204070_at
Figure 1052: PRO37200
Figure 1053A-C: DNA333515, NP_075463.1,
204072_s_at
Figure 1054: PRO88167
Figure 1055: DNA344343, NP_003586.1, 204079_at
Figure 1056: PRO61375
Figure 1057: DNA344344, NP_006186.1, 204082_at
Figure 1058: PRO22518
Figure 1059: DNA270476, NP_003591.1, 204092_s_at
Figure 1060: PRO58855
Figure 1061: DNA216689, NP_002975.1, 204103_at
Figure 1062: PRO34276
Figure 1063: DNA328522, NP_001769.2, 204118_at
Figure 1064: PRO2696
Figure 1065: DNA304489, NP_003495.1, 204126_s_at
Figure 1066: PRO71058
Figure 1067: DNA325824, NP_002906.1, 204128_s_at
Figure 1068: PRO82290
Figure 1069: DNA103333, NP_055705.1, 204135_at
Figure 1070: PRO4663
Figure 1071: DNA344345, NP_006470.1, 204146_at
Figure 1072: PRO61659
Figure 1073A-B: DNA344346, 7698815.10, 204156_at
Figure 1074: PRO95058
Figure 1075: DNA330040, NP_523240.1, 204159_at
Figure 1076: PRO59546
Figure 1077: DNA273694, NP_006092.1, 204162_at
Figure 1078: PRO61661
Figure 1079A-B: DNA254376, NP_055778.1,
204166_at
Figure 1080: PRO49486
Figure 1081: DNA272655, NP_001818.1, 204170_s_at
Figure 1082: PRO60781
Figure 1083: DNA330041, NP_000088.2, 204172_at
Figure 1084: PRO85324
Figure 1085: DNA328529, NP_001620.2, 204174_at
Figure 1086: PRO49814
Figure 1087: DNA226380, NP_001765.1, 204192_at
Figure 1088: PRO4695
Figure 1089A-B: DNA290230, NP_004341.1,
204197_s_at
Figure 1090: PRO70325
Figure 1091: DNA151798, NP_001797.1, 204203_at
Figure 1092: PRO12186
Figure 1093: DNA271778, NP_068594.1, 204205_at
Figure 1094: PRO60062
Figure 1095: DNA333754, NP_004868.1, 204220_at
Figure 1096: PRO88379
Figure 1097: DNA150812, NP_006842.1, 204222_s_at
Figure 1098: PRO12481
Figure 1099A-B: DNA287273, NP_006435.1,
204240_s_at
Figure 1100: PRO69545
Figure 1101: DNA330043, NP_001789.2, 204252_at
Figure 1102: PRO85326
Figure 1103A-B: DNA103527, NP_000367.1,
204254_s_at
Figure 1104: PRO4854
Figure 1105A-B: DNA103527, NM_000376,
204255_s_at
Figure 1106: PRO4854
Figure 1107: DNA228132, NP_076995.1, 204256_at
Figure 1108: PRO38595
Figure 1109: DNA273802, NP_066950.1, 204285_s_at
Figure 1110: PRO61763
Figure 1111: DNA273802, NM_021127, 204286_s_at
Figure 1112: PRO61763
Figure 1113: DNA344347, NP_002916.1, 204319_s_at
Figure 1114: PRO63255
Figure 1115: DNA330136, X76717, 204326_x_at
Figure 1116: PRO82583
Figure 1117: DNA327613, NP_005971.1, 204351_at
Figure 1118: PRO83622
Figure 1119A-D: DNA339387, NP_055625.2,
204373_s_at
Figure 1120: PRO91192
Figure 1121: DNA344348, NP_004477.2, 204384_at
Figure 1122: PRO95059
Figure 1123: DNA334269, NP_000231.1, 204388_s_at
Figure 1124: PRO59228
Figure 1125: DNA334269, NM_000240, 204389_at
Figure 1126: PRO59228
Figure 1127: DNA344349, NP_002241.1, 204401_at
Figure 1128: PRO4787
Figure 1129: DNA255402, NP_055288.1, 204405_x_at
Figure 1130: PRO50469
Figure 1131A-B: DNA254135, NP_060066.1,
204411_at
Figure 1132: PRO49250
Figure 1133: DNA327616, NP_075011.1, 204415_at
Figure 1134: PRO83624
Figure 1135: DNA327617, NP_006811.1, 204439_at
Figure 1136: PRO83625
Figure 1137A-B: DNA330049, NP_004514.2,
204444_at
Figure 1138: PRO85330
Figure 1139: DNA270496, NP_001316.1, 204459_at
Figure 1140: PRO58875
Figure 1141: DNA331075, NP_000601.2, 204489_s_at
Figure 1142: PRO86231
Figure 1143: DNA331075, NM_000610, 204490_s_at
Figure 1144: PRO86231
Figure 1145A-C: DNA344350, 418805.19, 204491_at
Figure 1146: PRO95060
Figure 1147: DNA194652, NP_001187.1, 204493_at
Figure 1148: PRO23974
Figure 1149A-B: DNA331311, NP_056054.1,

204500_s_at
Figure 1150: PRO86405
Figure 1151: DNA297387, NP_003494.1, 204510_at
Figure 1152: PRO58394
Figure 1153: DNA330051, NP_003431.1, 204523_at
Figure 1154: PRO85332
Figure 1155A-B: DNA272298, NP_055544.1,
204529_s_at
Figure 1156: PRO60555
Figure 1157: DNA82362, NP_001556.1, 204533_at
Figure 1158: PRO1718
Figure 1159: DNA225993, NP_000646.1, 204563_at
Figure 1160: PRO36456
Figure 1161: DNA151910, NP_004906.2, 204567_s_at
Figure 1162: PRO12754
Figure 1163: DNA328266, NP_005993.1, 204616_at
Figure 1164: PRO12125
Figure 1165: DNA344351, NP_006177.1, 204621_s_at
Figure 1166: PRO12850
Figure 1167: DNA344352, NM_173173, 204622_x_at
Figure 1168: PRO95061
Figure 1169: DNA226079, NP_001602.1, 204638_at
Figure 1170: PRO36542
Figure 1171: DNA226699, NP_000013.1, 204639_at
Figure 1172: PRO37162
Figure 1173: DNA254470, NP_002488.1, 204641_at
Figure 1174: PRO49578
Figure 1175A-B: DNA227097, NP_000101.1,
204646_at
Figure 1176: PRO37560
Figure 1177: DNA52729, M21121, 204655_at
Figure 1178: PRO91
Figure 1179: DNA344353, M11867, 204670_x_at
Figure 1180: PRO95062
Figure 1181: DNA327521, NP_002192.2, 204698_at
Figure 1182: PRO58320
Figure 1183: DNA271179, NP_004280.3, 204702_s_at
Figure 1184: PRO59497
Figure 1185A-B: DNA344354, NP_612565.1,
204709_s_at
Figure 1186: PRO95063
Figure 1187A-B: DNA335768, NP_000121.1,
204714_s_at
Figure 1188: PRO90077
Figure 1189A-B: DNA273690, NP_055602.1,
204720_s_at
Figure 1190: PRO61657
Figure 1191: DNA328698, NP_006144.1, 204725_s_at
Figure 1192: PRO12168
Figure 1193A-B: DNA83176, NP_003234.1, 204731_at
Figure 1194: PRO2620
Figure 1195A-B: DNA344355, NP_006193.1,
204735_at
Figure 1196: PRO95064
Figure 1197A-B: DNA325192, NP_038203.1,
204744_s_at
Figure 1198: PRO81753
Figure 1199: DNA330057, NP_005941.1, 204745_x_at
Figure 1200: PRO85337
Figure 1201: DNA287178, NP_001540.1, 204747_at
Figure 1202: PRO69467
Figure 1203A-B: DNA226070, NP_000954.1,
204748_at
Figure 1204: PRO36533
Figure 1205: DNA330058, NP_004529.2, 204749_at
Figure 1206: PRO85338
Figure 1207A-B: DNA270601, NP_002117.1,
204753_s_at
Figure 1208: PRO58973
Figure 1209: DNA329153, NP_001259.1, 204759_at
Figure 1210: PRO84786
Figure 1211: DNA328541, NP_004503.1, 204773_at
Figure 1212: PRO4843
Figure 1213: DNA328542, NP_055025.1, 204774_at
Figure 1214: PRO2577
Figure 1215: DNA227033, NP_002362.1, 204777_s_at
Figure 1216: PRO37496
Figure 1217: DNA332667, NP_000034.1, 204780_s_at
Figure 1218: PRO1207
Figure 1219: DNA344356, NM_152877, 204781_s_at
Figure 1220: PRO95065
Figure 1221: DNA344357, NP_000865.2, 204786_s_at
Figure 1222: PRO1011
Figure 1223: DNA253585, NP_004409.1, 204794_at
Figure 1224: PRO49183
Figure 1225A-B: DNA329907, NP_036423.1,
204817_at
Figure 1226: PRO85224
Figure 1227: DNA254127, NM_006994, 204820_s_at
Figure 1228: PRO49242
Figure 1229: DNA254127, U90548, 204821_at
Figure 1230: PRO49242
Figure 1231A-B: DNA269878, M86699, 204822_at
Figure 1232: PRO58276
Figure 1233: DNA255289, NP_055606.1, 204825_at
Figure 1234: PRO50363
Figure 1235: DNA344358, NP_002175.2, 204863_s_at
Figure 1236: PRO85478
Figure 1237: DNA344359, NM_175767, 204864_s_at
Figure 1238: PRO95066
Figure 1239: DNA333633, NM_014882, 204882_at
Figure 1240: PRO88275
Figure 1241: DNA330065, NP_055079.2, 204887_s_at
Figure 1242: PRO85345
Figure 1243: DNA226195, NP_000949.1, 204896_s_at
Figure 1244: PRO36658
Figure 1245: DNA344360, 334072.2, 204897_at
Figure 1246: PRO95067
Figure 1247: DNA329157, NP_004271.1, 204905_s_at
Figure 1248: PRO62861
Figure 1249A-B: DNA344361, NP_001549.1,
204912_at

Figure 1250: PRO2536
Figure 1251: DNA228014, NP_002153.1, 204949_at
Figure 1252: PRO38477
Figure 1253: DNA150427, NP_005599.1, 204960_at
Figure 1254: PRO12243
Figure 1255: DNA330067, NP_001800.1, 204962_s_at
Figure 1256: PRO60368
Figure 1257: DNA287399, NP_058197.1, 204972_at
Figure 1258: PRO69656
Figure 1259: DNA329158, NP_077013.1, 204985_s_at
Figure 1260: PRO84788
Figure 1261: DNA272427, NP_004799.1, 205005_s_at
Figure 1262: PRO60679
Figure 1263: DNA272427, NM_004808, 205006_s_at
Figure 1264: PRO60679
Figure 1265: DNA344362, NP_000666.2, 205013_s_at
Figure 1266: PRO4938
Figure 1267: DNA329534, NP_004615.2, 205019_s_at
Figure 1268: PRO2904
Figure 1269: DNA272312, NP_005188.1, 205022_s_at
Figure 1270: PRO60569
Figure 1271: DNA330069, NP_002866.2, 205024_s_at
Figure 1272: PRO85348
Figure 1273: DNA328297, NP_477097.1, 205034_at
Figure 1274: PRO59418
Figure 1275: DNA324992, NP_597680.1, 205047_s_at
Figure 1276: PRO81586
Figure 1277: DNA328551, NP_003823.1, 205048_s_at
Figure 1278: PRO84351
Figure 1279A-B: DNA83118, NP_000213.1,
205051_s_at
Figure 1280: PRO2598
Figure 1281: DNA254214, NP_001689.1, 205052_at
Figure 1282: PRO49326
Figure 1283A-B: DNA220750, NP_002199.2,
205055_at
Figure 1284: PRO34728
Figure 1285: DNA329025, NP_006199.1, 205066_s_at
Figure 1286: PRO4860
Figure 1287: DNA327632, NP_001302.1, 205081_at
Figure 1288: PRO83635
Figure 1289A-B: DNA344363, NP_005482.1,
205088_at
Figure 1290: PRO95068
Figure 1291: DNA344364, 331306.1, 205098_at
Figure 1292: PRO4949
Figure 1293: DNA226177, NP_001286.1, 205099_s_at
Figure 1294: PRO36640
Figure 1295: DNA192060, NP_002974.1, 205114_s_at
Figure 1296: PRO21960
Figure 1297: DNA344365, NP_008924.1, 205129_at
Figure 1298: PRO95069
Figure 1299: DNA299899, NP_002148.1, 205133_s_at
Figure 1300: PRO62760
Figure 1301: DNA328554, NP_038202.1, 205147_x_at
Figure 1302: PRO84354
Figure 1303A-B: DNA329160, NP_002821.1,
205171_at
Figure 1304: PRO84789
Figure 1305: DNA328810, NP_001770.1, 205173_x_at
Figure 1306: PRO2557
Figure 1307: DNA344366, NP_004476.1, 205184_at
Figure 1308: PRO59080
Figure 1309: DNA272443, NP_055531.1, 205213_at
Figure 1310: PRO60693
Figure 1311: DNA273535, NP_004217.1, 205214_at
Figure 1312: PRO61515
Figure 1313: DNA188333, NP_006410.1, 205242_at
Figure 1314: PRO21708
Figure 1315: DNA227447, NP_003193.1, 205254_x_at
Figure 1316: PRO37910
Figure 1317: DNA227447, NM_003202, 205255_x_at
Figure 1318: PRO37910
Figure 1319A-B: DNA188301, NP_002300.1,
205266_at
Figure 1320: PRO21834
Figure 1321: DNA332739, NP_006226.1, 205267_at
Figure 1322: PRO87518
Figure 1323: DNA227173, NP_001456.1, 205285_s_at
Figure 1324: PRO37636
Figure 1325A-B: DNA331483, NM_003672,
205288_at
Figure 1326: PRO86528
Figure 1327: DNA43320, DNA43320, 205289_at
Figure 1328: PRO313
Figure 1329: DNA219011, NP_001191.1, 205290_s_at
Figure 1330: PRO34479
Figure 1331A-B: DNA331484, NP_000869.1,
205291_at
Figure 1332: PRO3276
Figure 1333: DNA327019, NP_001406.1, 205321_at
Figure 1334: PRO83323
Figure 1335A-B: DNA269546, NP_055612.1,
205340_at
Figure 1336: PRO57962
Figure 1337: DNA326497, NM_000156, 205354_at
Figure 1338: PRO58046
Figure 1339: DNA336844, NP_003857.1, 205376_at
Figure 1340: PRO90913
Figure 1341A-C: DNA332571, NP_065209.1,
205390_s_at
Figure 1342: PRO12143
Figure 1343: DNA325568, NP_001265.1, 205393_s_at
Figure 1344: PRO12187
Figure 1345: DNA325568, NM_001274, 205394_at
Figure 1346: PRO12187
Figure 1347: DNA151830, NP_005893.1, 205397_x_at
Figure 1348: PRO62998
Figure 1349: DNA151830, NM_005902, 205398_s_at
Figure 1350: PRO62998
Figure 1351: DNA329010, NP_004942.1, 205419_at
Figure 1352: PRO23370

Figure 1353: DNA335207, NP_057531.2, 205429_s_at
Figure 1354: PRO89594
Figure 1355: DNA287337, NP_002096.1, 205436_s_at
Figure 1356: PRO69600
Figure 1357: DNA272221, NP_037431.1, 205449_at
Figure 1358: PRO60483
Figure 1359: DNA88194, NP_000724.1, 205456_at
Figure 1360: PRO2220
Figure 1361: DNA188355, NP_004582.1, 205476_at
Figure 1362: PRO21885
Figure 1363: DNA287224, NP_005092.1, 205483_s_at
Figure 1364: PRO69503
Figure 1365: DNA330084, NP_055265.1, 205484_at
Figure 1366: PRO9895
Figure 1367A-E: DNA334058, NP_000531.1,
205485_at
Figure 1368: PRO88622
Figure 1369: DNA225959, NP_006135.1, 205488_at
Figure 1370: PRO36422
Figure 1371: DNA226043, NP_006424.2, 205495_s_at
Figure 1372: PRO36506
Figure 1373A-B: DNA344367, NP_005392.1,
205503_at
Figure 1374: PRO24022
Figure 1375: DNA344368, NP_001481.2, 205505_at
Figure 1376: PRO95070
Figure 1377: DNA328566, NP_060446.1, 205511_at
Figure 1378: PRO84363
Figure 1379A-B: DNA334718, NP_004923.1,
205532_s_at
Figure 1380: PRO2196
Figure 1381: DNA344369, NP_036581.1, 205542_at
Figure 1382: PRO28528
Figure 1383: DNA344370, NP_006797.3, 205548_s_at
Figure 1384: PRO95071
Figure 1385: DNA331486, NM_002534, 205552_s_at
Figure 1386: PRO69559
Figure 1387: DNA256257, NP_055213.1, 205569_at
Figure 1388: PRO51301
Figure 1389A-B: DNA227714, NP_000852.1,
205579_at
Figure 1390: PRO38177
Figure 1391A-B: DNA327643, NP_055712.1,
205594_at
Figure 1392: PRO83644
Figure 1393: DNA344371, NP_073576.1, 205596_s_at
Figure 1394: PRO95072
Figure 1395: DNA329013, NP_005649.1, 205599_at
Figure 1396: PRO20128
Figure 1397: DNA90631, NP_000747.1, 205630_at
Figure 1398: PRO2519
Figure 1399: DNA88076, NP_001628.1, 205639_at
Figure 1400: PRO2640
Figure 1401: DNA344372, NP_003780.1, 205641_s_at
Figure 1402: PRO95073
Figure 1403A-B: DNA196641, NP_002340.1,
205668_at
Figure 1404: PRO25114
Figure 1405: DNA344373, NP_076992.1, 205673_s_at
Figure 1406: PRO95074
Figure 1407: DNA328570, NP_004040.1, 205681_at
Figure 1408: PRO37843
Figure 1409: DNA327644, NP_060395.2, 205684_s_at
Figure 1410: PRO83645
Figure 1411: DNA344374, NP_061989.1, 205687_at
Figure 1412: PRO95075
Figure 1413: DNA226234, NP_001766.1, 205692_s_at
Figure 1414: PRO36697
Figure 1415: DNA150621, NP_036595.1, 205704_s_at
Figure 1416: PRO12374
Figure 1417: DNA331817, NP_055154.3, 205707_at
Figure 1418: PRO86240
Figure 1419: DNA220761, NP_000880.1, 205718_at
Figure 1420: PRO34739
Figure 1421: DNA326483, NP_060346.1, 205748_s_at
Figure 1422: PRO82861
Figure 1423: DNA331318, NP_003636.1, 205768_s_at
Figure 1424: PRO51139
Figure 1425: DNA331318, NM_003645, 205769_at
Figure 1426: PRO51139
Figure 1427: DNA330091, NP_057461.1, 205771_s_at
Figure 1428: PRO85362
Figure 1429: DNA344375, NP_002176.2, 205798_at
Figure 1430: PRO95076
Figure 1431A-B: DNA344376, NP_733772.1,
205801_s_at
Figure 1432: PRO95077
Figure 1433: DNA194766, NP_079504.1, 205804_s_at
Figure 1434: PRO24046
Figure 1435: DNA344377, NP_064512.1, 205807_s_at
Figure 1436: PRO95078
Figure 1437: DNA103440, NP_031386.1, 205821_at
Figure 1438: PRO4767
Figure 1439: DNA75526, NP_001758.1, 205831_at
Figure 1440: PRO2013
Figure 1441A-B: DNA328574, NP_004963.1,
205841_at
Figure 1442: PRO84368
Figure 1443A-B: DNA328574, NM_004972,
205842_s_at
Figure 1444: PRO84368
Figure 1445A-B: DNA220746, NP_000876.1,
205884_at
Figure 1446: PRO34724
Figure 1447: DNA330095, NP_004732.1, 205895_s_at
Figure 1448: PRO85366
Figure 1449: DNA328576, NP_001328.1, 205898_at
Figure 1450: PRO4940
Figure 1451: DNA103307, NP_000238.1, 205904_at
Figure 1452: PRO4637
Figure 1453A-B: DNA339322, NP_003408.1,
205917_at

Figure 1454: PRO91128
Figure 1455A-B: DNA255292, NP_056374.1,
205933_at
Figure 1456: PRO50365
Figure 1457A-B: DNA270867, NP_006217.1,
205934_at
Figure 1458: PRO59203
Figure 1459: DNA329047, NP_006390.1, 205965_at
Figure 1460: PRO58425
Figure 1461: DNA196439, NP_003865.1, 205988_at
Figure 1462: PRO24934
Figure 1463A-B: DNA227747, NP_005798.1,
206007_at
Figure 1464: PRO38210
Figure 1465: DNA103281, NP_002899.1, 206036_s_at
Figure 1466: PRO4611
Figure 1467: DNA344378, NP_073715.1, 206042_x_at
Figure 1468: PRO95079
Figure 1469: DNA275181, NP_003081.1, 206055_s_at
Figure 1470: PRO62882
Figure 1471: DNA330096, NP_057051.1, 206060_s_at
Figure 1472: PRO37163
Figure 1473A-B: DNA344379, NP_006246.2,
206099_at
Figure 1474: PRO95080
Figure 1475: DNA83063, NP_004429.1, 206114_at
Figure 1476: PRO2068
Figure 1477A-B: DNA151420, NP_004421.1,
206115_at
Figure 1478: PRO12876
Figure 1479: DNA329006, NP_003142.1, 206118_at
Figure 1480: PRO12865
Figure 1481: DNA331657, NP_001707.1, 206126_at
Figure 1482: PRO23970
Figure 1483: DNA344380, NP_004953.1, 206159_at
Figure 1484: PRO2562
Figure 1485: DNA329005, NP_003028.1, 206181_at
Figure 1486: PRO12612
Figure 1487A-B: DNA344381, NP_055604.1,
206188_at
Figure 1488: PRO95081
Figure 1489A-B: DNA274141, NP_006460.2,
206245_s_at
Figure 1490: PRO62077
Figure 1491: DNA334388, NP_055141.2, 206324_s_at
Figure 1492: PRO88904
Figure 1493: DNA88224, NP_001829.1, 206337_at
Figure 1494: PRO2236
Figure 1495: DNA336220, NM_006123, 206342_x_at
Figure 1496: PRO91049
Figure 1497: DNA227700, NP_004769.1, 206361_at
Figure 1498: PRO38163
Figure 1499: DNA227208, NP_005351.2, 206363_at
Figure 1500: PRO37671
Figure 1501A-B: DNA330100, NP_055690.1,
206364_at
Figure 1502: PRO85369
Figure 1503: DNA329169, NP_002986.1, 206365_at
Figure 1504: PRO1610
Figure 1505: DNA329169, NM_002995, 206366_x_at
Figure 1506: PRO1610
Figure 1507A-B: DNA335332, NP_002640.2,
206369_s_at
Figure 1508: PRO89706
Figure 1509A-E: DNA333253, NP_066267.1,
206385_s_at
Figure 1510: PRO87958
Figure 1511: DNA326727, NP_001527.1, 206445_s_at
Figure 1512: PRO83069
Figure 1513: DNA153751, NP_005942.1, 206461_x_at
Figure 1514: PRO12925
Figure 1515: DNA288243, NP_002277.3, 206486_at
Figure 1516: PRO36451
Figure 1517: DNA268333, NP_001260.1, 206499_s_at
Figure 1518: PRO57322
Figure 1519: DNA344382, NP_003826.1, 206518_s_at
Figure 1520: PRO95082
Figure 1521A-B: DNA334589, NP_055073.1,
206546_at
Figure 1522: PRO89073
Figure 1523: DNA327663, NP_006771.1, 206565_x_at
Figure 1524: PRO83654
Figure 1525: DNA330103, NP_056179.1, 206584_at
Figure 1526: PRO19671
Figure 1527: DNA329172, NP_005254.1, 206589_at
Figure 1528: PRO84796
Figure 1529: DNA344383, NP_003846.1, 206618_at
Figure 1530: PRO4778
Figure 1531A-C: DNA328331, NP_004645.1,
206624_at
Figure 1532: PRO84195
Figure 1533: DNA227709, NP_000947.1, 206631_at
Figure 1534: PRO38172
Figure 1535: DNA335452, NP_004891.3, 206632_s_at
Figure 1536: PRO89808
Figure 1537: DNA327666, 7688312.1, 206653_at
Figure 1538: PRO83656
Figure 1539: DNA88374, NP_002095.1, 206666_at
Figure 1540: PRO2768
Figure 1541: DNA334470, NP_536859.1, 206687_s_at
Figure 1542: PRO88974
Figure 1543: DNA328590, NP_056948.2, 206707_x_at
Figure 1544: PRO84375
Figure 1545: DNA340145, NP_036439.1, 206710_s_at
Figure 1546: PRO91644
Figure 1547: DNA340152, NP_055300.1, 206726_at
Figure 1548: PRO91651
Figure 1549: DNA226427, NP_002251.1, 206785_s_at
Figure 1550: PRO36890
Figure 1551: DNA88195, NP_000064.1, 206804_at
Figure 1552: PRO2693
Figure 1553: DNA272165, NP_003319.1, 206828_at

Figure 1658: PRO84511
Figure 1659: DNA336246, NP_001767.2, 207691_x_at
Figure 1660: PRO90415
Figure 1661A-B: DNA226405, NP_006525.1,
207700_s_at
Figure 1662: PRO36868
Figure 1663: DNA333631, NP_031359.1, 207723_s_at
Figure 1664: PRO88273
Figure 1665: DNA329064, NP_060301.1, 207735_at
Figure 1666: PRO84724
Figure 1667: DNA325654, NP_054752.1, 207761_s_at
Figure 1668: PRO4348
Figure 1669A-B: DNA329179, NP_056958.1,
207785_s_at
Figure 1670: PRO84802
Figure 1671: DNA329180, NP_004428.1, 207793_s_at
Figure 1672: PRO84803
Figure 1673: DNA329000, NM_000648, 207794_at
Figure 1674: PRO84690
Figure 1675: DNA227722, NP_002253.1, 207795_s_at
Figure 1676: PRO38185
Figure 1677: DNA329181, NM_007334, 207796_x_at
Figure 1678: PRO84804
Figure 1679: DNA227494, NP_002158.1, 207826_s_at
Figure 1680: PRO37957
Figure 1681A-C: DNA335409, NP_057427.2,
207828_s_at
Figure 1682: PRO89771
Figure 1683: DNA329182, NP_065385.2, 207838_x_at
Figure 1684: PRO84805
Figure 1685: DNA330123, NP_008984.1, 207840_at
Figure 1686: PRO35080
Figure 1687: DNA344401, NP_002179.2, 207844_at
Figure 1688: PRO95090
Figure 1689: DNA217244, U25676, 207849_at
Figure 1690: PRO34286
Figure 1691: DNA330124, NP_002981.2, 207861_at
Figure 1692: PRO34107
Figure 1693: DNA109234, NP_000065.1, 207892_at
Figure 1694: PRO6517
Figure 1695: DNA344402, NP_002978.1, 207900_at
Figure 1696: PRO1717
Figure 1697A-B: DNA150910, NP_005566.1,
207904_s_at
Figure 1698: PRO12536
Figure 1699: DNA344403, NP_000579.2, 207906_at
Figure 1700: PRO95091
Figure 1701: DNA344404, NP_000870.1, 207952_at
Figure 1702: PRO69
Figure 1703: DNA227067, X06318, 207957_s_at
Figure 1704: PRO37530
Figure 1705A-B: DNA344405, NP_008912.1,
207978_s_at
Figure 1706: PRO85386
Figure 1707A-C: DNA254145, NP_004329.1,
207996_s_at
Figure 1708: PRO49260
Figure 1709A-B: DNA226403, NP_000711.1,
207998_s_at
Figure 1710: PRO36866
Figure 1711: DNA344406, NM_012411, 208010_s_at
Figure 1712: PRO95092
Figure 1713: DNA324249, NM_004510, 208012_x_at
Figure 1714: PRO80933
Figure 1715: DNA333763, NM_021708, 208071_s_at
Figure 1716: PRO88387
Figure 1717A-C: DNA331500, NP_003307.2,
208073_x_at
Figure 1718: PRO86537
Figure 1719: DNA331501, D84212, 208079_s_at
Figure 1720: PRO58855
Figure 1721A-B: DNA344407, NP_110384.1,
208082_x_at
Figure 1722: PRO95093
Figure 1723: DNA344408, NP_112182.1, 208103_s_at
Figure 1724: PRO80638
Figure 1725A-B: DNA335356, NP_000952.1,
208131_s_at
Figure 1726: PRO25026
Figure 1727: DNA325329, NP_004719.1, 208152_s_at
Figure 1728: PRO81872
Figure 1729: DNA344409, NP_002177.1, 208164_s_at
Figure 1730: PRO64957
Figure 1731: DNA210622, NP_057009.1, 208190_s_at
Figure 1732: PRO35016
Figure 1733: DNA36717, NP_000581.1, 208193_at
Figure 1734: PRO72
Figure 1735: DNA328611, NP_005816.2, 208206_s_at
Figure 1736: PRO84393
Figure 1737: DNA344410, NP_071431.2, 208303_s_at
Figure 1738: PRO28725
Figure 1739: DNA196361, NP_001828.1, 208304_at
Figure 1740: PRO24864
Figure 1741: DNA344411, X12544, 208306_x_at
Figure 1742: PRO95094
Figure 1743A-B: DNA344412, NP_006776.1,
208309_s_at
Figure 1744: PRO9824
Figure 1745A-C: DNA344413, NP_006729.3,
208325_s_at
Figure 1746: PRO95095
Figure 1747: DNA344414, NP_003813.1, 208337_s_at
Figure 1748: PRO62964
Figure 1749: DNA344415, NM_003822, 208343_s_at
Figure 1750: PRO62964
Figure 1751: DNA329576, NM_002745, 208351_s_at
Figure 1752: PRO64127
Figure 1753: DNA344416, NM_020480, 208353_x_at
Figure 1754: PRO95096
Figure 1755: DNA344417, NP_008999.2, 208382_s_at
Figure 1756: PRO95097
Figure 1757: DNA324250, NP_536349.1, 208392_x_at

Figure 1758: PRO80934
Figure 1759A-B: DNA344418, NP_005723.2,
208393_s_at
Figure 1760: PRO86236
Figure 1761: DNA344419, NP_004801.1, 208406_s_at
Figure 1762: PRO12190
Figure 1763A-B: DNA331315, NP_004622.1,
208433_s_at
Figure 1764: PRO70090
Figure 1765: DNA327690, NP_004022.1, 208436_s_at
Figure 1766: PRO83673
Figure 1767A-C: DNA331504, NP_000042.2,
208442_s_at
Figure 1768: PRO86540
Figure 1769: DNA331327, NP_036382.2, 208456_s_at
Figure 1770: PRO86414
Figure 1771: DNA326738, NP_004315.1, 208478_s_at
Figure 1772: PRO38101
Figure 1773: DNA344420, NM_006260, 208499_s_at
Figure 1774: PRO11602
Figure 1775: DNA344421, NP_005281.1, 208524_at
Figure 1776: PRO54695
Figure 1777: DNA344422, NP_619527.1, 208536_s_at
Figure 1778: PRO95098
Figure 1779: DNA330045, NP_005943.1, 208581_x_at
Figure 1780: PRO82583
Figure 1781: DNA225836, NP_006716.1, 208602_x_at
Figure 1782: PRO36299
Figure 1783: DNA344423, NP_066301.1, 208608_s_at
Figure 1784: PRO23346
Figure 1785: DNA281431, NP_004550.1, 208628_s_at
Figure 1786: PRO66271
Figure 1787: DNA324641, NP_005608.1, 208646_at
Figure 1788: PRO10849
Figure 1789: DNA344424, NP_006007.2, 208653_s_at
Figure 1790: PRO95099
Figure 1791: DNA344425, U87954, 208676_s_at
Figure 1792: PRO95100
Figure 1793: DNA304686, NP_002565.1, 208680_at
Figure 1794: PRO71112
Figure 1795A-B: DNA328619, BC001188, 208691_at
Figure 1796: PRO84401
Figure 1797: DNA287189, NP_002038.1, 208693_s_at
Figure 1798: PRO69475
Figure 1799: DNA344426, NP_036205.1, 208696_at
Figure 1800: PRO81195
Figure 1801: DNA325127, NP_001559.1, 208697_s_at
Figure 1802: PRO81699
Figure 1803A-B: DNA325944, NP_001960.2,
208708_x_at
Figure 1804: PRO82391
Figure 1805: DNA344427, NP_061899.1, 208716_s_at
Figure 1806: PRO177
Figure 1807: DNA344428, NP_003899.1, 208726_s_at
Figure 1808: PRO95101
Figure 1809: DNA344429, NP_004879.1, 208737_at
Figure 1810: PRO61194
Figure 1811: DNA344430, NM_006476, 208745_at
Figure 1812: PRO95102
Figure 1813: DNA287285, NP_005794.1, 208748_s_at
Figure 1814: PRO69556
Figure 1815: DNA344431, NP_631946.1, 208754_s_at
Figure 1816: PRO71113
Figure 1817: DNA324217, NP_004035.2, 208758_at
Figure 1818: PRO80908
Figure 1819: DNA344432, NP_060877.1, 208767_s_at
Figure 1820: PRO37687
Figure 1821: DNA344433, NP_002806.2, 208777_s_at
Figure 1822: PRO95103
Figure 1823: DNA287219, NP_110379.1, 208778_s_at
Figure 1824: PRO69498
Figure 1825: DNA329189, NP_009139.1, 208787_at
Figure 1826: PRO4911
Figure 1827: DNA225671, NP_001822.1, 208791_at
Figure 1828: PRO36134
Figure 1829A-B: DNA344434, NP_055818.2,
208798_x_at
Figure 1830: PRO95104
Figure 1831: DNA330145, NP_002788.1, 208799_at
Figure 1832: PRO84403
Figure 1833A-C: DNA330146, 1397486.26, 208806_at
Figure 1834: PRO85404
Figure 1835: DNA273521, NP_002070.1, 208813_at
Figure 1836: PRO61502
Figure 1837: DNA327699, BAA75062.1, 208815_x_at
Figure 1838: PRO83682
Figure 1839: DNA344435, NP_002789.1, 208827_at
Figure 1840: PRO82662
Figure 1841A-B: DNA83031, NP_001737.1,
208852_s_at
Figure 1842: PRO2564
Figure 1843: DNA227874, NP_003320.1, 208864_s_at
Figure 1844: PRO38337
Figure 1845: DNA344436, NP_113600.1, 208869_s_at
Figure 1846: PRO95105
Figure 1847: DNA328624, BC003562, 208891_at
Figure 1848: PRO59076
Figure 1849: DNA270713, NP_001937.1, 208892_s_at
Figure 1850: PRO59076
Figure 1851: DNA328625, NM_022652, 208893_s_at
Figure 1852: PRO84404
Figure 1853: DNA329221, NP_061984.1, 208894_at
Figure 1854: PRO4555
Figure 1855A-B: DNA324910, NP_061820.1,
208905_at
Figure 1856: PRO81514
Figure 1857: DNA326260, NP_001203.1, 208910_s_at
Figure 1858: PRO82667
Figure 1859: DNA226500, NP_005619.1, 208916_at
Figure 1860: PRO36963
Figure 1861: DNA325473, NP_006353.2, 208922_s_at
Figure 1862: PRO81996

Figure 1863: DNA329552, NP_063948.1, 208925_at
Figure 1864: PRO85097
Figure 1865: DNA326233, NP_000968.2, 208929_x_at
Figure 1866: PRO82645
Figure 1867: DNA327702, NP_006490.2, 208934_s_at
Figure 1868: PRO83684
Figure 1869: DNA327702, NM_006499, 208936_x_at
Figure 1870: PRO83684
Figure 1871: DNA344437, NP_036379.1, 208941_s_at
Figure 1872: PRO70339
Figure 1873A-B: DNA344438, D50683, 208944_at
Figure 1874: PRO95106
Figure 1875: DNA325900, NP_002297.1, 208949_s_at
Figure 1876: PRO82356
Figure 1877: DNA327661, NP_005522.1, 208966_x_at
Figure 1878: PRO83652
Figure 1879A-B: DNA344439, NP_002256.2,
208974_x_at
Figure 1880: PRO82739
Figure 1881A-B: DNA330153, L38951, 208975_s_at
Figure 1882: PRO82739
Figure 1883: DNA328629, NP_006079.1, 208977_x_at
Figure 1884: PRO84407
Figure 1885: DNA329522, NP_000433.2, 208981_at
Figure 1886: PRO85080
Figure 1887: DNA330155, 7692317.2, 208982_at
Figure 1888: PRO85407
Figure 1889: DNA329522, NM_000442, 208983_s_at
Figure 1890: PRO85080
Figure 1891: DNA330156, NP_003749.1, 208985_s_at
Figure 1892: PRO85408
Figure 1893: DNA344440, NP_644805.1, 208991_at
Figure 1894: PRO95107
Figure 1895: DNA331514, NM_003150, 208992_s_at
Figure 1896: PRO86548
Figure 1897: DNA227552, NP_003346.2, 208997_s_at
Figure 1898: PRO38015
Figure 1899A-B: DNA344441, AAG09407.1,
208999_at
Figure 1900: PRO95108
Figure 1901: DNA328630, NP_036293.1, 209004_s_at
Figure 1902: PRO84408
Figure 1903: DNA328631, AK027318, 209006_s_at
Figure 1904: PRO84409
Figure 1905: DNA328632, NP_064713.2, 209007_s_at
Figure 1906: PRO84410
Figure 1907: DNA328633, NP_004784.2, 209017_s_at
Figure 1908: PRO84411
Figure 1909: DNA327706, NP_006363.3, 209024_s_at
Figure 1910: PRO83688
Figure 1911: DNA344442, AF279899, 209034_at
Figure 1912: PRO95109
Figure 1913: DNA274967, AF233453, 209049_s_at
Figure 1914: PRO62700
Figure 1915A-C: DNA344443, NP_579890.1,
209052_s_at
Figure 1916: PRO81109
Figure 1917A-B: DNA331518, NM_133336,
209053_s_at
Figure 1918: PRO86550
Figure 1919A-B: DNA226405, NM_006534,
209060_x_at
Figure 1920: PRO36868
Figure 1921A-C: DNA344444, 1394903.34, 209061_at
Figure 1922: PRO95110
Figure 1923A-B: DNA226405, AF036892,
209062_x_at
Figure 1924: PRO36868
Figure 1925: DNA330160, NP_006285.1, 209066_x_at
Figure 1926: PRO85412
Figure 1927: DNA329194, NP_112740.1, 209067_s_at
Figure 1928: PRO84814
Figure 1929A-B: DNA324473, NP_002904.2,
209084_s_at
Figure 1930: PRO81135
Figure 1931A-B: DNA273483, AB007960,
209090_s_at
Figure 1932: DNA324318, NP_006755.2, 209100_at
Figure 1933: PRO80995
Figure 1934: DNA330118, NP_036389.2, 209102_s_at
Figure 1935: PRO85380
Figure 1936: DNA330163, NP_060308.1, 209104_s_at
Figure 1937: PRO85415
Figure 1938A-B: DNA344445, 104805.26, 209105_at
Figure 1939: PRO95111
Figure 1940: DNA344446, NP_004055.1, 209112_at
Figure 1941: PRO95112
Figure 1942: DNA344447, BC005127, 209122_at
Figure 1943: PRO95113
Figure 1944: DNA344448, NM_176895, 209147_s_at
Figure 1945: PRO95114
Figure 1946: DNA330166, NP_004688.2, 209161_at
Figure 1947: PRO85418
Figure 1948: DNA344449, 1448768.1, 209163_at
Figure 1949: PRO95115
Figure 1950: DNA344450, NP_001906.1, 209164_s_at
Figure 1951: PRO57071
Figure 1952A-C: DNA270403, NM_016343,
209172_s_at
Figure 1953: PRO58786
Figure 1954: DNA329196, NP_004573.2, 209181_s_at
Figure 1955: PRO84815
Figure 1956A-B: DNA344451, NP_733765.1,
209186_at
Figure 1957: PRO84419
Figure 1958: DNA189700, NP_005243.1, 209189_at
Figure 1959: PRO25619
Figure 1960: DNA226176, NP_003458.1, 209201_x_at
Figure 1961: PRO36639
Figure 1962: DNA326267, NP_004861.1, 209208_at
Figure 1963: PRO82674
Figure 1964: DNA103439, NP_001111.2, 209215_at

Figure 1965: PRO4766
Figure 1966: DNA330168, NP_006322.1, 209233_at
Figure 1967: PRO85420
Figure 1968: DNA344452, NM_007189, 209247_s_at
Figure 1969: PRO95116
Figure 1970: DNA344453, BC004949, 209251_x_at
Figure 1971: PRO84424
Figure 1972: DNA255255, NP_071437.3, 209267_s_at
Figure 1973: PRO50332
Figure 1974: DNA328650, DNA328650, 209286_at
Figure 1975: PRO84425
Figure 1976A-B: DNA344454, NP_006440.2,
209288_s_at
Figure 1977: PRO95117
Figure 1978: DNA328651, AF087853, 209304_x_at
Figure 1979: PRO82889
Figure 1980: DNA344455, BC024654, 209305_s_at
Figure 1981: PRO95118
Figure 1982: DNA344456, NP_001216.1, 209310_s_at
Figure 1983: PRO37559
Figure 1984: DNA344457, U65585, 209312_x_at
Figure 1985: PRO95119
Figure 1986A-B: DNA344458, NP_006611.1,
209316_s_at
Figure 1987: PRO12057
Figure 1988: DNA344459, U94829, 209325_s_at
Figure 1989: PRO95120
Figure 1990: DNA329200, NP_005040.1, 209336_at
Figure 1991: PRO84817
Figure 1992: DNA275106, NP_005058.2, 209339_at
Figure 1993: PRO62821
Figure 1994: DNA328655, 346677.3, 209341_s_at
Figure 1995: PRO84429
Figure 1996: DNA227208, NM_005360, 209347_s_at
Figure 1997: PRO37671
Figure 1998A-B: DNA328658, AF055376,
209348_s_at
Figure 1999: PRO84432
Figure 2000: DNA330170, AF109161, 209357_at
Figure 2001: PRO84807
Figure 2002A-B: DNA344460, NP_001745.2,
209360_s_at
Figure 2003: PRO95121
Figure 2004A-C: DNA344461, NP_061872.1,
209379_s_at
Figure 2005: PRO95122
Figure 2006: DNA330173, NP_006200.2, 209392_at
Figure 2007: PRO85423
Figure 2008: DNA339326, NP_004273.1, 209406_at
Figure 2009: PRO91131
Figure 2010: DNA330175, NP_006836.1, 209408_at
Figure 2011: PRO59681
Figure 2012A-B: DNA344462, NM_133650,
209447_at
Figure 2013: PRO95123
Figure 2014: DNA330121, NM_004180, 209451_at
Figure 2015: PRO85383
Figure 2016: DNA344463, NP_065737.1, 209459_s_at
Figure 2017: PRO95124
Figure 2018: DNA344464, NM_020686, 209460_at
Figure 2019: PRO95125
Figure 2020: DNA287304, AAH00040.1, 209461_x_at
Figure 2021: PRO69571
Figure 2022A-B: DNA344465, 347965.2, 209473_at
Figure 2023: PRO95126
Figure 2024: DNA336246, NM_001776, 209474_s_at
Figure 2025: PRO90415
Figure 2026: DNA324976, NP_005828.1, 209482_at
Figure 2027: PRO81571
Figure 2028: DNA324899, NP_002938.1, 209507_at
Figure 2029: PRO81503
Figure 2030: DNA274027, NP_004571.2, 209514_s_at
Figure 2031: PRO61971
Figure 2032A-B: DNA344466, NM_144767,
209534_x_at
Figure 2033: PRO95127
Figure 2034: DNA344467, NM_139265, 209536_s_at
Figure 2035: PRO82426
Figure 2036: DNA274949, NP_008904.1, 209538_at
Figure 2037: PRO62684
Figure 2038A-B: DNA344468, NP_004831.1,
209539_at
Figure 2039: PRO83388
Figure 2040A-C: DNA335383, NP_000609.1,
209540_at
Figure 2041: PRO19618
Figure 2042A-C: DNA335383, NM_000618,
209541_at
Figure 2043: PRO19618
Figure 2044: DNA329201, NP_055984.1, 209567_at
Figure 2045: PRO84818
Figure 2046: DNA344469, NP_003788.2, 209572_s_at
Figure 2047: PRO40888
Figure 2048A-C: DNA254145, NM_004338,
209573_s_at
Figure 2049: PRO49260
Figure 2050: DNA344470, NP_002060.3, 209576_at
Figure 2051: PRO95128
Figure 2052: DNA304797, NP_005935.3, 209582_s_at
Figure 2053: PRO71209
Figure 2054: DNA304797, NM_005944, 209583_s_at
Figure 2055: PRO71209
Figure 2056: DNA344471, NP_004119.1, 209595_at
Figure 2057: PRO95129
Figure 2058: DNA270689, NP_002042.1, 209602_s_at
Figure 2059: PRO59053
Figure 2060: DNA344472, 412986.6, 209603_at
Figure 2061: PRO95130
Figure 2062: DNA270689, NM_002051, 209604_s_at
Figure 2063: PRO59053
Figure 2064: DNA330186, NP_004327.1, 209642_at
Figure 2065: PRO85434

Figure 2066: DNA323856, NP_056455.1, 209669_s_at
Figure 2067: PRO80599
Figure 2068A-B: DNA344473, NP_008927.1,
209681_at
Figure 2069: PRO23299
Figure 2070A-B: DNA344474, NM_170662,
209682_at
Figure 2071: PRO95131
Figure 2072: DNA328264, NP_005183.2, 209714_s_at
Figure 2073: PRO12087
Figure 2074A-B: DNA328594, M37435, 209716_at
Figure 2075: PRO84379
Figure 2076A-C: DNA254412, NP_005656.2,
209717_at
Figure 2077: PRO49522
Figure 2078: DNA227124, NP_005118.1, 209732_at
Figure 2079: PRO37587
Figure 2080: DNA344475, AF113682, 209753_s_at
Figure 2081: PRO95132
Figure 2082: DNA344476, U09088, 209754_s_at
Figure 2083: PRO95133
Figure 2084: DNA324250, NM_080424, 209761_s_at
Figure 2085: PRO80934
Figure 2086A-B: DNA328675, NM_033274,
209765_at
Figure 2087: PRO84447
Figure 2088: DNA329178, NP_008979.2, 209770_at
Figure 2089: PRO84801
Figure 2090: DNA275195, NP_001025.1, 209773_s_at
Figure 2091: PRO62893
Figure 2092A-B: DNA255050, NP_065165.1,
209780_at
Figure 2093: PRO50138
Figure 2094A-B: DNA344477, AF222340,
209788_s_at
Figure 2095: PRO95134
Figure 2096: DNA336284, NP_001217.2, 209790_s_at
Figure 2097: PRO90442
Figure 2098: DNA226436, NP_001772.1, 209795_at
Figure 2099: PRO36899
Figure 2100: DNA327731, NP_003302.1, 209803_s_at
Figure 2101: PRO83707
Figure 2102: DNA271384, AAA61110.1, 209813_x_at
Figure 2103: PRO59683
Figure 2104: DNA326100, NP_006444.2, 209820_s_at
Figure 2105: PRO82528
Figure 2106: DNA225992, NP_003374.1, 209822_s_at
Figure 2107: PRO36455
Figure 2108: DNA344478, M17955, 209823_x_at
Figure 2109: PRO95135
Figure 2110: DNA336282, NP_001169.2, 209824_s_at
Figure 2111: PRO61686
Figure 2112: DNA327732, NP_036606.2, 209825_s_at
Figure 2113: PRO61801
Figure 2114A-B: DNA196499, AB002384, 209829_at
Figure 2115: PRO24988
Figure 2116: DNA344479, L05424, 209835_x_at
Figure 2117: DNA344480, AAH35133.1, 209840_s_at
Figure 2118: PRO95136
Figure 2119: DNA329207, NM_018334, 209841_s_at
Figure 2120: PRO220
Figure 2121: DNA344481, BC012398, 209845_at
Figure 2122: PRO95137
Figure 2123: DNA324805, NP_008978.1, 209846_s_at
Figure 2124: PRO81419
Figure 2125: DNA272753, NP_005780.1, 209853_s_at
Figure 2126: PRO60864
Figure 2127: DNA344482, NP_006829.1, 209861_s_at
Figure 2128: PRO61513
Figure 2129A-B: DNA325767, NP_476510.1,
209876_at
Figure 2130: PRO82238
Figure 2131: DNA226120, NP_002997.1, 209879_at
Figure 2132: PRO36583
Figure 2133A-C: DNA194808, NP_003606.2,
209884_s_at
Figure 2134: PRO24078
Figure 2135A-B: DNA344483, NP_056305.1,
209889_at
Figure 2136: PRO95138
Figure 2137: DNA334335, NP_065726.1, 209891_at
Figure 2138: PRO80882
Figure 2139: DNA254936, NP_009164.1, 209917_s_at
Figure 2140: PRO50026
Figure 2141: DNA299884, AB040875, 209921_at
Figure 2142: PRO70858
Figure 2143: DNA226887, NP_002529.1, 209925_at
Figure 2144: PRO37350
Figure 2145: DNA150133, AAD01646.1, 209933_s_at
Figure 2146: PRO12219
Figure 2147: DNA336245, AF005775, 209939_x_at
Figure 2148: PRO91070
Figure 2149: DNA344484, NM_139266, 209969_s_at
Figure 2150: PRO83711
Figure 2151: DNA344485, AF116615, 209971_x_at
Figure 2152: DNA226658, NP_003736.1, 209999_x_at
Figure 2153: PRO37121
Figure 2154: DNA226658, NM_003745, 210001_s_at
Figure 2155: PRO37121
Figure 2156A-B: DNA344486, NM_173844,
210017_at
Figure 2157: PRO95140
Figure 2158A-B: DNA344487, NM_006785,
210018_x_at
Figure 2159: PRO9824
Figure 2160: DNA255921, NP_000725.1, 210031_at
Figure 2161: PRO50974
Figure 2162: DNA344488, NP_002159.1, 210046_s_at
Figure 2163: PRO82489
Figure 2164: DNA326809, NP_036244.2, 210052_s_at
Figure 2165: PRO83142
Figure 2166: DNA328285, NP_002745.1, 210059_s_at

Figure 2167: PRO84161
Figure 2168: DNA344489, NP_057580.1, 210075_at
Figure 2169: PRO50605
Figure 2170: DNA334812, NP_002028.1, 210105_s_at
Figure 2171: PRO4624
Figure 2172A-C: DNA344490, 348003.19, 210108_at
Figure 2173: PRO95141
Figure 2174: DNA254310, NP_055226.1, 210109_at
Figure 2175: PRO49421
Figure 2176: DNA270010, NP_002342.1, 210116_at
Figure 2177: PRO58405
Figure 2178: DNA344491, 7763479.63, 210136_at
Figure 2179: PRO95142
Figure 2180: DNA333697, NP_003641.2, 210140_at
Figure 2181: PRO88328
Figure 2182: DNA256015, NP_002182.1, 210141_s_at
Figure 2183: PRO51063
Figure 2184: DNA344492, NP_077734.1, 210145_at
Figure 2185: PRO90384
Figure 2186: DNA340737, NM_172390, 210162_s_at
Figure 2187: PRO92688
Figure 2188: DNA330202, NP_005400.1, 210163_at
Figure 2189: PRO19838
Figure 2190: DNA287620, NP_004122.1, 210164_at
Figure 2191: PRO2081
Figure 2192: DNA335084, 233354.1, 210174_at
Figure 2193: PRO89492
Figure 2194: DNA330203, NP_003755.1, 210190_at
Figure 2195: PRO85449
Figure 2196: DNA186230, NP_006599.1, 210191_s_at
Figure 2197: PRO21476
Figure 2198: DNA344493, NP_003773.1, 210205_at
Figure 2199: PRO1756
Figure 2200: DNA344494, NP_000749.2, 210229_s_at
Figure 2201: PRO2055
Figure 2202: DNA344495, NM_134470, 210233_at
Figure 2203: PRO88491
Figure 2204: DNA328690, NP_524145.1, 210240_s_at
Figure 2205: PRO59660
Figure 2206: DNA287333, NP_005283.1, 210279_at
Figure 2207: PRO69597
Figure 2208A-B: DNA270015, NP_003444.1,
210281_s_at
Figure 2209: PRO58410
Figure 2210A-C: DNA194808, NM_003615,
210286_s_at
Figure 2211: PRO24078
Figure 2212: DNA272137, NP_000309.1, 210296_s_at
Figure 2213: PRO60406
Figure 2214A-B: DNA188419, NP_002011.1,
210316_at
Figure 2215: PRO21767
Figure 2216: DNA329213, NP_219491.1, 210321_at
Figure 2217: PRO2313
Figure 2218: DNA225528, NP_000610.1, 210354_at
Figure 2219: PRO35991
Figure 2220: DNA330207, BC001131, 210387_at
Figure 2221: PRO85451
Figure 2222A-B: DNA330208, AF164622,
210425_x_at
Figure 2223: PRO85452
Figure 2224: DNA344496, NP_599022.1, 210426_x_at
Figure 2225: PRO95143
Figure 2226: DNA329215, NP_036224.1, 210439_at
Figure 2227: PRO7424
Figure 2228: DNA344497, NP_002552.2, 210448_s_at
Figure 2229: PRO95144
Figure 2230: DNA344498, NM_133484, 210458_s_at
Figure 2231: PRO86554
Figure 2232: DNA326589, NP_060192.1, 210463_x_at
Figure 2233: PRO82947
Figure 2234: DNA323856, NM_015640, 210466_s_at
Figure 2235: PRO80599
Figure 2236A-B: DNA274461, M37712, 210473_s_at
Figure 2237: PRO62367
Figure 2238: DNA344499, NM_134262, 210479_s_at
Figure 2239: PRO95145
Figure 2240: DNA256385, NP_004470.1, 210506_at
Figure 2241: PRO51426
Figure 2242: DNA344500, NP_003367.2, 210512_s_at
Figure 2243: PRO84827
Figure 2244: DNA344501, NP_002118.1, 210514_x_at
Figure 2245: PRO50891
Figure 2246: DNA270066, AF078844, 210524_x_at
Figure 2247: PRO58459
Figure 2248: DNA344502, AF010447, 210528_at
Figure 2249: PRO95146
Figure 2250: DNA344503, NP_003769.1, 210540_s_at
Figure 2251: PRO1109
Figure 2252A-B: DNA344504, NP_004546.1,
210555_s_at
Figure 2253: PRO82622
Figure 2254A-B: DNA344505, NM_173164,
210556_at
Figure 2255: PRO95147
Figure 2256: DNA344506, NM_172211, 210557_x_at
Figure 2257: PRO95148
Figure 2258: DNA344507, NM_033379, 210559_s_at
Figure 2259: PRO70806
Figure 2260: DNA344508, U97075, 210563_x_at
Figure 2261: PRO95149
Figure 2262: DNA329217, AAH03406.1, 210571_s_at
Figure 2263: PRO84828
Figure 2264: DNA344509, AF241788, 210574_s_at
Figure 2265: PRO95150
Figure 2266: DNA327808, NM_002970, 210592_s_at
Figure 2267: PRO83769
Figure 2268: DNA227722, NM_002262, 210606_x_at
Figure 2269: PRO38185
Figure 2270: DNA330210, U03858, 210607_at
Figure 2271: PRO126
Figure 2272: DNA150511, AF000425, 210629_x_at

Figure 2273: PRO11557
Figure 2274: DNA344510, NP_003692.1, 210643_at
Figure 2275: PRO1292
Figure 2276: DNA227153, NP_002278.1, 210644_s_at
Figure 2277: PRO37616
Figure 2278A-C: DNA330214, D83077, 210645_s_at
Figure 2279: PRO12135
Figure 2280: DNA290260, NP_036555.1, 210646_x_at
Figure 2281: PRO70385
Figure 2282: DNA256521, NP_038459.1, 210690_at
Figure 2283: PRO51556
Figure 2284: DNA329218, NM_014412, 210691_s_at
Figure 2285: PRO84829
Figure 2286A-B: DNA335356, NM_000961,
210702_s_at
Figure 2287: PRO25026
Figure 2288: DNA329023, NP_066925.1, 210715_s_at
Figure 2289: PRO209
Figure 2290: DNA344511, BC015818, 210732_s_at
Figure 2291: PRO95151
Figure 2292: DNA103245, NM_002350, 210754_s_at
Figure 2293: PRO4575
Figure 2294: DNA194819, NP_667341.1, 210763_x_at
Figure 2295: PRO24086
Figure 2296: DNA344512, NP_001307.2, 210766_s_at
Figure 2297: PRO83174
Figure 2298: DNA103572, D14705, 210844_x_at
Figure 2299: PRO4896
Figure 2300: DNA344513, Y09392, 210847_x_at
Figure 2301A-C: DNA329220, NM_000051,
210858_x_at
Figure 2302: PRO84830
Figure 2303: DNA188234, NP_000630.1, 210865_at
Figure 2304: PRO21942
Figure 2305: DNA228132, NM_024090, 210868_s_at
Figure 2306: PRO38595
Figure 2307: DNA344514, AF098641, 210916_s_at
Figure 2308: PRO95153
Figure 2309: DNA344515, NP_000061.1, 210944_s_at
Figure 2310: PRO38022
Figure 2311: DNA344516, NM_003711, 210946_at
Figure 2312: PRO95154
Figure 2313: DNA344517, AF294627, 210948_s_at
Figure 2314: PRO95155
Figure 2315: DNA344518, NP_004453.1, 210950_s_at
Figure 2316: PRO81644
Figure 2317: DNA274027, NM_004580, 210951_x_at
Figure 2318: PRO61971
Figure 2319: DNA336282, NM_001178, 210971_s_at
Figure 2320: PRO61686
Figure 2321A-B: DNA344519, NP_000595.1,
210973_s_at
Figure 2322: PRO34231
Figure 2323: DNA344520, U47674, 210980_s_at
Figure 2324: PRO95156
Figure 2325: DNA269888, NP_002073.1, 210981_s_at
Figure 2326: PRO58286
Figure 2327: DNA329221, NM_019111, 210982_s_at
Figure 2328: PRO4555
Figure 2329: DNA238565, NP_005907.2, 210983_s_at
Figure 2330: PRO39210
Figure 2331: DNA151825, NP_005891.1, 210993_s_at
Figure 2332: PRO12900
Figure 2333: DNA344521, NM_002184, 211000_s_at
Figure 2334: PRO85478
Figure 2335: DNA150135, NP_055202.1, 211005_at
Figure 2336: PRO12232
Figure 2337: DNA273498, L12723, 211015_s_at
Figure 2338: PRO61480
Figure 2339: DNA344522, BC002526, 211016_x_at
Figure 2340: PRO95157
Figure 2341A-C: DNA344523, NP_000480.2,
211022_s_at
Figure 2342: PRO95158
Figure 2343: DNA287198, NP_006073.1, 211058_x_at
Figure 2344: PRO69484
Figure 2345: DNA328698, NM_006153, 211063_s_at
Figure 2346: PRO12168
Figure 2347: DNA326974, NM_000967, 211073_x_at
Figure 2348: PRO83285
Figure 2349A-B: DNA235639, NP_000206.1,
211108_s_at
Figure 2350: PRO38866
Figure 2351: DNA304765, M30894, 211144_x_at
Figure 2352: PRO71178
Figure 2353: DNA196439, NM_003874, 211190_x_at
Figure 2354: PRO24934
Figure 2355: DNA344524, U96627, 211192_s_at
Figure 2356: PRO95159
Figure 2357: DNA330221, NP_056071.1, 211207_s_at
Figure 2358: PRO85460
Figure 2359: DNA270010, NM_002351, 211209_x_at
Figure 2360: PRO58405
Figure 2361: DNA344525, AF100539, 211210_x_at
Figure 2362: PRO95160
Figure 2363: DNA344526, AF100542, 211211_x_at
Figure 2364: PRO95161
Figure 2365: DNA151022, NM_001345, 211272_s_at
Figure 2366: PRO12096
Figure 2367: DNA344527, NM_004130, 211275_s_at
Figure 2368: PRO95162
Figure 2369A-B: DNA344528, NM_002600,
211302_s_at
Figure 2370: PRO10691
Figure 2371A-C: DNA328811, NM_002222,
211323_s_at
Figure 2372: PRO84551
Figure 2373A-B: DNA339333, NP_005537.3,
211339_s_at
Figure 2374: PRO91137
Figure 2375: DNA103395, U80737, 211352_s_at
Figure 2376: PRO4723

Figure 2377: DNA327754, NP_150634.1, 211367_s_at
Figure 2378: PRO4526
Figure 2379A-B: DNA339371, NP_054742.1,
211383_s_at
Figure 2380: PRO91176
Figure 2381: DNA327755, NP_115957.1, 211458_s_at
Figure 2382: PRO83725
Figure 2383: DNA93439, NM_006564, 211469_s_at
Figure 2384: PRO4515
Figure 2385: DNA324183, NM_001935, 211478_s_at
Figure 2386: PRO80881
Figure 2387: DNA344529, BC001173, 211501_s_at
Figure 2388: PRO62214
Figure 2389: DNA344530, NM_003376, 211527_x_at
Figure 2390: PRO69153
Figure 2391: DNA344531, NP_001005.1, 211542_x_at
Figure 2392: PRO95163
Figure 2393: DNA269888, NM_002082, 211543_s_at
Figure 2394: PRO58286
Figure 2395: DNA226578, NM_004354, 211559_s_at
Figure 2396: PRO37041
Figure 2397: DNA329031, NP_004890.2, 211566_x_at
Figure 2398: PRO84699
Figure 2399: DNA226255, NP_003047.1, 211576_s_at
Figure 2400: PRO36718
Figure 2401: DNA331572, AF000426, 211581_x_at
Figure 2402: PRO86585
Figure 2403: DNA196752, AF031136, 211583_x_at
Figure 2404: PRO25202
Figure 2405: DNA344532, NP_631958.1, 211597_s_at
Figure 2406: PRO95164
Figure 2407: DNA275389, M30448, 211623_s_at
Figure 2408: PRO63052
Figure 2409: DNA344533, M24668, 211633_x_at
Figure 2410: PRO95165
Figure 2411: DNA344534, L06101, 211641_x_at
Figure 2412: DNA344535, M17565, 211654_x_at
Figure 2413A-B: DNA103553, NM_000176,
211671_s_at
Figure 2414: PRO4880
Figure 2415A-B: DNA255619, AF054589,
211675_s_at
Figure 2416: PRO50682
Figure 2417: DNA188293, NP_000407.1, 211676_s_at
Figure 2418: PRO21787
Figure 2419: DNA327760, NP_114430.1, 211685_s_at
Figure 2420: PRO83729
Figure 2421: DNA88515, L41270, 211688_x_at
Figure 2422: PRO2390
Figure 2423: DNA344536, NM_000968, 211710_x_at
Figure 2424: PRO95168
Figure 2425: DNA344537, NM_178014, 211714_x_at
Figure 2426: PRO10347
Figure 2427A-B: DNA274117, NP_612356.1,
211721_s_at
Figure 2428: PRO62054
Figure 2429: DNA329225, NP_006486.2, 211742_s_at
Figure 2430: PRO84833
Figure 2431: DNA344538, NM_148976, 211746_x_at
Figure 2432: PRO81959
Figure 2433: DNA344539, NP_036454.1, 211747_s_at
Figure 2434: PRO95169
Figure 2435: DNA344540, BC021088, 211750_x_at
Figure 2436: PRO84424
Figure 2437: DNA324147, NP_005774.2, 211758_x_at
Figure 2438: PRO80848
Figure 2439: DNA344541, BC005974, 211760_s_at
Figure 2440: PRO95170
Figure 2441: DNA254725, NM_002266, 211762_s_at
Figure 2442: PRO49824
Figure 2443: DNA340145, NM_012307, 211776_s_at
Figure 2444: PRO91644
Figure 2445: DNA344542, NM_001561, 211786_at
Figure 2446: PRO2023
Figure 2447: DNA344543, NP_003627.1, 211791_s_at
Figure 2448: PRO62306
Figure 2449: DNA331536, AAA60662.1, 211796_s_at
Figure 2450: PRO86563
Figure 2451: DNA344544, NM_052827, 211804_s_at
Figure 2452: PRO95171
Figure 2453A-B: DNA225940, NP_000144.1,
211810_s_at
Figure 2454: PRO36403
Figure 2455A-B: DNA328707, AAF03782.1,
211828_s_at
Figure 2456: PRO84466
Figure 2457: DNA344545, NM_138763, 211833_s_at
Figure 2458: PRO95172
Figure 2459: DNA344546, NP_757351.1, 211839_s_at
Figure 2460: PRO95173
Figure 2461A-B: DNA188192, NP_006130.1,
211856_x_at
Figure 2462: PRO21704
Figure 2463A-B: DNA188192, NM_006139,
211861_x_at
Figure 2464: PRO21704
Figure 2465: DNA225836, NM_006725, 211893_x_at
Figure 2466: PRO36299
Figure 2467: DNA344547, U66146, 211900_x_at
Figure 2468: PRO95174
Figure 2469: DNA226176, NM_003467, 211919_s_at
Figure 2470: PRO36639
Figure 2471: DNA272286, NM_001752, 211922_s_at
Figure 2472: PRO60544
Figure 2473: DNA344548, 7762146.13, 211929_at
Figure 2474: PRO95175
Figure 2475A-B: DNA272195, D21262, 211951_at
Figure 2476: DNA325941, NP_005339.1, 211969_at
Figure 2477: PRO82388
Figure 2478: DNA344549, 474771.15, 211974_x_at
Figure 2479: PRO95176
Figure 2480A-B: DNA344550, BC047523, 211984_at

Figure 2481: PRO4904
Figure 2482A-B: DNA344551, 7698619.16,
211985_s_at
Figure 2483: PRO95177
Figure 2484A-C: DNA327765, 1390535.1, 211986_at
Figure 2485: PRO83732
Figure 2486: DNA344552, NP_291032.1, 211990_at
Figure 2487: PRO85469
Figure 2488: DNA324768, NM_033554, 211991_s_at
Figure 2489: PRO4884
Figure 2490: DNA326406, NP_005315.1, 211999_at
Figure 2491: PRO11403
Figure 2492: DNA287433, NP_006810.1, 212009_s_at
Figure 2493: PRO69690
Figure 2494: DNA88197, X66733, 212014_x_at
Figure 2495: PRO2694
Figure 2496A-D: DNA103461, NP_002408.2,
212020_s_at
Figure 2497: PRO4788
Figure 2498A-D: DNA103461, NM_002417,
212022_s_at
Figure 2499: PRO4788
Figure 2500A-D: DNA226463, X65551, 212023_s_at
Figure 2501: PRO36926
Figure 2502: DNA328709, BC004151, 212048_s_at
Figure 2503: PRO37676
Figure 2504A-B: DNA344553, 7697666.18, 212063_at
Figure 2505: PRO95178
Figure 2506A-D: DNA344554, BAA25496.2,
212065_s_at
Figure 2507: PRO95179
Figure 2508: DNA344555, NP_065800.1, 212096_s_at
Figure 2509: PRO95180
Figure 2510: DNA325009, NP_001744.2, 212097_at
Figure 2511: PRO81600
Figure 2512: DNA344556, AF055029, 212098_at
Figure 2513: PRO95181
Figure 2514: DNA344557, 7763517.13, 212099_at
Figure 2515: PRO95182
Figure 2516A-B: DNA150956, BAA06685.1,
212110_at
Figure 2517: PRO12560
Figure 2518: DNA344558, AF070622, 212124_at
Figure 2519: PRO95183
Figure 2520: DNA151008, BC014044, 212125_at
Figure 2521: PRO12837
Figure 2522: DNA330242, BC007034, 212185_x_at
Figure 2523: PRO85477
Figure 2524: DNA330243, NP_006207.1, 212190_at
Figure 2525: PRO2584
Figure 2526: DNA326233, NM_000977, 212191_x_at
Figure 2527: PRO82645
Figure 2528A-C: DNA330244, 253946.17, 212195_at
Figure 2529: PRO85478
Figure 2530: DNA328437, NM_005801, 212227_x_at
Figure 2531: PRO84271
Figure 2532: DNA151120, M61906, 212240_s_at
Figure 2533: PRO12179
Figure 2534A-B: DNA329229, 1345070.7, 212249_at
Figure 2535: PRO84835
Figure 2536: DNA329182, NM_020524, 212259_s_at
Figure 2537: PRO84805
Figure 2538A-B: DNA344559, 332723.7, 212290_at
Figure 2539: PRO95184
Figure 2540: DNA344560, AL833829, 212291_at
Figure 2541: DNA328719, BC012895, 212295_s_at
Figure 2542: PRO84475
Figure 2543A-B: DNA344561, AL832633, 212299_at
Figure 2544: PRO95186
Figure 2545A-B: DNA344562, 319543.9, 212314_at
Figure 2546: PRO95187
Figure 2547A-B: DNA124122, NP_005602.2,
212331_at
Figure 2548: PRO6323
Figure 2549A-B: DNA124122, NM_005611,
212332_at
Figure 2550: PRO6323
Figure 2551: DNA287190, CAB43217.1, 212333_at
Figure 2552: PRO69476
Figure 2553: DNA344563, BC017742, 212334_at
Figure 2554: PRO95188
Figure 2555A-B: DNA344564, 254170.1, 212335_at
Figure 2556: PRO2759
Figure 2557A-B: DNA255527, D50525, 212337_at
Figure 2558: DNA344565, BC040726, 212359_s_at
Figure 2559A-B: DNA269762, BAA25456.1,
212368_at
Figure 2560: PRO58171
Figure 2561A-B: DNA344566, BAA25518.1,
212370_x_at
Figure 2562: PRO95190
Figure 2563A-C: DNA330249, AAA99177.1,
212372_at
Figure 2564: PRO85482
Figure 2565A-C: DNA344567, 020294.13, 212386_at
Figure 2566: PRO95191
Figure 2567A-C: DNA328725, AB007923, 212390_at
Figure 2568A-B: DNA328549, NP_002897.1,
212397_at
Figure 2569: PRO84350
Figure 2570A-B: DNA328549, NM_002906,
212398_at
Figure 2571: PRO84350
Figure 2572A-B: DNA344568, AK074108, 212400_at
Figure 2573A-B: DNA330250, NP_060727.1,
212406_s_at
Figure 2574: PRO85483
Figure 2575: DNA254828, NP_056417.1, 212408_at
Figure 2576: PRO49923
Figure 2577: DNA344569, 1454838.10, 212412_at
Figure 2578: PRO95192
Figure 2579: DNA330251, NP_059965.1, 212430_at

Figure 2580: PRO85484
Figure 2581: DNA304655, NP_079472.1, 212434_at
Figure 2582: PRO71082
Figure 2583A-B: DNA344570, 481983.1, 212446_s_at
Figure 2584: PRO95193
Figure 2585: DNA344571, AF052178, 212458_at
Figure 2586: PRO95194
Figure 2587: DNA151348, DNA151348, 212463_at
Figure 2588: PRO11726
Figure 2589: DNA344572, 226098.35, 212472_at
Figure 2590: PRO95195
Figure 2591A-B: DNA330252, NP_055447.1,
212473_s_at
Figure 2592: PRO85485
Figure 2593A-B: DNA344573, D26069, 212476_at
Figure 2594A-C: DNA344574, NP_597677.1,
212483_at
Figure 2595: PRO95197
Figure 2596: DNA344575, 7762745.4, 212498_at
Figure 2597: PRO95198
Figure 2598: DNA344576, NP_005185.2, 212501_at
Figure 2599: PRO91094
Figure 2600A-B: DNA344577, NP_116193.1,
212502_at
Figure 2601: PRO84485
Figure 2602: DNA344578, 1307005.1, 212511_at
Figure 2603: PRO95199
Figure 2604A-B: DNA344579, BC036190, 212522_at
Figure 2605: PRO95200
Figure 2606: DNA328733, AF038183, 212527_at
Figure 2607: PRO84486
Figure 2608: DNA344580, AL080111, 212530_at
Figure 2609: PRO95201
Figure 2610A-C: DNA344581, NP_056111.1,
212538_at
Figure 2611: PRO95202
Figure 2612: DNA65407, DNA65407, 212558_at
Figure 2613: PRO1276
Figure 2614A-D: DNA328737, 148650.1, 212560_at
Figure 2615: PRO84490
Figure 2616A-B: DNA254958, AL117448, 212561_at
Figure 2617: DNA344582, NP_056016.1, 212563_at
Figure 2618: PRO81715
Figure 2619: DNA344583, BC039084, 212568_s_at
Figure 2620: PRO95203
Figure 2621A-C: DNA331128, NP_065892.1,
212582_at
Figure 2622: PRO84841
Figure 2623A-B: DNA333749, NP_002829.2,
212587_s_at
Figure 2624: PRO88374
Figure 2625: DNA275100, DNA275100, 212589_at
Figure 2626: DNA331327, NM_012250, 212590_at
Figure 2627: PRO86414
Figure 2628: DNA331298, NM_014456, 212593_s_at
Figure 2629: PRO81909
Figure 2630: DNA272928, NP_055579.1, 212595_s_at
Figure 2631: PRO61012
Figure 2632: DNA344584, 253648.3, 212613_at
Figure 2633: PRO95204
Figure 2634A-B: DNA330258, BAA22955.2,
212619_at
Figure 2635: PRO85490
Figure 2636A-B: DNA344585, AL833311, 212621_at
Figure 2637: PRO95205
Figure 2638: DNA194679, BAA05062.1, 212623_at
Figure 2639: PRO23989
Figure 2640: DNA344586, AL050082, 212637_s_at
Figure 2641: PRO95206
Figure 2642A-C: DNA344587, NP_006725.2,
212641_at
Figure 2643: PRO95207
Figure 2644A-C: DNA344588, NM_006734,
212642_s_at
Figure 2645: PRO95208
Figure 2646: DNA329031, NM_004899, 212645_x_at
Figure 2647: PRO84699
Figure 2648: DNA344589, NP_000568.1, 212657_s_at
Figure 2649: PRO83789
Figure 2650A-B: DNA344590, D87076, 212660_at
Figure 2651: DNA344591, L34089, 212671_s_at
Figure 2652A-D: DNA344592, 032872.20, 212672_at
Figure 2653: PRO84830
Figure 2654: DNA344593, AF515797, 212681_at
Figure 2655A-B: DNA329901, BAA32291.2,
212683_at
Figure 2656: PRO85218
Figure 2657: DNA272355, L38935, 212697_at
Figure 2658: DNA326234, NM_033251, 212734_x_at
Figure 2659: PRO82646
Figure 2660: DNA290267, NP_005000.1, 212739_s_at
Figure 2661: PRO70399
Figure 2662A-B: DNA327779, 363462.9, 212741_at
Figure 2663: PRO83744
Figure 2664A-B: DNA273398, NM_015568,
212750_at
Figure 2665: PRO61398
Figure 2666A-B: DNA344594, NP_751911.1,
212757_s_at
Figure 2667: PRO95212
Figure 2668: DNA344595, AAH34232.1, 212771_at
Figure 2669: PRO95213
Figure 2670A-C: DNA344596, AB029032, 212779_at
Figure 2671: DNA290260, NM_012423, 212790_x_at
Figure 2672: PRO70385
Figure 2673A-B: DNA150479, BAA74900.1,
212792_at
Figure 2674: PRO12281
Figure 2675A-B: DNA344597, NP_055894.1,
212796_s_at
Figure 2676: PRO95215
Figure 2677: DNA328750, 7689361.1, 212812_at

Figure 2678: PRO84500
Figure 2679A-C: DNA336121, AB020663, 212820_at
Figure 2680A-B: DNA344598, BAB84995.1,
212823_s_at
Figure 2681: PRO95216
Figure 2682: DNA330171, CAA34971.1, 212827_at
Figure 2683: PRO85421
Figure 2684: DNA344599, 234498.36, 212847_at
Figure 2685: PRO95217
Figure 2686: DNA344600, AL713742, 212886_at
Figure 2687: PRO95218
Figure 2688: DNA344601, 989341.96, 212906_at
Figure 2689: PRO85986
Figure 2690: DNA271630, DNA271630, 212907_at
Figure 2691: DNA272939, NP_064582.1, 212922_s_at
Figure 2692: PRO61023
Figure 2693: DNA344602, BC045715, 212923_s_at
Figure 2694A-B: DNA344603, AB011164,
212929_s_at
Figure 2695A-B: DNA272008, BAA06684.1,
212932_at
Figure 2696: PRO60283
Figure 2697: DNA344604, NP_056156.2, 212949_at
Figure 2698: PRO80842
Figure 2699: DNA255330, AL359588, 212959_s_at
Figure 2700: DNA344605, U66042, 212961_x_at
Figure 2701: PRO50485
Figure 2702: DNA325417, NP_001742.1, 212971_at
Figure 2703: PRO69635
Figure 2704A-B: DNA344606, 474311.10, 212985_at
Figure 2705: PRO95220
Figure 2706: DNA344607, NM_147156, 212989_at
Figure 2707: PRO50467
Figure 2708: DNA344608, BC038387, 213010_at
Figure 2709A-C: DNA327783, DNA327783,
213015_at
Figure 2710: PRO83747
Figure 2711A-B: DNA253815, BAA20833.2,
213035_at
Figure 2712: PRO49218
Figure 2713A-B: DNA344609, NM_174953,
213036_x_at
Figure 2714: PRO95221
Figure 2715: DNA344610, NP_699172.1, 213038_at
Figure 2716: PRO95222
Figure 2717A-B: DNA329242, BAA76857.1,
213056_at
Figure 2718: PRO84847
Figure 2719: DNA323879, NP_003991.1, 213060_s_at
Figure 2720: PRO80622
Figure 2721A-C: DNA328757, 475076.9, 213069_at
Figure 2722: PRO84506
Figure 2723: DNA150837, CAA06743.1, 213083_at
Figure 2724: PRO12495
Figure 2725: DNA344611, NP_000975.2, 213084_x_at
Figure 2726: PRO95223
Figure 2727A-B: DNA331353, BAA76818.1,
213092_x_at
Figure 2728: PRO60758
Figure 2729: DNA270466, M12996, 213093_at
Figure 2730A-B: DNA339968, BAA76825.1,
213111_at
Figure 2731: PRO91476
Figure 2732: DNA330215, NP_060081.1, 213113_s_at
Figure 2733: PRO24295
Figure 2734: DNA326217, NP_004474.1, 213129_s_at
Figure 2735: PRO82630
Figure 2736: DNA344612, NM_006806, 213134_x_at
Figure 2737: PRO95224
Figure 2738: DNA287230, AAA36325.1, 213138_at
Figure 2739: PRO69509
Figure 2740: DNA330277, CAB45152.1, 213142_x_at
Figure 2741: PRO85506
Figure 2742A-B: DNA344613, 1330122.30, 213164_at
Figure 2743: PRO95225
Figure 2744: DNA344614, X17568, 213175_s_at
Figure 2745: PRO95226
Figure 2746: DNA344615, AF279370, 213186_at
Figure 2747: DNA344616, NP_705833.1, 213188_s_at
Figure 2748: PRO95227
Figure 2749: DNA339710, NP_116167.3, 213189_at
Figure 2750: PRO91439
Figure 2751: DNA344617, K02885, 213193_x_at
Figure 2752: DNA344618, 1501943.6, 213206_at
Figure 2753: PRO95229
Figure 2754: DNA344619, 1398007.8, 213226_at
Figure 2755: PRO95230
Figure 2756A-B: DNA344620, NP_065186.2,
213238_at
Figure 2757: PRO95231
Figure 2758A-B: DNA194850, BAA25458.1,
213243_at
Figure 2759: PRO24112
Figure 2760A-C: DNA344621, BAA20800.2,
213261_at
Figure 2761: PRO59767
Figure 2762A-B: DNA344622, AY217548, 213281_at
Figure 2763: PRO4671
Figure 2764: DNA260974, NP_006065.1, 213293_s_at
Figure 2765: PRO54720
Figure 2766A-B: DNA329248, BAA20816.1,
213302_at
Figure 2767: PRO84850
Figure 2768A-B: DNA331295, NM_002719,
213305_s_at
Figure 2769: PRO86394
Figure 2770A-B: DNA344623, NP_055999.1,
213309_at
Figure 2771: PRO95232
Figure 2772: DNA344624, AY074889, 213315_x_at
Figure 2773: PRO95233
Figure 2774: DNA344625, BC020923, 213317_at

Figure 2775: PRO95234
Figure 2776: DNA344626, AAH19339.1, 213320_at
Figure 2777: PRO95235
Figure 2778A-B: DNA344627, AF022789,
213327_s_at
Figure 2779: DNA287433, NM_006819, 213330_s_at
Figure 2780: PRO69690
Figure 2781A-B: DNA274793, BAA96028.1,
213365_at
Figure 2782: PRO62559
Figure 2783: DNA324853, NP_001007.2, 213377_x_at
Figure 2784: PRO81462
Figure 2785: DNA344628, 222320.2, 213385_at
Figure 2786: PRO95237
Figure 2787A-B: DNA344629, 7697344.6, 213416_at
Figure 2788: PRO95238
Figure 2789A-B: DNA331398, DNA331398,
213457_at
Figure 2790: PRO83924
Figure 2791A-B: DNA330285, 241020.1, 213469_at
Figure 2792: PRO85513
Figure 2793A-B: DNA344630, NP_055917.1,
213471_at
Figure 2794: PRO95239
Figure 2795: DNA328766, NP_006077.1, 213476_x_at
Figure 2796: PRO84514
Figure 2797A-B: DNA344631, NM_002265,
213507_s_at
Figure 2798: PRO82739
Figure 2799: DNA326639, NP_001229.1, 213523_at
Figure 2800: PRO82992
Figure 2801: DNA324005, NP_056529.1, 213524_s_at
Figure 2802: PRO11582
Figure 2803: DNA344632, BC022977, 213530_at
Figure 2804A-B: DNA344633, 062042.23,
213531_s_at
Figure 2805: PRO95240
Figure 2806: DNA254264, NP_689960.1, 213546_at
Figure 2807: PRO49375
Figure 2808: DNA344634, NM_144781, 213581_at
Figure 2809: PRO95241
Figure 2810: DNA344635, AAH15899.1, 213587_s_at
Figure 2811: PRO95242
Figure 2812: DNA326426, NP_004300.1, 213606_s_at
Figure 2813: PRO61246
Figure 2814A-C: DNA330292, NP_056045.2,
213618_at
Figure 2815: PRO85519
Figure 2816: DNA344636, BC045542, 213623_at
Figure 2817: PRO95243
Figure 2818: DNA344637, NP_005940.1, 213629_x_at
Figure 2819: PRO95244
Figure 2820: DNA326239, NP_006752.1, 213655_at
Figure 2821: PRO39530
Figure 2822: DNA325704, NM_004990, 213671_s_at
Figure 2823: PRO82188
Figure 2824: DNA344638, AK057596, 213703_at
Figure 2825: PRO95245
Figure 2826: DNA328629, NM_006088, 213726_x_at
Figure 2827: PRO84407
Figure 2828: DNA334387, NP_075563.2, 213727_x_at
Figure 2829: PRO88903
Figure 2830A-B: DNA344639, NP_036467.2,
213733_at
Figure 2831: PRO95246
Figure 2832: DNA326273, NM_001970, 213757_at
Figure 2833: PRO82678
Figure 2834: DNA327804, AF442151, 213797_at
Figure 2835: PRO69493
Figure 2836A-B: DNA344640, 7684018.188,
213803_at
Figure 2837: PRO95247
Figure 2838: DNA344641, 233172.5, 213852_at
Figure 2839: PRO95248
Figure 2840: DNA344642, 026641.16, 213888_s_at
Figure 2841: PRO95249
Figure 2842: DNA272347, NP_001011.1, 213890_x_at
Figure 2843: PRO60603
Figure 2844: DNA151041, X66087, 213906_at
Figure 2845: DNA333671, NP_005592.1, 213915_at
Figure 2846: PRO37543
Figure 2847: DNA327806, 242985.1, 213929_at
Figure 2848: PRO83767
Figure 2849: DNA344643, 1454455.7, 213931_at
Figure 2850: PRO95250
Figure 2851A-D: DNA339387, NM_014810,
213956_at
Figure 2852: PRO91192
Figure 2853: DNA344644, BC033755, 213958_at
Figure 2854: PRO95251
Figure 2855: DNA226014, NP_000230.1, 213975_s_at
Figure 2856: PRO36477
Figure 2857: DNA344645, AL050290, 213988_s_at
Figure 2858: PRO95252
Figure 2859: DNA344646, AF305069, 213996_at
Figure 2860: PRO86433
Figure 2861: DNA329136, NM_016391, 214011_s_at
Figure 2862: PRO84772
Figure 2863: DNA150990, NM_003641, 214022_s_at
Figure 2864: PRO12570
Figure 2865: DNA344647, BC013297, 214049_x_at
Figure 2866: PRO84853
Figure 2867: DNA330298, NP_005403.2, 214095_at
Figure 2868: PRO83772
Figure 2869: DNA330298, NM_005412, 214096_s_at
Figure 2870: PRO83772
Figure 2871: DNA344648, L43578, 214112_s_at
Figure 2872: DNA344649, NP_005096.1, 214113_s_at
Figure 2873: PRO37600
Figure 2874: DNA344650, 127586.127, 214129_at
Figure 2875: PRO95254
Figure 2876: DNA344651, 1500085.15, 214163_at

Figure 2877: PRO95255
Figure 2878: DNA344652, 236569.38, 214169_at
Figure 2879: PRO95256
Figure 2880: DNA329182, BC016852, 214177_s_at
Figure 2881: PRO84805
Figure 2882A-B: DNA269826, NP_003195.1,
214179_s_at
Figure 2883: PRO58228
Figure 2884: DNA344653, NM_000391, 214196_s_at
Figure 2885: PRO95257
Figure 2886: DNA331361, NP_003318.1, 214228_x_at
Figure 2887: PRO2398
Figure 2888: DNA344654, 264912.4, 214241_at
Figure 2889: PRO95258
Figure 2890: DNA344655, 202212.8, 214329_x_at
Figure 2891: PRO95259
Figure 2892: DNA344656, NP_203524.1, 214352_s_at
Figure 2893: PRO95260
Figure 2894: DNA304680, NM_007355, 214359_s_at
Figure 2895: PRO71106
Figure 2896: DNA273138, NP_005495.1, 214390_s_at
Figure 2897: PRO61182
Figure 2898: DNA344657, AK097004, 214402_s_at
Figure 2899: PRO95261
Figure 2900: DNA287630, NP_000160.1, 214430_at
Figure 2901: PRO2154
Figure 2902: DNA344658, BC039858, 214435_x_at
Figure 2903: PRO12184
Figure 2904A-B: DNA344659, NP_036213.1,
214446_at
Figure 2905: PRO37794
Figure 2906: DNA331744, NP_001326.2, 214450_at
Figure 2907: PRO1574
Figure 2908: DNA327812, NP_006408.2, 214453_s_at
Figure 2909: PRO83773
Figure 2910: DNA150971, NP_002249.1, 214470_at
Figure 2911: PRO12564
Figure 2912: DNA329253, NP_006128.1, 214551_s_at
Figure 2913: PRO84853
Figure 2914: DNA80218, U23772, 214567_s_at
Figure 2915: PRO1610
Figure 2916: DNA344660, AF001892, 214657_s_at
Figure 2917: PRO95262
Figure 2918: DNA330303, BAA05499.1, 214662_at
Figure 2919: PRO85528
Figure 2920: DNA328785, NP_004062.1, 214683_s_at
Figure 2921: PRO84531
Figure 2922: DNA344661, NP_006622.1, 214686_at
Figure 2923: PRO95263
Figure 2924A-B: DNA344662, AB002326,
214707_x_at
Figure 2925: DNA344663, AB046861, 214723_x_at
Figure 2926A-B: DNA334132, BAB21826.1,
214724_at
Figure 2927: PRO88686
Figure 2928A-B: DNA344664, 350410.3, 214787_at
Figure 2929: PRO95266
Figure 2930: DNA339733, NP_612411.2, 214791_at
Figure 2931: PRO91461
Figure 2932A-B: DNA344665, AAH42045.1,
214855_s_at
Figure 2933: PRO95267
Figure 2934A-E: DNA344666, L39064, 214950_at
Figure 2935: DNA344667, NP_009198.3, 214958_s_at
Figure 2936: PRO95269
Figure 2937A-B: DNA344668, NP_003023.1,
214971_s_at
Figure 2938: PRO54745
Figure 2939: DNA344669, NP_003819.1, 214975_s_at
Figure 2940: PRO95270
Figure 2941: DNA327532, NM_002065, 215001_s_at
Figure 2942: PRO71134
Figure 2943: DNA344670, U90551, 215071_s_at
Figure 2944: PRO85534
Figure 2945: DNA344671, 212023.3, 215100_at
Figure 2946: PRO23679
Figure 2947: DNA344672, 350922.19, 215133_s_at
Figure 2948: PRO95271
Figure 2949: DNA344673, AAH20773.1, 215136_s_at
Figure 2950: PRO84861
Figure 2951: DNA273371, NP_000364.1, 215165_x_at
Figure 2952: PRO61373
Figure 2953: DNA324015, NM_006335, 215171_s_at
Figure 2954: PRO80735
Figure 2955: DNA344674, NP_056420.1, 215172_at
Figure 2956: PRO95272
Figure 2957A-B: DNA150496, AB023212, 215175_at
Figure 2958: DNA324269, NP_006345.1, 215273_s_at
Figure 2959: PRO80952
Figure 2960A-B: DNA255050, NM_020432,
215286_s_at
Figure 2961: PRO50138
Figure 2962: DNA254588, AL049782, 215318_at
Figure 2963: DNA344675, 7763519.36, 215338_s_at
Figure 2964: PRO95273
Figure 2965: DNA336791, BC027954, 215345_x_at
Figure 2966: PRO90861
Figure 2967: DNA327831, NP_076956.1, 215380_s_at
Figure 2968: PRO83783
Figure 2969: DNA331570, AAH15794.1, 215440_s_at
Figure 2970: PRO84545
Figure 2971: DNA344676, NM_152876, 215719_x_at
Figure 2972: PRO95274
Figure 2973: DNA273821, X98258, 215731_s_at
Figure 2974: DNA344677, NP_000944.1, 215894_at
Figure 2975: PRO95275
Figure 2976: DNA330324, NP_002720.1, 215933_s_at
Figure 2977: PRO58034
Figure 2978: DNA344678, 1452291.4, 216133_at
Figure 2979: PRO23844
Figure 2980: DNA344679, AAA61033.1, 216191_s_at
Figure 2981: PRO95276

Figure 2982A-B: DNA344680, NM_015184,
216218_s_at
Figure 2983: PRO95277
Figure 2984: DNA344681, NM_173172, 216248_s_at
Figure 2985: PRO95278
Figure 2986: DNA326994, NP_055955.1, 216251_s_at
Figure 2987: PRO83301
Figure 2988: DNA344682, NM_152873, 216252_x_at
Figure 2989: PRO95279
Figure 2990A-C: DNA270933, NM_006766,
216361_s_at
Figure 2991: PRO59265
Figure 2992: DNA344683, X80821, 216563_at
Figure 2993: DNA287243, NP_004452.1, 216602_s_at
Figure 2994: PRO69518
Figure 2995A-C: DNA150435, NP_055444.1,
216620_s_at
Figure 2996: PRO12247
Figure 2997: DNA226699, NM_000022, 216705_s_at
Figure 2998: PRO37162
Figure 2999: DNA344684, BC026029, 216804_s_at
Figure 3000: PRO95280
Figure 3001: DNA329135, NP_002913.2, 216834_at
Figure 3002: PRO58102
Figure 3003: DNA227597, NP_000627.1, 216841_s_at
Figure 3004: PRO38060
Figure 3005: DNA344685, L76665, 216907_x_at
Figure 3006: PRO95281
Figure 3007: DNA328810, NM_001779, 216942_s_at
Figure 3008: PRO2557
Figure 3009A-C: DNA103378, U23850, 216944_s_at
Figure 3010: PRO4708
Figure 3011: DNA275181, NM_003090, 216977_x_at
Figure 3012: PRO62882
Figure 3013: DNA344686, NP_543157.1, 217025_s_at
Figure 3014: PRO95282
Figure 3015: DNA331366, L06797, 217028_at
Figure 3016: PRO4516
Figure 3017: DNA329073, NP_004830.1, 217080_s_at
Figure 3018: PRO84731
Figure 3019A-B: DNA328813, BAA76774.1,
217118_s_at
Figure 3020: PRO84553
Figure 3021: DNA227752, NM_001504, 217119_s_at
Figure 3022: PRO38215
Figure 3023A-B: DNA329269, BAA32292.2,
217122_s_at
Figure 3024: PRO84865
Figure 3025: DNA340209, NP_114093.1, 217123_x_at
Figure 3026: PRO91704
Figure 3027: DNA344687, NP_001893.2, 217127_at
Figure 3028: PRO84866
Figure 3029: DNA103549, M21624, 217143_s_at
Figure 3030: PRO4876
Figure 3031: DNA227786, NP_057472.1, 217147_s_at
Figure 3032: PRO38249
Figure 3033: DNA344688, NM_005949, 217165_x_at
Figure 3034: PRO95283
Figure 3035: DNA344689, NM_176786, 217212_s_at
Figure 3036: PRO95284
Figure 3037: DNA344690, D84140, 217235_x_at
Figure 3038: DNA151105, NP_005601.1, 217301_x_at
Figure 3039: PRO12857
Figure 3040: DNA344691, X69383, 217381_s_at
Figure 3041: PRO95286
Figure 3042: DNA344692, D13079, 217394_at
Figure 3043: PRO95287
Figure 3044: DNA344693, BC047570, 217403_s_at
Figure 3045: PRO95288
Figure 3046: DNA344694, 7697666.21, 217523_at
Figure 3047: PRO95289
Figure 3048: DNA344695, 023453.1, 217540_at
Figure 3049: PRO95290
Figure 3050: DNA344696, 346253.1, 217550_at
Figure 3051: PRO95291
Figure 3052: DNA344697, AK074970, 217724_at
Figure 3053: PRO95292
Figure 3054: DNA323856, AL080119, 217725_x_at
Figure 3055: PRO80599
Figure 3056: DNA325832, NP_068839.1, 217731_s_at
Figure 3057: PRO1869
Figure 3058: DNA325832, NM_021999, 217732_s_at
Figure 3059: PRO1869
Figure 3060A-B: DNA327847, 142131.14, 217738_at
Figure 3061: PRO2834
Figure 3062: DNA88541, NP_005737.1, 217739_s_at
Figure 3063: PRO2834
Figure 3064: DNA227205, NP_071404.1, 217744_s_at
Figure 3065: PRO37668
Figure 3066: DNA344698, NP_057001.1, 217751_at
Figure 3067: PRO95293
Figure 3068: DNA325910, NP_057110.2, 217776_at
Figure 3069: PRO82365
Figure 3070: DNA328819, NP_057145.1, 217783_s_at
Figure 3071: PRO84557
Figure 3072: DNA325873, NP_006100.2, 217786_at
Figure 3073: PRO82331
Figure 3074A-B: DNA254292, NP_004472.1,
217787_s_at
Figure 3075: PRO49403
Figure 3076A-B: DNA254292, NM_004481,
217788_s_at
Figure 3077: PRO49403
Figure 3078: DNA344699, NP_005709.1, 217818_s_at
Figure 3079: PRO80955
Figure 3080: DNA344700, BC032643, 217832_at
Figure 3081: PRO95294
Figure 3082: DNA344701, BC040844, 217834_s_at
Figure 3083: PRO95295
Figure 3084: DNA328823, NP_057421.1, 217838_s_at
Figure 3085: PRO84561
Figure 3086: DNA344702, NP_066952.1, 217848_s_at

Figure 3087: PRO11669
Figure 3088A-B: DNA324921, NP_073585.6,
217853_at
Figure 3089: PRO81523
Figure 3090: DNA344703, NP_002686.2, 217854_s_at
Figure 3091: PRO95296
Figure 3092: DNA344704, NP_060904.1, 217865_at
Figure 3093: PRO95297
Figure 3094: DNA335592, NP_036237.2, 217867_x_at
Figure 3095: PRO852
Figure 3096: DNA344705, NP_001247.2, 217879_at
Figure 3097: PRO95298
Figure 3098: DNA255145, NP_060917.1, 217882_at
Figure 3099: PRO50225
Figure 3100A-B: DNA325652, NP_057441.1,
217892_s_at
Figure 3101: PRO82143
Figure 3102: DNA330345, NP_055130.1, 217906_at
Figure 3103: PRO85566
Figure 3104: DNA328826, NP_004272.2, 217911_s_at
Figure 3105: PRO84564
Figure 3106: DNA344706, NP_751918.1, 217919_s_at
Figure 3107: PRO95299
Figure 3108: DNA287241, NP_056991.1, 217933_s_at
Figure 3109: PRO69516
Figure 3110A-B: DNA225648, NP_061165.1,
217941_s_at
Figure 3111: PRO36111
Figure 3112: DNA326730, NP_057037.1, 217950_at
Figure 3113: PRO83072
Figure 3114: DNA329273, NP_037374.1, 217957_at
Figure 3115: PRO84869
Figure 3116A-B: DNA272661, NP_443198.1,
217966_s_at
Figure 3117: PRO60787
Figure 3118A-B: DNA272661, NM_052966,
217967_s_at
Figure 3119: PRO60787
Figure 3120: DNA329546, NP_055214.1, 217979_at
Figure 3121: PRO296
Figure 3122: DNA227218, NP_003721.2, 217983_s_at
Figure 3123: PRO37681
Figure 3124: DNA227218, NM_003730, 217984_at
Figure 3125: PRO37681
Figure 3126: DNA328831, NP_057329.1, 217989_at
Figure 3127: PRO233
Figure 3128: DNA344707, NP_663768.1, 217991_x_at
Figure 3129: PRO95300
Figure 3130: DNA328832, NP_067022.1, 217995_at
Figure 3131: PRO84568
Figure 3132: DNA328833, BC018929, 217996_at
Figure 3133: PRO84569
Figure 3134: DNA328834, AF220656, 217997_at
Figure 3135: DNA287364, NP_031376.1, 218000_s_at
Figure 3136: PRO69625
Figure 3137: DNA326005, NP_057004.1, 218007_s_at
Figure 3138: PRO82446
Figure 3139: DNA273008, NP_003972.1, 218009_s_at
Figure 3140: PRO61079
Figure 3141: DNA339506, NP_060589.1, 218016_s_at
Figure 3142: PRO91277
Figure 3143: DNA325094, NP_079346.1, 218017_s_at
Figure 3144: PRO81671
Figure 3145: DNA328836, NP_054894.1, 218027_at
Figure 3146: PRO84572
Figure 3147A-B: DNA255183, NP_061900.1,
218035_s_at
Figure 3148: PRO50262
Figure 3149: DNA325978, NM_016359, 218039_at
Figure 3150: PRO82423
Figure 3151: DNA329276, NP_077001.1, 218069_at
Figure 3152: PRO12104
Figure 3153: DNA287261, NP_060344.1, 218081_at
Figure 3154: PRO69533
Figure 3155: DNA325169, NP_057494.2, 218085_at
Figure 3156: PRO81734
Figure 3157: DNA344708, NP_056207.2, 218086_at
Figure 3158: PRO95301
Figure 3159: DNA329278, NP_004495.1, 218092_s_at
Figure 3160: PRO84871
Figure 3161: DNA225639, NP_060831.1, 218096_at
Figure 3162: PRO36102
Figure 3163: DNA344709, NP_004540.1, 218101_s_at
Figure 3164: PRO82036
Figure 3165: DNA344710, NP_666499.1, 218105_s_at
Figure 3166: PRO62669
Figure 3167: DNA344711, NP_060699.2, 218139_s_at
Figure 3168: PRO95302
Figure 3169: DNA327857, NP_057386.1, 218142_s_at
Figure 3170: PRO83799
Figure 3171: DNA287235, NP_060598.1, 218156_s_at
Figure 3172: PRO69514
Figure 3173: DNA151377, NP_057132.1, 218170_at
Figure 3174: PRO11754
Figure 3175: DNA304470, NP_061100.1, 218172_s_at
Figure 3176: PRO71046
Figure 3177A-D: DNA340174, NP_064630.1,
218184_at
Figure 3178: PRO91669
Figure 3179: DNA344712, NP_036590.1, 218188_s_at
Figure 3180: PRO82887
Figure 3181A-C: DNA330360, NP_078789.1,
218204_s_at
Figure 3182: PRO85576
Figure 3183: DNA344713, NP_060641.2, 218218_at
Figure 3184: PRO95303
Figure 3185: DNA225650, NP_057246.1, 218234_at
Figure 3186: PRO36113
Figure 3187: DNA327858, NP_036473.1, 218238_at
Figure 3188: PRO83800
Figure 3189: DNA327858, NM_012341, 218239_s_at
Figure 3190: PRO83800

Figure 3191A-B: DNA344714, NP_037367.2,
218269_at
Figure 3192: PRO95304
Figure 3193: DNA329074, NP_064524.1, 218285_s_at
Figure 3194: PRO21326
Figure 3195A-B: DNA328853, NP_065702.2,
218319_at
Figure 3196: PRO84584
Figure 3197: DNA329281, NP_036526.2, 218336_at
Figure 3198: PRO84874
Figure 3199A-B: DNA344715, BAB47444.2,
218342_s_at
Figure 3200: PRO95305
Figure 3201: DNA328854, NP_056979.1, 218350_s_at
Figure 3202: PRO84585
Figure 3203A-B: DNA273415, NP_036442.2,
218355_at
Figure 3204: PRO61414
Figure 3205: DNA344716, NP_071921.1, 218373_at
Figure 3206: PRO95306
Figure 3207A-B: DNA330366, NP_073602.2,
218376_s_at
Figure 3208: PRO85581
Figure 3209: DNA328856, NP_068376.1, 218380_at
Figure 3210: PRO84586
Figure 3211: DNA327863, NP_055131.1, 218384_at
Figure 3212: PRO83804
Figure 3213: DNA255340, NP_060154.1, 218396_at
Figure 3214: PRO50409
Figure 3215: DNA344717, NP_663747.1, 218399_s_at
Figure 3216: PRO95307
Figure 3217A-B: DNA287192, NP_006178.1,
218400_at
Figure 3218: PRO69478
Figure 3219: DNA333245, NP_037454.2, 218404_at
Figure 3220: PRO87952
Figure 3221A-B: DNA344718, NP_076414.2,
218456_at
Figure 3222: PRO95308
Figure 3223: DNA328861, NP_057030.2, 218472_s_at
Figure 3224: PRO84589
Figure 3225: DNA327943, NP_055399.1, 218498_s_at
Figure 3226: PRO865
Figure 3227: DNA150648, NP_037464.1, 218507_at
Figure 3228: PRO11576
Figure 3229: DNA326550, NP_057663.1, 218529_at
Figure 3230: PRO224
Figure 3231: DNA327868, NP_060601.2, 218542_at
Figure 3232: PRO83809
Figure 3233: DNA255113, NP_073587.1, 218543_s_at
Figure 3234: PRO50195
Figure 3235: DNA330373, NP_060751.1, 218552_at
Figure 3236: PRO85587
Figure 3237: DNA344719, NP_059142.1, 218558_s_at
Figure 3238: PRO85588
Figure 3239: DNA329587, NP_036256.1, 218566_s_at
Figure 3240: PRO85121
Figure 3241: DNA325036, NP_060708.1, 218568_at
Figure 3242: PRO81625
Figure 3243A-B: DNA273435, NP_057532.1,
218585_s_at
Figure 3244: PRO61430
Figure 3245: DNA93548, NP_005758.1, 218589_at
Figure 3246: PRO4929
Figure 3247: DNA326916, NP_149061.1, 218592_s_at
Figure 3248: PRO83235
Figure 3249: DNA287642, NP_060934.1, 218597_s_at
Figure 3250: PRO9902
Figure 3251A-B: DNA254789, NP_057301.1,
218603_at
Figure 3252: PRO49887
Figure 3253A-B: DNA344720, NP_073600.2,
218618_s_at
Figure 3254: PRO95309
Figure 3255A-B: DNA339409, NP_057257.1,
218620_s_at
Figure 3256: PRO91214
Figure 3257: DNA327869, NP_057672.1, 218625_at
Figure 3258: PRO1898
Figure 3259: DNA339537, NP_060864.1, 218633_x_at
Figure 3260: PRO91303
Figure 3261: DNA344721, NP_057303.1, 218636_s_at
Figure 3262: PRO1477
Figure 3263A-B: DNA344722, NP_073606.1,
218648_at
Figure 3264: PRO95310
Figure 3265: DNA330378, NP_071741.2, 218663_at
Figure 3266: PRO81126
Figure 3267: DNA339660, NP_079491.1, 218670_at
Figure 3268: PRO91402
Figure 3269: DNA287291, NP_067036.1, 218676_s_at
Figure 3270: PRO69561
Figure 3271: DNA330379, NP_073562.1, 218689_at
Figure 3272: PRO85591
Figure 3273: DNA328873, NP_057041.1, 218698_at
Figure 3274: PRO84600
Figure 3275: DNA344723, NP_060320.1, 218712_at
Figure 3276: PRO95311
Figure 3277: DNA328874, NP_054778.1, 218723_s_at
Figure 3278: PRO84601
Figure 3279: DNA324251, NP_060880.2, 218726_at
Figure 3280: PRO80935
Figure 3281: DNA330382, NP_005724.1, 218755_at
Figure 3282: PRO61907
Figure 3283A-B: DNA344724, NP_054828.2,
218782_s_at
Figure 3284: PRO95312
Figure 3285: DNA335239, NP_060158.1, 218792_s_at
Figure 3286: PRO89625
Figure 3287: DNA344725, NP_060854.2, 218805_at
Figure 3288: PRO95313
Figure 3289: DNA256846, NP_059985.1, 218826_at

Figure 3290: PRO51777
Figure 3291: DNA255213, AK000364, 218829_s_at
Figure 3292: PRO50292
Figure 3293: DNA328879, NP_064570.1, 218845_at
Figure 3294: PRO84606
Figure 3295A-B: DNA344726, NP_004821.2,
218846_at
Figure 3296: PRO95314
Figure 3297: DNA330385, NP_057733.2, 218859_s_at
Figure 3298: PRO85594
Figure 3299: DNA330386, NP_057394.1, 218866_s_at
Figure 3300: PRO85595
Figure 3301: DNA344727, NP_060930.2, 218870_at
Figure 3302: PRO95315
Figure 3303: DNA330387, NP_036309.1, 218875_s_at
Figure 3304: PRO85596
Figure 3305: DNA327874, BC022791, 218880_at
Figure 3306: PRO4805
Figure 3307: DNA344728, NP_078806.1, 218881_s_at
Figure 3308: PRO95316
Figure 3309: DNA226633, NP_060376.1, 218886_at
Figure 3310: PRO37096
Figure 3311A-B: DNA335042, NP_060562.3,
218888_s_at
Figure 3312: PRO4401
Figure 3313: DNA344729, AK026953, 218889_at
Figure 3314: PRO95317
Figure 3315: DNA254380, NP_065112.1, 218918_at
Figure 3316: PRO49490
Figure 3317: DNA328364, NP_068577.1, 218921_at
Figure 3318: PRO84223
Figure 3319: DNA329333, NP_054886.1, 218936_s_at
Figure 3320: PRO84917
Figure 3321A-B: DNA344730, NP_055129.1,
218943_s_at
Figure 3322: PRO69459
Figure 3323: DNA334561, NP_068572.1, 218976_at
Figure 3324: PRO89050
Figure 3325: DNA329050, NP_057053.1, 218982_s_at
Figure 3326: PRO84712
Figure 3327A-B: DNA344731, NP_060101.1,
218986_s_at
Figure 3328: PRO51309
Figure 3329: DNA327211, NP_075053.2, 218989_x_at
Figure 3330: PRO71052
Figure 3331: DNA227194, NP_060765.1, 218999_at
Figure 3332: PRO37657
Figure 3333: DNA328884, NP_054884.1, 219006_at
Figure 3334: PRO84609
Figure 3335: DNA227187, NP_057703.1, 219014_at
Figure 3336: PRO37650
Figure 3337: DNA328885, NP_061108.2, 219017_at
Figure 3338: PRO50294
Figure 3339: DNA329293, NP_057136.1, 219037_at
Figure 3340: PRO84883
Figure 3341: DNA333718, NP_068595.2, 219066_at
Figure 3342: PRO88346
Figure 3343A-B: DNA344732, NP_060254.2,
219073_s_at
Figure 3344: PRO90806
Figure 3345: DNA327877, NP_065108.1, 219099_at
Figure 3346: PRO83816
Figure 3347: DNA344733, NP_079204.1, 219100_at
Figure 3348: PRO95318
Figure 3349: DNA287242, NP_127460.1, 219110_at
Figure 3350: PRO69517
Figure 3351: DNA304472, NP_057678.1, 219117_s_at
Figure 3352: PRO535
Figure 3353: DNA297191, NP_060962.2, 219148_at
Figure 3354: PRO70808
Figure 3355: DNA329295, NP_036549.1, 219155_at
Figure 3356: PRO84885
Figure 3357A-B: DNA331610, NM_025085,
219158_s_at
Figure 3358: PRO86609
Figure 3359: DNA328892, NM_021630, 219165_at
Figure 3360: PRO84616
Figure 3361: DNA330400, NP_078796.1, 219176_at
Figure 3362: PRO85608
Figure 3363A-B: DNA344734, NP_078914.1,
219178_at
Figure 3364: PRO95319
Figure 3365: DNA329223, NP_037517.1, 219183_s_at
Figure 3366: PRO84831
Figure 3367: DNA330401, NP_057377.1, 219191_s_at
Figure 3368: PRO85609
Figure 3369: DNA344735, NP_071451.1, 219209_at
Figure 3370: PRO83818
Figure 3371: DNA344736, NP_057614.1, 219210_s_at
Figure 3372: PRO95320
Figure 3373: DNA330403, NP_059110.1, 219211_at
Figure 3374: PRO85611
Figure 3375: DNA339627, NP_079000.1, 219221_at
Figure 3376: PRO91378
Figure 3377: DNA333832, NP_071411.1, 219222_at
Figure 3378: PRO88449
Figure 3379: DNA225594, NP_037404.1, 219229_at
Figure 3380: PRO36057
Figure 3381: DNA252224, NM_022073, 219232_s_at
Figure 3382: PRO48216
Figure 3383: DNA344737, NP_060796.1, 219243_at
Figure 3384: PRO84617
Figure 3385: DNA344738, NP_061195.2, 219255_x_at
Figure 3386: PRO19612
Figure 3387: DNA329296, NP_060328.1, 219258_at
Figure 3388: PRO84886
Figure 3389: DNA328895, NP_071762.2, 219259_at
Figure 3390: PRO1317
Figure 3391: DNA255020, NP_061918.1, 219297_at
Figure 3392: PRO50109
Figure 3393: DNA255939, NP_078876.1, 219315_s_at
Figure 3394: PRO50991

Figure 3395: DNA227784, NP_060383.1, 219343_at
Figure 3396: PRO38247
Figure 3397: DNA254710, NP_060382.1, 219352_at
Figure 3398: PRO49810
Figure 3399: DNA287174, AF161525, 219356_s_at
Figure 3400: PRO69464
Figure 3401A-B: DNA327885, NP_075601.1,
219369_s_at
Figure 3402: PRO82377
Figure 3403: DNA188342, NP_064510.1, 219386_s_at
Figure 3404: PRO21718
Figure 3405: DNA344739, NP_683866.1, 219423_x_at
Figure 3406: PRO95321
Figure 3407: DNA329014, NP_005746.2, 219424_at
Figure 3408: PRO9998
Figure 3409: DNA328902, NP_071750.1, 219452_at
Figure 3410: PRO84623
Figure 3411: DNA328367, NP_079108.2, 219456_s_at
Figure 3412: PRO84226
Figure 3413: DNA328367, NM_024832, 219457_s_at
Figure 3414: PRO84226
Figure 3415A-B: DNA199058, NP_060319.1,
219460_s_at
Figure 3416: PRO28533
Figure 3417: DNA325850, NP_076994.1, 219479_at
Figure 3418: PRO82312
Figure 3419: DNA344740, NP_079021.2, 219493_at
Figure 3420: PRO95322
Figure 3421A-B: DNA344741, NP_059120.2,
219505_at
Figure 3422: PRO95323
Figure 3423A-C: DNA330409, NM_022898,
219528_s_at
Figure 3424: PRO85617
Figure 3425: DNA329299, NP_004660.1, 219529_at
Figure 3426: PRO84888
Figure 3427: DNA334311, NP_073563.1, 219532_at
Figure 3428: PRO50477
Figure 3429: DNA344742, NP_003405.2, 219540_at
Figure 3430: PRO95324
Figure 3431: DNA256737, NP_060276.1, 219541_at
Figure 3432: PRO51671
Figure 3433: DNA330410, NP_060925.1, 219555_s_at
Figure 3434: PRO85618
Figure 3435: DNA225636, NP_065696.1, 219557_s_at
Figure 3436: PRO36099
Figure 3437: DNA336133, NP_078852.1, 219582_at
Figure 3438: PRO90333
Figure 3439: DNA325053, NP_060230.2, 219588_s_at
Figure 3440: PRO81637
Figure 3441: DNA344743, NP_006125.2, 219600_s_at
Figure 3442: PRO193
Figure 3443: DNA331601, NP_071915.1, 219628_at
Figure 3444: PRO85620
Figure 3445: DNA327892, NP_060470.1, 219648_at
Figure 3446: PRO83828
Figure 3447: DNA328915, NP_055056.2, 219654_at
Figure 3448: PRO84634
Figure 3449: DNA344744, NP_079352.1, 219675_s_at
Figure 3450: PRO95325
Figure 3451: DNA255161, NP_071430.1, 219684_at
Figure 3452: PRO50241
Figure 3453: DNA339552, NP_061922.1, 219696_at
Figure 3454: PRO91318
Figure 3455A-B: DNA330297, NP_065138.2,
219700_at
Figure 3456: PRO85524
Figure 3457A-B: DNA227762, NP_060169.1,
219734_at
Figure 3458: PRO38225
Figure 3459: DNA256481, NP_060269.1, 219757_s_at
Figure 3460: PRO51518
Figure 3461: DNA344745, NP_078896.1, 219765_at
Figure 3462: PRO95326
Figure 3463: DNA344746, NP_078987.2, 219777_at
Figure 3464: PRO95327
Figure 3465A-B: DNA330418, NP_060568.3,
219787_s_at
Figure 3466: PRO85623
Figure 3467: DNA344747, NP_690049.1, 219793_at
Figure 3468: PRO95328
Figure 3469: DNA324981, NP_076975.1, 219812_at
Figure 3470: PRO81575
Figure 3471: DNA331378, NP_079020.12, 219834_at
Figure 3472: PRO86449
Figure 3473: DNA287295, NP_078784.1, 219836_at
Figure 3474: PRO69564
Figure 3475: DNA344748, NP_066358.1, 219854_at
Figure 3476: PRO95329
Figure 3477: DNA255255, NM_022154, 219869_s_at
Figure 3478: PRO50332
Figure 3479: DNA344749, NP_079273.1, 219870_at
Figure 3480: PRO95330
Figure 3481: DNA254838, NP_078904.1, 219874_at
Figure 3482: PRO49933
Figure 3483: DNA328923, NP_075379.1, 219892_at
Figure 3484: PRO84640
Figure 3485: DNA330421, NP_057438.2, 219911_s_at
Figure 3486: PRO85626
Figure 3487A-C: DNA344750, NP_060606.2,
219918_s_at
Figure 3488: PRO95331
Figure 3489: DNA328924, NP_057150.2, 219933_at
Figure 3490: PRO84641
Figure 3491: DNA344751, NP_037396.2, 219945_at
Figure 3492: PRO95332
Figure 3493: DNA256345, AK000925, 219957_at
Figure 3494: PRO51387
Figure 3495: DNA218280, NP_068570.1, 219971_at
Figure 3496: PRO34332
Figure 3497: DNA325979, NP_060924.4, 219978_s_at
Figure 3498: PRO82424

Figure 3499: DNA330425, NP_078956.1, 219990_at
Figure 3500: PRO85630
Figure 3501: DNA333765, AK000812, 219994_at
Figure 3502: PRO88389
Figure 3503: DNA256141, NP_060893.1, 220030_at
Figure 3504: PRO51189
Figure 3505A-B: DNA344752, NP_037389.3,
220038_at
Figure 3506: PRO95333
Figure 3507A-B: DNA221079, NP_071445.1,
220066_at
Figure 3508: PRO34753
Figure 3509: DNA256091, NP_071385.1, 220094_s_at
Figure 3510: PRO51141
Figure 3511: DNA330431, NP_055198.1, 220118_at
Figure 3512: PRO85635
Figure 3513: DNA256803, AK001445, 220121_at
Figure 3514: PRO51734
Figure 3515: DNA227302, NP_037401.1, 220132_s_at
Figure 3516: PRO37765
Figure 3517: DNA344753, AK000388, 220161_s_at
Figure 3518: PRO95334
Figure 3519: DNA335568, NP_076927.1, 220177_s_at
Figure 3520: PRO89910
Figure 3521: DNA330434, NP_060842.1, 220235_s_at
Figure 3522: PRO85637
Figure 3523: DNA344754, NP_036551.3, 220334_at
Figure 3524: PRO95335
Figure 3525: DNA287186, NP_061134.1, 220358_at
Figure 3526: PRO69472
Figure 3527: DNA255964, NP_079113.1, 220416_at
Figure 3528: PRO51015
Figure 3529: DNA339549, NP_061834.1, 220418_at
Figure 3530: PRO91315
Figure 3531: DNA330438, NP_061026.1, 220485_s_at
Figure 3532: PRO50795
Figure 3533: DNA327214, NP_078991.2, 220495_s_at
Figure 3534: PRO83483
Figure 3535: DNA344755, NP_620591.1, 220558_x_at
Figure 3536: PRO95336
Figure 3537: DNA255798, NP_079265.1, 220576_at
Figure 3538: PRO50853
Figure 3539: DNA344756, NP_079282.1, 220577_at
Figure 3540: PRO95337
Figure 3541: DNA344757, NP_071767.2, 220587_s_at
Figure 3542: PRO95338
Figure 3543A-B: DNA334963, NP_116561.1,
220613_s_at
Figure 3544: PRO89395
Figure 3545: DNA227368, NP_057371.1, 220633_s_at
Figure 3546: PRO37831
Figure 3547A-B: DNA327908, NP_060988.2,
220651_s_at
Figure 3548: PRO83843
Figure 3549: DNA329306, NP_079149.2, 220655_at
Figure 3550: PRO84895
Figure 3551A-B: DNA327909, NP_064568.2,
220658_s_at
Figure 3552: PRO83844
Figure 3553: DNA329307, NP_037483.1, 220684_at
Figure 3554: PRO84896
Figure 3555: DNA323756, NP_057267.2, 220688_s_at
Figure 3556: PRO80512
Figure 3557: DNA330443, NP_061086.1, 220702_at
Figure 3558: PRO85644
Figure 3559: DNA344758, NP_061033.1, 220704_at
Figure 3560: PRO88381
Figure 3561A-B: DNA329308, NP_065705.2,
220735_s_at
Figure 3562: PRO84897
Figure 3563: DNA344759, NP_065857.1, 220773_s_at
Figure 3564: PRO50495
Figure 3565: DNA344760, NP_065089.1, 220888_s_at
Figure 3566: PRO95339
Figure 3567: DNA288247, NP_478059.1, 220892_s_at
Figure 3568: PRO70011
Figure 3569: DNA338124, NP_079419.1, 220918_at
Figure 3570: PRO90989
Figure 3571: DNA328940, NP_078893.1, 220933_s_at
Figure 3572: PRO84653
Figure 3573: DNA344761, NP_065126.1, 220944_at
Figure 3574: PRO95340
Figure 3575: DNA324246, NP_112188.1, 221004_s_at
Figure 3576: PRO80930
Figure 3577: DNA336778, NP_110407.2, 221020_s_at
Figure 3578: PRO90848
Figure 3579: DNA254520, NP_060952.1, 221039_s_at
Figure 3580: PRO49627
Figure 3581: DNA328945, NP_079177.2, 221081_s_at
Figure 3582: PRO84657
Figure 3583: DNA344762, NP_036613.1, 221092_at
Figure 3584: PRO89669
Figure 3585: DNA226227, NP_060872.1, 221111_at
Figure 3586: PRO36690
Figure 3587: DNA344763, NP_659508.1, 221223_x_at
Figure 3588: PRO86458
Figure 3589A-C: DNA332533, NP_068585.1,
221234_s_at
Figure 3590: PRO87347
Figure 3591: DNA328948, NP_110437.1, 221253_s_at
Figure 3592: PRO84659
Figure 3593: DNA330452, NP_112494.2, 221258_s_at
Figure 3594: PRO85653
Figure 3595: DNA344764, BC000158, 221267_s_at
Figure 3596: PRO95341
Figure 3597: DNA295327, NP_068575.1, 221271_at
Figure 3598: PRO70773
Figure 3599: DNA329312, NP_005205.2, 221331_x_at
Figure 3600: PRO84901
Figure 3601: DNA256061, NP_112183.1, 221428_s_at
Figure 3602: PRO51109
Figure 3603: DNA344765, NP_112487.1, 221434_s_at

Figure 3604: PRO70013
Figure 3605: DNA344766, 1163161.25, 221471_at
Figure 3606: PRO12237
Figure 3607: DNA324282, NP_002939.2, 221475_s_at
Figure 3608: PRO6360
Figure 3609: DNA227303, NP_004322.1, 221479_s_at
Figure 3610: PRO37766
Figure 3611A-B: DNA344767, NP_004767.1,
221484_at
Figure 3612: PRO59982
Figure 3613: DNA330456, NP_060571.1, 221520_s_at
Figure 3614: PRO85657
Figure 3615: DNA328952, NP_067067.1, 221524_s_at
Figure 3616: PRO84663
Figure 3617: DNA328953, NP_004086.1, 221539_at
Figure 3618: PRO70296
Figure 3619: DNA327526, NM_020676, 221552_at
Figure 3620: PRO83574
Figure 3621: DNA304486, NP_115497.1, 221553_at
Figure 3622: PRO71055
Figure 3623: DNA329317, NP_057353.1, 221558_s_at
Figure 3624: PRO81157
Figure 3625: DNA329095, NP_057000.2, 221565_s_at
Figure 3626: PRO77352
Figure 3627: DNA334699, NP_003937.1, 221567_at
Figure 3628: PRO89166
Figure 3629: DNA329319, NP_005440.1, 221601_s_at
Figure 3630: PRO1607
Figure 3631: DNA329319, NM_005449, 221602_s_at
Figure 3632: PRO1607
Figure 3633: DNA344768, NP_057059.2, 221618_s_at
Figure 3634: PRO95342
Figure 3635: DNA344769, NP_036464.1, 221641_s_at
Figure 3636: PRO95343
Figure 3637: DNA218280, NM_021798, 221658_s_at
Figure 3638: PRO34332
Figure 3639: DNA327927, NP_037390.2, 221666_s_at
Figure 3640: PRO57311
Figure 3641A-B: DNA344770, NP_055140.1,
221676_s_at
Figure 3642: PRO49875
Figure 3643: DNA194468, AF225418, 221679_s_at
Figure 3644: PRO23835
Figure 3645: DNA344771, AF094508, 221681_s_at
Figure 3646: DNA330460, NP_060255.2, 221685_s_at
Figure 3647: PRO85660
Figure 3648: DNA324690, NP_002511.1, 221691_x_at
Figure 3649: PRO58993
Figure 3650: DNA256141, NM_018423, 221696_s_at
Figure 3651: PRO51189
Figure 3652: DNA344772, NP_078943.1, 221704_s_at
Figure 3653: PRO90809
Figure 3654A-C: DNA328664, NM_007200,
221718_s_at
Figure 3655: PRO84437
Figure 3656A-B: DNA344773, 1505701.34, 221727_at
Figure 3657: PRO95345
Figure 3658: DNA328961, NP_443112.1, 221756_at
Figure 3659: PRO84667
Figure 3660: DNA328961, NM_052880, 221757_at
Figure 3661: PRO84667
Figure 3662A-C: DNA328965, BAB21809.1,
221778_at
Figure 3663: PRO51878
Figure 3664A-B: DNA344774, AL833316,
221824_s_at
Figure 3665: PRO95346
Figure 3666: DNA344775, NP_689501.1, 221864_at
Figure 3667: PRO95347
Figure 3668: DNA344776, 299937.3, 221897_at
Figure 3669: PRO95348
Figure 3670: DNA327933, 1452741.11, 221899_at
Figure 3671: PRO83865
Figure 3672A-B: DNA344777, AB020656, 221905_at
Figure 3673: DNA328971, AK000472, 221923_s_at
Figure 3674: PRO84674
Figure 3675: DNA329321, NP_112493.1, 221931_s_at
Figure 3676: PRO84906
Figure 3677A-B: DNA336655, BAB85561.1,
221971_x_at
Figure 3678: PRO90728
Figure 3679: DNA344778, 7696429.33, 221973_at
Figure 3680: PRO95350
Figure 3681: DNA331384, AK026326, 221985_at
Figure 3682: PRO86454
Figure 3683: DNA254739, NP_068766.1, 221987_s_at
Figure 3684: PRO49837
Figure 3685: DNA344779, AF218023, 221989_at
Figure 3686: PRO95351
Figure 3687: DNA344780, 127586.70, 222001_x_at
Figure 3688: PRO95352
Figure 3689A-C: DNA344781, NM_006738,
222024_s_at
Figure 3690: PRO95353
Figure 3691: DNA344782, AAH44933.1, 222039_at
Figure 3692: PRO95354
Figure 3693: DNA325036, NM_018238, 222132_s_at
Figure 3694: PRO81625
Figure 3695A-B: DNA339979, BAA95990.1,
222139_at
Figure 3696: PRO91487
Figure 3697: DNA329916, 338326.15, 222142_at
Figure 3698: PRO85231
Figure 3699A-B: DNA344783, 027987.100, 222145_at
Figure 3700: PRO95355
Figure 3701: DNA331386, AL079297, 222150_s_at
Figure 3702: DNA328975, NP_078807.1, 222155_s_at
Figure 3703: PRO47688
Figure 3704: DNA256784, NP_075069.1, 222209_s_at
Figure 3705: PRO51716
Figure 3706: DNA323915, NP_077306.1, 222217_s_at
Figure 3707: PRO703

Figure 3708: DNA287425, NP_060979.1, 222231_s_at
Figure 3709: PRO69682
Figure 3710: DNA344784, AAB26149.1, 222247_at
Figure 3711: PRO95356
Figure 3712: DNA344785, AL137750, 222262_s_at
Figure 3713: PRO95357
Figure 3714: DNA344786, 405457.25, 222303_at
Figure 3715: PRO95358
Figure 3716: DNA330470, 096828.1, 222307_at
Figure 3717: PRO85668
Figure 3718: DNA344787, 016338.1, 222371_at
Figure 3719: PRO95359
Figure 3720A-B: DNA324364, NP_037468.1,
222385_x_at
Figure 3721: PRO1314
Figure 3722: DNA335675, AJ251830, 222392_x_at
Figure 3723: PRO90003
Figure 3724: DNA227358, NP_057479.1, 222404_x_at
Figure 3725: PRO37821
Figure 3726: DNA344788, AK074898, 222405_at
Figure 3727: PRO95360
Figure 3728A-B: DNA344789, NM_014325,
222409_at
Figure 3729: PRO49875
Figure 3730: DNA327939, NP_060654.1, 222442_s_at
Figure 3731: PRO83869
Figure 3732: DNA344790, NM_005105, 222443_s_at
Figure 3733: PRO37600
Figure 3734A-B: DNA325652, NM_016357,
222457_s_at
Figure 3735: PRO82143
Figure 3736A-B: DNA256489, NP_079110.1,
222464_s_at
Figure 3737: PRO51526
Figure 3738: DNA331089, NP_057143.1, 222500_at
Figure 3739: PRO4984
Figure 3740: DNA329370, NP_060611.2, 222522_x_at
Figure 3741: PRO84949
Figure 3742A-B: DNA344791, AL834191, 222603_at
Figure 3743: PRO95361
Figure 3744: DNA330483, AK001472, 222608_s_at
Figure 3745: PRO85679
Figure 3746: DNA329330, NP_057130.1, 222609_s_at
Figure 3747: PRO84914
Figure 3748: DNA344792, BC035985, 222622_at
Figure 3749: PRO95362
Figure 3750: DNA329331, NP_005763.2, 222666_s_at
Figure 3751: PRO84915
Figure 3752: DNA344793, 1454336.17, 222669_s_at
Figure 3753: PRO95363
Figure 3754: DNA344794, NP_079170.1, 222684_s_at
Figure 3755: PRO95364
Figure 3756A-B: DNA344795, AF537091, 222685_at
Figure 3757: PRO95365
Figure 3758A-B: DNA344796, 998337.2, 222689_at
Figure 3759: PRO95366
Figure 3760: DNA339537, NM_018394, 222697_s_at
Figure 3761: PRO91303
Figure 3762: DNA323797, NP_078916.1, 222703_s_at
Figure 3763: PRO80547
Figure 3764: DNA344797, BC044575, 222734_at
Figure 3765: PRO95367
Figure 3766: DNA333586, 295181.4, 222735_at
Figure 3767: PRO84603
Figure 3768A-B: DNA344798, NM_014109,
222740_at
Figure 3769: PRO95368
Figure 3770: DNA335239, NM_017688, 222746_s_at
Figure 3771: PRO89625
Figure 3772A-B: DNA340168, NP_060163.2,
222761_at
Figure 3773: PRO91663
Figure 3774: DNA344799, BC005401, 222763_s_at
Figure 3775: PRO95369
Figure 3776A-B: DNA335042, NM_018092,
222774_s_at
Figure 3777: PRO4401
Figure 3778A-B: DNA344800, BC033901,
222787_s_at
Figure 3779: PRO95370
Figure 3780: DNA255044, DNA255044, 222833_at
Figure 3781A-B: DNA329438, NP_476516.1,
222837_s_at
Figure 3782: PRO85008
Figure 3783: DNA339367, NP_037469.1, 222841_s_at
Figure 3784: PRO91172
Figure 3785: DNA344801, AL834387, 222843_at
Figure 3786: PRO95371
Figure 3787A-B: DNA333626, DNA333626,
222846_at
Figure 3788: PRO88268
Figure 3789: DNA335638, NP_203130.1, 222847_s_at
Figure 3790: PRO48216
Figure 3791: DNA331389, NP_071428.2, 222848_at
Figure 3792: PRO81238
Figure 3793A-B: DNA344802, NP_064547.2,
222875_at
Figure 3794: PRO95372
Figure 3795: DNA344803, 321334.4, 222900_at
Figure 3796: PRO95373
Figure 3797: DNA344804, NP_005012.1, 222938_x_at
Figure 3798: PRO95374
Figure 3799: DNA330501, AK022792, 222958_s_at
Figure 3800: PRO85694
Figure 3801: DNA330503, NP_038466.2, 222991_s_at
Figure 3802: PRO85696
Figure 3803: DNA330504, NP_057575.2, 222993_at
Figure 3804: PRO84923
Figure 3805: DNA324548, NP_110409.2, 223020_at
Figure 3806: PRO81202
Figure 3807A-B: DNA344805, NP_057308.1,
223027_at

Figure 3808: PRO84924
Figure 3809A-B: DNA344806, NM_016224,
223028_s_at
Figure 3810: PRO84924
Figure 3811: DNA324707, NP_037369.1, 223032_x_at
Figure 3812: PRO81339
Figure 3813A-B: DNA256347, NP_065801.1,
223055_s_at
Figure 3814: PRO51389
Figure 3815A-B: DNA256347, NM_020750,
223056_s_at
Figure 3816: PRO51389
Figure 3817: DNA325295, NP_113641.1, 223058_at
Figure 3818: PRO81841
Figure 3819: DNA287216, NM_021154, 223062_s_at
Figure 3820: PRO69496
Figure 3821: DNA304492, NP_114405.1, 223065_s_at
Figure 3822: PRO1864
Figure 3823A-B: DNA328934, NP_061936.2,
223068_at
Figure 3824: PRO84649
Figure 3825A-B: DNA328934, NM_019063,
223069_s_at
Figure 3826: PRO84649
Figure 3827: DNA344807, NP_036609.1, 223072_s_at
Figure 3828: PRO95375
Figure 3829: DNA227294, NP_060225.1, 223076_s_at
Figure 3830: PRO37757
Figure 3831A-B: DNA329316, AF158555,
223079_s_at
Figure 3832: PRO84904
Figure 3833: DNA329349, NP_054861.1, 223100_s_at
Figure 3834: PRO84931
Figure 3835A-C: DNA339662, NP_110433.1,
223125_s_at
Figure 3836: PRO91404
Figure 3837: DNA330445, NP_112174.1, 223132_s_at
Figure 3838: PRO85646
Figure 3839: DNA325557, NP_115675.1, 223151_at
Figure 3840: PRO82060
Figure 3841: DNA329352, NP_057154.2, 223156_at
Figure 3842: PRO84932
Figure 3843A-B: DNA339969, BAA86461.1,
223162_s_at
Figure 3844: PRO91477
Figure 3845: DNA324924, NP_113631.1, 223164_at
Figure 3846: PRO81525
Figure 3847A-B: DNA344808, NP_067028.1,
223168_at
Figure 3848: PRO1200
Figure 3849A-B: DNA344809, AAH23525.1,
223176_at
Figure 3850: PRO95376
Figure 3851: DNA344810, NP_113665.1, 223179_at
Figure 3852: PRO84933
Figure 3853: DNA254276, NP_054896.1, 223180_s_at
Figure 3854: PRO49387
Figure 3855: DNA344811, NP_113675.2, 223182_s_at
Figure 3856: PRO95377
Figure 3857: DNA344812, AF201944, 223193_x_at
Figure 3858: PRO95378
Figure 3859: DNA323792, NP_113647.1, 223195_s_at
Figure 3860: PRO80542
Figure 3861: DNA339535, NP_060855.1, 223200_s_at
Figure 3862: PRO91301
Figure 3863A-B: DNA257461, NP_113607.1,
223217_s_at
Figure 3864: PRO52040
Figure 3865A-B: DNA257461, NM_031419,
223218_s_at
Figure 3866: PRO52040
Figure 3867: DNA327954, NP_113646.1, 223220_s_at
Figure 3868: PRO83879
Figure 3869: DNA340182, NP_068380.1, 223222_at
Figure 3870: PRO91677
Figure 3871: DNA344813, NP_114091.2, 223227_at
Figure 3872: PRO95379
Figure 3873: DNA344814, NP_060019.1, 223253_at
Figure 3874: PRO95380
Figure 3875: DNA330517, NP_115879.1, 223273_at
Figure 3876: PRO85707
Figure 3877: DNA344815, NP_116565.1, 223276_at
Figure 3878: PRO12050
Figure 3879A-B: DNA330522, NP_116071.2,
223287_s_at
Figure 3880: PRO85712
Figure 3881: DNA326962, NP_064711.1, 223290_at
Figure 3882: PRO83275
Figure 3883: DNA330523, BC001220, 223294_at
Figure 3884: PRO85713
Figure 3885: DNA257363, NP_115691.1, 223296_at
Figure 3886: PRO51950
Figure 3887: DNA329355, NP_150596.1, 223299_at
Figure 3888: PRO50434
Figure 3889: DNA329356, NP_115671.1, 223304_at
Figure 3890: PRO84935
Figure 3891: DNA330454, NP_112589.1, 223307_at
Figure 3892: PRO85655
Figure 3893: DNA344816, NM_020806, 223319_at
Figure 3894: PRO50495
Figure 3895: DNA329358, NP_115649.1, 223334_at
Figure 3896: PRO84937
Figure 3897A-B: DNA255756, L12052, 223358_s_at
Figure 3898: PRO50812
Figure 3899: DNA344817, NM_145071, 223377_x_at
Figure 3900: PRO86458
Figure 3901A-B: DNA344818, NP_055387.1,
223380_s_at
Figure 3902: PRO95381
Figure 3903: DNA344819, NP_663735.1, 223381_at
Figure 3904: PRO38881
Figure 3905A-B: DNA344820, NP_115644.1,

223382_s_at
Figure 3906: PRO84939
Figure 3907A-B: DNA344821, NM_032268,
223383_at
Figure 3908: PRO84939
Figure 3909: DNA340216, NP_115686.2, 223398_at
Figure 3910: PRO91711
Figure 3911: DNA339511, NP_060635.1, 223400_s_at
Figure 3912: PRO91282
Figure 3913: DNA324156, NP_115588.1, 223403_s_at
Figure 3914: PRO80856
Figure 3915: DNA344822, NP_115514.2, 223412_at
Figure 3916: PRO95382
Figure 3917: DNA329362, NP_060286.1, 223413_s_at
Figure 3918: PRO84941
Figure 3919: DNA329362, NM_017816, 223414_s_at
Figure 3920: PRO84941
Figure 3921: DNA255676, NP_060754.1, 223434_at
Figure 3922: PRO50738
Figure 3923: DNA330533, NP_058647.1, 223451_s_at
Figure 3924: PRO772
Figure 3925: DNA344823, BAA92078.1, 223457_at
Figure 3926: PRO95383
Figure 3927: DNA273418, AAG01157.1, 223480_s_at
Figure 3928: DNA327958, NP_115789.1, 223484_at
Figure 3929: PRO23554
Figure 3930: DNA329456, NP_057126.1, 223490_s_at
Figure 3931: PRO85023
Figure 3932: DNA338084, NP_006564.1, 223502_s_at
Figure 3933: PRO738
Figure 3934: DNA344824, AF255647, 223503_at
Figure 3935: PRO95384
Figure 3936: DNA333656, NP_115646.2, 223533_at
Figure 3937: PRO88295
Figure 3938: DNA330536, NP_115666.1, 223542_at
Figure 3939: PRO85722
Figure 3940A-B: DNA339971, BAA86587.1,
223617_x_at
Figure 3941: PRO91479
Figure 3942: DNA327028, NP_005291.1, 223620_at
Figure 3943: PRO37083
Figure 3944: DNA344825, BC002724, 223666_at
Figure 3945: PRO83126
Figure 3946: DNA344826, NP_006548.1, 223704_s_at
Figure 3947: PRO51385
Figure 3948: DNA344827, AF176013, 223722_at
Figure 3949: PRO95385
Figure 3950: DNA344828, NM_146388, 223743_s_at
Figure 3951: PRO95386
Figure 3952: DNA188735, NP_001506.1, 223758_s_at
Figure 3953: PRO26224
Figure 3954: DNA287253, NP_444268.1, 223774_at
Figure 3955: PRO69527
Figure 3956: DNA331132, NP_115524.1, 223798_at
Figure 3957: PRO86273
Figure 3958: DNA332645, NP_570138.1, 223809_at
Figure 3959: PRO61997
Figure 3960: DNA327200, NP_114156.1, 223836_at
Figure 3961: PRO1065
Figure 3962: DNA344829, NP_683699.1, 223851_s_at
Figure 3963: PRO95387
Figure 3964: DNA335398, AF132202, 223940_x_at
Figure 3965A-B: DNA344830, NM_004830,
223947_s_at
Figure 3966: PRO95388
Figure 3967: DNA335568, NM_024022, 223948_s_at
Figure 3968: PRO89910
Figure 3969: DNA327213, NM_032405, 223949_at
Figure 3970: PRO83482
Figure 3971: DNA344831, NM_013324, 223961_s_at
Figure 3972: PRO37588
Figure 3973: DNA324248, NM_004509, 223980_s_at
Figure 3974: PRO80932
Figure 3975: DNA344832, AF130059, 223991_s_at
Figure 3976: PRO95389
Figure 3977: DNA344833, NP_002594.1, 224046_s_at
Figure 3978: PRO95390
Figure 3979: DNA344834, NM_172234, 224156_x_at
Figure 3980: PRO95391
Figure 3981A-C: DNA227619, NP_054831.1,
224218_s_at
Figure 3982: PRO38082
Figure 3983: DNA324707, NM_013237, 224232_s_at
Figure 3984: PRO81339
Figure 3985: DNA329370, NM_018141, 224247_s_at
Figure 3986: PRO84949
Figure 3987: DNA344835, NP_115942.1, 224285_at
Figure 3988: PRO78450
Figure 3989: DNA330558, NP_057588.1, 224330_s_at
Figure 3990: PRO84950
Figure 3991: DNA344836, NP_115868.1, 224331_s_at
Figure 3992: PRO84951
Figure 3993: DNA344837, BC015060, 224345_x_at
Figure 3994: PRO86616
Figure 3995: DNA344838, NM_018725, 224361_s_at
Figure 3996: PRO19612
Figure 3997: DNA335328, NP_116010.1, 224367_at
Figure 3998: PRO89703
Figure 3999: DNA330334, NP_114402.1, 224368_s_at
Figure 4000: PRO85557
Figure 4001: DNA328323, NP_114148.2, 224428_s_at
Figure 4002: PRO69531
Figure 4003: DNA344839, NP_113668.2, 224450_s_at
Figure 4004: PRO95392
Figure 4005: DNA328885, NM_018638, 224453_s_at
Figure 4006: PRO50294
Figure 4007: DNA344840, NP_116186.1, 224461_s_at
Figure 4008: PRO95393
Figure 4009: DNA329373, NP_115722.1, 224467_s_at
Figure 4010: PRO84952
Figure 4011: DNA323732, NP_057260.2, 224472_x_at
Figure 4012: PRO80490

Figure 4013: DNA344841, BC006236, 224480_s_at
Figure 4014: PRO95394
Figure 4015A-C: DNA344842, AJ314646, 224482_s_at
Figure 4016: DNA344843, BC006384, 224507_s_at
Figure 4017: PRO95396
Figure 4018: DNA344844, 242250.1, 224508_at
Figure 4019: PRO95397
Figure 4020: DNA327977, NP_115886.1, 224518_s_at
Figure 4021: PRO83898
Figure 4022: DNA329374, NP_115735.1, 224523_s_at
Figure 4023: PRO84953
Figure 4024: DNA344845, NM_148902, 224553_s_at
Figure 4025: PRO95398
Figure 4026: DNA344846, 1453417.19, 224559_at
Figure 4027: PRO95399
Figure 4028A-E: DNA344847, AF001893, 224566_at
Figure 4029: PRO95400
Figure 4030: DNA334965, D87666, 224567_x_at
Figure 4031: DNA330569, BC020516, 224572_s_at
Figure 4032: DNA344848, NP_066972.1, 224583_at
Figure 4033: PRO82633
Figure 4034A-B: DNA334919, NP_536856.2,
224596_at
Figure 4035: PRO89354
Figure 4036: DNA344849, 1383705.7, 224601_at
Figure 4037: PRO95401
Figure 4038: DNA331396, 1357555.1, 224603_at
Figure 4039: PRO86461
Figure 4040: DNA255362, DNA255362, 224604_at
Figure 4041: DNA344850, BC017399, 224605_at
Figure 4042: PRO95402
Figure 4043: DNA344851, AF070636, 224609_at
Figure 4044: PRO95403
Figure 4045: DNA344852, 348196.115, 224610_at
Figure 4046: PRO95404
Figure 4047: DNA329376, BAA91036.1, 224632_at
Figure 4048: PRO84954
Figure 4049A-B: DNA344853, 361207.5, 224634_at
Figure 4050: PRO95405
Figure 4051: DNA344854, AK093442, 224654_at
Figure 4052: PRO95406
Figure 4053A-B: DNA344855, BAB21782.1,
224674_at
Figure 4054: PRO49364
Figure 4055A-B: DNA344856, AL161973, 224685_at
Figure 4056A-B: DNA330574, BAA86542.2,
224698_at
Figure 4057: PRO85755
Figure 4058: DNA329378, BC022990, 224714_at
Figure 4059: PRO84956
Figure 4060: DNA330577, NP_443076.1, 224715_at
Figure 4061: PRO85758
Figure 4062: DNA330579, NP_612434.1, 224719_s_at
Figure 4063: PRO85760
Figure 4064: DNA344857, NP_653202.1, 224733_at
Figure 4065: PRO95408
Figure 4066: DNA257352, DNA257352, 224739_at
Figure 4067: PRO51940
Figure 4068: DNA344858, 887619.58, 224741_x_at
Figure 4069: PRO95409
Figure 4070: DNA330581, NP_542399.1, 224753_at
Figure 4071: PRO82014
Figure 4072A-B: DNA344859, NP_065875.1,
224764_at
Figure 4073: PRO95410
Figure 4074: DNA336077, BC035511, 224783_at
Figure 4075: PRO90299
Figure 4076A-B: DNA333692, AB033075, 224790_at
Figure 4077: DNA228087, DNA228087, 224793_s_at
Figure 4078: PRO38550
Figure 4079A-B: DNA287330, BAA86479.1,
224799_at
Figure 4080: PRO69594
Figure 4081A-B: DNA330584, NP_065881.1,
224800_at
Figure 4082: PRO85764
Figure 4083A-B: DNA287330, AB032991, 224801_at
Figure 4084: DNA331397, AK001723, 224802_at
Figure 4085: PRO23259
Figure 4086: DNA344860, NP_699164.1, 224819_at
Figure 4087: PRO95411
Figure 4088A-B: DNA330559, BAB21791.1,
224832_at
Figure 4089: PRO85741
Figure 4090A-B: DNA330809, 336997.1, 224837_at
Figure 4091: PRO85973
Figure 4092A-B: DNA330522, NM_032682,
224838_at
Figure 4093: PRO85712
Figure 4094A-B: DNA344861, NP_597700.1,
224839_s_at
Figure 4095: PRO95412
Figure 4096A-B: DNA324748, NP_004108.1,
224840_at
Figure 4097: PRO36841
Figure 4098A-B: DNA344862, AF141346,
224841_x_at
Figure 4099: DNA344863, BC027989, 224847_at
Figure 4100: PRO95414
Figure 4101A-C: DNA329379, 010205.2, 224848_at
Figure 4102: PRO84957
Figure 4103: DNA344864, NP_116199.1, 224850_at
Figure 4104: PRO95415
Figure 4105A-B: DNA324748, NM_004117,
224856_at
Figure 4106: PRO36841
Figure 4107: DNA329381, D28589, 224870_at
Figure 4108A-B: DNA344865, NP_065871.1,
224909_s_at
Figure 4109: PRO95416
Figure 4110: DNA344866, AAH10736.1, 224913_s_at
Figure 4111: PRO95417

Figure 4112: DNA330591, NP_115865.1, 224919_at
Figure 4113: PRO85771
Figure 4114A-B: DNA344867, BC009948, 224925_at
Figure 4115: PRO95418
Figure 4116A-B: DNA228196, BAA92674.1,
224937_at
Figure 4117: PRO38661
Figure 4118: DNA336269, 346724.14, 224944_at
Figure 4119: PRO90430
Figure 4120: DNA344868, 7769724.1, 224989_at
Figure 4121: PRO95419
Figure 4122: DNA329384, NP_777581.1, 224990_at
Figure 4123: PRO84960
Figure 4124: DNA344869, BC034247, 225036_at
Figure 4125: PRO95420
Figure 4126: DNA344870, NP_061189.1, 225081_s_at
Figure 4127: PRO95421
Figure 4128: DNA330598, 1384569.2, 225086_at
Figure 4129: PRO85776
Figure 4130A-E: DNA329391, 233747.10, 225097_at
Figure 4131: PRO84967
Figure 4132A-B: DNA327993, 898436.7, 225133_at
Figure 4133: PRO81138
Figure 4134: DNA344871, BC037573, 225148_at
Figure 4135: PRO95422
Figure 4136: DNA344872, NP_079272.4, 225158_at
Figure 4137: PRO84969
Figure 4138: DNA344873, NM_024996, 225161_at
Figure 4139: PRO84969
Figure 4140: DNA330604, NP_277050.1, 225171_at
Figure 4141: PRO85782
Figure 4142: DNA330604, NM_033515, 225173_at
Figure 4143: PRO85782
Figure 4144: DNA344874, BC040556, 225175_s_at
Figure 4145: PRO95423
Figure 4146: DNA344875, AAH27990.1, 225178_at
Figure 4147: PRO83914
Figure 4148A-B: DNA344876, 335186.18, 225195_at
Figure 4149: PRO95424
Figure 4150: DNA336053, NP_110438.1, 225196_s_at
Figure 4151: PRO90282
Figure 4152: DNA344877, 233597.34, 225220_at
Figure 4153: PRO95425
Figure 4154: DNA344878, NP_542763.1, 225252_at
Figure 4155: PRO95426
Figure 4156A-B: DNA330605, 233102.7, 225265_at
Figure 4157: PRO85783
Figure 4158A-B: DNA258863, DNA258863,
225266_at
Figure 4159A-B: DNA344879, 7771332.17, 225285_at
Figure 4160: PRO95427
Figure 4161A-B: DNA330606, 475590.1, 225290_at
Figure 4162: PRO85784
Figure 4163: DNA344880, NP_149100.1, 225291_at
Figure 4164: PRO95428
Figure 4165: DNA339708, NP_116147.1, 225309_at
Figure 4166: PRO91438
Figure 4167: DNA344881, 1455093.11, 225315_at
Figure 4168: PRO95429
Figure 4169: DNA324422, DNA324422, 225331_at
Figure 4170: PRO81086
Figure 4171A-B: DNA344882, 331507.16, 225342_at
Figure 4172: PRO95430
Figure 4173: DNA344883, 475538.46, 225351_at
Figure 4174: PRO95431
Figure 4175: DNA344884, 475309.4, 225356_at
Figure 4176: PRO95432
Figure 4177A-B: DNA330742, 476805.1, 225363_at
Figure 4178: PRO85910
Figure 4179: DNA327965, NP_060760.1, 225367_at
Figure 4180: PRO83888
Figure 4181: DNA329401, NP_612403.2, 225386_s_at
Figure 4182: PRO84976
Figure 4183: DNA344885, NM_173647, 225414_at
Figure 4184: PRO95433
Figure 4185: DNA344886, NP_116258.1, 225439_at
Figure 4186: PRO52516
Figure 4187A-B: DNA330617, 336147.2, 225447_at
Figure 4188: PRO59923
Figure 4189: DNA330618, CAB55990.1, 225458_at
Figure 4190: PRO85793
Figure 4191: DNA344887, BC022333, 225470_at
Figure 4192: PRO95434
Figure 4193A-B: DNA328006, 234824.7, 225478_at
Figure 4194: PRO83924
Figure 4195A-B: DNA334963, NM_032943,
225496_s_at
Figure 4196: PRO89395
Figure 4197A-B: DNA344888, AL833216, 225519_at
Figure 4198: PRO95435
Figure 4199: DNA331675, NP_056255.1, 225520_at
Figure 4200: PRO86670
Figure 4201A-B: DNA344889, BAB33341.1,
225525_at
Figure 4202: PRO95436
Figure 4203: DNA330621, AAF71051.1, 225535_s_at
Figure 4204: PRO85795
Figure 4205: DNA328010, NP_149016.1, 225557_at
Figure 4206: PRO83928
Figure 4207A-B: DNA344890, NM_057170,
225558_at
Figure 4208: PRO95437
Figure 4209A-B: DNA344891, AL832362, 225570_at
Figure 4210: PRO95438
Figure 4211A-B: DNA329407, 234687.2, 225606_at
Figure 4212: PRO84980
Figure 4213A-B: DNA344892, AK074072, 225608_at
Figure 4214A-C: DNA344893, 197240.1, 225611_at
Figure 4215: PRO95440
Figure 4216: DNA331399, 994419.37, 225622_at
Figure 4217: PRO86463
Figure 4218A-B: DNA340041, AK024473, 225624_at

Figure 4219A-B: DNA331400, NP_060910.2,
225626_at
Figure 4220: PRO86464
Figure 4221A-B: DNA344894, BAA96062.2,
225629_s_at
Figure 4222: PRO95441
Figure 4223: DNA344895, 473880.39, 225636_at
Figure 4224: PRO95442
Figure 4225: DNA344896, NM_148170, 225647_s_at
Figure 4226: PRO95443
Figure 4227A-B: DNA288261, NP_037414.2,
225655_at
Figure 4228: PRO70021
Figure 4229: DNA344897, NP_612496.1, 225657_at
Figure 4230: PRO81096
Figure 4231A-B: DNA344898, NM_133646,
225662_at
Figure 4232: PRO95444
Figure 4233A-B: DNA344899, AF480462, 225665_at
Figure 4234: PRO95445
Figure 4235: DNA332522, 235504.1, 225685_at
Figure 4236: PRO87339
Figure 4237: DNA328012, BC017873, 225686_at
Figure 4238: PRO83930
Figure 4239: DNA329410, DNA329410, 225699_at
Figure 4240: PRO84982
Figure 4241: DNA304821, AAH11254.1, 225706_at
Figure 4242: PRO71227
Figure 4243: DNA344900, NP_689735.1, 225707_at
Figure 4244: PRO95446
Figure 4245: DNA344901, 1383664.3, 225710_at
Figure 4246: PRO95447
Figure 4247: DNA344902, 040422.37, 225711_at
Figure 4248: PRO95448
Figure 4249A-B: DNA330634, 243208.1, 225725_at
Figure 4250: PRO85806
Figure 4251A-B: DNA255834, BAA86514.1,
225727_at
Figure 4252: PRO50889
Figure 4253: DNA325290, NP_116294.1, 225751_at
Figure 4254: PRO81837
Figure 4255A-B: DNA344903, 232693.1, 225752_at
Figure 4256: PRO95449
Figure 4257A-B: DNA344904, 344455.25,
225766_s_at
Figure 4258: PRO60223
Figure 4259: DNA344905, BC044244, 225775_at
Figure 4260: PRO95450
Figure 4261: DNA328016, NP_542409.1, 225783_at
Figure 4262: PRO83934
Figure 4263: DNA344906, 033730.20, 225796_at
Figure 4264: PRO95451
Figure 4265: DNA344907, BC009508, 225799_at
Figure 4266: PRO84986
Figure 4267A-B: DNA328001, 246799.1, 225801_at
Figure 4268: PRO83920
Figure 4269: DNA330637, NP_478136.1, 225803_at
Figure 4270: PRO85809
Figure 4271: DNA344908, BC046199, 225834_at
Figure 4272: PRO95452
Figure 4273: DNA335325, 199593.7, 225835_at
Figure 4274: PRO89700
Figure 4275: DNA329417, 411336.1, 225842_at
Figure 4276: PRO84989
Figure 4277: DNA329418, NP_660152.1, 225850_at
Figure 4278: PRO19906
Figure 4279: DNA344909, 001697.17, 225857_s_at
Figure 4280: PRO95453
Figure 4281A-B: DNA258903, DNA258903,
225864_at
Figure 4282: DNA344910, BC035314, 225866_at
Figure 4283: PRO81453
Figure 4284A-B: DNA344911, NP_733837.1,
225887_at
Figure 4285: PRO95454
Figure 4286: DNA330642, NP_115494.1, 225898_at
Figure 4287: PRO85814
Figure 4288A-B: DNA331403, NP_150601.1,
225912_at
Figure 4289: PRO86467
Figure 4290: DNA344912, 232561.20, 225922_at
Figure 4291: PRO95455
Figure 4292A-B: DNA328790, 481415.9, 225927_at
Figure 4293: PRO84535
Figure 4294A-B: DNA344913, AL833201,
225929_s_at
Figure 4295: PRO95456
Figure 4296: DNA344914, BC032220, 225931_s_at
Figure 4297: PRO95457
Figure 4298A-B: DNA344915, AL390144,
225959_s_at
Figure 4299: PRO95458
Figure 4300: DNA344916, 202205.5, 225967_s_at
Figure 4301: PRO95459
Figure 4302A-B: DNA344917, BC037303, 225984_at
Figure 4303: PRO95460
Figure 4304A-B: DNA329423, BAB21799.1,
226003_at
Figure 4305: PRO84994
Figure 4306A-B: DNA335463, 246054.6, 226021_at
Figure 4307: PRO89818
Figure 4308A-B: DNA344918, 347857.19, 226025_at
Figure 4309: PRO95461
Figure 4310: DNA335659, 027830.2, 226034_at
Figure 4311: PRO89988
Figure 4312A-B: DNA344919, 331817.1, 226039_at
Figure 4313: PRO95462
Figure 4314: DNA344920, NP_079382.2, 226075_at
Figure 4315: PRO95463
Figure 4316A-B: DNA344921, 1500207.3, 226085_at
Figure 4317: PRO95464
Figure 4318A-B: DNA344922, NM_012081,

226099_at
Figure 4319: PRO37794
Figure 4320: DNA329425, BC008294, 226117_at
Figure 4321A-B: DNA344923, AK027859, 226118_at
Figure 4322: PRO95465
Figure 4323: DNA257557, DNA257557, 226123_at
Figure 4324: DNA330657, 198409.1, 226140_s_at
Figure 4325: PRO85829
Figure 4326: DNA344924, 243488.38, 226150_at
Figure 4327: PRO95466
Figure 4328A-B: DNA344925, BAB67795.1,
226184_at
Figure 4329: PRO95467
Figure 4330: DNA344926, 128514.91, 226193_x_at
Figure 4331: PRO95468
Figure 4332: DNA344927, NP_659489.1, 226199_at
Figure 4333: PRO91821
Figure 4334: DNA344928, AF306698, 226214_at
Figure 4335: PRO95469
Figure 4336A-B: DNA329428, 1446144.8, 226218_at
Figure 4337: PRO84999
Figure 4338A-B: DNA344929, 1445835.2, 226225_at
Figure 4339: PRO95470
Figure 4340: DNA344930, 7761926.1, 226233_at
Figure 4341: PRO95471
Figure 4342: DNA344931, BX248749, 226241_s_at
Figure 4343A-C: DNA344932, 987122.2, 226251_at
Figure 4344: PRO95473
Figure 4345: DNA344933, NP_071931.1, 226264_at
Figure 4346: PRO95474
Figure 4347: DNA330666, 199829.14, 226272_at
Figure 4348: PRO85838
Figure 4349: DNA344934, BC036402, 226275_at
Figure 4350: DNA344935, 347831.7, 226282_at
Figure 4351: PRO95476
Figure 4352: DNA328028, NP_005773.1, 226319_s_at
Figure 4353: PRO83945
Figure 4354: DNA328028, NM_005782, 226320_at
Figure 4355: PRO83945
Figure 4356: DNA344936, 7696668.2, 226333_at
Figure 4357: PRO95477
Figure 4358: DNA344937, 218237.1, 226350_at
Figure 4359: PRO95478
Figure 4360A-B: DNA331407, 198233.1, 226352_at
Figure 4361: PRO86471
Figure 4362: DNA329430, NP_116191.2, 226353_at
Figure 4363: PRO38524
Figure 4364A-B: DNA330675, 177663.2, 226372_at
Figure 4365: PRO85847
Figure 4366A-B: DNA344938, AL832599, 226390_at
Figure 4367: DNA335613, NP_116178.1, 226401_at
Figure 4368: PRO89948
Figure 4369: DNA344939, BC044951, 226410_at
Figure 4370: DNA344940, 407605.1, 226431_at
Figure 4371: PRO95480
Figure 4372A-B: DNA344941, 474795.3, 226438_at
Figure 4373: PRO95481
Figure 4374: DNA330678, 401430.1, 226444_at
Figure 4375: PRO85850
Figure 4376: DNA344942, AL390172, 226517_at
Figure 4377: PRO95482
Figure 4378: DNA344943, 334193.1, 226528_at
Figure 4379: PRO95483
Figure 4380: DNA304794, NP_115521.2, 226541_at
Figure 4381: PRO71206
Figure 4382: DNA344944, 978789.5, 226545_at
Figure 4383: PRO95484
Figure 4384A-B: DNA344945, 237667.2, 226568_at
Figure 4385: PRO95485
Figure 4386A-B: DNA328031, 331264.1, 226587_at
Figure 4387: PRO83948
Figure 4388: DNA344946, AK098194, 226609_at
Figure 4389: PRO95486
Figure 4390: DNA344947, AAM76703.1, 226610_at
Figure 4391: PRO95487
Figure 4392: DNA344948, AF514992, 226611_s_at
Figure 4393: DNA328033, 1446419.1, 226625_at
Figure 4394: PRO83949
Figure 4395: DNA344949, NP_689775.1, 226661_at
Figure 4396: PRO95489
Figure 4397: DNA338349, NM_173626, 226679_at
Figure 4398: PRO91021
Figure 4399A-B: DNA328035, 336832.2, 226682_at
Figure 4400: PRO83951
Figure 4401A-B: DNA344950, 239418.7, 226683_at
Figure 4402: PRO95490
Figure 4403A-C: DNA329129, NM_007203,
226694_at
Figure 4404: PRO84288
Figure 4405: DNA328037, AAH16969.1, 226702_at
Figure 4406: PRO83952
Figure 4407: DNA344951, NP_660202.1, 226707_at
Figure 4408: PRO95491
Figure 4409: DNA344952, 7762613.1, 226736_at
Figure 4410: PRO95492
Figure 4411A-B: DNA344953, NP_689561.1,
226738_at
Figure 4412: PRO95493
Figure 4413A-B: DNA344954, 7762967.1, 226756_at
Figure 4414: PRO95494
Figure 4415: DNA338085, NP_001538.2, 226757_at
Figure 4416: PRO90963
Figure 4417: DNA344955, 232416.1, 226759_at
Figure 4418: PRO95495
Figure 4419A-B: DNA344956, 898708.1, 226760_at
Figure 4420: PRO95496
Figure 4421A-B: DNA344957, AL832206, 226782_at
Figure 4422: PRO95497
Figure 4423A-B: DNA332574, 1383798.8, 226789_at
Figure 4424: PRO87370
Figure 4425A-B: DNA330694, 481455.4, 226810_at
Figure 4426: PRO85865

Figure 4427: DNA328038, 216863.2, 226811_at
Figure 4428: PRO83953
Figure 4429A-B: DNA344958, NP_115939.1,
226829_at
Figure 4430: PRO95498
Figure 4431: DNA344959, 221888.1, 226832_at
Figure 4432: PRO95499
Figure 4433: DNA344960, 999400.45, 226864_at
Figure 4434: PRO95500
Figure 4435: DNA344961, 255540.3, 226867_at
Figure 4436: PRO95501
Figure 4437: DNA344962, Z99705, 226878_at
Figure 4438: DNA344963, 366261.31, 226883_at
Figure 4439: PRO95503
Figure 4440: DNA330564, NP_115885.1, 226906_s_at
Figure 4441: PRO85746
Figure 4442: DNA328044, DNA328044, 226936_at
Figure 4443: PRO83958
Figure 4444: DNA154627, DNA154627, 226976_at
Figure 4445: DNA344964, 7696742.1, 226982_at
Figure 4446: PRO95504
Figure 4447: DNA344965, 7769585.1, 226991_at
Figure 4448: PRO95505
Figure 4449: DNA339717, NP_150281.1, 227006_at
Figure 4450: PRO91445
Figure 4451A-B: DNA275168, DNA275168,
227013_at
Figure 4452: PRO62870
Figure 4453: DNA344966, NP_065170.1, 227014_at
Figure 4454: PRO86261
Figure 4455A-B: DNA330705, 198782.1, 227020_at
Figure 4456: PRO85876
Figure 4457: DNA344967, 350955.33, 227030_at
Figure 4458: PRO95506
Figure 4459A-C: DNA344968, AB055890, 227039_at
Figure 4460: PRO95507
Figure 4461: DNA344969, 7769752.1, 227052_at
Figure 4462: PRO95508
Figure 4463: DNA336061, NP_660322.1, 227066_at
Figure 4464: PRO90288
Figure 4465: DNA344970, 7698705.3, 227074_at
Figure 4466: PRO95509
Figure 4467A-B: DNA344971, 7697931.24, 227110_at
Figure 4468: PRO95510
Figure 4469: DNA330709, 7692923.1, 227117_at
Figure 4470: PRO85880
Figure 4471: DNA344972, 7698297.2, 227124_at
Figure 4472: PRO95511
Figure 4473: DNA333713, 407443.5, 227125_at
Figure 4474: PRO88341
Figure 4475: DNA344973, AK098237, 227141_at
Figure 4476: PRO95512
Figure 4477: DNA340090, AAH07902.1, 227161_at
Figure 4478: PRO91590
Figure 4479A-B: DNA344974, NP_689899.1,
227166_at
Figure 4480: PRO38669
Figure 4481: DNA344975, NP_612350.1, 227172_at
Figure 4482: PRO95513
Figure 4483: DNA344976, 332013.1, 227177_at
Figure 4484: PRO95514
Figure 4485: DNA267411, NP_659443.1, 227182_at
Figure 4486: PRO57098
Figure 4487A-B: DNA344977, 408890.1, 227210_at
Figure 4488: PRO95515
Figure 4489: DNA344978, AL834179, 227237_x_at
Figure 4490: PRO95516
Figure 4491A-B: DNA344979, AL833296, 227239_at
Figure 4492: PRO95517
Figure 4493: DNA330717, 232831.10, 227290_at
Figure 4494: PRO85888
Figure 4495: DNA344980, BC042036, 227291_s_at
Figure 4496: PRO95518
Figure 4497A-B: DNA344981, 337195.1, 227318_at
Figure 4498: PRO95519
Figure 4499: DNA329446, NM_078468, 227322_s_at
Figure 4500: PRO85014
Figure 4501: DNA344982, AK097987, 227353_at
Figure 4502: PRO95520
Figure 4503: DNA336553, AK095177, 227354_at
Figure 4504: PRO90632
Figure 4505: DNA344983, 211443.3, 227357_at
Figure 4506: PRO95521
Figure 4507: DNA344984, 163230.9, 227361_at
Figure 4508: PRO95522
Figure 4509: DNA344985, BC036414, 227369_at
Figure 4510: PRO95523
Figure 4511: DNA344986, BC045695, 227379_at
Figure 4512: PRO95524
Figure 4513: DNA344987, 244251.8, 227383_at
Figure 4514: PRO95525
Figure 4515: DNA332679, 335037.7, 227396_at
Figure 4516: PRO87464
Figure 4517: DNA226872, NP_001955.1, 227404_s_at
Figure 4518: PRO37335
Figure 4519: DNA344988, 200338.2, 227410_at
Figure 4520: PRO95526
Figure 4521: DNA344989, NP_659486.1, 227413_at
Figure 4522: PRO95527
Figure 4523A-C: DNA344990, 410523.22, 227426_at
Figure 4524: PRO12910
Figure 4525A-B: DNA340206, NP_079420.2,
227438_at
Figure 4526: PRO91701
Figure 4527A-B: DNA328054, 233014.1, 227458_at
Figure 4528: PRO83968
Figure 4529: DNA344991, NP_005222.2, 227473_at
Figure 4530: PRO95528
Figure 4531A-B: DNA344992, AL832945, 227478_at
Figure 4532: PRO95529
Figure 4533: DNA344993, 221804.1, 227489_at
Figure 4534: PRO95530

Figure 4535: DNA344994, 197788.1, 227491_at
Figure 4536: PRO95531
Figure 4537: DNA344995, 1449825.8, 227503_at
Figure 4538: PRO95532
Figure 4539: DNA344996, 887619.55, 227517_s_at
Figure 4540: PRO95533
Figure 4541A-B: DNA331401, 336865.4, 227525_at
Figure 4542: PRO86465
Figure 4543: DNA340229, NP_443070.1, 227552_at
Figure 4544: PRO91724
Figure 4545: DNA344997, AAM09645.1, 227560_at
Figure 4546: PRO95534
Figure 4547A-B: DNA287193, BAA92611.1,
227606_s_at
Figure 4548: PRO69479
Figure 4549: DNA330730, BC010846, 227607_at
Figure 4550: PRO85899
Figure 4551A-B: DNA344998, NM_170709,
227627_at
Figure 4552: PRO95535
Figure 4553A-B: DNA344999, BC028212, 227645_at
Figure 4554: PRO95536
Figure 4555A-B: DNA345000, 1081047.29, 227670_at
Figure 4556: PRO95537
Figure 4557: DNA330734, NP_116143.2, 227686_at
Figure 4558: PRO85903
Figure 4559: DNA345001, 020646.23, 227697_at
Figure 4560: PRO95538
Figure 4561: DNA323723, NP_060658.1, 227700_x_at
Figure 4562: PRO80483
Figure 4563: DNA345002, AJ420488, 227708_at
Figure 4564: PRO95539
Figure 4565A-B: DNA333658, 1454272.17, 227755_at
Figure 4566: PRO88297
Figure 4567A-B: DNA345003, 232924.7, 227767_at
Figure 4568: PRO95540
Figure 4569: DNA332527, 028115.17, 227769_at
Figure 4570: PRO87344
Figure 4571: DNA339728, NP_542382.1, 227787_s_at
Figure 4572: PRO91456
Figure 4573: DNA345004, 196714.3, 227798_at
Figure 4574: PRO95541
Figure 4575: DNA345005, AL137420, 227818_at
Figure 4576: DNA345006, NP_689613.1, 227856_at
Figure 4577: PRO95543
Figure 4578: DNA260485, DNA260485, 227867_at
Figure 4579: PRO54411
Figure 4580: DNA336725, AY032883, 227877_at
Figure 4581: PRO90794
Figure 4582: DNA345007, 198947.2, 227889_at
Figure 4583: PRO95544
Figure 4584: DNA329481, NP_057234.2, 227915_at
Figure 4585: PRO60949
Figure 4586: DNA329456, NM_016042, 227916_x_at
Figure 4587: PRO85023
Figure 4588: DNA345008, 199363.8, 227930_at
Figure 4589: PRO95545
Figure 4590: DNA345009, 040316.1, 227944_at
Figure 4591: PRO95546
Figure 4592: DNA345010, 1101718.57, 227984_at
Figure 4593: PRO95547
Figure 4594: DNA150660, NP_057151.1, 228019_s_at
Figure 4595: PRO12397
Figure 4596: DNA345011, 241960.67, 228030_at
Figure 4597: PRO95548
Figure 4598: DNA345012, 156397.1, 228032_s_at
Figure 4599: PRO95549
Figure 4600: DNA334778, 1383803.1, 228049_x_at
Figure 4601: PRO89231
Figure 4602: DNA331655, 1449874.3, 228053_s_at
Figure 4603: PRO86651
Figure 4604: DNA330745, NP_612428.1, 228069_at
Figure 4605: PRO85913
Figure 4606: DNA345013, NP_694968.1, 228071_at
Figure 4607: PRO23647
Figure 4608: DNA345014, AAH25407.1, 228080_at
Figure 4609: PRO95550
Figure 4610: DNA345015, NP_694938.1, 228094_at
Figure 4611: PRO95551
Figure 4612: DNA330436, NP_037394.1, 228098_s_at
Figure 4613: PRO85639
Figure 4614: DNA151725, DNA151725, 228107_at
Figure 4615: PRO12014
Figure 4616A-C: DNA330747, 200650.1, 228109_at
Figure 4617: PRO85915
Figure 4618: DNA340579, BC040547, 228113_at
Figure 4619: PRO92247
Figure 4620A-B: DNA334022, NP_569713.1,
228167_at
Figure 4621: PRO88589
Figure 4622: DNA345016, CAD38596.1, 228245_s_at
Figure 4623: PRO95552
Figure 4624: DNA260948, DNA260948, 228273_at
Figure 4625: PRO54700
Figure 4626: DNA330755, BC020784, 228280_at
Figure 4627: PRO85923
Figure 4628: DNA345017, NP_659455.2, 228281_at
Figure 4629: PRO95553
Figure 4630: DNA340370, DNA340370, 228283_at
Figure 4631: PRO91834
Figure 4632: DNA339731, NP_612380.1, 228298_at
Figure 4633: PRO91459
Figure 4634: DNA345018, 333338.2, 228314_at
Figure 4635: PRO95554
Figure 4636A-B: DNA345019, 1453154.2, 228324_at
Figure 4637: PRO95555
Figure 4638: DNA345020, NM_174889, 228355_s_at
Figure 4639: PRO95556
Figure 4640: DNA336744, BC007609, 228361_at
Figure 4641: PRO90814
Figure 4642: DNA345021, 7769848.1, 228363_at
Figure 4643: PRO95557

Figure 4644: DNA345022, AF378122, 228376_at
Figure 4645: PRO95558
Figure 4646: DNA330759, 337444.1, 228390_at
Figure 4647: PRO85926
Figure 4648A-B: DNA330760, 330900.8, 228401_at
Figure 4649: PRO85927
Figure 4650A-B: DNA339727, NP_542179.1,
228410_at
Figure 4651: PRO91455
Figure 4652: DNA345023, NM_015975, 228483_s_at
Figure 4653: PRO95559
Figure 4654A-C: DNA330761, 388991.1, 228487_s_at
Figure 4655: PRO85928
Figure 4656A-B: DNA328454, NP_057525.1,
228496_s_at
Figure 4657: PRO4330
Figure 4658: DNA345024, 412954.22, 228532_at
Figure 4659: PRO95560
Figure 4660: DNA336376, 234038.1, 228560_at
Figure 4661: PRO91061
Figure 4662: DNA345025, 1453417.9, 228582_x_at
Figure 4663: PRO95561
Figure 4664: DNA150004, DNA150004, 228592_at
Figure 4665: PRO4644
Figure 4666: DNA345026, BC035088, 228654_at
Figure 4667: PRO95562
Figure 4668A-B: DNA345027, 7698079.3, 228658_at
Figure 4669: PRO95563
Figure 4670: DNA335393, 025911.1, 228708_at
Figure 4671: PRO89758
Figure 4672A-B: DNA345028, 7695185.17, 228722_at
Figure 4673: PRO95564
Figure 4674: DNA330772, 286623.2, 228729_at
Figure 4675: PRO85937
Figure 4676: DNA257559, NP_116272.1, 228737_at
Figure 4677: PRO52129
Figure 4678: DNA328082, BC014851, 228762_at
Figure 4679: PRO83994
Figure 4680: DNA345029, 998974.45, 228809_at
Figure 4681: PRO95565
Figure 4682: DNA260010, DNA260010, 228812_at
Figure 4683: DNA330777, DNA330777, 228869_at
Figure 4684: PRO85941
Figure 4685: DNA345030, 7693726.1, 228879_at
Figure 4686: PRO95566
Figure 4687: DNA345031, 021903.1, 228910_at
Figure 4688: PRO95567
Figure 4689: DNA345032, 1087130.10, 228931_at
Figure 4690: PRO95568
Figure 4691: DNA329447, BC016981, 228948_at
Figure 4692: PRO85015
Figure 4693A-B: DNA345033, AY198415, 228964_at
Figure 4694: PRO95569
Figure 4695A-B: DNA340099, BC028424, 228980_at
Figure 4696: PRO91599
Figure 4697: DNA345034, AL137573, 229007_at
Figure 4698: PRO95570
Figure 4699A-B: DNA336693, NP_277037.1,
229016_s_at
Figure 4700: PRO90766
Figure 4701: DNA330786, 233085.1, 229029_at
Figure 4702: PRO85950
Figure 4703: DNA336085, DNA336085, 229041_s_at
Figure 4704: PRO90304
Figure 4705: DNA330777, 330848.1, 229045_at
Figure 4706: PRO85941
Figure 4707: DNA345035, BAC04479.1, 229065_at
Figure 4708: PRO95571
Figure 4709: DNA330790, NP_116133.1, 229070_at
Figure 4710: PRO85954
Figure 4711: DNA330791, 7697349.2, 229072_at
Figure 4712: PRO85955
Figure 4713: DNA332520, 344561.1, 229101_at
Figure 4714: PRO87337
Figure 4715A-B: DNA345036, 468481.1, 229116_at
Figure 4716: PRO95572
Figure 4717A-D: DNA345037, 903479.18, 229287_at
Figure 4718: PRO95573
Figure 4719: DNA333664, 237320.4, 229295_at
Figure 4720: PRO88303
Figure 4721A-B: DNA255352, AB033060, 229354_at
Figure 4722: DNA345038, NM_024711, 229367_s_at
Figure 4723: PRO95574
Figure 4724: DNA345039, 199232.2, 229390_at
Figure 4725: PRO57551
Figure 4726: DNA255197, DNA255197, 229391_s_at
Figure 4727: PRO50276
Figure 4728: DNA335178, AF402776, 229437_at
Figure 4729: PRO69678
Figure 4730: DNA330797, 211332.1, 229442_at
Figure 4731: PRO85961
Figure 4732: DNA328090, 007911.2, 229450_at
Figure 4733: PRO84001
Figure 4734A-B: DNA237810, DNA237810,
229490_s_at
Figure 4735: PRO38918
Figure 4736: DNA338094, AK093350, 229521_at
Figure 4737: PRO90970
Figure 4738: DNA330799, 481875.1, 229551_x_at
Figure 4739: PRO85963
Figure 4740: DNA334937, BAB71227.1, 229553_at
Figure 4741: PRO89370
Figure 4742A-B: DNA345040, 451858.13, 229572_at
Figure 4743: PRO95575
Figure 4744A-B: DNA345041, AL834393, 229594_at
Figure 4745: DNA345042, NP_689831.1, 229603_at
Figure 4746: PRO95577
Figure 4747: DNA345043, 401253.39, 229604_at
Figure 4748: PRO95578
Figure 4749: DNA345044, BC025714, 229606_at
Figure 4750: PRO95579
Figure 4751: DNA333760, 098138.1, 229629_at

Figure 4752: PRO88384
Figure 4753: DNA345045, BC034328, 229638_at
Figure 4754: DNA345046, AL833184, 229686_at
Figure 4755: PRO95581
Figure 4756: DNA334491, 428695.5, 229725_at
Figure 4757: PRO88993
Figure 4758A-B: DNA227985, NP_055107.1,
229733_s_at
Figure 4759: PRO38448
Figure 4760: DNA345047, 979808.6, 229764_at
Figure 4761: PRO95582
Figure 4762: DNA330807, 334422.1, 229814_at
Figure 4763: PRO85971
Figure 4764: DNA345048, 7683061.1, 229841_at
Figure 4765: PRO95583
Figure 4766: DNA345049, NP_694579.1, 229901_at
Figure 4767: PRO81858
Figure 4768: DNA333743, 243761.3, 229937_x_at
Figure 4769: PRO88368
Figure 4770: DNA345050, 221062.1, 229954_at
Figure 4771: PRO95584
Figure 4772A-B: DNA345051, NP_722579.1,
229971_at
Figure 4773: PRO6017
Figure 4774: DNA345052, NP_689413.1, 229980_s_at
Figure 4775: PRO69560
Figure 4776: DNA330811, 1382987.2, 230000_at
Figure 4777: PRO85975
Figure 4778: DNA338348, BAC03808.1, 230012_at
Figure 4779: PRO91019
Figure 4780: DNA345053, AL834186, 230060_at
Figure 4781: PRO95585
Figure 4782: DNA332487, DNA332487, 230110_at
Figure 4783: PRO87315
Figure 4784: DNA345054, 064937.11, 230141_at
Figure 4785: PRO95586
Figure 4786: DNA345055, NP_065391.1, 230170_at
Figure 4787: PRO88
Figure 4788: DNA345056, AL831898, 230179_at
Figure 4789: PRO95587
Figure 4790A-B: DNA345057, AL713763, 230180_at
Figure 4791: PRO95588
Figure 4792: DNA345058, AL832695, 230192_at
Figure 4793: DNA345059, 229293.16, 230206_at
Figure 4794: PRO95590
Figure 4795: DNA345060, 7692383.1, 230226_s_at
Figure 4796: PRO95591
Figure 4797: DNA345061, AK058039, 230292_at
Figure 4798: PRO95592
Figure 4799: DNA330818, 212282.1, 230304_at
Figure 4800: PRO85982
Figure 4801: DNA345062, 403834.1, 230383_x_at
Figure 4802: PRO95593
Figure 4803: DNA330822, 332195.1, 230391_at
Figure 4804: PRO85986
Figure 4805A-B: DNA345063, 234102.72, 230425_[?]
Figure 4806: PRO95594
Figure 4807: DNA345064, NP_653312.1, 230434_at
Figure 4808: PRO95595
Figure 4809: DNA330712, 1452648.12, 230466_s_at
Figure 4810: PRO85883
Figure 4811A-B: DNA330824, 333480.5, 230489_at
Figure 4812: PRO85988
Figure 4813: DNA332672, 335924.1, 230494_at
Figure 4814: PRO87457
Figure 4815: DNA332827, NP_660356.1, 230563_at
Figure 4816: PRO87594
Figure 4817: DNA345065, 234921.2, 230570_at
Figure 4818: PRO95596
Figure 4819A-C: DNA254793, NP_055987.1,
230618_s_at
Figure 4820: PRO49890
Figure 4821: DNA328098, 402974.1, 230653_at
Figure 4822: PRO84008
Figure 4823: DNA257789, NP_116219.1, 230656_s_at
Figure 4824: PRO52338
Figure 4825: DNA340247, DNA340247, 230753_at
Figure 4826: PRO91742
Figure 4827: DNA345066, AAH29505.1, 230756_at
Figure 4828: PRO95597
Figure 4829: DNA336379, 401125.10, 230795_at
Figure 4830: PRO90514
Figure 4831: DNA345067, 1132645.25, 230805_at
Figure 4832: PRO95598
Figure 4833: DNA332685, 234194.1, 230836_at
Figure 4834: PRO87470
Figure 4835: DNA338109, 211204.3, 230866_at
Figure 4836: PRO90980
Figure 4837: DNA336019, DNA336019, 230970_at
Figure 4838: DNA345068, 407233.3, 231093_at
Figure 4839: PRO95599
Figure 4840: DNA329405, AL117452, 231094_s_at
Figure 4841: DNA345069, 895820.1, 231106_at
Figure 4842: PRO95600
Figure 4843: DNA329473, 370473.13, 231124_x_at
Figure 4844: PRO85038
Figure 4845A-B: DNA226303, DNA226303,
231259_s_at
Figure 4846: PRO36766
Figure 4847A-B: DNA339703, NP_115970.2,
231396_s_at
Figure 4848: PRO91433
Figure 4849: DNA338354, DNA338354, 231576_at
Figure 4850: PRO91025
Figure 4851: DNA150808, M55542, 231577_s_at
Figure 4852: PRO12478
Figure 4853: DNA345070, NP_006630.1, 231747_at
Figure 4854: PRO34958
Figure 4855: DNA330839, NP_060908.1, 231769_at
Figure 4856: PRO86002
Figure 4857: DNA331119, NP_005433.2, 231776_at
[?]igure 4858: PRO50745

Figure 4859: DNA335123, AK027521, 231837_at
Figure 4860: PRO89526
Figure 4861: DNA345071, 1512952.7, 231866_at
Figure 4862: PRO95601
Figure 4863A-C: DNA339989, BAB21817.1,
231899_at
Figure 4864: PRO91497
Figure 4865A-B: DNA329476, 205127.1, 231929_at
Figure 4866: PRO85040
Figure 4867A-B: DNA256267, BAB13444.1,
231956_at
Figure 4868: PRO51311
Figure 4869: DNA345072, 978672.3, 232000_at
Figure 4870: PRO95602
Figure 4871: DNA345073, NP_056475.1, 232024_at
Figure 4872: PRO95603
Figure 4873: DNA323732, NM_016176, 232032_x_at
Figure 4874: PRO80490
Figure 4875: DNA330852, 1383611.1, 232138_at
Figure 4876: PRO86015
Figure 4877: DNA329094, NP_077285.1, 232160_s_at
Figure 4878: PRO84746
Figure 4879: DNA345074, 1077685.1, 232230_at
Figure 4880: PRO95604
Figure 4881: DNA345075, AJ278112, 232278_s_at
Figure 4882: PRO95605
Figure 4883: DNA329393, AF367998, 232296_s_at
Figure 4884: PRO84969
Figure 4885: DNA330862, 339154.9, 232304_at
Figure 4886: PRO86025
Figure 4887A-B: DNA340232, NP_443169.1,
232382_s_at
Figure 4888: PRO91727
Figure 4889: DNA328117, U25029, 232431_at
Figure 4890: PRO84024
Figure 4891: DNA340435, DNA340435, 232504_at
Figure 4892: DNA329286, NP_005691.2, 232510_s_at
Figure 4893: PRO69644
Figure 4894: DNA330868, 337037.1, 232584_at
Figure 4895: PRO86031
Figure 4896: DNA340361, DNA340361, 232615_at
Figure 4897: DNA345076, 143540.3, 232682_at
Figure 4898: PRO95606
Figure 4899: DNA330869, 406591.1, 232687_at
Figure 4900: PRO86032
Figure 4901: DNA270329, DNA270329, 232737_s_at
Figure 4902: PRO58716
Figure 4903: DNA330870, 227719.1, 232883_at
Figure 4904: PRO86033
Figure 4905: DNA325531, NM_032379, 232914_s_at
Figure 4906: PRO82038
Figure 4907: DNA345077, AK022251, 233089_at
Figure 4908: PRO95607
Figure 4909: DNA336161, NP_060857.2, 233252_s_at
Figure 4910: PRO90356
Figure 4911A-B: DNA340168, NM_017693,
233255_s_at
Figure 4912: PRO91663
Figure 4913: DNA324156, NM_032212, 233341_s_at
Figure 4914: PRO80856
Figure 4915: DNA331423, AF176071, 233467_s_at
Figure 4916A-B: DNA331391, NP_065947.1,
233734_s_at
Figure 4917: PRO49998
Figure 4918: DNA335477, 209190.1, 233800_at
Figure 4919: PRO89830
Figure 4920A-B: DNA345078, 474673.14,
233849_s_at
Figure 4921: PRO95608
Figure 4922: DNA329481, NM_016150, 233857_s_at
Figure 4923: PRO60949
Figure 4924A-B: DNA338110, 1382987.31, 233880_at
Figure 4925: PRO90981
Figure 4926: DNA345079, NP_057023.2, 233970_s_at
Figure 4927: PRO84916
Figure 4928: DNA331687, D13078, 234013_at
Figure 4929: PRO86682
Figure 4930: DNA333607, 211626.1, 234151_at
Figure 4931: PRO88251
Figure 4932: DNA345080, 401293.1, 234260_at
Figure 4933: PRO95609
Figure 4934A-B: DNA345081, NP_057422.2,
234304_s_at
Figure 4935: PRO95610
Figure 4936: DNA330881, NP_067004.3, 234306_s_at
Figure 4937: PRO1138
Figure 4938: DNA329312, NM_005214, 234362_s_at
Figure 4939: PRO84901
Figure 4940: DNA345082, 1452291.29, 234398_at
Figure 4941: PRO95611
Figure 4942: DNA345083, S60795, 234402_at
Figure 4943: PRO95612
Figure 4944: DNA345084, NP_443104.1, 234408_at
Figure 4945: PRO20110
Figure 4946: DNA345085, AAA61109.1, 234440_at
Figure 4947: PRO95613
Figure 4948A-C: DNA339394, NP_055768.2,
234660_s_at
Figure 4949: PRO91199
Figure 4950: DNA345086, BAB15056.1, 234785_at
Figure 4951: PRO95614
Figure 4952: DNA345087, X04937, 234819_at
Figure 4953: PRO95615
Figure 4954: DNA345088, CAA29554.1, 234849_at
Figure 4955: PRO95616
Figure 4956A-C: DNA345089, AJ238394,
234928_x_at
Figure 4957: PRO95617
Figure 4958: DNA330882, 406739.1, 234974_at
Figure 4959: PRO86044
Figure 4960: DNA345090, NM_052913, 234994_at
Figure 4961: PRO95618

Figure 4962: DNA258761, DNA258761, 235019_at
Figure 4963A-B: DNA345091, 135369.13, 235020_at
Figure 4964: PRO95619
Figure 4965: DNA339413, DNA339413, 235046_at
Figure 4966A-B: DNA345092, 292261.1, 235048_at
Figure 4967: PRO95620
Figure 4968A-B: DNA340485, BAC56923.1,
235085_at
Figure 4969: PRO92206
Figure 4970: DNA345093, 337920.2, 235104_at
Figure 4971: PRO95621
Figure 4972: DNA328146, BC025376, 235117_at
Figure 4973: PRO84051
Figure 4974: DNA333752, 200228.1, 235199_at
Figure 4975: PRO88377
Figure 4976: DNA345094, 1384081.2, 235203_at
Figure 4977: PRO95622
Figure 4978: DNA330896, 250896.1, 235213_at
Figure 4979: PRO86057
Figure 4980: DNA345095, 131102.1, 235230_at
Figure 4981: PRO95623
Figure 4982: DNA324093, NP_620156.1, 235256_s_at
Figure 4983: PRO80802
Figure 4984: DNA336016, DNA336016, 235291_s_at
Figure 4985: DNA345096, 237100.26, 235292_at
Figure 4986: PRO95624
Figure 4987: DNA330898, 227608.1, 235299_at
Figure 4988: PRO86059
Figure 4989A-B: DNA345097, NP_783161.1,
235306_at
Figure 4990: PRO86060
Figure 4991: DNA328151, 982500.1, 235352_at
Figure 4992: PRO84056
Figure 4993A-C: DNA345098, AL832877, 235410_at
Figure 4994: PRO95625
Figure 4995A-B: DNA345099, AF133211, 235421_at
Figure 4996: PRO95626
Figure 4997A-B: DNA345100, NP_689737.1,
235425_at
Figure 4998: PRO95627
Figure 4999A-B: DNA345101, 979268.1, 235440_at
Figure 5000: PRO95628
Figure 5001: DNA257872, DNA257872, 235457_at
Figure 5002: DNA330906, NP_116171.2, 235458_at
Figure 5003: PRO86067
Figure 5004A-B: DNA345102, AAH30800.1,
235463_s_at
Figure 5005: PRO95629
Figure 5006: DNA345103, NP_689629.1, 235509_at
Figure 5007: PRO95630
Figure 5008: DNA330912, 984873.1, 235609_at
Figure 5009: PRO86073
Figure 5010A-B: DNA336026, AB095926, 235643_at
Figure 5011: DNA345104, 1448915.1, 235680_at
Figure 5012: PRO95631
Figure 5013: DNA336165, AF368463, 235706_at
Figure 5014: PRO84371
Figure 5015: DNA345105, NP_689674.1, 235745_at
Figure 5016: PRO95632
Figure 5017A-B: DNA335175, DNA335175,
235971_at
Figure 5018: PRO89566
Figure 5019A-B: DNA345106, 244378.1, 236125_at
Figure 5020: PRO49375
Figure 5021: DNA336348, 1512910.2, 236203_at
Figure 5022: PRO90492
Figure 5023: DNA331211, 392245.1, 236226_at
Figure 5024: PRO86341
Figure 5025: DNA335691, DNA335691, 236280_at
Figure 5026: PRO12646
Figure 5027: DNA345107, AF488410, 236313_at
Figure 5028A-B: DNA345108, AF318353, 236322_at
Figure 5029: PRO95634
Figure 5030: DNA329312, AF414120, 236341_at
Figure 5031: PRO84901
Figure 5032: DNA333653, 325998.1, 236435_at
Figure 5033: PRO88292
Figure 5034: DNA345109, 7763130.1, 236471_at
Figure 5035: PRO95635
Figure 5036: DNA328168, 179804.1, 236474_at
Figure 5037: PRO84071
Figure 5038: DNA345110, 7691553.11, 236488_s_at
Figure 5039: PRO95636
Figure 5040: DNA330934, DNA330934, 236595_at
Figure 5041: PRO86095
Figure 5042: DNA330935, 229915.1, 236610_at
Figure 5043: PRO86096
Figure 5044: DNA345111, 414146.8, 236717_at
Figure 5045: PRO95637
Figure 5046: DNA329491, DNA329491, 236787_at
Figure 5047: DNA330939, 214517.1, 236796_at
Figure 5048: PRO86100
Figure 5049: DNA345112, AK074237, 236984_at
Figure 5050: PRO95638
Figure 5051: DNA330943, 1042935.2, 237009_at
Figure 5052: PRO86104
Figure 5053: DNA345113, 7762795.1, 237105_at
Figure 5054: PRO95639
Figure 5055A-B: DNA226536, NM_003234,
237215_s_at
Figure 5056: PRO36999
Figure 5057: DNA345114, BC032694, 237559_at
Figure 5058: PRO78081
Figure 5059: DNA328178, 985267.1, 237839_at
Figure 5060: PRO84081
Figure 5061: DNA330950, 983684.2, 237953_at
Figure 5062: PRO86111
Figure 5063A-B: DNA345115, 062186.18, 238002_at
Figure 5064: PRO60111
Figure 5065: DNA345116, BC033490, 238018_at
Figure 5066: PRO95640
Figure 5067A-B: DNA330952, 333610.10,

238021_s_at
Figure 5068: PRO86113
Figure 5069: DNA345117, 333610.2, 238022_at
Figure 5070: PRO95641
Figure 5071: DNA345118, 337083.5, 238075_at
Figure 5072: PRO95642
Figure 5073: DNA329492, 017295.1, 238156_at
Figure 5074: PRO85053
Figure 5075: DNA345119, 331249.6, 238520_at
Figure 5076: PRO95643
Figure 5077: DNA329495, 1447201.1, 238581_at
Figure 5078: PRO85056
Figure 5079: DNA329497, 232064.1, 238619_at
Figure 5080: PRO85058
Figure 5081A-B: DNA345120, 1400266.11, 238649_at
Figure 5082: PRO95644
Figure 5083: DNA334895, 172305.1, 238787_at
Figure 5084: PRO89333
Figure 5085: DNA328188, 7688626.1, 238875_at
Figure 5086: PRO84091
Figure 5087: DNA345121, 255109.1, 238900_at
Figure 5088: PRO95645
Figure 5089: DNA329500, 214454.1, 238950_at
Figure 5090: PRO85061
Figure 5091A-C: DNA345122, NM_018136,
239002_at
Figure 5092: PRO95646
Figure 5093A-B: DNA345123, 086440.4, 239151_at
Figure 5094: PRO95647
Figure 5095: DNA335753, 408088.2, 239179_at
Figure 5096: PRO90062
Figure 5097: DNA345124, 7685093.8, 239237_at
Figure 5098: PRO95648
Figure 5099: DNA345125, 401336.15, 239288_at
Figure 5100: PRO95649
Figure 5101: DNA333746, 332697.1, 239294_at
Figure 5102: PRO88371
Figure 5103: DNA345126, AL713733, 239412_at
Figure 5104: PRO95650
Figure 5105: DNA329502, 210572.1, 239427_at
Figure 5106: PRO85063
Figure 5107: DNA330983, 305289.1, 239448_at
Figure 5108: PRO86142
Figure 5109: DNA345127, 1397901.50, 239629_at
Figure 5110: PRO95651
Figure 5111: DNA333632, 247565.1, 240064_at
Figure 5112: PRO88274
Figure 5113: DNA330314, 026641.5, 240265_at
Figure 5114: PRO85538
Figure 5115: DNA340269, DNA340269, 240572_s_at
Figure 5116: PRO91765
Figure 5117A-B: DNA345128, NM_175571,
240646_at
Figure 5118: PRO86060
Figure 5119: DNA345129, 217952.1, 240789_at
Figure 5120: PRO95652
Figure 5121: DNA345130, 231676.2, 240951_at
Figure 5122: PRO95653
Figure 5123: DNA345131, NM_139273, 240983_s_at
Figure 5124: PRO95654
Figure 5125: DNA345132, 227682.1, 241393_at
Figure 5126: PRO95655
Figure 5127: DNA345133, BC016950, 241682_at
Figure 5128: PRO95656
Figure 5129: DNA345134, 212515.1, 241819_at
Figure 5130: PRO24261
Figure 5131: DNA331011, 979953.1, 241859_at
Figure 5132: PRO86169
Figure 5133: DNA345135, AK074645, 241869_at
Figure 5134: PRO95657
Figure 5135: DNA329506, NP_387510.1, 241937_s_at
Figure 5136: PRO85067
Figure 5137: DNA345136, 264653.1, 241956_at
Figure 5138: PRO95658
Figure 5139: DNA331015, 109159.1, 242031_at
Figure 5140: PRO86173
Figure 5141: DNA345137, 072859.8, 242146_at
Figure 5142: PRO95659
Figure 5143: DNA345138, 1502644.28, 242520_s_at
Figure 5144: PRO95660
Figure 5145A-B: DNA345139, AB067489, 242665_at
Figure 5146: DNA331031, 405967.1, 242669_at
Figure 5147: PRO86189
Figure 5148A-B: DNA345140, NM_015979,
242706_s_at
Figure 5149: PRO85734
Figure 5150: DNA345141, 7698324.1, 242939_at
Figure 5151: PRO95662
Figure 5152: DNA329507, 407430.1, 242943_at
Figure 5153: PRO85068
Figure 5154: DNA335321, 350834.1, 243049_at
Figure 5155: PRO89696
Figure 5156: DNA345142, 011019.14, 243124_at
Figure 5157: PRO95663
Figure 5158: DNA345143, AL833716, 243166_at
Figure 5159: PRO95664
Figure 5160A-B: DNA329508, 142131.16, 243296_at
Figure 5161: PRO85069
Figure 5162: DNA345144, 407288.1, 243386_at
Figure 5163: PRO95665
Figure 5164: DNA345145, 994948.45, 243405_at
Figure 5165: PRO95666
Figure 5166: DNA331051, 306804.1, 243469_at
Figure 5167: PRO86209
Figure 5168A-B: DNA345146, 331965.1, 243495_s_at
Figure 5169: PRO52796
Figure 5170: DNA333748, 394811.1, 243602_at
Figure 5171: PRO88373
Figure 5172: DNA345147, 315972.1, 243788_at
Figure 5173: PRO95667
Figure 5174: DNA345148, 086440.19, 243937_x_at
Figure 5175: PRO95668

Figure 5176A-B: DNA329494, 978990.1, 243999_at
Figure 5177: PRO85055
Figure 5178: DNA345149, 1009940.1, 244042_x_at
Figure 5179: PRO95669
Figure 5180: DNA335678, 432509.1, 244044_at
Figure 5181: PRO90006
Figure 5182: DNA334339, DNA334339, 244267_at
Figure 5183: PRO86220
Figure 5184: DNA345150, 333325.3, 244308_at
Figure 5185: PRO95670
Figure 5186: DNA328237, 337066.49, 244383_at
Figure 5187: PRO84140
Figure 5188A-B: DNA345151, NP_689742.2,
244509_at
Figure 5189: PRO95671
Figure 5190: DNA334446, 207194.3, 244579_at
Figure 5191: PRO88952
Figure 5192: DNA333766, 215245.1, 244598_at
Figure 5193: PRO88390
Figure 5194: DNA345152, 032035.3, 244764_at
Figure 5195: PRO95672
Figure 5196: DNA331069, DNA331069, 244798_at
Figure 5197: PRO86226
Figure 5198A-B: DNA328729, BAA11496.1,
D80001_at
Figure 5199: PRO38526
Figure 5200: DNA328961, BC011049, DNA36995_at
Figure 5201: PRO84667
Figure 5202: DNA304492, NM_032016,
DNA45409_at
Figure 5203: PRO1864
Figure 5204: DNA327200, NM_031950,
DNA59602_at
Figure 5205: PRO1065
Figure 5206: DNA345153, BC031639, DNA61875_at
Figure 5207: PRO83478
Figure 5208: DNA345154, NP_002174.1,
DNA82348_at
Figure 5209: PRO2021
Figure 5210: DNA327667, NP_065392.1,
DNA84141_at
Figure 5211: PRO83135
Figure 5212: DNA325850, NM_024089,
DNA84917_at
Figure 5213: PRO82312
Figure 5214: DNA325654, NM_014033,
DNA92232_at
Figure 5215: PRO4348
Figure 5216A-B: DNA345155, NM_153837,
DNA96860_at
Figure 5217: PRO6017
Figure 5218: DNA96866, DNA96866, DNA96866_at
Figure 5219: PRO6015
Figure 5220: DNA331073, NP_112184.1,
DNA101926_at
Figure 5221: PRO86229
Figure 5222: DNA108681, DNA108681,
DNA108681_at
Figure 5223: PRO6492
Figure 5224: DNA329215, NM_012092,
DNA108917_at
Figure 5225: PRO7424
Figure 5226: DNA345156, BC047595, DNA119482_at
Figure 5227: PRO9850
Figure 5228A-B: DNA345157, BAA86515.1,
DNA132162_at
Figure 5229: PRO95673
Figure 5230: DNA345158, BC044246, DNA139546_at
Figure 5231: PRO95674
Figure 5232: DNA324246, NM_030926,
DNA143288_at
Figure 5233: PRO80930
Figure 5234A-B: DNA150956, D31887,
DNA150956_at
Figure 5235: DNA304833, NP_443163.1,
DNA161000_at
Figure 5236: PRO71240
Figure 5237: DNA330417, NP_085144.1,
DNA164989_at
Figure 5238: PRO21341
Figure 5239: DNA345159, BC050675, P_Z93700_at
Figure 5240: PRO95675
Figure 5241: DNA329207, AL442092, P_X52226_at
Figure 5242: PRO220
Figure 5243: DNA345160, BC025407, P_X52238_at
Figure 5244: PRO95676
Figure 5245: DNA345161, BC009955, P_Z34109_at
Figure 5246A-B: DNA330610, BAB15739.1,
P_A37063_at
Figure 5247: PRO85787
Figure 5248: DNA328250, NP_443164.1, P_Z65107_at
Figure 5249: PRO82061
Figure 5250: DNA304469, NP_149078.1, P_A37079_at
Figure 5251: PRO71045
Figure 5252: DNA345162, NM_153206, P_Z65110_at
Figure 5253: PRO95678
Figure 5254: DNA345163, NM_171846, P_A37128_at
Figure 5255: PRO95679
Figure 5256A-C: DNA345164, NM_020477,
NM_000037_at
Figure 5257: PRO95680
Figure 5258: DNA109234, NM_000074,
NM_000074_at
Figure 5259: PRO6517
Figure 5260: DNA325711, NM_000075,
NM_000075_at
Figure 5261: PRO4873
Figure 5262: DNA227514, NP_000152.1,
NM_000161_at
Figure 5263: PRO37977
Figure 5264: DNA287630, NM_000169,
NM_000169_at

Figure 5265: PRO2154
Figure 5266: DNA328612, NP_000166.2,
NM_000175_at
Figure 5267: PRO84394
Figure 5268: DNA76511, NP_000197.1,
NM_000206_at
Figure 5269: PRO2539
Figure 5270A-B: DNA220748, NM_000210,
NM_000210_at
Figure 5271: PRO34726
Figure 5272: DNA88450, NM_000235, NM_000235_at
Figure 5273: PRO2795
Figure 5274: DNA226014, NM_000239,
NM_000239_at
Figure 5275: PRO36477
Figure 5276: DNA227071, NM_000269,
NM_000269_at
Figure 5277: PRO37534
Figure 5278: DNA226078, NP_000296.1,
NM_000305_at
Figure 5279: PRO36541
Figure 5280: DNA226082, NP_000301.1,
NM_000310_at
Figure 5281: PRO36545
Figure 5282A-B: DNA226395, NM_000321,
NM_000321_at
Figure 5283: PRO36858
Figure 5284A-C: DNA345165, AF039704,
NM_000391_at
Figure 5285: DNA227081, NP_000390.2,
NM_000399_at
Figure 5286: PRO37544
Figure 5287: DNA76514, NM_000418, NM_000418_at
Figure 5288: PRO2540
Figure 5289: DNA88549, M28526, NM_000442_at
Figure 5290: PRO2408
Figure 5291A-E: DNA226238, NM_000540,
NM_000540_at
Figure 5292A-B: PRO36701
Figure 5293: DNA83046, M31516, NM_000574_at
Figure 5294: PRO2569
Figure 5295A-B: DNA227659, NM_000579,
NM_000579_at
Figure 5296: PRO38122
Figure 5297: DNA345166, NM_000584,
NM_000584_at
Figure 5298: PRO74
Figure 5299: DNA345167, NM_000588,
NM_000588_at
Figure 5300: PRO95682
Figure 5301: DNA36717, NM_000590, NM_000590_at
Figure 5302: PRO72
Figure 5303: DNA345168, NM_000593,
NM_000593_at
Figure 5304: PRO36996
Figure 5305: DNA218655, M10988, NM_000594_at
Figure 5306: PRO34451
Figure 5307: DNA35629, NM_000595, NM_000595_at
Figure 5308: PRO7
Figure 5309: DNA225829, M59040, NM_000610_at
Figure 5310: PRO36292
Figure 5311: DNA345169, NP_000607.1,
NM_000616_at
Figure 5312: PRO2222
Figure 5313: DNA225528, NM_000619,
NM_000619_at
Figure 5314: PRO35991
Figure 5315: DNA227597, NM_000636,
NM_000636_at
Figure 5316: PRO38060
Figure 5317: DNA188234, NM_000639,
NM_000639_at
Figure 5318: PRO21942
Figure 5319: DNA331493, NM_000647,
NM_000647_at
Figure 5320: PRO84690
Figure 5321: DNA225993, NM_000655,
NM_000655_at
Figure 5322: PRO36456
Figure 5323: DNA89242, NM_000700, NM_000700_at
Figure 5324: PRO2907
Figure 5325: DNA88194, NM_000733, NM_000733_at
Figure 5326: PRO2220
Figure 5327: DNA90631, NM_000756, NM_000756_at
Figure 5328: PRO2519
Figure 5329: DNA345170, NM_000758,
NM_000758_at
Figure 5330: PRO2055
Figure 5331A-B: DNA226870, DNA226870,
NM_000791_at
Figure 5332: PRO37333
Figure 5333: DNA151820, NM_000860,
NM_000860_at
Figure 5334: PRO12194
Figure 5335A-B: DNA345171, NP_000868.1,
NM_000877_at
Figure 5336: PRO2590
Figure 5337A-B: DNA331484, NM_000878,
NM_000878_at
Figure 5338: PRO3276
Figure 5339: DNA345172, NM_000879,
NM_000879_at
Figure 5340: PRO69
Figure 5341A-B: DNA220746, NM_000885,
NM_000885_at
Figure 5342: PRO34724
Figure 5343: DNA220761, NM_000889,
NM_000889_at
Figure 5344: PRO34739
Figure 5345A-B: DNA345173, NM_138822,
NM_000919_at
Figure 5346: PRO95683

Figure 5347: DNA326011, NP_000933.1,
NM_000942_at
Figure 5348: PRO2720
Figure 5349: DNA227709, NM_000956,
NM_000956_at
Figure 5350: PRO38172
Figure 5351: DNA226195, NM_000958,
NM_000958_at
Figure 5352: PRO36658
Figure 5353A-B: DNA226070, NM_000963,
NM_000963_at
Figure 5354: PRO36533
Figure 5355A-B: DNA333708, NM_001066,
NM_001066_at
Figure 5356: PRO21928
Figure 5357A-B: DNA150748, NM_001114,
NM_001114_at
Figure 5358: PRO12446
Figure 5359: DNA225584, NM_001154,
NM_001154_at
Figure 5360: PRO36047
Figure 5361A-B: DNA325972, NM_001211,
NM_001211_at
Figure 5362: PRO82417
Figure 5363: DNA327718, NM_033307,
NM_001225_at
Figure 5364: PRO83697
Figure 5365: DNA287267, NP_001228.1,
NM_001237_at
Figure 5366: PRO37015
Figure 5367: DNA226177, NM_001295,
NM_001295_at
Figure 5368: PRO36640
Figure 5369: DNA331744, NM_001335,
NM_001335_at
Figure 5370: PRO1574
Figure 5371: DNA226182, NP_001391.2,
NM_001400_at
Figure 5372: PRO36645
Figure 5373: DNA227344, NP_001403.1,
NM_001412_at
Figure 5374: PRO37807
Figure 5375: DNA97300, NP_001407.1,
NM_001416_at
Figure 5376: PRO3647
Figure 5377: DNA188346, NM_001459,
NM_001459_at
Figure 5378: PRO21766
Figure 5379: DNA227752, X95876, NM_001504_at
Figure 5380: PRO38215
Figure 5381: DNA329941, NM_001552,
NM_001552_at
Figure 5382: PRO85249
Figure 5383A-B: DNA345174, NM_001558,
NM_001558_at
Figure 5384: PRO2536
Figure 5385A-B: DNA345175, NM_001559,
NM_001559_at
Figure 5386: PRO23394
Figure 5387: DNA218677, L12964, NM_001561_at
Figure 5388: PRO34455
Figure 5389: DNA82362, NM_001565, NM_001565_at
Figure 5390: PRO1718
Figure 5391A-B: DNA226364, NP_001612.1,
NM_001621_at
Figure 5392: PRO36827
Figure 5393: DNA88076, NM_001637, NM_001637_at
Figure 5394: PRO2640
Figure 5395: DNA188736, U00115, NM_001706_at
Figure 5396: PRO26296
Figure 5397A-B: DNA83031, NM_001746,
NM_001746_at
Figure 5398: PRO2564
Figure 5399: DNA150725, NM_001747,
NM_001747_at
Figure 5400: PRO12792
Figure 5401: DNA227480, NP_001739.1,
NM_001748_at
Figure 5402: PRO37943
Figure 5403: DNA345176, 348151.15, NM_001759_at
Figure 5404: PRO95684
Figure 5405: DNA103588, L27706, NM_001762_at
Figure 5406: PRO4912
Figure 5407: DNA75526, NM_001767, NM_001767_at
Figure 5408: PRO2013
Figure 5409: DNA328387, NM_001769,
NM_001769_at
Figure 5410: PRO4769
Figure 5411: DNA226380, NM_001774,
NM_001774_at
Figure 5412: PRO4695
Figure 5413: DNA226234, NM_001775,
NM_001775_at
Figure 5414: PRO36697
Figure 5415: DNA328522, NM_001778,
NM_001778_at
Figure 5416: PRO2696
Figure 5417: DNA226436, NM_001781,
NM_001781_at
Figure 5418: PRO36899
Figure 5419: DNA227573, NP_001780.1,
NM_001789_at
Figure 5420: PRO38036
Figure 5421: DNA329940, NM_001814,
NM_001814_at
Figure 5422: PRO2679
Figure 5423: DNA225671, NM_001831,
NM_001831_at
Figure 5424: PRO36134
Figure 5425: DNA196361, NM_001837,
NM_001837_at
Figure 5426: PRO24864

Figure 5427: DNA88224, NM_001838, NM_001838_at
Figure 5428: PRO2236
Figure 5429: DNA227606, NM_001881,
NM_001881_at
Figure 5430: PRO38069
Figure 5431: DNA225804, DNA225804,
NM_001908_at
Figure 5432: PRO3344
Figure 5433: DNA225661, NP_001944.1,
NM_001953_at
Figure 5434: PRO36124
Figure 5435: DNA226872, NM_001964,
NM_001964_at
Figure 5436: PRO37335
Figure 5437: DNA325595, NP_001966.1,
NM_001975_at
Figure 5438: PRO38010
Figure 5439: DNA226133, NM_001992,
NM_001992_at
Figure 5440: PRO36596
Figure 5441: DNA226892, DNA226892,
NM_002053_at
Figure 5442: PRO12478
Figure 5443: DNA88352, NM_002076, NM_002076_at
Figure 5444: PRO2759
Figure 5445: DNA88374, NM_002104, NM_002104_at
Figure 5446: PRO2768
Figure 5447: DNA151752, NM_002133,
NM_002133_at
Figure 5448: PRO12886
Figure 5449: DNA228014, NM_002162,
NM_002162_at
Figure 5450: PRO38477
Figure 5451A-B: DNA345177, NP_002173.1,
NM_002182_at
Figure 5452: PRO6177
Figure 5453: DNA345178, NM_002185,
NM_002185_at
Figure 5454: PRO95685
Figure 5455: DNA345179, NM_002186,
NM_002186_at
Figure 5456: PRO64957
Figure 5457: DNA345180, NM_002188,
NM_002188_at
Figure 5458: PRO95686
Figure 5459A-B: DNA220744, NP_002194.1,
NM_002203_at
Figure 5460: PRO34722
Figure 5461A-B: DNA88423, NP_002200.1,
NM_002209_at
Figure 5462: PRO2784
Figure 5463A-B: DNA325306, NM_002211,
NM_002211_at
Figure 5464: PRO81851
Figure 5465: DNA345181, NP_689926.1,
NM_002219_at
Figure 5466: PRO95687
Figure 5467A-C: DNA328811, D26070,
NM_002222_at
Figure 5468: PRO84551
Figure 5469: DNA226359, DNA226359,
NM_002228_at
Figure 5470: PRO36822
Figure 5471: DNA103320, NM_002229,
NM_002229_at
Figure 5472: PRO4650
Figure 5473: DNA345182, NM_002250,
NM_002250_at
Figure 5474: PRO4787
Figure 5475: DNA150971, NM_002258,
NM_002258_at
Figure 5476: PRO12564
Figure 5477: DNA226427, NM_002260,
NM_002260_at
Figure 5478: PRO36890
Figure 5479A-B: DNA345183, AJ000673,
NM_002262_at
Figure 5480: DNA345184, BC036703, NM_002265_at
Figure 5481: PRO82739
Figure 5482: DNA288243, NM_002286,
NM_002286_at
Figure 5483: PRO36451
Figure 5484A-B: DNA188301, NM_002309,
NM_002309_at
Figure 5485: PRO21834
Figure 5486: DNA151012, NM_009588,
NM_002341_at
Figure 5487: PRO11604
Figure 5488A-B: DNA196641, NM_002349,
NM_002349_at
Figure 5489: PRO25114
Figure 5490: DNA103245, M16038, NM_002350_at
Figure 5491: PRO4575
Figure 5492: DNA227033, NM_002371,
NM_002371_at
Figure 5493: PRO37496
Figure 5494: DNA345185, NP_002380.3,
NM_002389_at
Figure 5495: PRO95689
Figure 5496: DNA103554, J03569, NM_002394_at
Figure 5497: PRO4881
Figure 5498: DNA97290, NM_002512, NM_002512_at
Figure 5499: PRO3637
Figure 5500: DNA88035, NM_002526, NM_002526_at
Figure 5501: PRO2135
Figure 5502: DNA345186, NM_175080,
NM_002561_at
Figure 5503: PRO95690
Figure 5504A-B: DNA329120, NM_002569,
NM_002569_at
Figure 5505: PRO2752
Figure 5506: DNA83130, NM_002674, NM_002674_at

Figure 5507: PRO2096
Figure 5508: DNA345187, NP_002698.1,
NM_002707_at
Figure 5509: DNA227090, NP_002750.1,
NM_002759_at
Figure 5510: PRO37553
Figure 5511: DNA345188, NP_002795.2,
NM_002804_at
Figure 5512: PRO81979
Figure 5513A-B: DNA345189, NM_002844,
NM_002844_at
Figure 5514: PRO95691
Figure 5515: DNA227063, NM_002858,
NM_002858_at
Figure 5516: PRO37526
Figure 5517: DNA219225, NP_002874.1,
NM_002883_at
Figure 5518: PRO34531
Figure 5519: DNA88607, NP_002892.1,
NM_002901_at
Figure 5520: PRO2863
Figure 5521: DNA103281, NM_002908,
NM_002908_at
Figure 5522: PRO4611
Figure 5523: DNA216508, NM_002981,
NM_002981_at
Figure 5524: PRO34260
Figure 5525: DNA192060, NM_002983,
NM_002983_at
Figure 5526: PRO21960
Figure 5527: DNA216689, NM_002984,
NM_002984_at
Figure 5528: PRO34276
Figure 5529: DNA329241, NP_003002.1,
NM_003011_at
Figure 5530: PRO84846
Figure 5531: DNA329005, NM_003037,
NM_003037_at
Figure 5532: PRO12612
Figure 5533A-B: DNA326573, NP_003063.2,
NM_003072_at
Figure 5534: PRO82935
Figure 5535: DNA345190, NM_139276,
NM_003150_at
Figure 5536: PRO95692
Figure 5537: DNA227447, X59871, NM_003202_at
Figure 5538: PRO37910
Figure 5539A-B: DNA226536, X01060,
NM_003234_at
Figure 5540: PRO36999
Figure 5541A-B: DNA83176, NM_003243,
NM_003243_at
Figure 5542: PRO2620
Figure 5543: DNA227874, NM_003329,
NM_003329_at
Figure 5544: PRO38337
Figure 5545: DNA103421, NP_003366.1,
NM_003375_at
Figure 5546: PRO4749
Figure 5547: DNA345191, X71635, NM_003467_at
Figure 5548: PRO4516
Figure 5549: DNA304489, NM_003504,
NM_003504_at
Figure 5550: PRO71058
Figure 5551: DNA227239, NM_003506,
NM_003506_at
Figure 5552: PRO37702
Figure 5553: DNA150990, X84958, NM_003641_at
Figure 5554: PRO12570
Figure 5555: DNA333697, NM_003650,
NM_003650_at
Figure 5556: PRO88328
Figure 5557: DNA151802, AB004066, NM_003670_at
Figure 5558: PRO12890
Figure 5559: DNA227213, NP_003671.1,
NM_003680_at
Figure 5560: PRO37676
Figure 5561: DNA228010, NM_003688,
NM_003688_at
Figure 5562: PRO38473
Figure 5563: DNA345192, U88326, NM_003745_at
Figure 5564: PRO12771
Figure 5565: DNA345193, NM_148974,
NM_003790_at
Figure 5566: PRO95693
Figure 5567: DNA227921, NM_003798,
NM_003798_at
Figure 5568: PRO38384
Figure 5569: DNA345194, NP_003798.2,
NM_003807_at
Figure 5570: PRO5810
Figure 5571: DNA84130, U37518, NM_003810_at
Figure 5572: PRO1096
Figure 5573A-B: DNA200236, NP_003807.1,
NM_003816_at
Figure 5574: PRO34137
Figure 5575: DNA345195, NM_003839,
NM_003839_at
Figure 5576: PRO20114
Figure 5577: DNA345196, NM_003853,
NM_003853_at
Figure 5578: PRO36013
Figure 5579: DNA345197, NM_003855,
NM_003855_at
Figure 5580: PRO4778
Figure 5581: DNA325749, NP_003868.1,
NM_003877_at
Figure 5582: PRO12839
Figure 5583: DNA331776, NM_003897,
NM_003897_at
Figure 5584: PRO84760
Figure 5585: DNA227329, NP_004031.1,

NM_004040_at
Figure 5586: PRO37792
Figure 5587: DNA328570, NM_004049,
NM_004049_at
Figure 5588: PRO37843
Figure 5589: DNA88173, S93414, NM_004079_at
Figure 5590: PRO2210
Figure 5591: DNA103208, NM_004099,
NM_004099_at
Figure 5592: PRO4538
Figure 5593: DNA287620, NM_004131,
NM_004131_at
Figure 5594: PRO2081
Figure 5595: DNA227562, NP_004139.1,
NM_004148_at
Figure 5596: PRO38025
Figure 5597: DNA331392, NM_004195,
NM_004195_at
Figure 5598: PRO364
Figure 5599: DNA103394, U81800, NM_004207_at
Figure 5600: PRO4722
Figure 5601: DNA345198, NP_004212.3,
NM_004221_at
Figure 5602: PRO95694
Figure 5603: DNA345199, NP_004224.1,
NM_004233_at
Figure 5604: PRO2225
Figure 5605: DNA329130, NP_004286.2,
NM_004295_at
Figure 5606: PRO20124
Figure 5607: DNA287240, NM_004335,
NM_004335_at
Figure 5608: PRO29371
Figure 5609: DNA329008, NP_004337.2,
NM_004346_at
Figure 5610: PRO12832
Figure 5611: DNA226578, U47414, NM_004354_at
Figure 5612: PRO37041
Figure 5613: DNA345200, NP_620599.1,
NM_004357_at
Figure 5614: PRO95695
Figure 5615A-B: DNA151420, NM_004430,
NM_004430_at
Figure 5616: PRO12876
Figure 5617: DNA328541, NM_004512,
NM_004512_at
Figure 5618: PRO4843
Figure 5619A-C: DNA345201, NP_757366.1,
NM_004513_at
Figure 5620: PRO95696
Figure 5621: DNA328262, U57094, NM_004580_at
Figure 5622: PRO84153
Figure 5623: DNA226737, NM_004585,
NM_004585_at
Figure 5624: PRO37200
Figure 5625A-B: DNA345202, NM_033300,
NM_004631_at
Figure 5626: PRO95697
Figure 5627: DNA227700, NM_004778,
NM_004778_at
Figure 5628: PRO38163
Figure 5629: DNA151675, NM_004800,
NM_004800_at
Figure 5630: PRO11975
Figure 5631: DNA345203, NM_004810,
NM_004810_at
Figure 5632: PRO12190
Figure 5633: DNA345204, AJ420587, NM_004830_at
Figure 5634: PRO95698
Figure 5635: DNA345205, AL117422, NM_004844_at
Figure 5636: PRO95699
Figure 5637: DNA329010, NM_004951,
NM_004951_at
Figure 5638: PRO23370
Figure 5639: DNA227563, NP_004946.1,
NM_004955_at
Figure 5640: PRO38026
Figure 5641A-B: DNA103316, M54968,
NM_004985_at
Figure 5642: PRO4646
Figure 5643: DNA151043, NP_005004.1,
NM_005013_at
Figure 5644: PRO12099
Figure 5645: DNA227909, NP_005024.1,
NM_005033_at
Figure 5646: PRO38372
Figure 5647: DNA227124, NM_005127,
NM_005127_at
Figure 5648: PRO37587
Figure 5649: DNA328264, NM_005192,
NM_005192_at
Figure 5650: PRO12087
Figure 5651: DNA329159, NP_005195.2,
NM_005204_at
Figure 5652: PRO4660
Figure 5653: DNA88259, L15006, NM_005214_at
Figure 5654: PRO2254
Figure 5655: DNA189700, NM_005252,
NM_005252_at
Figure 5656: PRO25619
Figure 5657: DNA325989, NP_005304.3,
NM_005313_at
Figure 5658: PRO2732
Figure 5659: DNA225961, NM_005317,
NM_005317_at
Figure 5660: PRO36424
Figure 5661: DNA196628, NM_005327,
NM_005327_at
Figure 5662: PRO25105
Figure 5663: DNA227208, AF055377, NM_005360_at
Figure 5664: PRO37671
Figure 5665: DNA103269, NP_005366.1,

NM_005375_at
Figure 5666: PRO4599
Figure 5667: DNA188207, D28124, NM_005380_at
Figure 5668: PRO21719
Figure 5669: DNA153752, NP_005372.1,
NM_005381_at
Figure 5670: PRO12926
Figure 5671: DNA227376, NP_005393.1,
NM_005402_at
Figure 5672: PRO37839
Figure 5673A-B: DNA331302, NP_005424.1,
NM_005433_at
Figure 5674: PRO12922
Figure 5675: DNA88410, NM_005534, NM_005534_at
Figure 5676: PRO2778
Figure 5677: DNA226262, NM_005563,
NM_005563_at
Figure 5678: PRO36725
Figure 5679: DNA333671, NM_005601,
NM_005601_at
Figure 5680: PRO37543
Figure 5681: DNA150427, NM_005608,
NM_005608_at
Figure 5682: PRO12243
Figure 5683: DNA345206, NM_005627,
NM_005627_at
Figure 5684: PRO86741
Figure 5685: DNA226500, NM_005628,
NM_005628_at
Figure 5686: PRO36963
Figure 5687: DNA329013, NM_005658,
NM_005658_at
Figure 5688: PRO20128
Figure 5689: DNA226610, M80254, NM_005729_at
Figure 5690: PRO37073
Figure 5691A-B: DNA345207, NM_133482,
NM_005732_at
Figure 5692: PRO95700
Figure 5693: DNA88541, NM_005746, NM_005746_at
Figure 5694: PRO2834
Figure 5695: DNA93548, NM_005767, NM_005767_at
Figure 5696: PRO4929
Figure 5697: DNA227695, AF097358, NM_005810_at
Figure 5698: PRO38158
Figure 5699: DNA150959, NM_005822,
NM_005822_at
Figure 5700: PRO11599
Figure 5701: DNA328516, NM_005842,
NM_005842_at
Figure 5702: PRO12323
Figure 5703: DNA151825, NM_005900,
NM_005900_at
Figure 5704: PRO12900
Figure 5705: DNA345208, NM_130439,
NM_005962_at
Figure 5706: PRO95701
Figure 5707: DNA328266, NM_006002,
NM_006002_at
Figure 5708: PRO12125
Figure 5709: DNA225959, NM_006144,
NM_006144_at
Figure 5710: PRO36422
Figure 5711: DNA28759, NM_006159, NM_006159_at
Figure 5712: PRO2520
Figure 5713: DNA329015, NP_006155.2,
NM_006164_at
Figure 5714: PRO84691
Figure 5715A-B: DNA151841, M59465,
NM_006290_at
Figure 5716: PRO12904
Figure 5717: DNA103371, NP_006361.1,
NM_006370_at
Figure 5718: PRO4701
Figure 5719: DNA189708, AF155568, NM_006372_at
Figure 5720: PRO23166
Figure 5721: DNA150430, NM_006396,
NM_006396_at
Figure 5722: PRO12770
Figure 5723: DNA227112, NM_006406,
NM_006406_at
Figure 5724: PRO37575
Figure 5725: DNA227795, NM_006429,
NM_006429_at
Figure 5726: PRO38258
Figure 5727: DNA329225, NM_006495,
NM_006495_at
Figure 5728: PRO84833
Figure 5729: DNA226277, X91790, NM_006499_at
Figure 5730: PRO36740
Figure 5731: DNA103253, NP_006507.1,
NM_006516_at
Figure 5732: PRO4583
Figure 5733A-B: DNA331802, AF012108,
NM_006534_at
Figure 5734: PRO86743
Figure 5735: DNA93439, Y13248, NM_006564_at
Figure 5736: PRO4515
Figure 5737: DNA227751, NM_006566,
NM_006566_at
Figure 5738: PRO38214
Figure 5739A-B: DNA345209, NP_006697.2,
NM_006706_at
Figure 5740: PRO95702
Figure 5741: DNA225836, U66142, NM_006725_at
Figure 5742: PRO36299
Figure 5743: DNA226260, NP_006760.1,
NM_006769_at
Figure 5744: PRO36723
Figure 5745: DNA227190, NP_006830.1,
NM_006839_at
Figure 5746: PRO37653
Figure 5747: DNA324897, NM_006854,

NM_006854_at
Figure 5748: PRO12468
Figure 5749A-B: DNA103449, NM_006931,
NM_006931_at
Figure 5750: PRO4776
Figure 5751: DNA324805, NM_007047,
NM_007047_at
Figure 5752: PRO81419
Figure 5753: DNA328271, NM_007057,
NM_007057_at
Figure 5754: PRO81868
Figure 5755: DNA329189, NM_007208,
NM_007208_at
Figure 5756: PRO4911
Figure 5757: DNA103440, NM_007360,
NM_007360_at
Figure 5758: PRO4767
Figure 5759A-B: DNA345210, BC028412,
NM_012081_at
Figure 5760: PRO37794
Figure 5761: DNA326809, NM_012112,
NM_012112_at
Figure 5762: PRO83142
Figure 5763A-B: DNA151707, NP_036273.1,
NM_012141_at
Figure 5764: PRO12884
Figure 5765: DNA345211, NM_012449,
NM_012449_at
Figure 5766: PRO28528
Figure 5767: DNA150621, NM_012463,
NM_012463_at
Figure 5768: PRO12374
Figure 5769: DNA331485, NM_012483,
NM_012483_at
Figure 5770: PRO86529
Figure 5771: DNA331519, NM_012485,
NM_012484_at
Figure 5772: PRO86551
Figure 5773: DNA227302, NM_013269,
NM_013269_at
Figure 5774: PRO37765
Figure 5775: DNA225594, NM_013272,
NM_013272_at
Figure 5776: PRO36057
Figure 5777: DNA103481, NP_037417.1,
NM_013285_at
Figure 5778: PRO4808
Figure 5779: DNA196426, NM_013308,
NM_013308_at
Figure 5780: PRO24924
Figure 5781: DNA227125, AF132297, NM_013324_at
Figure 5782: PRO37588
Figure 5783: DNA150648, NM_013332,
NM_013332_at
Figure 5784: PRO11576
Figure 5785: DNA345212, AB025219, NM_013416_at
Figure 5786: PRO84354
Figure 5787: DNA345213, NM_014044,
NM_014044_at
Figure 5788: PRO95703
Figure 5789A-C: DNA227619, NM_014112,
NM_014112_at
Figure 5790: PRO38082
Figure 5791: DNA331817, NM_014339,
NM_014339_at
Figure 5792: PRO86240
Figure 5793: DNA227351, AF191020, NM_014367_at
Figure 5794: PRO37814
Figure 5795: DNA329546, NM_014399,
NM_014399_at
Figure 5796: PRO296
Figure 5797: DNA330084, NM_014450,
NM_014450_at
Figure 5798: PRO9895
Figure 5799: DNA227252, U96628, NM_014456_at
Figure 5800: PRO37715
Figure 5801A-B: DNA277809, D87465,
NM_014767_at
Figure 5802: PRO64556
Figure 5803A-B: DNA151685, NP_055610.1,
NM_014795_at
Figure 5804: PRO12883
Figure 5805A-B: DNA227353, NM_014822,
NM_014822_at
Figure 5806: PRO37816
Figure 5807: DNA150805, NM_014888,
NM_014888_at
Figure 5808: PRO11583
Figure 5809: DNA103333, NM_014890,
NM_014890_at
Figure 5810: PRO4663
Figure 5811: DNA328274, NM_014891,
NM_014891_at
Figure 5812: PRO12912
Figure 5813A-B: DNA304464, NM_014918,
NM_014918_at
Figure 5814: PRO71042
Figure 5815A-B: DNA345214, NP_619520.1,
NM_014966_at
Figure 5816: PRO12282
Figure 5817: DNA330103, NM_015364,
NM_015364_at
Figure 5818: PRO19671
Figure 5819: DNA345215, NM_015392,
NM_015392_at
Figure 5820: PRO95704
Figure 5821: DNA226662, NP_057043.1,
NM_015959_at
Figure 5822: PRO37125
Figure 5823: DNA330096, NM_015967,
NM_015967_at
Figure 5824: PRO37163

Figure 5825A-B: DNA345216, AF077041,
NM_016081_at
Figure 5826: PRO95705
Figure 5827: DNA328831, NM_016245,
NM_016245_at
Figure 5828: PRO233
Figure 5829: DNA227352, AF110777, NM_016283_at
Figure 5830: PRO37815
Figure 5831: DNA330421, NM_016354,
NM_016354_at
Figure 5832: PRO85626
Figure 5833A-B: DNA328454, NM_016441,
NM_016441_at
Figure 5834: PRO4330
Figure 5835: DNA345217, NP_057546.1,
NM_016462_at
Figure 5836: PRO23604
Figure 5837: DNA227364, NP_057635.1,
NM_016551_at
Figure 5838: PRO37827
Figure 5839: DNA326550, NM_016579,
NM_016579_at
Figure 5840: PRO224
Figure 5841: DNA327869, NM_016588,
NM_016588_at
Figure 5842: PRO1898
Figure 5843: DNA227187, NM_016619,
NM_016619_at
Figure 5844: PRO37650
Figure 5845: DNA326078, NM_016641,
NM_016641_at
Figure 5846: PRO38464
Figure 5847: DNA227294, NM_017755,
NM_017755_at
Figure 5848: PRO37757
Figure 5849: DNA226633, NM_017906,
NM_017906_at
Figure 5850: PRO37096
Figure 5851: DNA336491, AK027630, NM_018092_at
Figure 5852: PRO4401
Figure 5853A-B: DNA345218, BC034607,
NM_018123_at
Figure 5854: PRO95706
Figure 5855: DNA227194, NM_018295,
NM_018295_at
Figure 5856: PRO37657
Figure 5857: DNA226227, NM_018402,
NM_018402_at
Figure 5858: PRO36690
Figure 5859: DNA287642, NM_018464,
NM_018464_at
Figure 5860: PRO9902
Figure 5861: DNA345219, AF116708, NM_018630_at
Figure 5862: DNA304494, AF212365, NM_018725_at
Figure 5863: PRO71061
Figure 5864: DNA227929, NP_061932.1,
NM_019059_at
Figure 5865: PRO38392
Figure 5866: DNA227268, NP_061955.1,
NM_019082_at
Figure 5867: PRO37731
Figure 5868: DNA226256, J00194, NM_019111_at
Figure 5869: PRO36719
Figure 5870: DNA329552, NM_019895,
NM_019895_at
Figure 5871: PRO85097
Figure 5872: DNA329074, NM_020139,
NM_020139_at
Figure 5873: PRO21326
Figure 5874: DNA329553, NM_020150,
NM_020150_at
Figure 5875: PRO38313
Figure 5876: DNA227280, NP_064615.1,
NM_020230_at
Figure 5877: PRO37743
Figure 5878: DNA227720, NP_065161.1,
NM_020428_at
Figure 5879: PRO38183
Figure 5880: DNA225636, NM_020645,
NM_020645_at
Figure 5881: PRO36099
Figure 5882: DNA150992, NP_066362.1,
NM_021034_at
Figure 5883: PRO12572
Figure 5884: DNA329023, NM_021102,
NM_021102_at
Figure 5885: PRO209
Figure 5886: DNA227121, NM_021105,
NM_021105_at
Figure 5887: PRO37584
Figure 5888: DNA345220, NM_021129,
NM_021129_at
Figure 5889: PRO11669
Figure 5890A-B: DNA333179, AF231512,
NM_021618_at
Figure 5891: PRO87901
Figure 5892: DNA326379, NP_067639.1,
NM_021626_at
Figure 5893: PRO302
Figure 5894: DNA345221, BC004348, NM_021798_at
Figure 5895: PRO10273
Figure 5896: DNA331834, AF246221, NM_021999_at
Figure 5897: PRO86760
Figure 5898: DNA304835, NP_071327.1,
NM_022044_at
Figure 5899: PRO71242
Figure 5900: DNA330378, NM_022346,
NM_022346_at
Figure 5901: PRO81126
Figure 5902: DNA328902, NM_022355,
NM_022355_at
Figure 5903: PRO84623

Figure 5904: DNA328895, NM_022367,
NM_022367_at
Figure 5905: PRO1317
Figure 5906A-B: DNA329024, BAA25532.2,
AB011178_at
Figure 5907: PRO84696
Figure 5908: DNA345222, NP_612213.2,
AF007152_at
Figure 5909: PRO95708
Figure 5910: DNA66487, NM_002467, HSMYC1_at
Figure 5911: PRO1213
Figure 5912A-B: DNA325227, NP_005338.1,
HSRNABIP_at
Figure 5913: PRO81785
Figure 5914: DNA345223, Y00790, HSTCRGR_at
Figure 5915: PRO95709
Figure 5916: DNA103258, DNA103258,
HSINTASA_at
Figure 5917: PRO4588
Figure 5918: DNA288259, NP_114172.1,
HUMCYCB_at
Figure 5919: PRO4676
Figure 5920A-B: DNA227134, NP_000918.1,
HUMMDR1_at
Figure 5921: PRO37597
Figure 5922: DNA329025, NM_006208,
HUMPC1Q1_at
Figure 5923: PRO4860
Figure 5924: DNA345224, X15260, HUMTCRGC_at
Figure 5925: DNA150552, AAB97011.1,
AF040965_at
Figure 5926: PRO12326
Figure 5927: DNA331095, NP_005216.1, HUME2F_at
Figure 5928: PRO86245
Figure 5929: DNA151041, DNA151041, P_V84330_at
Figure 5930: PRO12849
Figure 5931: DNA329276, NM_024096, AK024843_at
Figure 5932: PRO12104
Figure 5933: DNA151120, DNA151120,
HUMP13KIN_at
Figure 5934: PRO12179
Figure 5935: DNA345225, NM_138341, P_Z29229_at
Figure 5936: PRO95710
Figure 5937: DNA345226, NP_663781.1,
AK024570_at
Figure 5938: PRO11652
Figure 5939: DNA287190, AL049943, HSM800284_at
Figure 5940: DNA345227, NP_005660.1,
HUMPOLLA_at
Figure 5941: PRO95711
Figure 5942: DNA151434, DNA151434, P_X04382_at
Figure 5943: PRO11802
Figure 5944: DNA345228, NP_079522.1, P_V61478_at
Figure 5945: PRO95712
Figure 5946A-C: DNA345229, NM_015293,
AB018339_at
Figure 5947: PRO95713
Figure 5948: DNA345230, M12886, HUMTCBYY_at
Figure 5949: PRO95714
Figure 5950A-C: DNA302013, NM_023037,
HSU50534_at
Figure 5951: PRO71030
Figure 5952A-B: DNA328284, NP_056356.1,
P_X37553_at
Figure 5953: PRO84160
Figure 5954A-B: DNA345231, 331792.1,
HSM801131_at
Figure 5955: PRO24965
Figure 5956: DNA151774, DNA151774, P_X85042_at
Figure 5957: PRO12052
Figure 5958A-B: DNA169926, DNA169926,
AB032991_at
Figure 5959: PRO23259
Figure 5960A-B: DNA345232, NM_006996,
HSA237724_at
Figure 5961: PRO23299
Figure 5962A-B: DNA329269, AB007916,
AB007916_at
Figure 5963A-B: DNA193917, AL050367,
HSM800541_at
Figure 5964: DNA330906, NM_032782, P_A51904_at
Figure 5965: PRO86067
Figure 5966: DNA193996, DNA193996, P_A40502_at
Figure 5967: PRO23400
Figure 5968: DNA194141, DNA194141, P_X37431_at
Figure 5969: PRO23535
Figure 5970: DNA228132, AK027031, AK027031_at
Figure 5971: PRO38595
Figure 5972: DNA345233, AL136919, P_Z51682_at
Figure 5973: PRO95715
Figure 5974: DNA328288, BC020517, AK022938_at
Figure 5975: PRO69876
Figure 5976: DNA345234, AK026962, AK026962_at
Figure 5977: PRO95716
Figure 5978: DNA331098, AY052405, AX047348_at
Figure 5979: PRO86248
Figure 5980: DNA345235, 221966.14,
AI984778_RC_at
Figure 5981: PRO95717
Figure 5982: DNA345236, 330869.67, AV762213_at
Figure 5983: PRO95718
Figure 5984: DNA210194, DNA210194,
HSM802254_at
Figure 5985: DNA331856, BC022522, 237658.8_at
Figure 5986: PRO71209
Figure 5987: DNA194527, DNA194527, 399617.1_at
Figure 5988: PRO23884
Figure 5989: DNA345237, 196714.4, 196714.2_at
Figure 5990: PRO95719
Figure 5991: DNA345238, 001697.46, 001697.5_at
Figure 5992: PRO95720
Figure 5993: DNA345239, AAH35779.1, 399901.2_at

Figure 5994: PRO95721
Figure 5995: DNA338349, BC035900, 428335.22_at
Figure 5996: PRO91021
Figure 5997: DNA164635, DNA164635,
DNA164635_at
Figure 5998: DNA326749, NP_116101.1,
DNA167237_at
Figure 5999: PRO23238
Figure 6000: DNA210622, NM_015925,
NM_015925_at
Figure 6001: PRO35016
Figure 6002: DNA345240, 098138.2, P_Q74306_at
Figure 6003: PRO95722
Figure 6004: DNA330438, NM_018556,
NM_018556_at
Figure 6005: PRO50795
Figure 6006: DNA345241, NM_018384,
NM_018384_at
Figure 6007: PRO95723
Figure 6008: DNA254520, NM_018482,
NM_018482_at
Figure 6009: PRO49627
Figure 6010: DNA254470, NM_002497,
NM_002497_at
Figure 6011: PRO49578
Figure 6012A-B: DNA331400, NM_018440,
NM_018440_at
Figure 6013: PRO86464
Figure 6014: DNA254414, NP_054898.1,
NM_014179_at
Figure 6015: PRO49524
Figure 6016: DNA255340, NM_017684,
NM_017684_at
Figure 6017: PRO50409
Figure 6018: DNA253811, NP_004410.2,
NM_004419_at
Figure 6019: PRO49214
Figure 6020: DNA255921, NM_000734,
NM_000734_at
Figure 6021: PRO50974
Figure 6022: DNA345242, BC002342, NM_014325_at
Figure 6023: PRO49875
Figure 6024: DNA255161, NM_022147,
NM_022147_at
Figure 6025: PRO50241
Figure 6026: DNA330123, NM_007053,
NM_007053_at
Figure 6027: PRO35080
Figure 6028: DNA327812, NM_006417,
NM_006417_at
Figure 6029: PRO83773
Figure 6030: DNA304717, NM_000389,
NM_000389_at
Figure 6031: PRO71143
Figure 6032: DNA328431, NM_001826,
NM_001826_at
Figure 6033: PRO45093
Figure 6034A-B: DNA333574, NM_002829,
NM_002829_at
Figure 6035: PRO88221
Figure 6036: DNA345243, L38616, NM_004899_at
Figure 6037: PRO95724
Figure 6038: DNA287207, NM_006325,
NM_006325_at
Figure 6039: PRO39268
Figure 6040: DNA329172, NM_005263,
NM_005263_at
Figure 6041: PRO84796
Figure 6042: DNA345244, NP_036229.1,
NM_012097_at
Figure 6043: PRO71114
Figure 6044: DNA256257, NM_014398,
NM_014398_at
Figure 6045: PRO51301
Figure 6046A-B: DNA221079, NM_022162,
NM_022162_at
Figure 6047: PRO34753
Figure 6048: DNA255454, NP_060834.1,
NM_018364_at
Figure 6049: PRO50521
Figure 6050A-B: DNA254789, NM_016217,
NM_016217_at
Figure 6051: PRO49887
Figure 6052A-B: DNA254376, NM_014963,
NM_014963_at
Figure 6053: PRO49486
Figure 6054: DNA254214, NM_001698,
NM_001698_at
Figure 6055: PRO49326
Figure 6056: DNA345245, BC015815, NM_006994_at
Figure 6057: PRO49242
Figure 6058: DNA253802, NP_055569.1,
NM_014754_at
Figure 6059: PRO49207
Figure 6060: DNA255269, AL110271, NM_015462_at
Figure 6061: PRO50346
Figure 6062: DNA256521, NM_013431,
NM_013431_at
Figure 6063: PRO51556
Figure 6064A-B: DNA345246, NM_138292,
NM_000051_at
Figure 6065: PRO95725
Figure 6066: DNA256533, NM_006114,
NM_006114_at
Figure 6067: PRO51565
Figure 6068A-B: DNA287273, NM_006444,
NM_006444_at
Figure 6069: PRO69545
Figure 6070: DNA330223, NP_001790.1,
NM_001799_at
Figure 6071: PRO49730
Figure 6072: DNA254350, NM_004052,

NM_004052_at
Figure 6073: PRO49461
Figure 6074: DNA254163, S73813, NM_001776_at
Figure 6075: PRO49277
Figure 6076: DNA328876, NP_060582.1,
NM_018112_at
Figure 6077: PRO84603
Figure 6078: DNA329900, M87338, NM_002914_at
Figure 6079: PRO81549
Figure 6080: DNA330040, NM_078626,
NM_001262_at
Figure 6081: PRO59546
Figure 6082: DNA339592, NP_071401.2,
NM_022118_at
Figure 6083: PRO91353
Figure 6084: DNA329575, NP_004699.1,
NM_004708_at
Figure 6085: PRO61403
Figure 6086: DNA277083, M84489, NM_002745_at
Figure 6087: PRO64127
Figure 6088: DNA327690, NM_004031,
NM_004031_at
Figure 6089: PRO83673
Figure 6090: DNA272066, NM_002940,
NM_002940_at
Figure 6091: PRO60337
Figure 6092: DNA345247, BC012125, NM_022154_at
Figure 6093: PRO50332
Figure 6094A-B: DNA254616, NM_004482,
NM_004482_at
Figure 6095: PRO49718
Figure 6096: DNA255402, NM_014473,
NM_014473_at
Figure 6097: PRO50469
Figure 6098: DNA328296, NP_061059.1,
NM_018589_at
Figure 6099: PRO51817
Figure 6100: DNA345248, NM_006639,
NM_006639_at
Figure 6101: PRO34958
Figure 6102: DNA287241, NM_015907,
NM_015907_at
Figure 6103: PRO69516
Figure 6104: DNA254380, NM_020379,
NM_020379_at
Figure 6105: PRO49490
Figure 6106A-B: DNA345249, AAH38115.1,
NM_017631_at
Figure 6107: PRO95726
Figure 6108: DNA287221, NP_057407.1,
NM_016323_at
Figure 6109: PRO69500
Figure 6110: DNA252224, AK025273, NM_022073_at
Figure 6111: PRO48216
Figure 6112A-B: DNA254218, NP_001914.2,
NM_001923_at
Figure 6113: PRO49330
Figure 6114: DNA329033, NM_005384,
NM_005384_at
Figure 6115: PRO84700
Figure 6116A-C: DNA345250, NP_002751.1,
NM_002760_at
Figure 6117: PRO59148
Figure 6118: DNA273060, NM_001255,
NM_001255_at
Figure 6119: PRO61125
Figure 6120: DNA345251, NP_694858.1,
NM_002270_at
Figure 6121: PRO60223
Figure 6122: DNA269750, NP_002919.1,
NM_002928_at
Figure 6123: PRO58159
Figure 6124: DNA327927, NM_013258,
NM_013258_at
Figure 6125: PRO57311
Figure 6126: DNA330057, NM_005950,
NM_005950_at
Figure 6127: PRO85337
Figure 6128A-B: DNA345252, AL136911,
NM_016357_at
Figure 6129: PRO82143
Figure 6130: DNA329118, NM_021874,
NM_021874_at
Figure 6131: PRO83123
Figure 6132A-B: DNA345253, NM_174956,
NM_005173_at
Figure 6133: PRO95727
Figure 6134: DNA256737, NM_017806,
NM_017806_at
Figure 6135: PRO51671
Figure 6136: DNA329253, NM_006137,
NM_006137_at
Figure 6137: PRO84853
Figure 6138: DNA254570, NP_055484.1,
NM_014669_at
Figure 6139: PRO49673
Figure 6140: DNA254416, NP_060915.1,
NM_018445_at
Figure 6141: PRO49526
Figure 6142A-C: DNA328497, NM_005502,
NM_005502_at
Figure 6143: PRO84319
Figure 6144A-B: DNA330366, NM_022765,
NM_022765_at
Figure 6145: PRO85581
Figure 6146: DNA328471, NP_005848.2,
NM_005857_at
Figure 6147: PRO84297
Figure 6148: DNA324742, NM_001760,
NM_001760_at
Figure 6149: PRO81367
Figure 6150A-B: DNA255183, NM_019027,

NM_019027_at
Figure 6151: PRO50262
Figure 6152: DNA256141, AL353940, NM_018423_at
Figure 6153: PRO51189
Figure 6154: DNA255145, NM_018447,
NM_018447_at
Figure 6155: PRO50225
Figure 6156: DNA256762, AK022882, NM_022451_at
Figure 6157: PRO51695
Figure 6158: DNA345254, NM_020437,
NM_020437_at
Figure 6159: PRO86261
Figure 6160: DNA329584, NP_005032.1,
NM_005041_at
Figure 6161: PRO85118
Figure 6162: DNA345255, AY184205, NM_015180_at
Figure 6163: PRO95728
Figure 6164: DNA327521, NM_002201,
NM_002201_at
Figure 6165: PRO58320
Figure 6166: DNA331323, NM_001259,
NM_001259_at
Figure 6167: PRO86412
Figure 6168: DNA272655, NM_001827,
NM_001827_at
Figure 6169: PRO60781
Figure 6170A-B: DNA345256, NP_665702.1,
NM_004619_at
Figure 6171: PRO20111
Figure 6172: DNA345257, NM_003835,
NM_003835_at
Figure 6173: PRO95729
Figure 6174: DNA345258, NM_002925,
NM_002925_at
Figure 6175: PRO63255
Figure 6176: DNA345259, NM_006538,
NM_006538_at
Figure 6177: PRO84980
Figure 6178: DNA270717, U31382, NM_004485_at
Figure 6179: PRO59080
Figure 6180: DNA152786, NP_057215.1,
NM_016131_at
Figure 6181: PRO10928
Figure 6182: DNA345260, NM_022168,
NM_022168_at
Figure 6183: PRO95730
Figure 6184A-B: DNA327674, NM_002748,
NM_002748_at
Figure 6185: PRO83661
Figure 6186: DNA325648, NP_037409.2,
NM_013277_at
Figure 6187: PRO82139
Figure 6188: DNA256561, NM_019604,
NM_019604_at
Figure 6189: PRO51592
Figure 6190: DNA329585, NP_005499.1,
NM_005508_at
Figure 6191: PRO85119
Figure 6192: DNA345261, NM_005290,
NM_005290_at
Figure 6193: PRO54695
Figure 6194: DNA328915, NM_014241,
NM_014241_at
Figure 6195: PRO84634
Figure 6196: DNA256089, D88308, NM_003645_at
Figure 6197: PRO51139
Figure 6198: DNA255215, AF207600, NM_018638_at
Figure 6199: PRO50294
Figure 6200A-B: DNA256807, NM_016255,
NM_016255_at
Figure 6201: PRO51738
Figure 6202: DNA255213, DNA255213,
NM_017780_at
Figure 6203: PRO50292
Figure 6204: DNA255386, NP_037518.1,
NM_013386_at
Figure 6205: PRO50454
Figure 6206A-B: DNA254292, DNA254292,
NM_004481_at
Figure 6207: PRO49403
Figure 6208: DNA260974, NM_006074,
NM_006074_at
Figure 6209: PRO54720
Figure 6210: DNA345262, NP_055118.1,
NM_014303_at
Figure 6211: PRO49256
Figure 6212: DNA331119, NM_005442,
NM_005442_at
Figure 6213: PRO50745
Figure 6214: DNA345263, NM_022468,
NM_022468_at
Figure 6215: PRO51432
Figure 6216: DNA254543, NP_006799.1,
NM_006808_at
Figure 6217: PRO49648
Figure 6218: DNA255088, NP_003249.1,
NM_003258_at
Figure 6219: PRO50174
Figure 6220: DNA253798, NP_002632.1,
NM_002641_at
Figure 6221: PRO49203
Figure 6222: DNA287425, NM_018509,
NM_018509_at
Figure 6223: PRO69682
Figure 6224: DNA295327, NM_021803,
NM_021803_at
Figure 6225: PRO70773
Figure 6226: DNA273523, NP_002154.1,
NM_002163_at
Figure 6227: PRO61504
Figure 6228: DNA271189, L22075, NM_006572_at
Figure 6229: PRO59506

Figure 6230: DNA333731, NP_055165.1,
NM_014350_at
Figure 6231: PRO88357
Figure 6232: DNA325507, NP_005842.1,
NM_005851_at
Figure 6233: PRO69461
Figure 6234: DNA294794, NM_002870,
NM_002870_at
Figure 6235: PRO70754
Figure 6236: DNA328303, NP_056525.1,
NM_015710_at
Figure 6237: PRO84173
Figure 6238: DNA345264, AL137399, NM_006785_at
Figure 6239: DNA327858, AF120334, NM_012341_at
Figure 6240: PRO83800
Figure 6241: DNA331122, NP_005728.2,
NM_005737_at
Figure 6242: PRO86265
Figure 6243: DNA289528, NM_004311,
NM_004311_at
Figure 6244: PRO70286
Figure 6245: DNA329123, NM_002882,
NM_002882_at
Figure 6246: PRO84765
Figure 6247: DNA339428, NP_057604.1,
NM_016520_at
Figure 6248: PRO91233
Figure 6249: DNA329038, NP_055704.1,
NM_014889_at
Figure 6250: PRO84705
Figure 6251: DNA345265, NP_004216.1,
NM_004225_at
Figure 6252: PRO95732
Figure 6253: DNA329587, NM_012124,
NM_012124_at
Figure 6254: PRO85121
Figure 6255A-B: DNA329248, AB002359,
AB002359_at
Figure 6256A-B: DNA255619, DNA255619,
AF054589_at
Figure 6257: PRO50682
Figure 6258A-B: DNA330255, AK025499,
HSM800958_at
Figure 6259: PRO85488
Figure 6260A-B: DNA255050, AL136883,
HSM801851_at
Figure 6261: PRO50138
Figure 6262: DNA328529, NM_001629, P_Z36336_at
Figure 6263: PRO49814
Figure 6264A-B: DNA329039, NP_056250.2,
AK027070_at
Figure 6265: PRO84706
Figure 6266: DNA328509, NM_006748, HSU44403_at
Figure 6267: PRO57996
Figure 6268: DNA345266, AF067023, NM_001363_at
Figure 6269A-B: DNA345267, NM_020453,
AB040920_at
Figure 6270: PRO95734
Figure 6271A-B: DNA331898, AF058925,
AF058925_at
Figure 6272: PRO86787
Figure 6273: DNA345268, NM_032479, AF151109_at
Figure 6274: PRO84951
Figure 6275: DNA331901, AL117515, AB029015_at
Figure 6276: DNA256422, AJ227900, HSA227900_at
Figure 6277: DNA254610, Z48633, HSHRTPSN_at
Figure 6278: DNA345269, NM_015660,
HSM800796_at
Figure 6279: PRO95735
Figure 6280: DNA256846, NM_017515, AK023080_at
Figure 6281: PRO51777
Figure 6282: DNA331902, NP_619634.1,
HSSOM172M_at
Figure 6283: PRO86790
Figure 6284: DNA329040, NP_005524.1,
HSU72882_at
Figure 6285: PRO84707
Figure 6286: DNA256796, AF083127, AF083127_at
Figure 6287: DNA345270, AAH06437.1,
AK024476_at
Figure 6288: PRO82523
Figure 6289A-B: DNA256299, BAB21793.1,
AB051489_at
Figure 6290: PRO51343
Figure 6291: DNA330259, NP_008944.1,
HSM801707_at
Figure 6292: PRO49366
Figure 6293: DNA331132, NM_032148,
HSM801796_at
Figure 6294: PRO86273
Figure 6295: DNA255964, NM_024837, AK025125_at
Figure 6296: PRO51015
Figure 6297: DNA256061, NM_030921, AF267864_at
Figure 6298: PRO51109
Figure 6299: DNA329078, NP_112200.2,
HSM801679_at
Figure 6300: PRO23253
Figure 6301: DNA345271, NP_001275.1,
NM_001284_at
Figure 6302: PRO22838
Figure 6303: DNA304710, NM_001540,
NM_001540_at
Figure 6304: PRO71136
Figure 6305: DNA330023, NM_001924,
NM_001924_at
Figure 6306: PRO85308
Figure 6307: DNA275385, NM_002094,
NM_002094_at
Figure 6308: PRO63048
Figure 6309: DNA328418, NM_003407,
NM_003407_at
Figure 6310: PRO84261

Figure 6311: DNA345272, NM_004128,
NM_004128_at
Figure 6312: PRO95736
Figure 6313: DNA331133, U63830, NM_004180_at
Figure 6314: PRO86274
Figure 6315: DNA287203, NP_006182.1,
NM_006191_at
Figure 6316: PRO69487
Figure 6317: DNA325920, NM_012111,
NM_012111_at
Figure 6318: PRO82373
Figure 6319: DNA253807, NM_020529,
NM_020529_at
Figure 6320: PRO49210
Figure 6321: DNA329925, NM_001537,
NM_001537_at
Figure 6322: PRO85239
Figure 6323: DNA289526, NM_004024,
NM_004024_at
Figure 6324: PRO70282
Figure 6325: DNA269766, NP_005646.1,
NM_005655_at
Figure 6326: PRO58175
Figure 6327: DNA329047, NM_006399,
NM_006399_at
Figure 6328: PRO58425
Figure 6329: DNA274167, AF026166, NM_006431_at
Figure 6330: PRO62097
Figure 6331: DNA254572, NM_006585,
NM_006585_at
Figure 6332: PRO49675
Figure 6333: DNA328591, NP_006635.1,
NM_006644_at
Figure 6334: PRO84376
Figure 6335: DNA255289, NM_014791,
NM_014791_at
Figure 6336: PRO50363
Figure 6337: DNA345273, X15183, HSHSP90R_at
Figure 6338: PRO95737
Figure 6339: DNA271847, NM_001539,
NM_001539_at
Figure 6340: PRO60127
Figure 6341: DNA270929, M88279, NM_002014_at
Figure 6342: PRO59262
Figure 6343: DNA329106, AF042081, NM_003022_at
Figure 6344: PRO83360
Figure 6345: DNA345274, NM_174886,
NM_003244_at
Figure 6346: PRO95738
Figure 6347: DNA253585, NM_004418,
NM_004418_at
Figure 6348: PRO49183
Figure 6349A-B: DNA275334, NP_112162.1,
NM_004749_at
Figure 6350: PRO63009
Figure 6351A-B: DNA270923, NM_004817,
NM_004817_at
Figure 6352: PRO59256
Figure 6353: DNA345275, NM_005572,
NM_005572_at
Figure 6354: PRO80660
Figure 6355A-B: DNA328473, NP_006473.1,
NM_006482_at
Figure 6356: PRO84299
Figure 6357: DNA326736, NM_006666,
NM_006666_at
Figure 6358: PRO83076
Figure 6359: DNA290235, NP_057121.1,
NM_016037_at
Figure 6360: PRO70335
Figure 6361: DNA331135, D43950, HUMKG1DD_at
Figure 6362: DNA273498, DNA273498,
HUMHSP70H_at
Figure 6363: PRO61480
Figure 6364: DNA270689, X58072, NM_002051_at
Figure 6365: PRO59053
Figure 6366: DNA271973, NM_002731,
NM_002731_at
Figure 6367: PRO60248
Figure 6368A-B: DNA345276, S65186,
NM_005546_at
Figure 6369: PRO95739
Figure 6370: DNA274202, NP_006804.1,
NM_006813_at
Figure 6371: PRO62131
Figure 6372: DNA328601, NM_015675,
NM_015675_at
Figure 6373: PRO84384
Figure 6374: DNA329050, NM_015969,
NM_015969_at
Figure 6375: PRO84712
Figure 6376: DNA326116, NM_016292,
NM_016292_at
Figure 6377: PRO82542
Figure 6378A-B: DNA329122, D87119,
NM_021643_at
Figure 6379: PRO84764
Figure 6380: DNA255418, L43575, HUMUNKN_at
Figure 6381: DNA345277, AK026038, AB046774_at
Figure 6382: PRO95740
Figure 6383: DNA339707, NP_116119.1, P_T31854_at
Figure 6384: PRO91437
Figure 6385: DNA328923, NM_023003, AF255922_at
Figure 6386: PRO84640
Figure 6387: DNA345278, NM_025006, AK023435_at
Figure 6388: PRO95741
Figure 6389: DNA255219, NP_078936.1,
AK026226_at
Figure 6390: PRO50298
Figure 6391: DNA345279, AAH14655.1,
IR1875335_at
Figure 6392: PRO84549

Figure 6393: DNA256091, NM_022102, AK024611_at
Figure 6394: PRO51141
Figure 6395: DNA254838, NM_024628, AK026841_at
Figure 6396: PRO49933
Figure 6397: DNA330548, AK025645, AK025645_at
Figure 6398: PRO85732
Figure 6399: DNA329355, NM_033280, P_V40521_at
Figure 6400: PRO50434
Figure 6401A-B: DNA256267, AB046838,
AB046838_at
Figure 6402: DNA327954, NM_031458, P_D00629_at
Figure 6403: PRO83879
Figure 6404: DNA255798, NM_024989, AK022439_at
Figure 6405: PRO50853
Figure 6406: DNA329384, NM_174921, P_Z33372_at
Figure 6407: PRO84960
Figure 6408: DNA345280, AB089319, P_Z24893_at
Figure 6409: PRO95742
Figure 6410: DNA255913, AL050125, HSM800425_at
Figure 6411: PRO50966
Figure 6412: DNA325379, NP_116136.1,
HSM800835_at
Figure 6413: PRO81913
Figure 6414: DNA254596, DNA254596, AF026941_at
Figure 6415: PRO49699
Figure 6416A-B: DNA254801, AL080209,
HSM800735_at
Figure 6417: PRO49897
Figure 6418: DNA255700, DNA255700,
HSM801128_at
Figure 6419A-B: DNA328853, NM_020651,
AF302505_at
Figure 6420: PRO84584
Figure 6421: DNA330854, AK023113, AK023113_at
Figure 6422: PRO86017
Figure 6423A-B: DNA345281, 198947.4,
AK023271_at
Figure 6424: PRO6012
Figure 6425: DNA345282, 154551.19, 154551.10_at
Figure 6426: PRO95743
Figure 6427A-B: DNA345283, 1327517.49,
994387.65_at
Figure 6428: PRO95744
Figure 6429: DNA257363, NM_032315, 203633.4_at
Figure 6430: PRO51950
Figure 6431: DNA345284, NM_145810, 475113.7_at
Figure 6432: PRO69531
Figure 6433: DNA345285, 200333.3,
200333.3_CON_at
Figure 6434: PRO95745
Figure 6435: DNA304068, NP_653250.1,
1091656.1_at
Figure 6436: PRO71035
Figure 6437A-B: DNA338079, AL831953,
337352.17_at
Figure 6438: PRO90959
Figure 6439: DNA258677, DNA258677, 404505.1_at
Figure 6440: DNA345286, 1452432.11, 359193.13_at
Figure 6441: PRO95746
Figure 6442A-B: DNA345287, NM_032550,
481857.16_at
Figure 6443: PRO95747
Figure 6444: DNA259902, DNA259902, 475431.4_at
Figure 6445: PRO53832
Figure 6446: DNA345288, 1499607.2, 210883.2_at
Figure 6447: PRO95748
Figure 6448: DNA345289, 1449133.1, 109254.1_at
Figure 6449: PRO95749
Figure 6450: DNA345290, 332730.8, 332730.8_at
Figure 6451: PRO95750
Figure 6452: DNA345291, 407233.2, 407233.2_at
Figure 6453: PRO95751
Figure 6454: DNA345292, NM_144601, 197670.7_at
Figure 6455: PRO95752
Figure 6456: DNA259663, DNA259663, 215119.2_at
Figure 6457: DNA345293, 408339.15, 221433.12_at
Figure 6458: PRO95753
Figure 6459: DNA287258, NP_542786.1,
228321.19_at
Figure 6460: PRO52174
Figure 6461: DNA329626, 1089565.1, 1089565.1_at
Figure 6462: PRO85155
Figure 6463: DNA259852, DNA259852, 099349.1_at
Figure 6464: PRO53782

The following numbered paragraphs (paras.) contain further statements of various aspects of the present invention:-
1. Isolated nucleic acid comprising at least 80% nucleic acid sequence identity to a nucleotide sequence encoding the polypeptide as shown in any one of the SEQ ID NOs 1-6464.
2. Isolated nucleic acid comprising at least 80% nucleic acid sequence identity to a nucleotide sequence comprising the full-length coding sequence of the nucleotide sequence as shown in any one of the SEQ ID NOs 1-6464.
3. A vector comprising the nucleic acid of Para 1.
4. The vector of Para 3 operably linked to control sequences recognized by a host cell transformed with the vector.
5. A host cell comprising the vector of Para 3.
6. The host cell of Para 5, wherein said cell is a CHO cell, an *E*.*coli* cell or a yeast cell.
7. A process for producing a PRO polypeptide comprising culturing the host cell of Para 6 under conditions suitable for expression of said PRO polypeptide and recovering said PRO polypeptide from the cell culture.
8. An isolated polypeptide comprising at least 80% amino acid sequence identity to an amino acid sequence of the polypeptide as shown in any one of the SEQ ID NOs 1-6464.
9. A chimeric molecule comprising a polypeptide according to Para 8 fused to a heterologous amino acid sequence.
10. The chimeric molecule of Para 9, wherein said heterologous amino acid sequence is an epitope tag sequence or an Fc region of an immunoglobulin.
11. An antibody which specifically binds to a polypeptide according to Para 8.
12. The antibody of Para 11, wherein said antibody is a monoclonal antibody, a humanized antibody or a single-chain antibody.
13. A composition of matter comprising (a) a polypeptide of Para 8, (b) an agonist of said polypeptide, (c) an antagonist of said polypeptide, or (d) an antibody that binds to said polypeptide, in combination with a carrier.
14. The composition of matter of Para 13, wherein said carrier is a pharmaceutically acceptable carrier.
15. The composition of matter of Para 14 comprising a therapeutically effective amount of (a), (b), (c) or (d).
16. An article of manufacture, comprising:
   a container;
   a label on said container; and
   a composition of matter comprising (a) a polypeptide of Para 8, (b) an agonist of said polypeptide, (c) an antagonist of said polypeptide, or (d) an antibody that binds to said polypeptide, contained within said container, wherein label on said container indicates that said composition of matter can be used for treating an immune related disease.
17. A method of treating an immune related disorder in a mammal in need thereof comprising administering to said mammal a therapeutically effective amount of (a) a polypeptide of Para 8, (b) an agonist of said polypeptide, (c) an antagonist of said polypeptide, or (d) an antibody that binds to said polypeptide.
18. The method of Para 17, wherein the immune related disorder is systemic lupus erythematosis, rheumatoid arthritis, osteoarthritis, juvenile chronic arthritis, a spondyloarthropathy, systemic sclerosis, an idiopathic inflammatory myopathy, Sjögren's syndrome, systemic vasculitis, sarcoidosis, autoimmune hemolytic anemia, autoimmune thrombocytopenia, thyroiditis, diabetes mellitus, immune-mediated renal disease, a demyelinating disease of the central or peripheral nervous system, idiopathic demyelinating polyneuropathy, Guillain-Barré syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, contact dermatitis, psoriasis, an allergic disease, asthma, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease.
19. A method for determining the presence of a PRO polypeptide of the invention as described in any one of SEQ ID NOs 1-6464, in a sample suspected of containing said polypeptide, said method comprising exposing said sample to an anti-PRO antibody, where the and determining binding of said antibody to a component of said sample.
20. A method of diagnosing an immune related disease in a mammal, said method comprising detecting the level of expression of a gene encoding a PRO polypeptide of the invention as described in any one of SEQ ID NOs 1-6464, (a) in a test sample of tissue cells obtained from the mammal, and (b) in a control sample of known normal tissue cells of the same cell type, wherein a higher or lower level of expression of said gene in the test sample as compared to the control sample is indicative of the presence of an immune related disease in the mammal from which the test tissue cells were obtained.
21. A method of diagnosing an immune related disease in a mammal, said method comprising (a) contacting a PRO polypeptide of the invention as described in any one of SEQ ID NOs 1-6464, anti-PRO antibody with a test sample of tissue cells obtained from said mammal and (b) detecting the formation of a complex between the antibody and the polypeptide in the test sample, wherein formation of said complex is indicative of the presence of an immune related disease in the mammal from which the test tissue cells were obtained.
22. A method of identifying a compound that inhibits the activity of a PRO polypeptide of the invention as described in any one of SEQ ID NOs 1-6464, said method comprising contacting cells which normally respond to said polypeptide with (a) said polypeptide and (b) a candidate compound, and determining the lack responsiveness by said cell to (a).
23. A method of identifying a compound that inhibits the expression of a gene encoding a PRO polypeptide of the invention as described in any one of SEQ ID NOs 1-6464, said method comprising contacting cells which normally express said polypeptide with a candidate compound, and determining the lack of expression said gene.
24. The method of Para 23, wherein said candidate compound is an antisense nucleic acid.
25. A method of identifying a compound that mimics the activity of a PRO polypeptide of the invention as described in any one of SEQ ID NOs 1-6464, said method comprising contacting cells which normally respond to said polypeptide with a candidate compound, and determining the responsiveness by said cell to said candidate compound.
26. A method of stimulating the immune response in a mammal, said method comprising administering to said mammal an effective amount of a PRO polypeptide of the invention as described in any one of SEQ ID NOs 1-6464, antagonist, wherein said immune response is stimulated.
27. A method of diagnosing an inflammatory immune response in a mammal, said method comprising detecting the level of expression of a gene encoding a PRO polypeptide of the invention as described in any one of SEQ ID NOs 1-6464, (a) in a test sample of tissue cells obtained from the mammal, and (b) in a control sample of known normal tissue cells of the same cell type, wherein a higher or lower level of expression of said gene in the test sample as compared to the control sample is indicative of the presence of an inflammatory immune response in the mammal from which the test tissue cells were obtained.

## Claims

1. Isolated nucleic acid comprising at least 80% nucleic acid sequence identity to a nucleotide sequence encoding the polypeptide as shown in SEQ ID NO: 2120 or a nucleotide sequence comprising the full-length coding sequence of the nucleotide sequence as shown in SEQ ID NO: 2119.

2. A vector comprising the nucleic acid of Claim 1.

3. A host cell comprising the vector of Claim 2.

4. A process for producing a PRO polypeptide comprising culturing the host cell of Claim 3 under conditions suitable for expression of said PRO polypeptide and recovering said PRO polypeptide from the cell culture.

5. An isolated polypeptide comprising at least 80% amino acid sequence identity to an amino acid sequence of the polypeptide as shown in SEQ ID NO: 2120.

6. A chimeric molecule comprising a polypeptide according to Claim 5 fused to a heterologous amino acid sequence.

7. An antibody which specifically binds to a polypeptide according to Claim 5.

8. A composition of matter comprising (a) a polypeptide of Claim 5, (b) an agonist of said polypeptide, (c) an antagonist of said polypeptide, or (d) an antibody that binds to said polypeptide, in combination with a carrier, wherein said carrier is optionally a pharmaceutically acceptable carrier.

9. A method of treating an immune related disorder in a mammal in need thereof comprising administering to said mammal a therapeutically effective amount of (a) a polypeptide of Claim 5, (b) an agonist of said polypeptide, (c) an antagonist of said polypeptide, or (d) an antibody that binds to said polypeptide.

10. The method of Claim 9, wherein the immune related disorder is systemic lupus erythematosis, rheumatoid arthritis, osteoarthritis, juvenile chronic arthritis, a spondyloarthropathy, systemic sclerosis, an idiopathic inflammatory myopathy, Sjögren's syndrome, systemic vasculitis, sarcoidosis, autoimmune hemolytic anemia, autoimmune thrombocytopenia, thyroiditis, diabetes mellitus, immune-mediated renal disease, a demyelinating disease of the central or peripheral nervous system, idiopathic demyelinating polyneuropathy, Guillain-Barré syndrome, a chronic inflammatory demyelinating polyneuropathy, a hepatobiliary disease, infectious or autoimmune chronic active hepatitis, primary biliary cirrhosis, granulomatous hepatitis, sclerosing cholangitis, inflammatory bowel disease, gluten-sensitive enteropathy, Whipple's disease, an autoimmune or immune-mediated skin disease, a bullous skin disease, erythema multiforme, contact dermatitis, psoriasis, an allergic disease, asthma, allergic rhinitis, atopic dermatitis, food hypersensitivity, urticaria, an immunologic disease of the lung, eosinophilic pneumonias, idiopathic pulmonary fibrosis, hypersensitivity pneumonitis, a transplantation associated disease, graft rejection or graft-versus-host-disease.

11. A method for determining the presence of a PRO polypeptide of the invention as described in SEQ ID NO: 2120, in a sample suspected of containing said polypeptide, said method comprising exposing said sample to an anti-PRO antibody and determining binding of said antibody to a component of said sample.

12. A method of diagnosing an immune related disease in a mammal, said method comprising detecting the level of expression of a gene encoding a PRO polypeptide of the invention as described in SEQ ID NO: 2120, (a) in a test sample of tissue cells obtained from the mammal, and (b) in a control sample of known normal tissue cells of the same cell type, wherein a higher or lower level of expression of said gene in the test sample as compared to the control sample is indicative of the presence of an immune related disease in the mammal from which the test tissue cells were obtained.

13. A method of diagnosing an immune related disease in a mammal, said method comprising (a) contacting a PRO polypeptide of the invention as described in SEQ ID NO: 2120, anti-PRO antibody with a test sample of tissue cells obtained from said mammal and (b) detecting the formation of a complex between the antibody and the polypeptide in the test sample, wherein formation of said complex is indicative of the presence of an immune related disease in the mammal from which the test tissue cells were obtained.

14. A method of identifying a compound that inhibits the activity of a PRO polypeptide of the invention as described in SEQ ID NO: 2120, said method comprising contacting cells which normally respond to said polypeptide with (a) said polypeptide and (b) a candidate compound, and determining the lack of responsiveness by said cell to (a).

15. A method of identifying a compound that inhibits the expression of a gene encoding a PRO polypeptide of the invention as described in SEQ ID NO: 2120, said method comprising contacting cells which normally express said polypeptide with a candidate compound, and determining the lack of expression of said gene.

16. A method of identifying a compound that mimics the activity of a PRO polypeptide of the invention as described in SEQ ID NO: 2120, said method comprising contacting cells which normally respond to said polypeptide with a candidate compound, and determining the responsiveness by said cell to said candidate compound.

17. A method of stimulating the immune response in a mammal, said method comprising administering to said mammal an effective amount of a PRO polypeptide of the invention as described in SEQ ID NO: 2120, or antagonist, wherein said immune response is stimulated.

18. A method of diagnosing an inflammatory immune response in a mammal, said method comprising detecting the level of expression of a gene encoding a PRO polypeptide of the invention as described in SEQ ID NO: 2120, (a) in a test sample of tissue cells obtained from the mammal, and (b) in a control sample of known normal tissue cells of the same cell type, wherein a higher or lower level of expression of said gene in the test sample as compared to the control sample is indicative of the presence of an inflammatory immune response in the mammal from which the test tissue cells were obtained.
